# EUROPEAN PATENT SPECIFICATION

(11) **EP 2 563 113 B1**
(45) Date of publication and mention of the grant of the patent: **29.11.2017**
(21) Application number: 11777981.9
(22) Date of filing: 27.04.2011
(51) Int. Cl.: A01H 5/00, C12N 15/82, C12N 15/29, C07K 14/415

(54) **MODIFIED PYR/PYL RECEPTORS ACTIVATED BY ORTHOGONAL LIGANDS**
DURCH ORTHOGONALE LIGANDEN AKTIVIERTE MODIFIZIERTE PYR/PYL-REZEPTOREN
RÉCEPTEURS DE PYR/PYL MODIFIÉS ACTIVÉS PAR DES LIGANDS ORTHOGONAUX

(30) Priority: 19.01.2011 US 434407 P; 28.04.2010 US 328999 P
(43) Date of publication of application: 06.03.2013
(73) Proprietor: The Regents of the University of California, Oakland, CA 94607 (US)
(72) Inventor: CUTLER, Sean R., Riverside California 92507 (US); PARK, Sang-Youl, Riverside California 92507 (US)
(74) Representative: J A Kemp
(86) International application number: PCT/US2011/034202
(87) International publication number: WO 2011/139798

(56) References cited:
- WO-A2-2010/093954
- US-A1- 2004 148 654
- US-A1- 2005 244 971
- US-A1- 2006 179 518
- US-A1- 2009 105 238
- US-A1- 2009 320 152
- SANG-YOUL PARK ET AL: "Abscisic Acid Inhibits Type 2C Protein Phosphatases via the PYR/PYL Family of START Proteins", SCIENCE, AMERICAN ASSOCIATION FOR THE ADVANCEMENT OF SCIENCE, WASHINGTON, DC; US, vol. 324, 1 May 2009 (2009-05-01), pages 1068-1071, XP007915938, ISSN: 0036-8075, DOI: 10.1126/SCIENCE.1173041 [retrieved on 2009-04-30]

### SEQUENCE LISTING

<110> Cutler, Sean R. Park, Sang-Youl The Regents of the University of California
<120> Modified PYR/PYL Receptors Activated by
   Orthogonal Ligands
<130> 81906-804547
<140> WO PCT/US11/34202
   <141> 2011-04-27
<150> US 61/328,999
   <151> 2010-04-28
<150> US 61/434,407
   <151> 2011-01-19
<160> 179
<170> FastSEQ for Windows Version 4.0
<210> 1
   <211> 191
   <212> PRT
   <213> Arabidopsis thaliana
<220>
   <223> thale cress PYR/PYL receptor, Pyrabactin resistance 1, abscisic acid receptor PYR1 (PYR1), ABI1-binding protein 6 (ABIP6), regulatory components of ABA receptor 11 (RCAR11), At4g17870, T6K21.50
<400> 1
   Met Pro Ser Glu Leu Thr Pro Glu Glu Arg Ser Glu Leu Lys Asn Ser
    1               5                  10                  15
   Ile Ala Glu Phe His Thr Tyr Gln Leu Asp Pro Gly Ser Cys Ser Ser
               20                  25                  30
   Leu His Ala Gln Arg Ile His Ala Pro Pro Glu Leu Val Trp Ser Ile
           35                  40                  45
   Val Arg Arg Phe Asp Lys Pro Gln Thr Tyr Lys His Phe Ile Lys Ser
       50                  55                  60
   Cys Ser Val Glu Gln Asn Phe Glu Met Arg Val Gly Cys Thr Arg Asp
   65                  70                  75                  80
   Val Ile Val Ile Ser Gly Leu Pro Ala Asn Thr Ser Thr Glu Arg Leu
                   85                  90                  95
   Asp Ile Leu Asp Asp Glu Arg Arg Val Thr Gly Phe Ser Ile Ile Gly
               100                 105                 110
   Gly Glu His Arg Leu Thr Asn Tyr Lys Ser Val Thr Thr Val His Arg
           115                 120                 125
   Phe Glu Lys Glu Asn Arg Ile Trp Thr Val Val Leu Glu Ser Tyr Val
       130                 135                 140
   Val Asp Met Pro Glu Gly Asn Ser Glu Asp Asp Thr Arg Met Phe Ala
   145                 150                 155                 160
   Asp Thr Val Val Lys Leu Asn Leu Gln Lys Leu Ala Thr Val Ala Glu
                   165                 170                 175
   Ala Met Ala Arg Asn Ser Gly Asp Gly Ser Gly Ser Gln Val Thr
               180                 185                 190
<210> 2
   <211> 221
   <212> PRT
   <213> Arabidopsis thaliana
<220>
   <223> thale cress PYR/PYL receptor, abscisic acid receptor PYL1, PYR1-like protein 1 (PYL1), ABI1-binding protein 6 (ABIP6), regulatory components of ABA receptor 9 (RCAR12), At5g46790, MZA15.21
<400> 2
   Met Ala Asn Ser Glu Ser Ser Ser Ser Pro Val Asn Glu Glu Glu Asn
    1               5                  10                  15
   Ser Gln Arg Ile Ser Thr Leu His His Gln Thr Met Pro Ser Asp Leu
               20                  25                  30
   Thr Gln Asp Glu Phe Thr Gln Leu Ser Gln Ser Ile Ala Glu Phe His
           35                  40                  45
   Thr Tyr Gln Leu Gly Asn Gly Arg Cys Ser Ser Leu Leu Ala Gln Arg
       50                  55                  60
   Ile His Ala Pro Pro Glu Thr Val Trp Ser Val Val Arg Arg Phe Asp
   65                  70                  75                  80
   Arg Pro Gln Ile Tyr Lys His Phe Ile Lys Ser Cys Asn Val Ser Glu
                   85                  90                  95
   Asp Phe Glu Met Arg Val Gly Cys Thr Arg Asp Val Asn Val Ile Ser
               100                 105                 110
   Gly Leu Pro Ala Asn Thr Ser Arg Glu Arg Leu Asp Leu Leu Asp Asp
           115                 120                 125
   Asp Arg Arg Val Thr Gly Phe Ser Ile Thr Gly Gly Glu His Arg Leu
       130                 135                 140
   Arg Asn Tyr Lys Ser Val Thr Thr Val His Arg Phe Glu Lys Glu Glu
   145                 150                 155                 160
   Glu Glu Glu Arg Ile Trp Thr Val Val Leu Glu Ser Tyr Val Val Asp
                   165                 170                 175
   Val Pro Glu Gly Asn Ser Glu Glu Asp Thr Arg Leu Phe Ala Asp Thr
               180                 185                 190
   Val Ile Arg Leu Asn Leu Gln Lys Leu Ala Ser Ile Thr Glu Ala Met
           195                 200                 205
   Asn Arg Asn Asn Asn Asn Asn Asn Ser Ser Gln Val Arg
       210                 215                 220
<210> 3
   <211> 190
   <212> PRT
   <213> Arabidopsis thaliana
<220>
   <223> thale cress PYR/PYL receptor, abscisic acid receptor PYL2, PYR1-like protein 2 (PYL2), ABI1-binding protein 6 (ABIP6), regulatory components of ABA receptor 14 (RCAR14), Bet v I allergen family protein, At2g26040, T19L18.15
<400> 3
   Met Ser Ser Ser Pro Ala Val Lys Gly Leu Thr Asp Glu Glu Gln Lys
    1               5                  10                  15
   Thr Leu Glu Pro Val Ile Lys Thr Tyr His Gln Phe Glu Pro Asp Pro
               20                  25                  30
   Thr Thr Cys Thr Ser Leu Ile Thr Gln Arg Ile His Ala Pro Ala Ser
           35                  40                  45
   Val Val Trp Pro Leu Ile Arg Arg Phe Asp Asn Pro Glu Arg Tyr Lys
       50                  55                  60
   His Phe Val Lys Arg Cys Arg Leu Ile Ser Gly Asp Gly Asp Val Gly
   65                  70                  75                  80
   Ser Val Arg Glu Val Thr Val Ile Ser Gly Leu Pro Ala Ser Thr Ser
                   85                  90                  95
   Thr Glu Arg Leu Glu Phe Val Asp Asp Asp His Arg Val Leu Ser Phe
               100                 105                 110
   Arg Val Val Gly Gly Glu His Arg Leu Lys Asn Tyr Lys Ser Val Thr
           115                 120                 125
   Ser Val Asn Glu Phe Leu Asn Gln Asp Ser Gly Lys Val Tyr Thr Val
       130                 135                 140
   Val Leu Glu Ser Tyr Thr Val Asp Ile Pro Glu Gly Asn Thr Glu Glu
   145                 150                 155                 160
   Asp Thr Lys Met Phe Val Asp Thr Val Val Lys Leu Asn Leu Gln Lys
                   165                 170                 175
   Leu Gly Val Ala Ala Thr Ser Ala Pro Met His Asp Asp Glu
               180                 185                 190
<210> 4
   <211> 209
   <212> PRT
   <213> Arabidopsis thaliana
<220>
   <223> thale cress PYR/PYL receptor, abscisic acid receptor PYL3, PYR1-like protein 3 (PYL3), regulatory components of ABA receptor 13 (RCAR13), At1g73000, F3N23.20
<400> 4
   Met Asn Leu Ala Pro Ile His Asp Pro Ser Ser Ser Ser Thr Thr Thr
    1               5                  10                  15
   Thr Ser Ser Ser Thr Pro Tyr Gly Leu Thr Lys Asp Glu Phe Ser Thr
               20                  25                  30
   Leu Asp Ser Ile Ile Arg Thr His His Thr Phe Pro Arg Ser Pro Asn
           35                  40                  45
   Thr Cys Thr Ser Leu Ile Ala His Arg Val Asp Ala Pro Ala His Ala
       50                  55                  60
   Ile Trp Arg Phe Val Arg Asp Phe Ala Asn Pro Asn Lys Tyr Lys His
   65                  70                  75                  80
   Phe Ile Lys Ser Cys Thr Ile Arg Val Asn Gly Asn Gly Ile Lys Glu
                   85                  90                  95
   Ile Lys Val Gly Thr Ile Arg Glu Val Ser Val Val Ser Gly Leu Pro
               100                 105                 110
   Ala Ser Thr Ser Val Glu Ile Leu Glu Val Leu Asp Glu Glu Lys Arg
           115                 120                 125
   Ile Leu Ser Phe Arg Val Leu Gly Gly Glu His Arg Leu Asn Asn Tyr
       130                 135                 140
   Arg Ser Val Thr Ser Val Asn Glu Phe Val Val Leu Glu Lys Asp Lys
   145                 150                 155                 160
   Lys Lys Arg Val Tyr Ser Val Val Leu Glu Ser Tyr Ile Val Asp Ile
                   165                 170                 175
   Pro Gln Gly Asn Thr Glu Glu Asp Thr Arg Met Phe Val Asp Thr Val
               180                 185                 190
   Val Lys Ser Asn Leu Gln Asn Leu Ala Val Ile Ser Thr Ala Ser Pro
           195                 200                 205
   Thr
<210> 5
   <211> 207
   <212> PRT
   <213> Arabidopsis thaliana
<220>
   <223> thale cress PYR/PYL receptor, abscisic acid receptor PYL4, PYR1-like protein 4 (PYL4), ABI1-binding protein 2 (ABIP2), regulatory components of ABA receptor 10 (RCAR10), At2g38310, T19C21.20
<400> 5
   Met Leu Ala Val His Arg Pro Ser Ser Ala Val Ser Asp Gly Asp Ser
    1               5                  10                  15
   Val Gln Ile Pro Met Met Ile Ala Ser Phe Gln Lys Arg Phe Pro Ser
               20                  25                  30
   Leu Ser Arg Asp Ser Thr Ala Ala Arg Phe His Thr His Glu Val Gly
           35                  40                  45
   Pro Asn Gln Cys Cys Ser Ala Val Ile Gln Glu Ile Ser Ala Pro Ile
       50                  55                  60
   Ser Thr Val Trp Ser Val Val Arg Arg Phe Asp Asn Pro Gln Ala Tyr
   65                  70                  75                  80
   Lys His Phe Leu Lys Ser Cys Ser Val Ile Gly Gly Asp Gly Asp Asn
                   85                  90                  95
   Val Gly Ser Leu Arg Gln Val His Val Val Ser Gly Leu Pro Ala Ala
               100                 105                 110
   Ser Ser Thr Glu Arg Leu Asp Ile Leu Asp Asp Glu Arg His Val Ile
           115                 120                 125
   Ser Phe Ser Val Val Gly Gly Asp His Arg Leu Ser Asn Tyr Arg Ser
       130                 135                 140
   Val Thr Thr Leu His Pro Ser Pro Ile Ser Gly Thr Val Val Val Glu
   145                 150                 155                 160
   Ser Tyr Val Val Asp Val Pro Pro Gly Asn Thr Lys Glu Glu Thr Cys
                   165                 170                 175
   Asp Phe Val Asp Val Ile Val Arg Cys Asn Leu Gln Ser Leu Ala Lys
               180                 185                 190
   Ile Ala Glu Asn Thr Ala Ala Glu Ser Lys Lys Lys Met Ser Leu
           195                 200                 205
<210> 6
   <211> 203
   <212> PRT
   <213> Arabidopsis thaliana
<220>
   <223> thale cress PYR/PYL receptor, abscisic acid receptor PYL5, PYR1-like protein 5 (PYL5), ABI1-binding protein 3 (ABIP3), regulatory components of ABA receptor 8 (RCAR8), Bet v I allergen family protein, At5g05440, K18I23.25
<400> 6
   Met Arg Ser Pro Val Gln Leu Gln His Gly Ser Asp Ala Thr Asn Gly
    1               5                  10                  15
   Phe His Thr Leu Gln Pro His Asp Gln Thr Asp Gly Pro Ile Lys Arg
               20                  25                  30
   Val Cys Leu Thr Arg Gly Met His Val Pro Glu His Val Ala Met His
           35                  40                  45
   His Thr His Asp Val Gly Pro Asp Gln Cys Cys Ser Ser Val Val Gln
       50                  55                  60
   Met Ile His Ala Pro Pro Glu Ser Val Trp Ala Leu Val Arg Arg Phe
   65                  70                  75                  80
   Asp Asn Pro Lys Val Tyr Lys Asn Phe Ile Arg Gln Cys Arg Ile Val
                   85                  90                  95
   Gln Gly Asp Gly Leu His Val Gly Asp Leu Arg Glu Val Met Val Val
               100                 105                 110
   Ser Gly Leu Pro Ala Val Ser Ser Thr Glu Arg Leu Glu Ile Leu Asp
           115                 120                 125
   Glu Glu Arg His Val Ile Ser Phe Ser Val Val Gly Gly Asp His Arg
       130                 135                 140
   Leu Lys Asn Tyr Arg Ser Val Thr Thr Leu His Ala Ser Asp Asp Glu
   145                 150                 155                 160
   Gly Thr Val Val Val Glu Ser Tyr Ile Val Asp Val Pro Pro Gly Asn
                   165                 170                 175
   Thr Glu Glu Glu Thr Leu Ser Phe Val Asp Thr Ile Val Arg Cys Asn
               180                 185                 190
   Leu Gln Ser Leu Ala Arg Ser Thr Asn Arg Gln
           195                 200
<210> 7
   <211> 215
   <212> PRT
   <213> Arabidopsis thaliana
<220>
   <223> thale cress PYR/PYL receptor, abscisic acid receptor PYL6, PYR1-like protein 6 (PYL6), ABI1-binding protein 5 (ABIP5), regulatory components of ABA receptor 9 (RCAR9), Bet v I allergen family protein, At2g40330, T7M7.15
<400> 7
   Met Pro Thr Ser Ile Gln Phe Gln Arg Ser Ser Thr Ala Ala Glu Ala
    1               5                  10                  15
   Ala Asn Ala Thr Val Arg Asn Tyr Pro His His His Gln Lys Gln Val
               20                  25                  30
   Gln Lys Val Ser Leu Thr Arg Gly Met Ala Asp Val Pro Glu His Val
           35                  40                  45
   Glu Leu Ser His Thr His Val Val Gly Pro Ser Gln Cys Phe Ser Val
       50                  55                  60
   Val Val Gln Asp Val Glu Ala Pro Val Ser Thr Val Trp Ser Ile Leu
   65                  70                  75                  80
   Ser Arg Phe Glu His Pro Gln Ala Tyr Lys His Phe Val Lys Ser Cys
                   85                  90                  95
   His Val Val Ile Gly Asp Gly Arg Glu Val Gly Ser Val Arg Glu Val
               100                 105                 110
   Arg Val Val Ser Gly Leu Pro Ala Ala Phe Ser Leu Glu Arg Leu Glu
           115                 120                 125
   Ile Met Asp Asp Asp Arg His Val Ile Ser Phe Ser Val Val Gly Gly
       130                 135                 140
   Asp His Arg Leu Met Asn Tyr Lys Ser Val Thr Thr Val His Glu Ser
   145                 150                 155                 160
   Glu Glu Asp Ser Asp Gly Lys Lys Arg Thr Arg Val Val Glu Ser Tyr
                   165                 170                 175
   Val Val Asp Val Pro Ala Gly Asn Asp Lys Glu Glu Thr Cys Ser Phe
               180                 185                 190
   Ala Asp Thr Ile Val Arg Cys Asn Leu Gln Ser Leu Ala Lys Leu Ala
           195                 200                 205
   Glu Asn Thr Ser Lys Phe Ser
       210                 215
<210> 8
   <211> 211
   <212> PRT
   <213> Arabidopsis thaliana
<220>
   <223> thale cress PYR/PYL receptor, abscisic acid receptor PYL7, PYR1-like protein 7 (PYL7), ABI1-binding protein 7 (ABIP7), regulatory components of ABA receptor 2 (RCAR2), At4g01026
<400> 8
   Met Glu Met Ile Gly Gly Asp Asp Thr Asp Thr Glu Met Tyr Gly Ala
    1               5                  10                  15
   Leu Val Thr Ala Gln Ser Leu Arg Leu Arg His Leu His His Cys Arg
               20                  25                  30
   Glu Asn Gln Cys Thr Ser Val Leu Val Lys Tyr Ile Gln Ala Pro Val
           35                  40                  45
   His Leu Val Trp Ser Leu Val Arg Arg Phe Asp Gln Pro Gln Lys Tyr
       50                  55                  60
   Lys Pro Phe Ile Ser Arg Cys Thr Val Asn Gly Asp Pro Glu Ile Gly
   65                  70                  75                  80
   Cys Leu Arg Glu Val Asn Val Lys Ser Gly Leu Pro Ala Thr Thr Ser
                   85                  90                  95
   Thr Glu Arg Leu Glu Gln Leu Asp Asp Glu Glu His Ile Leu Gly Ile
               100                 105                 110
   Asn Ile Ile Gly Gly Asp His Arg Leu Lys Asn Tyr Ser Ser Ile Leu
           115                 120                 125
   Thr Val His Pro Glu Met Ile Asp Gly Arg Ser Gly Thr Met Val Met
       130                 135                 140
   Glu Ser Phe Val Val Asp Val Pro Gln Gly Asn Thr Lys Asp Asp Thr
   145                 150                 155                 160
   Cys Tyr Phe Val Glu Ser Leu Ile Lys Cys Asn Leu Lys Ser Leu Ala
                   165                 170                 175
   Cys Val Ser Glu Arg Leu Ala Ala Gln Asp Ile Thr Asn Ser Ile Ala
               180                 185                 190
   Thr Phe Cys Asn Ala Ser Asn Gly Tyr Arg Glu Lys Asn His Thr Glu
           195                 200                 205
   Thr Asn Leu
       210
<210> 9
   <211> 188
   <212> PRT
   <213> Arabidopsis thaliana
<220>
   <223> thale cress PYR/PYL receptor, abscisic acid receptor PYL8, PYR1-like protein 8 (PYL8), ABI1-binding protein 1 (ABIP1), regulatory components of ABA receptor 3 (RCAR3), At5g53160, MFH8.10
<400> 9
   Met Glu Ala Asn Gly Ile Glu Asn Leu Thr Asn Pro Asn Gln Glu Arg
    1               5                  10                  15
   Glu Phe Ile Arg Arg His His Lys His Glu Leu Val Asp Asn Gln Cys
               20                  25                  30
   Ser Ser Thr Leu Val Lys His Ile Asn Ala Pro Val His Ile Val Trp
           35                  40                  45
   Ser Leu Val Arg Arg Phe Asp Gln Pro Gln Lys Tyr Lys Pro Phe Ile
       50                  55                  60
   Ser Arg Cys Val Val Lys Gly Asn Met Glu Ile Gly Thr Val Arg Glu
   65                  70                  75                  80
   Val Asp Val Lys Ser Gly Leu Pro Ala Thr Arg Ser Thr Glu Arg Leu
                   85                  90                  95
   Glu Leu Leu Asp Asp Asn Glu His Ile Leu Ser Ile Arg Ile Val Gly
               100                 105                 110
   Gly Asp His Arg Leu Lys Asn Tyr Ser Ser Ile Ile Ser Leu His Pro
           115                 120                 125
   Glu Thr Ile Glu Gly Arg Ile Gly Thr Leu Val Ile Glu Ser Phe Val
       130                 135                 140
   Val Asp Val Pro Glu Gly Asn Thr Lys Asp Glu Thr Cys Tyr Phe Val
   145                 150                 155                 160
   Glu Ala Leu Ile Lys Cys Asn Leu Lys Ser Leu Ala Asp Ile Ser Glu
                   165                 170                 175
   Arg Leu Ala Val Gln Asp Thr Thr Glu Ser Arg Val
               180                 185
<210> 10
   <211> 187
   <212> PRT
   <213> Arabidopsis thaliana
<220>
   <223> thale cress PYR/PYL receptor, abscisic acid receptor PYL9, PYR1-like protein 9 (PYL9), ABI1-binding protein 4 (ABIP4), regulatory components of ABA receptor 1 (RCAR1), At1g01360, F6F3.16
<400> 10
   Met Met Asp Gly Val Glu Gly Gly Thr Ala Met Tyr Gly Gly Leu Glu
    1               5                  10                  15
   Thr Val Gln Tyr Val Arg Thr His His Gln His Leu Cys Arg Glu Asn
               20                  25                  30
   Gln Cys Thr Ser Ala Leu Val Lys His Ile Lys Ala Pro Leu His Leu
           35                  40                  45
   Val Trp Ser Leu Val Arg Arg Phe Asp Gln Pro Gln Lys Tyr Lys Pro
       50                  55                  60
   Phe Val Ser Arg Cys Thr Val Ile Gly Asp Pro Glu Ile Gly Ser Leu
   65                  70                  75                  80
   Arg Glu Val Asn Val Lys Ser Gly Leu Pro Ala Thr Thr Ser Thr Glu
                   85                  90                  95
   Arg Leu Glu Leu Leu Asp Asp Glu Glu His Ile Leu Gly Ile Lys Ile
               100                 105                 110
   Ile Gly Gly Asp His Arg Leu Lys Asn Tyr Ser Ser Ile Leu Thr Val
           115                 120                 125
   His Pro Glu Ile Ile Glu Gly Arg Ala Gly Thr Met Val Ile Glu Ser
       130                 135                 140
   Phe Val Val Asp Val Pro Gln Gly Asn Thr Lys Asp Glu Thr Cys Tyr
   145                 150                 155                 160
   Phe Val Glu Ala Leu Ile Arg Cys Asn Leu Lys Ser Leu Ala Asp Val
                   165                 170                 175
   Ser Glu Arg Leu Ala Ser Gln Asp Ile Thr Gln
               180                 185
<210> 11
   <211> 183
   <212> PRT
   <213> Arabidopsis thaliana
<220>
   <223> thale cress PYR/PYL receptor, abscisic acid receptor PYL10, PYR1-like protein 10 (PYL10), ABI1-binding protein 8 (ABIP8), regulatory components of ABA receptor 4 (RCAR4), At4g27920, T13J8.30
<400> 11
   Met Asn Gly Asp Glu Thr Lys Lys Val Glu Ser Glu Tyr Ile Lys Lys
    1               5                  10                  15
   His His Arg His Glu Leu Val Glu Ser Gln Cys Ser Ser Thr Leu Val
               20                  25                  30
   Lys His Ile Lys Ala Pro Leu His Leu Val Trp Ser Ile Val Arg Arg
           35                  40                  45
   Phe Asp Glu Pro Gln Lys Tyr Lys Pro Phe Ile Ser Arg Cys Val Val
       50                  55                  60
   Gln Gly Lys Lys Leu Glu Val Gly Ser Val Arg Glu Val Asp Leu Lys
   65                  70                  75                  80
   Ser Gly Leu Pro Ala Thr Lys Ser Thr Glu Val Leu Glu Ile Leu Asp
                   85                  90                  95
   Asp Asn Glu His Ile Leu Gly Ile Arg Ile Val Gly Gly Asp His Arg
               100                 105                 110
   Leu Lys Asn Tyr Ser Ser Thr Ile Ser Leu His Ser Glu Thr Ile Asp
           115                 120                 125
   Gly Lys Thr Gly Thr Leu Ala Ile Glu Ser Phe Val Val Asp Val Pro
       130                 135                 140
   Glu Gly Asn Thr Lys Glu Glu Thr Cys Phe Phe Val Glu Ala Leu Ile
   145                 150                 155                 160
   Gln Cys Asn Leu Asn Ser Leu Ala Asp Val Thr Glu Arg Leu Gln Ala
                   165                 170                 175
   Glu Ser Met Glu Lys Lys Ile
               180
<210> 12
   <211> 161
   <212> PRT
   <213> Arabidopsis thaliana
<220>
   <223> thale cress PYR/PYL receptor, abscisic acid receptor PYL11, PYR1-like protein 11 (PYL11), regulatory components of ABA receptor 5 (RCAR5), Bet v I allergen family protein, At5g45860, K15I22.6
<400> 12
   Met Glu Thr Ser Gln Lys Tyr His Thr Cys Gly Ser Thr Leu Val Gln
    1               5                  10                  15
   Thr Ile Asp Ala Pro Leu Ser Leu Val Trp Ser Ile Leu Arg Arg Phe
               20                  25                  30
   Asp Asn Pro Gln Ala Tyr Lys Gln Phe Val Lys Thr Cys Asn Leu Ser
           35                  40                  45
   Ser Gly Asp Gly Gly Glu Gly Ser Val Arg Glu Val Thr Val Val Ser
       50                  55                  60
   Gly Leu Pro Ala Glu Phe Ser Arg Glu Arg Leu Asp Glu Leu Asp Asp
   65                  70                  75                  80
   Glu Ser His Val Met Met Ile Ser Ile Ile Gly Gly Asp His Arg Leu
                   85                  90                  95
   Val Asn Tyr Arg Ser Lys Thr Met Ala Phe Val Ala Ala Asp Thr Glu
               100                 105                 110
   Glu Lys Thr Val Val Val Glu Ser Tyr Val Val Asp Val Pro Glu Gly
           115                 120                 125
   Asn Ser Glu Glu Glu Thr Thr Ser Phe Ala Asp Thr Ile Val Gly Phe
       130                 135                 140
   Asn Leu Lys Ser Leu Ala Lys Leu Ser Glu Arg Val Ala His Leu Lys
   145                 150                 155                 160
   Leu
<210> 13
   <211> 159
   <212> PRT
   <213> Arabidopsis thaliana
<220>
   <223> thale cress PYR/PYL receptor, abscisic acid receptor PYL12, PYR1-like protein 12 (PYL212), regulatory components of ABA receptor 6 (RCAR6), Bet v I allergen family protein, At5g45870, K15I22.7
<400> 13
   Met Lys Thr Ser Gln Glu Gln His Val Cys Gly Ser Thr Val Val Gln
    1               5                   10                  15
   Thr Ile Asn Ala Pro Leu Pro Leu Val Trp Ser Ile Leu Arg Arg Phe
               20                  25                  30
   Asp Asn Pro Lys Thr Phe Lys His Phe Val Lys Thr Cys Lys Leu Arg
           35                  40                  45
   Ser Gly Asp Gly Gly Glu Gly Ser Val Arg Glu Val Thr Val Val Ser
       50                  55                  60
   Asp Leu Pro Ala Ser Phe Ser Leu Glu Arg Leu Asp Glu Leu Asp Asp
   65                  70                  75                  80
   Glu Ser His Val Met Val Ile Ser Ile Ile Gly Gly Asp His Arg Leu
                   85                  90                  95
   Val Asn Tyr Gln Ser Lys Thr Thr Val Phe Val Ala Ala Glu Glu Glu
               100                 105                 110
   Lys Thr Val Val Val Glu Ser Tyr Val Val Asp Val Pro Glu Gly Asn
           115                 120                 125
   Thr Glu Glu Glu Thr Thr Leu Phe Ala Asp Thr Ile Val Gly Cys Asn
       130                 135                 140
   Leu Arg Ser Leu Ala Lys Leu Ser Glu Lys Met Met Glu Leu Thr
   145                 150                 155
<210> 14
   <211> 164
   <212> PRT
   <213> Arabidopsis thaliana
<220>
   <223> thale cress PYR/PYL receptor, abscisic acid receptor PYL13, PYR1-like protein 13 (PYL13), regulatory components of ABA receptor 7 (RCAR7), At4g18620, F28A21.30
<400> 14
   Met Glu Ser Ser Lys Gln Lys Arg Cys Arg Ser Ser Val Val Glu Thr
    1               5                  10                  15
   Ile Glu Ala Pro Leu Pro Leu Val Trp Ser Ile Leu Arg Ser Phe Asp
               20                  25                  30
   Lys Pro Gln Ala Tyr Gln Arg Phe Val Lys Ser Cys Thr Met Arg Ser
           35                  40                  45
   Gly Gly Gly Gly Gly Lys Gly Gly Glu Gly Lys Gly Ser Val Arg Asp
       50                  55                  60
   Val Thr Leu Val Ser Gly Phe Pro Ala Asp Phe Ser Thr Glu Arg Leu
   65                  70                  75                  80
   Glu Glu Leu Asp Asp Glu Ser His Val Met Val Val Ser Ile Ile Gly
                   85                  90                  95
   Gly Asn His Arg Leu Val Asn Tyr Lys Ser Lys Thr Lys Val Val Ala
               100                 105                 110
   Ser Pro Glu Asp Met Ala Lys Lys Thr Val Val Val Glu Ser Tyr Val
           115                 120                 125
   Val Asp Val Pro Glu Gly Thr Ser Glu Glu Asp Thr Ile Phe Phe Val
       130                 135                 140
   Asp Asn Ile Ile Arg Tyr Asn Leu Thr Ser Leu Ala Lys Leu Thr Lys
   145                 150                 155                 160
   Lys Met Met Lys
<210> 15
   <211> 191
   <212> PRT
   <213> Brassica oleracea
<220>
   <223> wild cabbage Streptomyces cyclase/dehydrase family protein, locus tag 40.t00062, GenBank Accession No. ABD65175.1
<400> 15
   Met Pro Ser Gln Leu Thr Pro Glu Glu Arg Ser Glu Leu Ala Gln Ser
    1               5                  10                  15
   Ile Ala Glu Phe His Thr Tyr His Leu Gly Pro Gly Ser Cys Ser Ser
               20                  25                  30
   Leu His Ala Gln Arg Ile His Ala Pro Pro Glu Ile Val Trp Ser Val
           35                  40                  45
   Val Arg Arg Phe Asp Lys Pro Gln Thr Tyr Lys His Phe Ile Lys Ser
       50                  55                  60
   Cys Ser Val Glu Asp Gly Phe Glu Met Arg Val Gly Cys Thr Arg Ala
   65                  70                  75                  80
   Val Asn Val Ile Ser Gly Leu Pro Ala Asn Thr Ser Thr Glu Arg Leu
                   85                  90                  95
   Asp Ile Leu Asp Asp Glu Arg Arg Val Thr Gly Phe Ser Ile Ile Gly
               100                 105                 110
   Gly Glu His Arg Leu Thr Asn Tyr Lys Ser Val Thr Thr Val His Arg
           115                 120                 125
   Phe Glu Lys Glu Arg Arg Ile Trp Thr Val Val Leu Glu Ser Tyr Val
       130                 135                 140
   Val Asp Met Pro Glu Gly Asn Ser Glu Asp Asp Thr Arg Met Phe Ala
   145                 150                 155                 160
   Asp Thr Val Val Lys Leu Asn Leu Gln Lys Leu Ala Thr Val Thr Glu
                   165                 170                 175
   Ala Met Ala Arg Asn Ala Gly Asp Gly Ser Gly Ala Gln Val Thr
               180                 185                 190
<210> 16
   <211> 281
   <212> PRT
   <213> Brassica oleracea
<220>
   <223> wild cabbage Streptomyces cyclase/dehydrase family protein, locus tag 23.t00047, GenBank Accession No. ABD65631.1
<400> 16
   Met Pro Ser Glu Leu Thr Gln Glu Glu Arg Ser Lys Leu Thr Gln Ser
    1               5                  10                  15
   Ile Ser Glu Phe His Thr Tyr His Leu Gly Pro Gly Ser Cys Ser Ser
               20                  25                  30
   Leu His Ala Gln Arg Ile His Ala Pro Pro Glu Ile Val Trp Ser Val
           35                  40                  45
   Val Arg Gln Phe Asp Lys Pro Gln Thr Tyr Lys His Phe Ile Lys Ser
       50                  55                  60
   Cys Ser Val Glu Glu Gly Phe Glu Met Arg Val Gly Cys Thr Arg Asp
   65                  70                  75                  80
   Val Ile Val Ile Ser Gly Leu Pro Ala Asn Thr Ser Thr Glu Arg Leu
                   85                  90                  95
   Asp Met Leu Asp Asp Glu Arg Arg Val Thr Gly Phe Ser Ile Ile Gly
               100                 105                 110
   Gly Glu His Arg Leu Lys Asn Tyr Lys Ser Val Thr Thr Val His Arg
           115                 120                 125
   Phe Glu Arg Glu Arg Arg Ile Trp Thr Val Val Leu Glu Ser Tyr Val
       130                 135                 140
   Val Asp Met Pro Glu Gly Asn Ser Glu Asp Asp Thr Arg Met Phe Ala
   145                 150                 155                 160
   Asp Thr Val Val Lys Leu Asn Leu Gln Lys Leu Ala Thr Val Thr Glu
                   165                 170                 175
   Ala Met Ala Arg Asn Ala Gly Asp Gly Arg Gly Ser Arg Glu Thr Thr
               180                 185                 190
   Cys Arg Glu Ser Phe His Leu Ile Thr Ala Phe Glu Lys Gln Arg Gln
           195                 200                 205
   Ile Thr Glu Pro Thr Val Tyr Gln Asn Pro Pro Tyr His Thr Gly Met
       210                 215                 220
   Thr Pro Glu Pro Arg Thr Ser Thr Val Phe Ile Glu Leu Glu Asp His
   225                 230                 235                 240
   Arg Thr Leu Pro Gly Asn Leu Thr Pro Thr Thr Glu Glu His Leu Gln
                   245                 250                 255
   Arg Met Tyr Gln Arg Phe Trp Gly Ile Arg Gln Leu Gln Arg Pro Arg
               260                 265                 270
   Gln Ser Phe Gly Glu Arg Gln Ser Ile
           275                 280
<210> 17
   <211> 453
   <212> PRT
   <213> Vitis vinifera
<220>
   <223> wine grape cultivar PN40024 unnamed protein product, locus tag GSVIVT00015766001, GenBank Accession No. CAO63410.1
<400> 17
   Met Gln Met Lys Tyr Leu Glu Gly Lys Gln Asn Leu Met Glu Glu Lys
    1               5                  10                  15
   Gly Glu Lys Gln Cys Ile Pro Met Asp Leu Ala Val Arg Glu Ala Gln
               20                  25                  30
   Phe Lys Gly Ser Leu Leu Asp Arg Ile Thr Trp Leu Glu Gln Arg Leu
           35                  40                  45
   His Lys Leu Ser Leu Gln Leu Glu Thr Arg Ser Lys Gln Gln Pro His
       50                  55                  60
   Pro Ser Arg Met Gln Thr Ala Gly Glu Thr Ser Ser Arg His Gly Pro
   65                  70                  75                  80
   Lys Lys Glu Leu Ser Cys Ser Phe Pro Val Phe Ser Thr Arg Asn His
                   85                  90                  95
   Asn His Gly His Lys Gln Thr Ser Gln Phe His Val Pro Arg Phe Glu
               100                 105                 110
   Tyr Gln Glu Gly Gly Arg Glu Asn Pro Ala Val Val Ile Thr Lys Leu
           115                 120                 125
   Thr Pro Phe His His Pro Lys Ile Ile Thr Ile Leu Phe Pro Ile Ser
       130                 135                 140
   Asn Tyr Phe Ile Ile Phe Phe Phe Leu Thr Phe Asp Thr Lys Lys Gln
   145                 150                 155                 160
   Tyr Pro Leu Leu Phe Pro Ile Leu Pro Ser Arg Phe Leu Pro Ile Ser
                   165                 170                 175
   His Leu Ile Thr Gln Glu Ile Glu Lys Tyr Lys Thr Ser Ser His Phe
               180                 185                 190
   Ser Ser Pro Ala Ser Leu Phe Ala Ala Met Asn Lys Ala Glu Thr Ser
           195                 200                 205
   Ser Met Ala Glu Ala Glu Ser Glu Asp Ser Glu Thr Thr Thr Pro Thr
       210                 215                 220
   Thr His His Leu Thr Ile Pro Pro Gly Leu Thr Gln Pro Glu Phe Gln
   225                 230                 235                 240
   Glu Leu Ala His Ser Ile Ser Glu Phe His Thr Tyr Gln Val Gly Pro
                   245                 250                 255
   Gly Gln Cys Ser Ser Leu Leu Ala Gln Arg Val His Ala Pro Leu Pro
               260                 265                 270
   Thr Val Trp Ser Val Val Arg Arg Phe Asp Lys Pro Gln Thr Tyr Lys
           275                 280                 285
   His Phe Ile Lys Ser Cys His Val Glu Asp Gly Phe Glu Met Arg Val
       290                 295                 300
   Gly Cys Leu Arg Asp Val Asn Val Ile Ser Gly Leu Pro Ala Glu Thr
   305                 310                 315                 320
   Ser Thr Glu Arg Leu Asp Ile Leu Asp Asp Glu Arg His Val Thr Gly
                   325                 330                 335
   Phe Ser Ile Ile Gly Gly Glu His Arg Leu Arg Asn Tyr Arg Ser Val
               340                 345                 350
   Thr Thr Asn His Gly Gly Glu Ile Trp Thr Val Val Leu Glu Ser Tyr
           355                 360                 365
   Val Val Asp Met Pro Glu Gly Asn Thr Glu Glu Asp Thr Arg Leu Phe
       370                 375                 380
   Ala Asp Thr Val Val Lys Leu Asn Leu Gln Lys Leu Ala Ser Val Thr
   385                 390                 395                 400
   Glu Val Ser Gln Ser Cys Asn Tyr Pro Cys Gln Phe His Ile Ile Glu
                   405                 410                 415
   Asn Glu Asp Ile Gln Pro Glu Glu Met Asn Leu Gly Val Leu Thr Thr
               420                 425                 430
   Ser Ile Glu Glu Gln Arg Lys Lys Lys Arg Val Val Ala Met Lys Asp
           435                 440                 445
   Gly Ser Thr Ser Ser
       450
<210> 18
   <211> 195
   <212> PRT
   <213> Vitis vinifera
<220>
   <223> wine grape cultivar Pinot Noir hypothetical protein, clone ENTAV 115, locus tag VITISV_033963, GenBank Accession No. CAN64657.1
<220>
   <221> VARIANT
   <222> (193)...(193)
   <223> Xaa = any amino acid
<400> 18
   Met Ala Glu Ala Glu Ser Glu Asp Ser Glu Thr Thr Thr Pro Thr Thr
    1               5                  10                  15
   His His Leu Thr Ile Pro Pro Gly Leu Thr Gln Pro Glu Phe Gln Glu
               20                  25                  30
   Leu Ala His Ser Ile Ser Glu Phe His Thr Tyr Gln Val Gly Pro Gly
           35                  40                  45
   Gln Cys Ser Ser Leu Leu Ala Gln Arg Val His Ala Pro Leu Pro Thr
       50                  55                  60
   Val Trp Ser Val Val Arg Arg Phe Asp Lys Pro Gln Thr Tyr Lys His
   65                  70                  75                  80
   Phe Ile Lys Ser Cys His Val Glu Asp Gly Phe Glu Met Arg Val Gly
                   85                  90                  95
   Cys Leu Arg Asp Val Asn Val Ile Ser Gly Leu Pro Ala Glu Thr Ser
               100                 105                 110
   Thr Glu Arg Leu Asp Ile Leu Asp Asp Glu Arg His Val Thr Gly Phe
           115                 120                 125
   Ser Ile Ile Gly Gly Glu His Arg Leu Arg Asn Tyr Arg Ser Val Thr
       130                 135                 140
   Thr Val His Glu Tyr Gln Asn His Gly Gly Glu Ile Trp Thr Val Val
   145                 150                 155                 160
   Leu Glu Ser Tyr Val Val Asp Met Pro Glu Gly Asn Thr Glu Glu Asp
                   165                 170                 175
   Thr Arg Leu Phe Ala Asp Thr Val Val Lys Leu Asn Leu Ser Glu Ala
               180                 185                 190
   Xaa Arg Arg
           195
<210> 19
   <211> 217
   <212> PRT
   <213> Medicago truncatula
<220>
   <223> barrel medic unknown protein, clone MTYFD_FE_FF_FG1G-N-24, GenBank Accession No. ACJ85026.1
<400> 19
   Met Glu Lys Ala Glu Ser Ser Thr Ala Ser Thr Ser Asp Gln Asp Ser
    1               5                  10                  15
   Asp Glu Asn His Arg Thr Gln His His Leu Thr Leu Pro Ser Gly Leu
               20                  25                  30
   Arg Gln His Glu Phe Asp Ser Leu Ile Pro Phe Ile Asn Ser His His
           35                  40                  45
   Thr Tyr Leu Ile Gly Pro Asn Gln Cys Ser Thr Leu Leu Ala Gln Arg
       50                  55                  60
   Ile His Ala Pro Pro Gln Thr Val Trp Ser Val Val Arg Ser Phe Asp
   65                  70                  75                  80
   Lys Pro Gln Ile Tyr Lys His Ile Ile Lys Ser Cys Ser Leu Lys Glu
                   85                  90                  95
   Gly Phe Gln Met Lys Val Gly Cys Thr Arg Asp Val Asn Val Ile Ser
               100                 105                 110
   Gly Leu Pro Ala Ala Thr Ser Thr Glu Arg Leu Asp Val Leu Asp Asp
           115                 120                 125
   Glu Arg Arg Val Thr Gly Phe Ser Ile Ile Gly Gly Glu His Arg Leu
       130                 135                 140
   Lys Asn Tyr Arg Ser Val Thr Ser Val His Gly Phe Gly Asp Gly Asp
   145                 150                 155                 160
   Asn Gly Gly Glu Ile Trp Thr Val Val Leu Glu Ser Tyr Val Val Asp
                   165                 170                 175
   Val Pro Glu Gly Asn Thr Glu Glu Asp Thr Arg Leu Phe Ala Asp Thr
               180                 185                 190
   Val Val Lys Leu Asn Leu Gln Lys Leu Ala Ser Val Thr Glu Gly Lys
           195                 200                 205
   Asn Arg Asp Gly Asp Gly Lys Ser His
       210                 215
<210> 20
   <211> 212
   <212> PRT
   <213> Oryza sativa
<220>
   <223> rice Japonica Group, cultivar Nipponbare, conserved hypothetical protein Os10g0573400, GenBank Accession No. NP_00106570.1
<400> 20
   Met Glu Gln Gln Glu Glu Val Pro Pro Pro Pro Ala Gly Leu Gly Leu
    1               5                  10                  15
   Thr Ala Glu Glu Tyr Ala Gln Val Arg Ala Thr Val Glu Ala His His
               20                  25                  30
   Arg Tyr Ala Val Gly Pro Gly Gln Cys Ser Ser Leu Leu Ala Gln Arg
           35                  40                  45
   Ile His Ala Pro Pro Ala Ala Val Trp Ala Val Val Arg Arg Phe Asp
       50                  55                  60
   Cys Pro Gln Val Tyr Lys His Phe Ile Arg Ser Cys Val Leu Arg Pro
   65                  70                  75                  80
   Asp Pro His His Asp Asp Asn Gly Asn Asp Leu Arg Pro Gly Arg Leu
                   85                  90                  95
   Arg Glu Val Ser Val Ile Ser Gly Leu Pro Ala Ser Thr Ser Thr Glu
               100                 105                 110
   Arg Leu Asp Leu Leu Asp Asp Ala His Arg Val Phe Gly Phe Thr Ile
           115                 120                 125
   Thr Gly Gly Glu His Arg Leu Arg Asn Tyr Arg Ser Val Thr Thr Val
       130                 135                 140
   Ser Gln Leu Asp Glu Ile Cys Thr Leu Val Leu Glu Ser Tyr Ile Val
   145                 150                 155                 160
   Asp Val Pro Asp Gly Asn Thr Glu Asp Asp Thr Arg Leu Phe Ala Asp
                   165                 170                 175
   Thr Val Ile Arg Leu Asn Leu Gln Lys Leu Lys Ser Val Ser Glu Ala
               180                 185                 190
   Asn Ala Asn Ala Ala Ala Ala Ala Ala Ala Pro Pro Pro Pro Pro Pro
           195                 200                 205
   Ala Ala Ala Glu
       210
<210> 21
   <211> 212
   <212> PRT
   <213> Zea mays
<220>
   <223> maize cyclase/dehydrase family protein, clone 306819, GenBank Accession No. ACG40002.1
<400> 21
   Met Asp Gln Gln Gly Ala Gly Gly Asp Ala Glu Val Pro Ala Gly Leu
   1 5 10 15
   Gly Leu Thr Ala Ala Glu Tyr Glu Gln Leu Arg Ser Thr Val Asp Ala
   20 25 30
   His His Arg Tyr Ala Val Gly Glu Gly Gln Cys Ser Ser Leu Leu Ala
   35 40 45
   Gln Arg Ile His Ala Pro Pro Glu Ala Val Trp Ala Val Val Arg Arg
   50 55 60
   Phe Asp Cys Pro Gln Val Tyr Lys His Phe Ile Arg Ser Cys Ala Leu
   65 70 75 80
   Arg Pro Asp Pro Glu Ala Gly Asp Ala Leu Cys Pro Gly Arg Leu Arg
   85 90 95
   Glu Val Ser Val Ile Ser Gly Leu Pro Ala Ser Thr Ser Thr Glu Arg
   100 105 110
   Leu Asp Leu Leu Asp Asp Ala Ala Arg Val Phe Gly Phe Ser Ile Thr
   115 120 125
   Gly Gly Glu His Arg Leu Arg Asn Tyr Arg Ser Val Thr Thr Val Ser
   130 135 140
   Glu Leu Ala Val Pro Ala Ile Cys Thr Val Val Leu Glu Ser Tyr Val
   145 150 155 160
   Val Asp Val Pro Asp Gly Asn Thr Glu Asp Asp Thr Arg Leu Phe Ala
   165 170 175
   Asp Thr Val Ile Arg Leu Asn Leu Gln Lys Leu Lys Ser Val Ala Glu
   180 185 190
   Ala Asn Ala Ala Glu Ala Ala Ala Thr Thr Asn Ser Val Leu Leu Pro
   195 200 205
   Arg Pro Ala Glu
   210
<210> 22
   <211> 212
   <212> PRT
   <213> Zea mays
<220>
   <223> maize cyclase/dehydrase family protein, clone 241996, GenBank Accession No. ACG34473.1
<220>
   <221> VARIANT
   <222> (11)...(11)
   <223> Xaa = any amino acid
<400> 22
   Met Asp Gln Gln Gly Ala Gly Gly Asp Ala Xaa Val Pro Ala Gly Leu
   1 5 10 15
   Gly Leu Thr Ala Ala Glu Tyr Glu Gln Leu Arg Ser Thr Val Asp Ala
   20 25 30
   His His Arg Tyr Ala Val Gly Glu Gly Gln Cys Ser Ser Leu Leu Ala
   35 40 45
   Gln Arg Ile His Ala Pro Pro Glu Ala Val Trp Ala Val Val Arg Arg
   50 55 60
   Phe Asp Cys Pro Gln Val Tyr Lys His Phe Ile Arg Ser Cys Ala Leu
   65 70 75 80
   Arg Pro Asp Pro Glu Ala Gly Asp Ala Leu Cys Pro Gly Arg Leu Arg
   85 90 95
   Glu Val Ser Val Ile Ser Gly Leu Pro Ala Ser Thr Ser Thr Glu Arg
   100 105 110
   Leu Asp Leu Leu Asp Asp Ala Ala Arg Val Phe Gly Phe Ser Ile Thr
           115                 120                 125
   Gly Gly Glu His Arg Leu Arg Asn Tyr Arg Ser Val Thr Thr Val Ser
       130                 135                 140
   Glu Leu Ala Asp Pro Ala Ile Cys Thr Val Val Leu Glu Ser Tyr Val
   145                 150                 155                 160
   Val Asp Val Pro Asp Gly Asn Thr Glu Asp Asp Thr Arg Leu Phe Ala
                   165                 170                 175
   Asp Thr Val Ile Arg Leu Asn Leu Gln Lys Leu Lys Ser Val Thr Glu
               180                 185                 190
   Ala Asn Ala Ala Glu Ala Ala Ala Thr Thr Asn Ser Val Leu Leu Pro
           195                 200                 205
   Arg Pro Ala Glu
       210
<210> 23
   <211> 233
   <212> PRT
   <213> Vitis vinifera
<220>
   <223> wine grape cultivar PN40024 unnamed protein product, locus tag GSVIVT00032173001, GenBank Accession No. CAO43790.1
<400> 23
   Met Asp Pro His His His His Gly Leu Thr Glu Glu Glu Phe Arg Ala
    1               5                  10                  15
   Leu Glu Pro Ile Ile Gln Asn Tyr His Thr Phe Glu Pro Ser Pro Asn
               20                  25                  30
   Thr Cys Thr Ser Leu Ile Thr Gln Lys Ile Asp Ala Pro Ala Gln Val
           35                  40                  45
   Val Trp Pro Phe Val Arg Ser Phe Glu Asn Pro Gln Lys Tyr Lys His
       50                  55                  60
   Phe Ile Lys Asp Cys Thr Met Arg Gly Asp Gly Gly Val Gly Ser Ile
   65                  70                  75                  80
   Arg Glu Val Thr Val Val Ser Gly Leu Pro Ala Ser Thr Ser Thr Glu
                   85                  90                  95
   Arg Leu Glu Ile Leu Asp Asp Glu Lys His Ile Leu Ser Phe Arg Val
               100                 105                 110
   Val Gly Gly Glu His Arg Leu Asn Asn Tyr Arg Ser Val Thr Ser Val
           115                 120                 125
   Asn Asp Phe Ser Lys Glu Gly Lys Asp Tyr Thr Ile Val Leu Glu Ser
       130                 135                 140
   Tyr Ile Val Asp Ile Pro Glu Gly Asn Thr Gly Glu Asp Thr Lys Met
   145                 150                 155                 160
   Phe Val Asp Thr Val Val Lys Leu Asn Leu Gln Lys Leu Ala Val Val
                   165                 170                 175
   Ala Ile Thr Ser Leu His Glu Asn Glu Glu Ile Ala Asp Asn Glu Gly
               180                 185                 190
   Pro Ser Arg Glu Ile Ser Leu Gln Ser Glu Thr Glu Ser Ala Glu Arg
           195                 200                 205
   Gly Asp Glu Arg Arg Asp Gly Asp Gly Pro Ser Lys Ala Cys Asn Arg
       210                 215                 220
   Asn Glu Trp His Cys Thr Thr Lys Glu
   225                 230
<210> 24
   <211> 207
   <212> PRT
   <213> Oryza sativa
<220>
   <223> rice Japonica Group, cultivar Nipponbare, Bet v I allergen-like protein, clone P0495C02.29, GenBank Accession No. BAD25659.1
<400> 24
   Met Glu Pro His Met Glu Arg Ala Leu Arg Glu Ala Val Ala Ser Glu
    1               5                  10                  15
   Ala Glu Arg Arg Glu Leu Glu Gly Val Val Arg Ala His His Thr Phe
               20                  25                  30
   Pro Ala Ala Glu Arg Ala Ala Gly Pro Gly Arg Arg Pro Thr Cys Thr
           35                  40                  45
   Ser Leu Val Ala Gln Arg Val Asp Ala Pro Leu Ala Ala Val Trp Pro
       50                  55                  60
   Ile Val Arg Gly Phe Ala Asn Pro Gln Arg Tyr Lys His Phe Ile Lys
   65                  70                  75                  80
   Ser Cys Glu Leu Ala Ala Gly Asp Gly Ala Thr Val Gly Ser Val Arg
                   85                  90                  95
   Glu Val Ala Val Val Ser Gly Leu Pro Ala Ser Thr Ser Thr Glu Arg
               100                 105                 110
   Leu Glu Ile Leu Asp Asp Asp Arg His Val Leu Ser Phe Arg Val Val
           115                 120                 125
   Gly Gly Asp His Arg Leu Arg Asn Tyr Arg Ser Val Thr Ser Val Thr
       130                 135                 140
   Glu Phe Ser Ser Pro Ser Ser Pro Pro Arg Pro Tyr Cys Val Val Val
   145                 150                 155                 160
   Glu Ser Tyr Val Val Asp Val Pro Glu Gly Asn Thr Glu Glu Asp Thr
                   165                 170                 175
   Arg Met Phe Thr Asp Thr Val Val Lys Leu Asn Leu Gln Lys Leu Ala
               180                 185                 190
   Ala Val Ala Thr Ser Ser Ser Pro Pro Ala Ala Gly Asn His His
           195                 200                 205
<210> 25
   <211> 210
   <212> PRT
   <213> Oryza sativa
<220>
   <223> rice Indica Group, cultivar 93-11, hypothetical protein OsI_06433, GenBank Accession No. EAY85077.1
<400> 25
   Met Glu Pro His Met Glu Arg Ala Leu Arg Glu Ala Val Ala Ser Glu
    1               5                  10                  15
   Ala Glu Arg Arg Glu Leu Glu Gly Val Val Arg Ala His His Thr Phe
               20                  25                  30
   Pro Ala Ala Glu Arg Ala Ala Gly Pro Gly Arg Arg Pro Thr Cys Thr
           35                  40                  45
   Ser Leu Val Ala Gln Arg Val Asp Ala Pro Leu Ala Ala Val Trp Pro
       50                  55                  60
   Ile Val Arg Gly Phe Ala Asn Pro Gln Arg Tyr Lys His Phe Ile Lys
   65                  70                  75                  80
   Ser Cys Glu Leu Ala Ala Gly Asp Gly Ala Thr Val Gly Ser Val Arg
                   85                  90                  95
   Glu Val Ala Val Val Ser Gly Leu Pro Ala Ser Thr Ser Thr Glu Arg
               100                 105                 110
   Leu Glu Ile Leu Asp Asp Asp Arg His Val Leu Ser Phe Arg Val Val
           115                 120                 125
   Gly Gly Asp His Arg Leu Arg Asn Tyr Arg Ser Val Thr Ser Val Thr
       130                 135                 140
   Glu Phe Ser Ser Pro Ser Ser Pro Pro Ser Pro Pro Arg Pro Tyr Cys
   145                 150                 155                 160
   Val Val Val Glu Ser Tyr Val Val Asp Val Pro Glu Gly Asn Thr Glu
                   165                 170                 175
   Glu Asp Thr Arg Met Phe Thr Asp Thr Val Val Lys Leu Asn Leu Gln
               180                 185                 190
   Lys Leu Ala Ala Val Ala Thr Ser Ser Ser Pro Pro Ala Ala Gly Asn
           195                 200                 205
   His His
       210
<210> 26
   <211> 200
   <212> PRT
   <213> Zea mays
<220>
   <223> maize strain B73 unknown protein, clone ZM_BFb0151H07, GenBank Accession No. ACF82013.1
<400> 26
   Met Pro Tyr Thr Ala Pro Arg Pro Ser Pro Gln Gln His Ser Arg Val
    1               5                  10                  15
   Leu Ser Gly Gly Gly Ala Lys Ala Ala Ser His Gly Ala Ser Cys Ala
               20                  25                  30
   Ala Val Pro Ala Glu Val Ala Arg His His Glu His Ala Ala Arg Ala
           35                  40                  45
   Gly Gln Cys Cys Ser Ala Val Val Gln Ala Ile Ala Ala Pro Val Gly
       50                  55                  60
   Ala Val Trp Ser Val Val Arg Arg Phe Asp Arg Pro Gln Ala Tyr Lys
   65                  70                  75                  80
   His Phe Ile Arg Ser Cys Arg Leu Val Gly Gly Gly Asp Val Ala Val
                   85                  90                  95
   Gly Ser Val Arg Glu Val Arg Val Val Ser Gly Leu Pro Ala Thr Ser
               100                 105                 110
   Ser Arg Glu Arg Leu Glu Ile Leu Asp Asp Glu Arg Arg Val Leu Ser
           115                 120                 125
   Phe Arg Val Val Gly Gly Glu His Arg Leu Ala Asn Tyr Arg Ser Val
       130                 135                 140
   Thr Thr Val His Glu Ala Gly Ala Gly Ala Gly Thr Gly Thr Val Val
   145                 150                 155                 160
   Val Glu Ser Tyr Val Val Asp Val Pro His Gly Asn Thr Ala Asp Glu
                   165                 170                 175
   Thr Arg Val Phe Val Asp Thr Ile Val Arg Cys Asn Leu Gln Ser Leu
               180                 185                 190
   Ala Arg Thr Ala Glu Arg Leu Ala
           195                 200
<210> 27
   <211> 215
   <212> PRT
   <213> Vitis vinifera
<220>
   <223> wine grape cultivar PN40024 unnamed protein product, locus tag GSVIVT00037390001, GenBank Accession No. CAO48777.1
<400> 27
   Met Pro Ser Asn Pro Pro Lys Ser Ser Leu Val Val His Arg Ile Asn
    1               5                  10                  15
   Ser Pro Asn Ser Ile Thr Thr Ala Thr Thr Ala Ser Ala Ala Ala Asn
               20                  25                  30
   Asn His Asn Thr Ser Thr Met Pro Pro His Lys Gln Val Pro Asp Ala
           35                  40                  45
   Val Ser Arg His His Thr His Val Val Gly Pro Asn Gln Cys Cys Ser
       50                  55                  60
   Ala Val Val Gln Gln Ile Ala Ala Pro Val Ser Thr Val Trp Ser Val
   65                  70                  75                  80
   Val Arg Arg Phe Asp Asn Pro Gln Ala Tyr Lys His Phe Val Lys Ser
                   85                  90                  95
   Cys His Val Val Val Gly Asp Gly Asp Val Gly Thr Leu Arg Glu Val
               100                 105                 110
   His Val Ile Ser Gly Leu Pro Ala Ala Asn Ser Thr Glu Arg Leu Glu
           115                 120                 125
   Ile Leu Asp Asp Glu Arg His Val Leu Ser Phe Ser Val Ile Gly Gly
       130                 135                 140
   Asp His Arg Leu Ser Asn Tyr Arg Ser Val Thr Thr Leu His Pro Ser
   145                 150                 155                 160
   Pro Ser Ser Thr Gly Thr Val Val Leu Glu Ser Tyr Val Val Asp Ile
                   165                 170                 175
   Pro Pro Gly Asn Thr Lys Glu Asp Thr Cys Val Phe Val Asp Thr Ile
               180                 185                 190
   Val Arg Cys Asn Leu Gln Ser Leu Ala Gln Ile Ala Glu Asn Ala Ala
           195                 200                 205
   Gly Cys Lys Arg Ser Ser Ser
       210                 215
<210> 28
   <211> 213
   <212> PRT
   <213> Nicotiana tabacum
<220>
   <223> tobacco hypothetical protein, gene c17, GenBank Accession No. CAI84653.1
<400> 28
   Met Pro Pro Ser Ser Pro Asp Ser Ser Val Leu Leu Gln Arg Ile Ser
    1              5                   10                  15
   Ser Asn Thr Thr Pro Asp Phe Ala Cys Lys Gln Ser Gln Gln Leu Gln
               20                  25                  30
   Arg Arg Thr Met Pro Ile Pro Cys Thr Thr Gln Val Pro Asp Ser Val
           35                  40                  45
   Val Arg Phe His Thr His Pro Val Gly Pro Asn Gln Cys Cys Ser Ala
       50                  55                  60
   Val Ile Gln Arg Ile Ser Ala Pro Val Ser Thr Val Trp Ser Val Val
   65                  70                  75                  80
   Arg Arg Phe Asp Asn Pro Gln Ala Tyr Lys His Phe Val Lys Ser Cys
                   85                  90                  95
   His Val Ile Val Gly Asp Gly Asp Val Gly Thr Leu Arg Glu Val Arg
               100                 105                 110
   Val Ile Ser Gly Leu Pro Ala Ala Ser Ser Thr Glu Arg Leu Glu Ile
           115                 120                 125
   Leu Asp Asp Glu Arg His Val Ile Ser Phe Ser Val Val Gly Gly Asp
       130                 135                 140
   His Arg Leu Ala Asn Tyr Arg Ser Val Thr Thr Leu His Pro Glu Pro
   145                 150                 155                 160
   Ser Gly Asp Gly Thr Thr Ile Val Val Glu Ser Tyr Val Val Asp Val
                   165                 170                 175
   Pro Pro Gly Asn Thr Arg Asp Glu Thr Cys Val Phe Val Asp Thr Ile
               180                 185                 190
   Val Lys Cys Asn Leu Thr Ser Leu Ser Gln Ile Ala Val Asn Val Asn
           195                 200                 205
   Arg Arg Lys Asp Ser
       210
<210> 29
   <211> 208
   <212> PRT
   <213> Oryza sativa
<220>
   <223> rice Indica Group, cultivar 93-11, hypothetical protein OsI_04285, GenBank Accession No. EAY76350.1
<400> 29
   Met Pro Tyr Ala Ala Val Arg Pro Ser Pro Pro Pro Gln Leu Ser Arg
    1               5                  10                  15
   Pro Ile Gly Ser Gly Ala Gly Gly Gly Lys Ala Cys Pro Ala Val Pro
               20                  25                  30
   Cys Glu Val Ala Arg Tyr His Glu His Ala Val Gly Ala Gly Gln Cys
           35                  40                  45
   Cys Ser Thr Val Val Gln Ala Ile Ala Ala Pro Ala Asp Ala Val Trp
       50                  55                  60
   Ser Val Val Arg Arg Phe Asp Arg Pro Gln Ala Tyr Lys Lys Phe Ile
   65                  70                  75                  80
   Lys Ser Cys Arg Leu Val Asp Gly Asp Gly Gly Glu Val Gly Ser Val
                   85                  90                  95
   Arg Glu Val Arg Val Val Ser Gly Leu Pro Ala Thr Ser Ser Arg Glu
               100                 105                 110
   Arg Leu Glu Val Leu Asp Asp Asp Arg Arg Val Leu Ser Phe Arg Ile
           115                 120                 125
   Val Gly Gly Glu His Arg Leu Ala Asn Tyr Arg Ser Val Thr Thr Val
       130                 135                 140
   His Glu Ala Ala Ala Pro Ala Met Ala Val Val Val Glu Ser Tyr Val
   145                 150                 155                 160
   Val Asp Val Pro Pro Gly Asn Thr Trp Glu Glu Thr Arg Val Phe Val
                   165                 170                 175
   Asp Thr Ile Val Arg Cys Asn Leu Gln Ser Leu Ala Arg Thr Val Glu
               180                 185                 190
   Arg Leu Ala Pro Glu Ala Pro Arg Ala Asn Gly Ser Ile Asp His Ala
           195                 200                 205
<210> 30
   <211> 208
   <212> PRT
   <213> Oryza sativa
<220>
   <223> rice Japonica Group, cultivar Nipponbare, Bet v I allergen-like protein, gene B1088C09.11, clone B1088C09, GenBank Accession No. BAB68102.1
<400> 30
   Met Pro Tyr Ala Ala Val Arg Pro Ser Pro Pro Pro Gln Leu Ser Arg
    1               5                  10                  15
   Pro Ile Gly Ser Gly Ala Gly Gly Gly Lys Ala Cys Pro Ala Val Pro
               20                  25                  30
   Cys Glu Val Ala Arg Tyr His Glu His Ala Val Gly Ala Gly Gln Cys
           35                  40                  45
   Phe Ser Thr Val Val Gln Ala Ile Ala Ala Pro Ala Asp Ala Val Trp
       50                  55                  60
   Ser Val Val Arg Arg Phe Asp Arg Pro Gln Ala Tyr Lys Lys Phe Ile
   65                  70                  75                  80
   Lys Ser Cys Arg Leu Val Asp Gly Asp Gly Gly Glu Val Gly Ser Val
                   85                  90                  95
   Arg Glu Val Arg Val Val Ser Gly Leu Pro Ala Thr Ser Ser Arg Glu
               100                 105                 110
   Arg Leu Glu Val Leu Asp Asp Asp Arg Arg Val Leu Ser Phe Arg Ile
           115                 120                 125
   Val Gly Gly Glu His Arg Leu Ala Asn Tyr Arg Ser Val Thr Thr Val
       130                 135                 140
   His Glu Ala Ala Ala Pro Ala Met Ala Val Val Val Glu Ser Tyr Val
   145                 150                 155                 160
   Val Asp Val Pro Pro Gly Asn Thr Trp Glu Glu Thr Arg Val Phe Val
                   165                 170                 175
   Asp Thr Ile Val Arg Cys Asn Leu Gln Ser Leu Ala Arg Thr Val Glu
               180                 185                 190
   Arg Leu Ala Pro Glu Ala Pro Arg Ala Asn Gly Ser Ile Asp His Ala
           195                 200                 205
<210> 31
   <211> 213
   <212> PRT
   <213> Picea sitchensis
<220>
   <223> Sitka spruce cultivar FB3-425, unknown protein, clone WS0276_P02, GenBank Accession No. ABK22940.1
<400> 31
   Met Asp Ile Ile Ala Gly Phe Asp Gln Leu Ser Phe Arg Leu Ser Gly
    1               5                  10                  15
   Ala Ser Lys Gln Ile Thr Lys Thr Gly Ala Val Gln Tyr Leu Lys Gly
               20                  25                  30
   Glu Glu Gly Tyr Gly Glu Trp Leu Lys Glu Val Met Gly Arg Tyr His
           35                  40                  45
   Tyr His Ser His Asp Gly Ala Arg Glu Cys Arg Cys Ser Ser Val Val
       50                  55                  60
   Val Gln Gln Val Glu Ala Pro Val Ser Val Val Trp Ser Leu Val Arg
   65                  70                  75                  80
   Arg Phe Asp Gln Pro Gln Val Tyr Lys His Phe Val Ser Asn Cys Phe
                   85                  90                  95
   Met Arg Gly Asp Leu Lys Val Gly Cys Leu Arg Glu Val Arg Val Val
               100                 105                 110
   Ser Gly Leu Pro Ala Ala Thr Ser Thr Glu Arg Leu Asp Ile Leu Asp
           115                 120                 125
   Glu Glu Arg His Ile Leu Ser Phe Ser Ile Val Gly Gly Asp His Arg
       130                 135                 140
   Leu Asn Asn Tyr Arg Ser Ile Thr Thr Leu His Glu Thr Leu Ile Asn
   145                 150                 155                 160
   Gly Lys Pro Gly Thr Ile Val Ile Glu Ser Tyr Val Leu Asp Val Pro
                   165                 170                 175
   His Gly Asn Thr Lys Glu Glu Thr Cys Leu Phe Val Asp Thr Ile Val
               180                 185                 190
   Lys Cys Asn Leu Gln Ser Leu Ala His Val Ser Asn His Leu Asn Ser
           195                 200                 205
   Thr His Arg Cys Leu
       210
<210> 32
   <211> 207
   <212> PRT
   <213> Oryza sativa
<220>
   <223> rice Japonica Group, cultivar Nipponbare, hypothetical protein Os06g0562200, Bet v I allergen family protein, GenBank Accession No. NP_001057874.1
<400> 32
   Met Glu Ala His Val Glu Arg Ala Leu Arg Glu Gly Leu Thr Glu Glu
    1               5                  10                  15
   Glu Arg Ala Ala Leu Glu Pro Ala Val Met Ala His His Thr Phe Pro
               20                  25                  30
   Pro Ser Thr Thr Thr Ala Thr Thr Ala Ala Ala Thr Cys Thr Ser Leu
           35                  40                  45
   Val Thr Gln Arg Val Ala Ala Pro Val Arg Ala Val Trp Pro Ile Val
       50                  55                  60
   Arg Ser Phe Gly Asn Pro Gln Arg Tyr Lys His Phe Val Arg Thr Cys
   65                  70                  75                  80
   Ala Leu Ala Ala Gly Asp Gly Ala Ser Val Gly Ser Val Arg Glu Val
                   85                  90                  95
   Thr Val Val Ser Gly Leu Pro Ala Ser Thr Ser Thr Glu Arg Leu Glu
               100                 105                 110
   Met Leu Asp Asp Asp Arg His Ile Ile Ser Phe Arg Val Val Gly Gly
           115                 120                 125
   Gln His Arg Leu Arg Asn Tyr Arg Ser Val Thr Ser Val Thr Glu Phe
       130                 135                 140
   Gln Pro Pro Ala Ala Gly Pro Gly Pro Ala Pro Pro Tyr Cys Val Val
   145                 150                 155                 160
   Val Glu Ser Tyr Val Val Asp Val Pro Asp Gly Asn Thr Ala Glu Asp
                   165                 170                 175
   Thr Arg Met Phe Thr Asp Thr Val Val Lys Leu Asn Leu Gln Met Leu
               180                 185                 190
   Ala Ala Val Ala Glu Asp Ser Ser Ser Ala Ser Arg Arg Arg Asp
           195                 200                 205
<210> 33
   <211> 216
   <212> PRT
   <213> Oryza sativa
<220>
   <223> rice Japonica Group, cultivar Nipponbare, hypothetical protein Os05g0473000, Streptomyces cyclase/dehydrase family protein, GenBank Accession No. NP_001055819.1
<400> 33
   Met Pro Tyr Thr Ala Pro Arg Pro Ser Pro Pro Gln His Ser Arg Ile
    1               5                  10                  15
   Gly Gly Cys Gly Gly Gly Gly Val Leu Lys Ala Ala Gly Ala Ala Gly
               20                  25                  30
   His Ala Ala Ser Cys Val Ala Val Pro Ala Glu Val Ala Arg His His
           35                  40                  45
   Glu His Ala Ala Gly Val Gly Gln Cys Cys Ser Ala Val Val Gln Ala
       50                  55                  60
   Ile Ala Ala Pro Val Asp Ala Val Trp Ser Val Val Arg Arg Phe Asp
   65                  70                  75                  80
   Arg Pro Gln Ala Tyr Lys His Phe Ile Arg Ser Cys Arg Leu Leu Asp
                   85                  90                  95
   Gly Asp Gly Asp Gly Gly Ala Val Ala Val Gly Ser Val Arg Glu Val
               100                 105                 110
   Arg Val Val Ser Gly Leu Pro Ala Thr Ser Ser Arg Glu Arg Leu Glu
           115                 120                 125
   Ile Leu Asp Asp Glu Arg Arg Val Leu Ser Phe Arg Val Val Gly Gly
       130                 135                 140
   Glu His Arg Leu Ser Asn Tyr Arg Ser Val Thr Thr Val His Glu Thr
   145                 150                 155                 160
   Ala Ala Gly Ala Ala Ala Ala Val Val Val Glu Ser Tyr Val Val Asp
                   165                 170                 175
   Val Pro His Gly Asn Thr Ala Asp Glu Thr Arg Met Phe Val Asp Thr
               180                 185                 190
   Ile Val Arg Cys Asn Leu Gln Ser Leu Ala Arg Thr Ala Glu Gln Leu
           195                 200                 205
   Ala Leu Ala Ala Pro Arg Ala Ala
       210                 215
<210> 34
   <211> 212
   <212> PRT
   <213> Vitis vinifera
<220>
   <223> wine grape cultivar PN40024 unnamed protein product, locus tag GSVIVT00029365001, GenBank Accession No. CAO41436.1
<400> 34
   Met Pro Ser Ser Leu Gln Leu His Arg Ile Asn Asn Ile Asp Pro Thr
    1               5                  10                  15
   Thr Val Ala Val Ala Ala Thr Ala Ala Val Asn Cys His Lys Gln Ser
               20                  25                  30
   Arg Thr Pro Leu Arg Cys Ala Thr Pro Val Pro Asp Ala Val Ala Ser
           35                  40                  45
   Tyr His Ala His Ala Val Gly Pro His Gln Cys Cys Ser Met Val Val
       50                  55                  60
   Gln Thr Thr Ala Ala Ala Leu Pro Thr Val Trp Ser Val Val Arg Arg
   65                  70                  75                  80
   Phe Asp Asn Pro Gln Ala Tyr Lys His Phe Leu Lys Ser Cys His Val
                   85                  90                  95
   Ile Phe Gly Asp Gly Asp Ile Gly Thr Leu Arg Glu Val His Val Val
               100                 105                 110
   Ser Gly Leu Pro Ala Glu Ser Ser Thr Glu Arg Leu Glu Ile Leu Asp
           115                 120                 125
   Asp Glu Arg His Val Leu Ser Phe Ser Val Val Gly Gly Asp His Arg
       130                 135                 140
   Leu Cys Asn Tyr Arg Ser Val Thr Thr Leu His Pro Ser Pro Thr Gly
   145                 150                 155                 160
   Thr Gly Thr Val Val Val Glu Ser Tyr Val Val Asp Ile Pro Pro Gly
                   165                 170                 175
   Asn Thr Lys Glu Asp Thr Cys Val Phe Val Asp Thr Ile Val Lys Cys
               180                 185                 190
   Asn Leu Gln Ser Leu Ala Gln Met Ser Glu Lys Leu Thr Asn Asn Asn
           195                 200                 205
   Arg Asn Ser Ser
       210
<210> 35
   <211> 218
   <212> PRT
   <213> Zea mays
<220>
   <223> maize cyclase/dehydrase family protein, clone 1678999, GenBank Accession No. ACG30334.1
<400> 35
   Met Pro Cys Leu Gln Ala Ser Ser Pro Gly Ser Met Pro Tyr Gln His
   1               5                   10                  15
   His Gly Arg Gly Val Gly Cys Ala Ala Glu Ala Gly Ala Ala Val Gly
               20                  25                  30
   Ala Ser Ala Gly Thr Gly Thr Arg Cys Gly Ala His Asp Gly Glu Val
           35                  40                  45
   Pro Ala Glu Ala Ala Arg His His Glu His Ala Ala Pro Gly Pro Gly
       50                  55                  60
   Arg Cys Cys Ser Ala Val Val Gln Arg Val Ala Ala Pro Ala Glu Ala
   65                  70                  75                  80
   Val Trp Ser Val Val Arg Arg Phe Asp Gln Pro Gln Ala Tyr Lys Arg
                   85                  90                  95
   Phe Val Arg Ser Cys Ala Leu Leu Ala Gly Asp Gly Gly Val Gly Thr
               100                 105                 110
   Leu Arg Glu Val Arg Val Val Ser Gly Leu Pro Ala Ala Ser Ser Arg
           115                 120                 125
   Glu Arg Leu Glu Val Leu Asp Asp Glu Ser His Val Leu Ser Phe Arg
       130                 135                 140
   Val Val Gly Gly Glu His Arg Leu Gln Asn Tyr Leu Ser Val Thr Thr
   145                 150                 155                 160
   Val His Pro Ser Pro Ala Ala Pro Asp Ala Ala Thr Val Val Val Glu
                   165                 170                 175
   Ser Tyr Val Val Asp Val Pro Pro Gly Asn Thr Pro Glu Asp Thr Arg
               180                 185                 190
   Val Phe Val Asp Thr Ile Val Lys Cys Asn Leu Gln Ser Leu Ala Thr
           195                 200                 205
   Thr Ala Glu Lys Leu Ala Leu Ala Ala Val
       210                 215
<210> 36
   <211> 179
   <212> PRT
   <213> Physcomitrella patens
<220>
   <223> Physcomitrella patens subsp. patens moss, ecotype Gransden 2004, hypothetical protein, predicted protein, locus tag PHYPADRAFT_222359, GenBank Accession No. XP_001778048.1
<400> 36
   Met Gln Thr Lys Gly Arg Gln Ala Asp Phe Gln Thr Leu Leu Glu Gly
   1               5                   10                  15
   Gln Gln Asp Leu Ile Cys Arg Phe His Arg His Glu Leu Gln Pro His
               20                  25                  30
   Gln Cys Gly Ser Ile Leu Leu Gln Leu Ile Lys Ala Pro Val Glu Thr
           35                  40                  45
   Val Trp Ser Val Ala Arg Ser Phe Asp Lys Pro Gln Val Tyr Lys Arg
       50                  55                  60
   Phe Ile Gln Thr Cys Glu Ile Ile Glu Gly Asp Gly Gly Val Gly Ser
   65                  70                  75                  80
   Ile Arg Glu Val Arg Leu Val Ser Ser Ile Pro Ala Thr Ser Ser Ile
                   85                  90                  95
   Glu Arg Leu Glu Ile Leu Asp Asp Glu Glu His Ile Ile Ser Phe Arg
               100                 105                 110
   Val Leu Gly Gly Gly His Arg Leu Gln Asn Tyr Trp Ser Val Thr Ser
           115                 120                 125
   Leu His Ser His Glu Ile Asp Gly Gln Met Gly Thr Leu Val Leu Glu
       130                 135                 140
   Ser Tyr Val Val Asp Ile Pro Glu Gly Asn Thr Arg Glu Glu Thr His
   145                 150                 155                 160
   Met Phe Val Asp Thr Val Val Arg Cys Asn Leu Lys Ala Leu Ala Gln
                   165                 170                 175
   Val Ser Glu
<210> 37
   <211> 229
   <212> PRT
   <213> Oryza sativa
<220>
   <223> rice Indica Group, cultivar 93-11, hypothetical protein OsI_11160, GenBank Accession No. EAY89631.1
<400> 37
   Met Pro Cys Ile Pro Ala Ser Ser Pro Gly Ile Pro His Gln His Gln
   1               5                   10                  15
   His Gln His His Arg Ala Leu Ala Gly Val Gly Met Ala Val Gly Cys
               20                  25                  30
   Ala Ala Glu Ala Ala Val Ala Ala Ala Gly Val Ala Gly Thr Arg Cys
           35                  40                  45
   Gly Ala His Asp Gly Glu Val Pro Met Glu Val Ala Arg His His Glu
       50                  55                  60
   His Ala Glu Pro Gly Ser Gly Arg Cys Cys Ser Ala Val Val Gln His
   65                  70                  75                  80
   Val Ala Ala Pro Ala Pro Ala Val Trp Ser Val Val Arg Arg Phe Asp
                   85                  90                  95
   Gln Pro Gln Ala Tyr Lys Arg Phe Val Arg Ser Cys Ala Leu Leu Ala
               100                 105                 110
   Gly Asp Gly Gly Val Gly Thr Leu Arg Glu Val Arg Val Val Ser Gly
           115                 120                 125
   Leu Pro Ala Ala Ser Ser Arg Glu Arg Leu Glu Ile Leu Asp Asp Glu
       130                 135                 140
   Ser His Val Leu Ser Phe Arg Val Val Gly Gly Glu His Arg Leu Lys
   145                 150                 155                 160
   Asn Tyr Leu Ser Val Thr Thr Val His Pro Ser Pro Ser Ala Pro Thr
                   165                 170                 175
   Ala Ala Thr Val Val Val Glu Ser Tyr Val Val Asp Val Pro Pro Gly
               180                 185                 190
   Asn Thr Pro Glu Asp Thr Arg Val Phe Val Asp Thr Ile Val Lys Cys
           195                 200                 205
   Asn Leu Gln Ser Leu Ala Lys Thr Ala Glu Lys Leu Ala Ala Gly Ala
       210                 215                 220
   Arg Ala Ala Gly Ser
   225
<210> 38
   <211> 229
   <212> PRT
   <213> Oryza sativa
<220>
   <223> rice Japonica Group, cultivar Nipponbare, hypothetical protein Os03g0297600, Streptomyces cyclase/dehydrase family protein, GenBank Accession No. NP_001049838.1
<400> 38
   Met Pro Cys Ile Pro Ala Ser Ser Pro Gly Ile Pro His Gln His Gln
    1               5                  10                  15
   His Gln His His Arg Ala Leu Ala Gly Val Gly Met Ala Val Gly Cys
               20                  25                  30
   Ala Ala Glu Ala Ala Val Ala Ala Ala Gly Val Ala Gly Thr Arg Cys
           35                  40                  45
   Gly Ala His Asp Gly Glu Val Pro Met Glu Val Ala Arg His His Glu
       50                  55                  60
   His Ala Glu Pro Gly Ser Gly Arg Cys Cys Ser Ala Val Val Gln His
   65                  70                  75                  80
   Val Ala Ala Pro Ala Ala Ala Val Trp Ser Val Val Arg Arg Phe Asp
                   85                  90                  95
   Gln Pro Gln Ala Tyr Lys Arg Phe Val Arg Ser Cys Ala Leu Leu Ala
               100                 105                 110
   Gly Asp Gly Gly Val Gly Thr Leu Arg Glu Val Arg Val Val Ser Gly
           115                 120                 125
   Leu Pro Ala Ala Ser Ser Arg Glu Arg Leu Glu Ile Leu Asp Asp Glu
       130                 135                 140
   Ser His Val Leu Ser Phe Arg Val Val Gly Gly Glu His Arg Leu Lys
   145                 150                 155                 160
   Asn Tyr Leu Ser Val Thr Thr Val His Pro Ser Pro Ser Ala Pro Thr
                   165                 170                 175
   Ala Ala Thr Val Val Val Glu Ser Tyr Val Val Asp Val Pro Pro Gly
               180                 185                 190
   Asn Thr Pro Glu Asp Thr Arg Val Phe Val Asp Thr Ile Val Lys Cys
           195                 200                 205
   Asn Leu Gln Ser Leu Ala Lys Thr Ala Glu Lys Leu Ala Ala Gly Ala
       210                 215                 220
   Arg Ala Ala Gly Ser
                                 225
<210> 39
   <211> 205
   <212> PRT
   <213> Medicago truncatula
<220>
   <223> barrel medic unknown protein, clone MTYFP_FQ_FR_FS1G-H-19, GenBank Accession No. ACJ85898.1
<400> 39
   Met Pro Ser Pro Val Gln Phe Gln Arg Phe Asp Ser Asn Thr Ala Ile
    1               5                  10                  15
   Thr Asn Gly Val Asn Cys Pro Lys Gln Ile Gln Ala Cys Arg Tyr Ala
               20                  25                  30
   Leu Ser Ser Leu Lys Pro Thr Val Ser Val Pro Glu Thr Val Val Asp
           35                  40                  45
   His His Met His Val Val Gly Gln Asn Gln Cys Tyr Ser Val Val Ile
       50                  55                  60
   Gln Thr Ile Asn Ala Ser Val Ser Thr Val Trp Ser Val Val Arg Arg
   65                  70                  75                  80
   Phe Asp Tyr Pro Gln Gly Tyr Lys His Phe Val Lys Ser Cys Asn Val
                   85                  90                  95
   Val Ala Ser Gly Asp Gly Ile Arg Val Gly Ala Leu Arg Glu Val Arg
               100                 105                 110
   Leu Val Ser Gly Leu Pro Ala Val Ser Ser Thr Glu Arg Leu Asp Ile
           115                 120                 125
   Leu Asp Glu Glu Arg His Val Ile Ser Phe Ser Val Val Gly Gly Val
       130                 135                 140
   His Arg Cys Arg Asn Tyr Arg Ser Val Thr Thr Leu His Gly Asp Gly
   145                 150                 155                 160
   Asn Gly Gly Thr Val Val Ile Glu Ser Tyr Val Val Asp Val Pro Gln
                   165                 170                 175
   Gly Asn Thr Lys Glu Glu Thr Cys Ser Phe Ala Asp Thr Ile Val Arg
               180                 185                 190
   Cys Asn Leu Gln Ser Leu Val Gln Ile Ala Glu Lys Leu
           195                 200                 205
<210> 40
   <211> 212
   <212> PRT
   <213> Zea mays
<220>
   <223> maize AT-rich element binding factor 3, clone 1458362, GenBank Accession No. ACG26321.1
<400> 40
   Met Pro Phe Ala Ala Ser Arg Thr Ser Gln Gln Gln His Ser Arg Val
    1               5                  10                  15
   Ala Thr Asn Gly Arg Ala Val Ala Val Cys Ala Gly His Ala Gly Val
               20                  25                  30
   Pro Asp Glu Val Ala Arg His His Glu His Ala Val Ala Ala Gly Gln
           35                  40                  45
   Cys Cys Ala Ala Met Val Gln Ser Ile Ala Ala Pro Val Asp Ala Val
       50                  55                  60
   Trp Ser Leu Val Arg Arg Phe Asp Gln Pro Gln Arg Tyr Lys Arg Phe
   65                  70                  75                  80
   Ile Arg Ser Cys His Leu Val Asp Gly Asp Gly Ala Glu Val Gly Ser
                   85                  90                  95
   Val Arg Glu Leu Leu Leu Val Ser Gly Leu Pro Ala Glu Ser Ser Arg
               100                 105                 110
   Glu Arg Leu Glu Ile Arg Asp Asp Glu Arg Arg Val Ile Ser Phe Arg
           115                 120                 125
   Val Leu Gly Gly Asp His Arg Leu Ala Asn Tyr Arg Ser Val Thr Thr
       130                 135                 140
   Val His Glu Ala Ala Pro Ser Gln Asp Gly Arg Pro Leu Thr Met Val
   145                 150                 155                 160
   Val Glu Ser Tyr Val Val Asp Val Pro Pro Gly Asn Thr Val Glu Glu
                   165                 170                 175
   Thr Arg Ile Phe Val Asp Thr Ile Val Arg Cys Asn Leu Gln Ser Leu
               180                 185                 190
   Glu Gly Thr Val Ile Arg Gln Leu Glu Ile Ala Ala Met Pro His Asp
           195                 200                 205
   Asp Asn Gln Asn
       210
<210> 41
   <211> 233
   <212> PRT
   <213> Zea mays
<220>
   <223> maize strain B73 unknown protein, clone ZM_BFb0105O18, GenBank Accession No. ACF87013.1
<400> 41
   Met Arg Glu Arg Asn Ser Ser Ile Asp Gln Glu His Gln Arg Gly Ser
    1               5                  10                  15
   Ser Ser Arg Ser Thr Met Pro Phe Ala Ala Ser Arg Thr Ser Gln Gln
               20                  25                  30
   Gln His Ser Arg Val Ala Thr Asn Gly Arg Ala Val Ala Val Cys Ala
           35                  40                  45
   Gly His Ala Gly Val Pro Asp Glu Val Ala Arg His His Glu His Ala
       50                  55                  60
   Val Ala Ala Gly Gln Cys Cys Ala Ala Met Val Gln Ser Ile Ala Ala
   65                  70                  75                  80
   Pro Val Asp Ala Val Trp Ser Leu Val Arg Arg Phe Asp Gln Pro Gln
                   85                  90                  95
   Arg Tyr Lys Arg Phe Ile Arg Ser Cys His Leu Val Asp Gly Asp Gly
               100                 105                 110
   Ala Glu Val Gly Ser Val Arg Glu Leu Leu Leu Val Ser Gly Leu Pro
           115                 120                 125
   Ala Glu Ser Ser Arg Glu Arg Leu Glu Ile Arg Asp Asp Glu Arg Arg
       130                 135                 140
   Val Ile Ser Phe Arg Val Leu Gly Gly Asp His Arg Leu Ala Asn Tyr
   145                 150                 155                 160
   Arg Ser Val Thr Thr Val His Glu Ala Ala Pro Ser Gln Asp Gly Arg
                   165                 170                 175
   Pro Leu Thr Met Val Val Glu Ser Tyr Val Val Asp Val Pro Pro Gly
               180                 185                 190
   Asn Thr Val Glu Glu Thr Arg Ile Phe Val Asp Thr Ile Val Arg Cys
           195                 200                 205
   Asn Leu Gln Ser Leu Glu Gly Thr Val Ile Arg Gln Leu Glu Ile Ala
       210                 215                 220
   Ala Met Pro His Asp Asp Asn Gln Asn
   225                 230
<210> 42
   <211> 194
   <212> PRT
   <213> Physcomitrella patens
<220>
   <223> Physcomitrella patens subsp. patens moss, ecotype Gransden 2004, hypothetical protein, predicted protein, locus tag PHYPADRAFT_209242, GenBank Accession No. XP_001762113.1
<400> 42
   Met Met Gln Glu Lys Gln Gly Arg Pro Asp Phe Gln Phe Leu Leu Glu
    1               5                  10                  15
   Gly Gln Gln Asp Leu Ile Cys Arg Phe His Lys His Glu Leu Leu Pro
               20                  25                  30
   His Gln Cys Gly Ser Ile Leu Leu Gln Gln Ile Lys Ala Pro Val Gln
           35                  40                  45
   Thr Val Trp Leu Ile Val Arg Arg Phe Asp Glu Pro Gln Val Tyr Lys
       50                  55                  60
   Arg Phe Ile Gln Arg Cys Asp Ile Val Glu Gly Asp Gly Val Val Gly
   65                  70                  75                  80
   Ser Ile Arg Glu Val Gln Leu Val Ser Ser Ile Pro Ala Thr Ser Ser
                   85                  90                  95
   Ile Glu Arg Leu Glu Ile Leu Asp Asp Glu Glu His Ile Ile Ser Phe
               100                 105                 110
   Arg Val Leu Gly Gly Gly His Arg Leu Gln Asn Tyr Trp Ser Val Thr
           115                 120                 125
   Ser Leu His Arg His Glu Ile Gln Gly Gln Met Gly Thr Leu Val Leu
       130                 135                 140
   Glu Ser Tyr Val Val Asp Ile Pro Asp Gly Asn Thr Arg Glu Glu Thr
   145                 150                 155                 160
   His Thr Phe Val Asp Thr Val Val Arg Cys Asn Leu Lys Ala Leu Ala
                   165                 170                 175
   Gln Val Ser Glu Gln Lys His Leu Leu Asn Ser Asn Glu Lys Pro Ala
               180                 185                 190
   Ala Pro
<210> 43
   <211> 191
   <212> PRT
   <213> Vitis vinifera
<220>
   <223> wine grape cultivar PN40024 unnamed protein product, locus tag GSVIVT00035869001, GenBank Accession No. CAO48052.1
<400> 43
   Met Lys Val Tyr Ser Pro Ser Gln Ile Leu Ala Glu Arg Gly Pro Arg
    1               5                  10                  15
   Ala Gln Ala Met Gly Asn Leu Tyr His Thr His His Leu Leu Pro Asn
               20                  25                  30
   Gln Cys Ser Ser Leu Val Val Gln Thr Thr Asp Ala Pro Leu Pro Gln
           35                  40                  45
   Val Trp Ser Met Val Arg Arg Phe Asp Arg Pro Gln Ser Tyr Lys Arg
       50                  55                  60
   Phe Val Arg Gly Cys Thr Leu Arg Arg Gly Lys Gly Gly Val Gly Ser
   65                  70                  75                  80
   Val Arg Glu Val Asn Ile Val Ser Gly Leu Pro Ala Glu Ile Ser Leu
                   85                  90                  95
   Glu Arg Leu Asp Lys Leu Asp Asp Asp Leu His Val Met Arg Phe Thr
               100                 105                 110
   Val Ile Gly Gly Asp His Arg Leu Ala Asn Tyr His Ser Thr Leu Thr
           115                 120                 125
   Leu His Glu Asp Glu Glu Asp Gly Val Arg Lys Thr Val Val Met Glu
       130                 135                 140
   Ser Tyr Val Val Asp Val Pro Gly Gly Asn Ser Ala Gly Glu Thr Cys
   145                 150                 155                 160
   Tyr Phe Ala Asn Thr Ile Ile Gly Phe Asn Leu Lys Ala Leu Ala Ala
                   165                 170                 175
   Val Thr Glu Thr Met Ala Leu Lys Ala Asn Ile Pro Ser Gly Phe
               180                 185                 190
<210> 44
   <211> 217
   <212> PRT
   <213> Physcomitrella patens
<220>
   <223> Physcomitrella patens subsp. patens moss, ecotype Gransden 2004, hypothetical protein, predicted protein, locus tag PHYPADRAFT_132509, GenBank Accession No. XP_001767821.1
<400> 44
   Met Gln Gln Val Lys Gly Arg Gln Asp Phe Gln Arg Leu Leu Glu Ala
    1               5                  10                  15
   Gln Gln Asp Leu Ile Cys Arg Tyr His Thr His Glu Leu Lys Ala His
               20                  25                  30
   Gln Cys Gly Ser Ile Leu Leu Gln Gln Ile Lys Val Pro Leu Pro Ile
           35                  40                  45
   Val Trp Ala Ile Val Arg Ser Phe Asp Lys Pro Gln Val Tyr Lys Arg
       50                  55                  60
   Phe Ile Gln Thr Cys Lys Ile Thr Glu Gly Asp Gly Gly Val Gly Ser
   65                  70                  75                  80
   Ile Arg Glu Val His Leu Val Ser Ser Val Pro Ala Thr Cys Ser Ile
                   85                  90                  95
   Glu Arg Leu Glu Ile Leu Asp Asp Glu Lys His Ile Ile Ser Phe Arg
               100                 105                 110
   Val Leu Gly Gly Gly His Arg Leu Gln Asn Tyr Ser Ser Val Ser Ser
           115                 120                 125
   Leu His Glu Leu Glu Val Glu Gly His Pro Cys Thr Leu Val Leu Glu
       130                 135                 140
   Ser Tyr Met Val Asp Ile Pro Asp Gly Asn Thr Arg Glu Glu Thr His
   145                 150                 155                 160
   Met Phe Val Asp Thr Val Val Arg Cys Asn Leu Lys Ser Leu Ala Gln
                   165                 170                 175
   Ile Ser Glu Gln Gln Tyr Asn Lys Asp Cys Leu Gln Gln Lys Gln His
               180                 185                 190
   Asp Gln Gln Gln Met Tyr Gln Gln Arg His Pro Pro Leu Pro Pro Ile
           195                 200                 205
   Pro Ile Thr Asp Lys Asn Met Glu Arg
       210                 215
<210> 45
   <211> 195
   <212> PRT
   <213> Physcomitrella patens
<220>
   <223> Physcomitrella patens subsp. patens moss, ecotype Gransden 2004, hypothetical protein, predicted protein, locus tag PHYPADRAFT_213389, GenBank Accession No. XP_001767012.1
<400> 45
   Met Arg Phe Asp Ile Gly His Asn Asp Val Arg Gly Phe Phe Thr Cys
    1               5                  10                  15
   Glu Glu Glu His Ala Tyr Ala Leu His Ser Gln Thr Val Glu Leu Asn
               20                  25                  30
   Gln Cys Gly Ser Ile Leu Met Gln Gln Ile His Ala Pro Ile Glu Val
           35                  40                  45
   Val Trp Ser Ile Val Arg Ser Phe Gly Ser Pro Gln Ile Tyr Lys Lys
       50                  55                  60
   Phe Ile Gln Ala Cys Ile Leu Thr Val Gly Asp Gly Gly Val Gly Ser
   65                  70                  75                  80
   Ile Arg Glu Val Phe Leu Val Ser Gly Val Pro Ala Thr Ser Ser Ile
                       85                  90                  95
   Glu Arg Leu Glu Ile Leu Asp Asp Glu Lys His Val Phe Ser Phe Arg
                   100                 105                 110
   Val Leu Lys Gly Gly His Arg Leu Gln Asn Tyr Arg Ser Val Thr Thr
           115                 120                 125
   Leu His Glu Gln Glu Val Asn Gly Arg Gln Thr Thr Thr Val Leu Glu
       130                 135                 140
   Ser Tyr Val Val Asp Val Pro Asp Gly Asn Thr Arg Glu Glu Thr His
   145                 150                 155                 160
   Met Phe Ala Asp Thr Val Val Met Cys Asn Leu Lys Ser Leu Ala Gln
                   165                 170                 175
   Val Ala Glu Trp Arg Ala Met Gln Gly Ile Thr Gln Gln Leu Ser Thr
               180                 185                 190
   Ser Ser Leu
           195
<210> 46
   <211> 172
   <212> PRT
   <213> Vitis vinifera
<220>
   <223> wine grape cultivar Pinot Noir hypothetical protein, clone ENTAV 115, locus tag VITISV_004947, GenBank Accession No. CAN72620.1
<400> 46
   Met Gly Asn Leu Tyr His Thr His His Leu Leu Pro Asn Gln Cys Ser
    1               5                  10                  15
   Ser Leu Val Val Gln Thr Thr Asp Ala Pro Leu Pro Gln Val Trp Ser
               20                  25                  30
   Met Val Arg Arg Phe Asp Arg Pro Gln Ser Tyr Lys Arg Phe Val Arg
           35                  40                  45
   Gly Cys Thr Leu Arg Arg Gly Lys Gly Gly Val Gly Ser Val Arg Glu
       50                  55                  60
   Val Asn Ile Val Ser Gly Leu Pro Ala Glu Ile Ser Leu Glu Arg Leu
   65                  70                  75                  80
   Asp Lys Leu Asp Asp Asp Leu His Val Met Arg Phe Thr Val Ile Gly
                   85                  90                  95
   Gly Asp His Arg Leu Ala Asn Tyr His Ser Thr Leu Thr Leu His Glu
               100                 105                 110
   Asp Glu Glu Asp Gly Val Arg Lys Thr Val Val Met Glu Ser Tyr Val
           115                 120                 125
   Val Asp Val Pro Gly Gly Asn Ser Ala Gly Glu Thr Cys Tyr Phe Ala
       130                 135                 140
   Asn Thr Ile Ile Gly Phe Asn Leu Lys Ala Leu Ala Ala Val Thr Glu
   145                 150                 155                 160
   Thr Met Ala Leu Lys Ala Asn Ile Pro Ser Gly Phe
                   165                 170
<210> 47
   <211> 196
   <212> PRT
   <213> Picea sitchensis
<220>
   <223> Sitka spruce cultivar FB3-425, unknown protein, clone WS0281_I24, GenBank Accession No. ABK23752.1
<400> 47
   Met Glu Asp Leu Ser Ser Trp Arg Glu Gly Arg Ala Met Trp Leu Gly
    1               5                   10                  15
   Asn Pro Pro Ser Glu Ser Glu Leu Val Cys Arg His His Arg His Glu
               20                  25                  30
   Leu Gln Gly Asn Gln Cys Ser Ser Phe Leu Val Lys His Ile Arg Ala
           35                  40                  45
   Pro Val His Leu Val Trp Ser Ile Val Arg Thr Phe Asp Gln Pro Gln
       50                  55                  60
   Lys Tyr Lys Pro Phe Val His Ser Cys Ser Val Arg Gly Gly Ile Thr
   65                  70                  75                  80
   Val Gly Ser Ile Arg Asn Val Asn Val Lys Ser Gly Leu Pro Ala Thr
                   85                  90                  95
   Ala Ser Glu Glu Arg Leu Glu Ile Leu Asp Asp Asn Glu His Val Phe
               100                 105                 110
   Ser Ile Lys Ile Leu Gly Gly Asp His Arg Leu Gln Asn Tyr Ser Ser
           115                 120                 125
   Ile Ile Thr Val His Pro Glu Ile Ile Asp Gly Arg Pro Gly Thr Leu
       130                 135                 140
   Val Ile Glu Ser Tyr Val Val Asp Val Pro Glu Gly Asn Thr Arg Glu
   145                 150                 155                 160
   Glu Thr Arg Phe Phe Val Glu Ala Leu Val Lys Cys Asn Leu Lys Ser
                   165                 170                 175
   Leu Ala Asp Val Ser Glu Arg Leu Ala Ser Gln His His Thr Glu Leu
               180                 185                 190
   Leu Glu Arg Thr
           195
<210> 48
   <211> 185
   <212> PRT
   <213> Solanum tuberosum
<220>
   <223> potato cultivar Kuras, CAPIP1-like protein, clone 153D02, similar to Casicum annuum CAPIP1, GenBank Accession No. ABB29920.1
<400> 48
   Met Asn Ala Asn Gly Phe Cys Gly Val Glu Lys Glu Tyr Ile Arg Lys
   1               5                   10                  15
   His His Leu His Glu Pro Lys Glu Asn Gln Cys Ser Ser Phe Leu Val
               20                  25                  30
   Lys His Ile Arg Ala Pro Val His Leu Val Trp Ser Leu Val Arg Arg
           35                  40                  45
   Phe Asp Gln Pro Gln Lys Tyr Lys Pro Phe Ile Ser Arg Cys Ile Val
       50                  55                  60
   Gln Gly Asp Leu Glu Ile Gly Ser Leu Arg Glu Val Asp Val Lys Ser
   65                  70                  75                  80
   Gly Leu Pro Ala Thr Thr Ser Thr Glu Arg Leu Glu Leu Leu Asp Asp
                   85                  90                  95
   Glu Glu His Ile Leu Ser Val Arg Ile Val Gly Gly Asp His Arg Leu
               100                 105                 110
   Arg Asn Tyr Ser Ser Val Ile Ser Val His Pro Glu Val Ile Asp Gly
           115                 120                 125
   Arg Pro Gly Thr Val Val Leu Glu Ser Phe Val Val Asp Val Pro Glu
       130                 135                 140
   Gly Asn Thr Lys Asp Glu Thr Cys Tyr Phe Val Glu Ala Leu Ile Asn
   145                 150                 155                 160
   Cys Asn Leu Lys Ser Leu Ala Asp Ile Ser Glu Arg Val Ala Val Gln
                   165                 170                 175
   Asp Arg Thr Glu Pro Ile Asp Gln Val
               180                 185
<210> 49
   <211> 190
   <212> PRT
   <213> Medicago truncatula
<220>
   <223> barrel medic unknown protein, clone MTYFP_FQ_FR_FS1G-E-17, GenBank Accession No. ACJ85952.1
<400> 49
   Met Asn Asn Gly Cys Glu Gln Gln Gln Tyr Ser Val Ile Glu Thr Gln
   1               5                   10                  15
   Tyr Ile Arg Arg His His Lys His Asp Leu Arg Asp Asn Gln Cys Ser
               20                  25                  30
   Ser Ala Leu Val Lys His Ile Lys Ala Pro Val His Leu Val Trp Ser
           35                  40                  45
   Leu Val Arg Arg Phe Asp Gln Pro Gln Lys Tyr Lys Pro Phe Ile Ser
       50                  55                  60
   Arg Cys Ile Met Gln Gly Asp Leu Ser Ile Gly Ser Val Arg Glu Val
   65                  70                  75                  80
   Asn Val Lys Ser Gly Leu Pro Ala Thr Thr Ser Thr Glu Arg Leu Glu
                   85                  90                  95
   Gln Leu Asp Asp Glu Glu His Ile Leu Gly Ile Arg Ile Val Gly Gly
               100                 105                 110
   Asp His Arg Leu Arg Asn Tyr Ser Ser Ile Ile Thr Val His Pro Gly
           115                 120                 125
   Val Ile Asp Gly Arg Pro Gly Thr Met Val Ile Glu Ser Phe Val Val
       130                 135                 140
   Asp Val Pro Glu Gly Asn Thr Lys Asp Glu Thr Cys Tyr Phe Val Glu
   145                 150                 155                 160
   Ala Leu Ile Arg Tyr Asn Leu Ser Ser Leu Ala Asp Val Ser Glu Arg
                   165                 170                 175
   Met Ala Val Gln Gly Arg Thr Asp Pro Ile Asn Ile Asn Pro
               180                 185                 190
<210> 50
   <211> 185
   <212> PRT
   <213> Vitis vinifera
<220>
   <223> wine grape cultivar PN40024 unnamed protein product, locus tag GSVIVT00002440001, GenBank Accession No. CA065816.1
<400> 50
   Met Ser Gly Tyr Gly Cys Ile Lys Met Glu Asp Glu Tyr Ile Arg Arg
    1               5                  10                  15
   His His Arg His Glu Ile Arg Asp Asn Gln Cys Ser Ser Ser Leu Val
               20                  25                  30
   Lys His Ile Lys Ala Pro Val His Leu Val Trp Ser Leu Val Arg Ser
           35                  40                  45
   Phe Asp Gln Pro Gln Lys Tyr Lys Pro Phe Val Ser Arg Cys Ile Val
       50                  55                  60
   Gln Gly Asp Leu Glu Ile Gly Ser Val Arg Glu Val Asn Val Lys Ser
   65                  70                  75                  80
   Gly Leu Pro Ala Thr Thr Ser Thr Glu Arg Leu Glu Leu Leu Asp Asp
                   85                  90                  95
   Glu Glu His Ile Phe Gly Met Arg Ile Val Gly Gly Asp His Arg Leu
               100                 105                 110
   Lys Asn Tyr Ser Ser Ile Val Thr Val His Pro Glu Ile Ile Asp Gly
           115                 120                 125
   Arg Pro Gly Thr Leu Val Ile Glu Ser Phe Val Val Asp Val Pro Asp
       130                 135                 140
   Gly Asn Thr Lys Asp Glu Thr Cys Tyr Phe Val Glu Ala Leu Ile Lys
   145                 150                 155                 160
   Cys Asn Leu Lys Ser Leu Ala Asp Val Ser Glu Arg Leu Ala Ile Gln
                   165                 170                 175
   Asp Arg Thr Glu Pro Ile Asp Arg Met
               180                 185
<210> 51
   <211> 185
   <212> PRT
   <213> Vitis vinifera
<220>
   <223> wine grape cultivar PN40024 unnamed protein product, locus tag GSVIVT00006507001, GenBank Accession No. CAO69376.1
<400> 51
   Met Asn Gly Asn Gly Leu Ser Ser Met Glu Ser Glu Tyr Ile Arg Arg
    1               5                  10                  15
   His His Arg His Glu Pro Ala Glu Asn Gln Cys Ser Ser Ala Leu Val
               20                  25                  30
   Lys His Ile Lys Ala Pro Val Pro Leu Val Trp Ser Leu Val Arg Arg
           35                  40                  45
   Phe Asp Gln Pro Gln Lys Tyr Lys Pro Phe Ile Ser Arg Cys Val Val
       50                  55                  60
   Gln Gly Asn Leu Glu Ile Gly Ser Leu Arg Glu Val Asp Val Lys Ser
   65                  70                  75                  80
   Gly Leu Pro Ala Thr Thr Ser Thr Glu Arg Leu Glu Leu Leu Asp Asp
                   85                  90                  95
   Asp Glu His Ile Leu Ser Met Arg Ile Ile Gly Gly Asp His Arg Leu
               100                 105                 110
   Arg Asn Tyr Ser Ser Ile Ile Ser Leu His Pro Glu Ile Ile Asp Gly
           115                 120                 125
   Arg Pro Gly Thr Met Val Ile Glu Ser Tyr Val Val Asp Val Pro Glu
       130                 135                 140
   Gly Asn Thr Lys Asp Glu Thr Cys Tyr Phe Val Glu Ala Leu Ile Lys
   145                 150                 155                 160
   Cys Asn Leu Lys Ser Leu Ala Asp Val Ser Glu Arg Leu Ala Val Gln
                   165                 170                 175
   Asp Arg Thr Glu Pro Ile Asp Arg Met
               180                 185
<210> 52
   <211> 208
   <212> PRT
   <213> Oryza sativa
<220>
   <223> rice Japonica Group, cultivar Nipponbare, hypothetical protein OsJ_21703, GenBank Accession No. EAZ37364.1
<400> 52
   Met Glu Ala His Val Glu Arg Ala Leu Arg Glu Gly Leu Thr Glu Glu
    1               5                  10                  15
   Glu Arg Ala Ala Leu Glu Pro Ala Val Met Ala His His Thr Phe Pro
               20                  25                  30
   Pro Ser Thr Thr Thr Ala Thr Thr Ala Ala Ala Thr Cys Thr Ser Leu
           35                  40                  45
   Val Thr Gln Arg Val Ala Ala Pro Val Arg Ala Val Trp Pro Ile Val
       50                  55                  60
   Arg Ser Phe Gly Asn Pro Gln Arg Tyr Lys His Phe Val Arg Thr Cys
   65                  70                  75                  80
   Ala Leu Ala Ala Gly Asn Gly Pro Ser Phe Gly Ser Val Arg Glu Val
                   85                  90                  95
   Thr Val Val Ser Gly Pro Ser Arg Leu Pro Pro Gly Thr Glu Arg Leu
               100                 105                 110
   Glu Met Leu Asp Asp Asp Arg His Ile Ile Ser Phe Arg Val Val Gly
               115                 120                 125
   Gly Gln His Arg Leu Arg Asn Tyr Arg Ser Val Thr Ser Val Thr Glu
       130                 135                 140
   Phe Gln Pro Pro Ala Ala Gly Pro Gly Pro Ala Pro Pro Tyr Cys Val
   145                 150                 155                 160
   Val Val Glu Ser Tyr Val Val Asp Val Pro Asp Gly Asn Thr Ala Glu
                   165                 170                 175
   Asp Thr Arg Met Phe Thr Asp Thr Val Val Lys Leu Asn Leu Gln Met
               180                 185                 190
   Leu Ala Ala Val Ala Glu Asp Ser Ser Ser Ala Ser Arg Arg Arg Asp
           195                 200                 205
<210> 53
   <211> 186
   <212> PRT
   <213> Capsicum annuum
<220>
   <223> pepper cultivar hanbyul, CAPIP1 protein, GenBank Accession No. AAT35532.1
<400> 53
   Met Met Asn Ala Asn Gly Phe Ser Gly Val Glu Lys Glu Tyr Ile Arg
    1               5                  10                  15
   Lys His His Leu His Gln Pro Lys Glu Asn Gln Cys Ser Ser Phe Leu
               20                  25                  30
   Val Lys His Ile Arg Ala Pro Val His Leu Val Trp Ser Leu Val Arg
           35                  40                  45
   Arg Phe Asp Gln Pro Gln Lys Tyr Lys Pro Phe Val Ser Arg Cys Ile
       50                  55                  60
   Ala Gln Gly Asp Leu Glu Ile Gly Ser Leu Arg Glu Val Asp Val Lys
   65                  70                  75                  80
   Ser Gly Leu Pro Ala Thr Thr Ser Thr Glu Arg Leu Glu Leu Leu Asp
                   85                  90                  95
   Asp Glu Glu His Ile Leu Ser Phe Arg Ile Ile Gly Gly Asp His Arg
               100                 105                 110
   Leu Arg Asn Tyr Ser Ser Ile Ile Ser Leu His Pro Glu Val Ile Asp
           115                 120                 125
   Gly Arg Pro Gly Thr Leu Val Ile Glu Ser Phe Val Val Asp Val Pro
       130                 135                 140
   Gln Gly Asn Thr Lys Asp Glu Thr Cys Tyr Phe Val Glu Ala Leu Ile
   145                 150                 155                 160
   Asn Cys Asn Leu Lys Ser Leu Ala Asp Val Ser Glu Arg Leu Ala Val
                   165                 170                 175
   Gln Asp Arg Thr Glu Pro Ile Asp Gln Val
               180                 185
<210> 54
   <211> 186
   <212> PRT
   <213> Populus trichocarpa
<220>
   <223> California poplar (Western balsam poplar, black cottonwood) cultivar 383-2499 (Nisqually-1), unknown protein, clone PX0011_1113, GenBank Accession No. ABK92491.1
<400> 54
   Met Asn Gly Ser Asp Ala Tyr Ser Ala Thr Glu Ala Gln Tyr Val Arg
    1               5                  10                  15
   Arg His His Lys His Glu Pro Arg Glu Asn Gln Cys Thr Ser Ala Leu
               20                  25                  30
   Val Lys His Ile Lys Ala Pro Ala His Leu Val Trp Ser Leu Val Arg
           35                  40                  45
   Arg Phe Asp Gln Pro Gln Arg Tyr Lys Pro Phe Val Ser Arg Cys Val
       50                  55                  60
   Met Asn Gly Glu Leu Gly Ile Gly Ser Val Arg Glu Val Asn Val Lys
   65                  70                  75                  80
   Ser Gly Leu Pro Ala Thr Thr Ser Thr Glu Arg Leu Glu Leu Leu Asp
                   85                  90                  95
   Asp Glu Glu His Ile Leu Gly Val Gln Ile Val Gly Gly Asp His Arg
               100                 105                 110
   Leu Lys Asn Tyr Ser Ser Ile Met Thr Val His Pro Glu Phe Ile Asp
           115                 120                 125
   Gly Arg Pro Gly Thr Leu Val Ile Glu Ser Phe Ile Val Asp Val Pro
       130                 135                 140
   Asp Gly Asn Thr Lys Asp Glu Thr Cys Tyr Phe Val Glu Ala Leu Ile
   145                 150                 155                 160
   Arg Cys Asn Leu Lys Ser Leu Ala Asp Val Ser Glu Arg Met Ala Val
                   165                 170                 175
   Gln Asp Arg Val Glu Pro Val Asn Gln Phe
               180                 185
<210> 55
   <211> 185
   <212> PRT
   <213> Capsicum annuum
<220>
   <223> pepper cultivar hanbyul, PIP1 protein, GenBank Accession No. ABF72432.1
<400> 55
   Met Asn Ala Asn Gly Phe Ser Gly Val Glu Lys Glu Tyr Ile Arg Lys
    1               5                  10                  15
   His His Leu His Gln Pro Lys Glu Asn Gln Cys Ser Ser Phe Leu Val
               20                  25                  30
   Lys His Ile Arg Ala Pro Val His Leu Val Trp Ser Leu Val Arg Arg
           35                  40                  45
   Phe Asp Gln Pro Gln Lys Tyr Lys Pro Phe Val Ser Arg Cys Ile Ala
       50                  55                  60
   Gln Gly Asp Leu Glu Ile Gly Ser Leu Arg Glu Val Asp Val Lys Ser
   65                  70                  75                  80
   Gly Leu Pro Ala Thr Thr Ser Thr Glu Arg Leu Glu Leu Leu Asp Asp
                   85                  90                  95
   Glu Glu His Ile Leu Ser Phe Arg Ile Ile Gly Gly Asp His Arg Leu
               100                 105                 110
   Arg Asn Tyr Ser Ser Ile Ile Ser Leu His Pro Glu Val Ile Asp Gly
           115                 120                 125
   Arg Pro Gly Thr Leu Val Ile Glu Ser Phe Val Val Asp Val Pro Gln
       130                 135                 140
   Gly Asn Thr Lys Asp Glu Thr Cys Tyr Phe Val Glu Ala Leu Ile Asn
   145                 150                 155                 160
   Cys Asn Leu Lys Ser Leu Ala Asp Val Ser Glu Arg Leu Ala Val Gln
                   165                 170                 175
   Asp Arg Thr Glu Pro Ile Asp Gln Val
               180                 185
<210> 56
   <211> 186
   <212> PRT
   <213> Populus trichocarpa x Populus deltoides
<220>
   <223> California poplar (Western balsam poplar, black cottonwood) x Eastern cottonwood, cultivar H11-11, unknown protein, clone WS0133_I04, GenBank Accession No. ABK96505.1
<400> 56
   Met Asn Gly Ser Asp Ala Tyr Ser Ala Thr Glu Ala Gln Tyr Val Arg
    1              5                   10                  15
   Arg His His Lys His Glu Pro Arg Glu Asn Gln Cys Thr Ser Ala Leu
               20                  25                  30
   Val Lys His Ile Lys Ala Pro Ala His Leu Val Trp Ser Leu Val Arg
           35                  40                  45
   Arg Phe Asp Gln Pro Gln Arg Tyr Lys Pro Phe Val Ser Arg Cys Val
       50                  55                  60
   Met Asn Gly Glu Leu Gly Ile Gly Ser Val Arg Glu Val Asn Val Lys
   65                  70                  75                  80
   Ser Gly Leu Pro Ala Thr Thr Ser Thr Glu Arg Leu Glu Leu Leu Asp
                   85                  90                  95
   Asp Glu Glu His Ile Leu Gly Val Gln Ile Val Gly Gly Asp His Arg
               100                 105                 110
   Leu Lys Asn Tyr Ser Ser Ile Met Thr Val His Pro Glu Phe Ile Asp
           115                 120                 125
   Gly Arg Pro Gly Thr Leu Val Ile Glu Ser Phe Ile Val Asp Val Pro
       130                 135                 140
   Asp Gly Asn Thr Lys Asp Glu Thr Cys Tyr Phe Val Lys Ala Leu Ile
   145                 150                 155                 160
   Arg Cys Asn Leu Lys Ser Leu Ala Asp Val Ser Glu Arg Met Ala Val
                   165                 170                 175
   Gln Asp Arg Val Glu Pro Val Asn Gln Phe
               180                 185
<210> 57
   <211> 188
   <212> PRT
   <213> Pisum sativum
<220>
   <223> pea AT-rich element binding factor 3 (PsATF, ATF3), potential transcription factor, GenBank Accession No. AAV85853.1
<400> 57
   Met Asn Asn Gly Gly Glu Gln Tyr Ser Ala Ile Glu Thr Gln Tyr Ile
   1               5                   10                  15
   Arg Arg Arg His Lys His Asp Leu Arg Asp Asn Gln Cys Ser Ser Ala
               20                  25                  30
   Leu Val Lys His Ile Lys Ala Pro Val His Leu Val Trp Ser Leu Val
           35                  40                  45
   Arg Arg Phe Asp Gln Pro Gln Lys Tyr Lys Pro Phe Val Ser Arg Cys
       50                  55                  60
   Ile Met Gln Gly Asp Leu Gly Ile Gly Ser Val Arg Glu Val Asn Val
   65                  70                  75                  80
   Lys Ser Gly Leu Pro Ala Thr Thr Ser Thr Glu Arg Leu Glu Gln Leu
                   85                  90                  95
   Asp Asp Glu Glu His Ile Leu Gly Ile Arg Ile Val Gly Gly Asp His
               100                 105                 110
   Arg Leu Arg Asn Tyr Ser Ser Val Ile Thr Val His Pro Glu Val Ile
           115                 120                 125
   Asp Gly Arg Pro Gly Thr Met Val Ile Glu Ser Phe Val Val Asp Val
       130                 135                 140
   Pro Glu Gly Asn Thr Arg Asp Glu Thr Cys Tyr Phe Val Glu Ala Leu
   145                 150                 155                 160
   Ile Arg Gly Asn Leu Ser Ser Leu Ala Asp Val Ser Glu Arg Met Ala
                   165                 170                 175
   Val Gln Gly Arg Thr Asp Pro Ile Asn Val Asn Pro
               180                 185
<210> 58
   <211> 177
   <212> PRT
   <213> Vitis vinifera
<220>
   <223> wine grape cultivar PN40024 unnamed protein product, locus tag GSVIVT00027009001, GenBank Accession No. CAO39744.1
<400> 58
   Met Glu Ala Gln Val Ile Cys Arg His His Ala His Glu Pro Arg Glu
   1               5                   10                  15
   Asn Gln Cys Ser Ser Val Leu Val Arg His Val Lys Ala Pro Ala Asn
               20                  25                  30
   Leu Val Trp Ser Leu Val Arg Arg Phe Asp Gln Pro Gln Lys Tyr Lys
           35                  40                  45
   Pro Phe Val Ser Arg Cys Val Val Gln Gly Asp Leu Arg Ile Gly Ser
       50                  55                  60
   Val Arg Glu Val Asn Val Lys Thr Gly Leu Pro Ala Thr Thr Ser Thr
   65                  70                  75                  80
   Glu Arg Leu Glu Leu Phe Asp Asp Asp Glu His Val Leu Gly Ile Lys
                   85                  90                  95
   Ile Leu Asp Gly Asp His Arg Leu Arg Asn Tyr Ser Ser Val Ile Thr
               100                 105                 110
   Val His Pro Glu Ile Ile Asp Gly Arg Pro Gly Thr Leu Val Ile Glu
           115                 120                 125
   Ser Phe Val Val Asp Val Pro Glu Gly Asn Thr Lys Asp Asp Thr Cys
       130                 135                 140
   Tyr Phe Val Arg Ala Leu Ile Asn Cys Asn Leu Lys Cys Leu Ala Glu
   145                 150                 155                 160
   Val Ser Glu Arg Met Ala Met Leu Gly Arg Val Glu Pro Ala Asn Ala
                   165                 170                 175
   Val
<210> 59
   <211> 178
   <212> PRT
   <213> Vitis vinifera
<220>
   <223> wine grape cultivar Pinot Noir hypothetical protein, clone ENTAV 115, locus tag VITISV_004915, GenBank Accession No. CAN82501.1
<400> 59
   Met Met Glu Ala Gln Val Ile Cys Arg His His Ala His Glu Pro Arg
    1               5                  10                  15
   Glu Asn Gln Cys Ser Ser Val Leu Val Arg His Val Lys Ala Pro Ala
               20                  25                  30
   Asn Leu Val Trp Ser Leu Val Arg Arg Phe Asp Gln Pro Gln Lys Tyr
           35                  40                  45
   Lys Pro Phe Val Ser Arg Cys Val Val Gln Gly Asp Leu Arg Ile Gly
       50                  55                  60
   Ser Val Arg Glu Val Asn Val Lys Thr Gly Leu Pro Ala Thr Thr Ser
   65                  70                  75                  80
   Thr Glu Arg Leu Glu Leu Phe Asp Asp Asp Glu His Val Leu Gly Ile
                   85                  90                  95
   Lys Ile Leu Asp Gly Asp His Arg Leu Arg Asn Tyr Ser Ser Val Ile
               100                 105                 110
   Thr Val His Pro Glu Ile Ile Asp Gly Arg Pro Gly Thr Leu Val Ile
           115                 120                 125
   Glu Ser Phe Val Val Asp Val Pro Glu Gly Asn Thr Lys Asp Asp Thr
       130                 135                 140
   Cys Tyr Phe Val Arg Ala Leu Ile Asn Cys Asn Leu Lys Cys Leu Ala
   145                 150                 155                 160
   Glu Val Ser Glu Arg Met Ala Met Leu Gly Arg Val Glu Pro Ala Asn
                   165                 170                 175
   Ala Val
<210> 60
   <211> 193
   <212> PRT
   <213> Arachis hypogaea
<220>
   <223> peanut pathogenesis-induced protein (PIP), GenBank Accession No. ACG76109.1
<220>
   <221> VARIANT
   <222> (162)...(162)
   <223> Xaa = any amino acid
<400> 60
   Met Met Asn Gly Ser Cys Gly Gly Gly Gly Gly Gly Glu Ala Tyr Gly
    1               5                  10                  15
   Ala Ile Glu Ala Gln Tyr Ile Arg Arg His His Arg His Glu Pro Arg
               20                  25                  30
   Asp Asn Gln Cys Thr Ser Ala Leu Val Lys His Ile Arg Ala Pro Val
           35                  40                  45
   His Leu Val Trp Ser Leu Val Arg Arg Phe Asp Gln Pro Gln Lys Tyr
       50                  55                  60
   Lys Pro Phe Val Ser Arg Cys Ile Met Gln Gly Asp Leu Gly Ile Gly
   65                  70                  75                  80
   Ser Val Arg Glu Val Asn Val Lys Ser Gly Leu Pro Ala Thr Thr Ser
                   85                  90                  95
   Thr Glu Arg Leu Glu Gln Leu Asp Asp Glu Glu His Ile Leu Gly Ile
               100                 105                 110
   Arg Ile Val Gly Gly Asp His Arg Leu Arg Asn Tyr Ser Ser Ile Ile
           115                 120                 125
   Thr Val His Pro Glu Val Ile Glu Gly Arg Pro Gly Thr Met Val Ile
       130                 135                 140
   Glu Ser Phe Val Val Asp Val Pro Asp Gly Asn Thr Lys Asp Glu Thr
   145                 150                 155                 160
   Cys Xaa Phe Val Glu Ala Leu Ile Arg Cys Asn Leu Ser Ser Leu Ala
                   165                 170                 175
   Asp Val Ser Glu Arg Met Ala Val Gln Gly Arg Thr Asp Pro Ile Asn
               180                 185                 190
   Gln
<210> 61
   <211> 217
   <212> PRT
   <213> Zea mays
<220>
   <223> maize AT-rich element binding factor 3, clone 300908, GenBank Accession No. ACG39386.1
<400> 61
   Met Val Val Glu Met Asp Gly Gly Val Gly Val Ala Ala Gly Gly Gly
    1               5                  10                  15
   Gly Gly Ala Gln Thr Pro Ala Pro Ala Pro Pro Arg Arg Trp Arg Leu
               20                  25                  30
   Ala Asp Glu Arg Cys Asp Leu Arg Ala Met Glu Thr Asp Tyr Val Arg
           35                  40                  45
   Arg Phe His Arg His Glu Pro Arg Asp His Gln Cys Ser Ser Ala Val
       50                  55                  60
   Ala Lys His Ile Lys Ala Pro Val His Leu Val Trp Ser Leu Val Arg
   65                  70                  75                  80
   Arg Phe Asp Gln Pro Gln Leu Phe Lys Pro Phe Val Ser Arg Cys Glu
                   85                  90                  95
   Met Lys Gly Asn Ile Glu Ile Gly Ser Val Arg Glu Val Asn Val Lys
               100                 105                 110
   Ser Gly Leu Pro Ala Thr Arg Ser Thr Glu Arg Leu Glu Leu Leu Asp
           115                 120                 125
   Asp Asp Glu Arg Ile Leu Ser Val Arg Phe Val Gly Gly Asp His Arg
       130                 135                 140
   Leu Gln Asn Tyr Ser Ser Ile Leu Thr Val His Pro Glu Val Ile Asp
   145                 150                 155                 160
   Gly Arg Pro Gly Thr Leu Val Ile Glu Ser Phe Val Val Asp Val Pro
                   165                 170                 175
   Asp Gly Asn Thr Lys Asp Glu Thr Cys Tyr Phe Val Glu Ala Leu Leu
               180                 185                 190
   Lys Cys Asn Leu Arg Ser Leu Ala Glu Val Ser Glu Gly Gln Val Ile
           195                 200                 205
   Met Asp Gln Thr Glu Pro Leu Asp Arg
       210                 215
<210> 62
   <211> 217
   <212> PRT
   <213> Zea mays
<220>
   <223> maize strain B73, unknown protein, clone ZM_BFb0036A01, GenBank Accession No. ACF80077.1
<400> 62
   Met Val Val Glu Met Asp Gly Gly Val Gly Val Ala Ala Ala Gly Gly
    1               5                  10                  15
   Gly Gly Ala Gln Thr Pro Ala Pro Pro Pro Pro Arg Arg Trp Arg Leu
               20                  25                  30
   Ala Asp Glu Arg Cys Asp Leu Arg Ala Met Glu Thr Asp Tyr Val Arg
           35                  40                  45
   Arg Phe His Arg His Glu Pro Arg Asp His Gln Cys Ser Ser Ala Val
       50                  55                  60
   Ala Lys His Ile Lys Ala Pro Val His Leu Val Trp Ser Leu Val Arg
   65                  70                  75                  80
   Arg Phe Asp Gln Pro Gln Leu Phe Lys Pro Phe Val Ser Arg Cys Glu
                   85                  90                  95
   Met Lys Gly Asn Ile Glu Ile Gly Ser Val Arg Glu Val Asn Val Lys
               100                 105                 110
   Ser Gly Leu Pro Ala Thr Arg Ser Thr Glu Arg Leu Glu Leu Leu Asp
           115                 120                 125
   Asp Asp Glu Arg Ile Leu Ser Val Arg Phe Val Gly Gly Asp His Arg
       130                 135                 140
   Leu Gln Asn Tyr Ser Ser Ile Leu Thr Val His Pro Glu Val Ile Asp
   145                 150                 155                 160
   Gly Arg Pro Gly Thr Leu Val Ile Glu Ser Phe Val Val Asp Val Pro
                   165                 170                 175
   Asp Gly Asn Thr Lys Asp Glu Thr Cys Tyr Phe Val Glu Ala Leu Leu
               180                 185                 190
   Lys Cys Asn Leu Arg Ser Leu Ala Glu Val Ser Glu Gly Gln Val Ile
           195                 200                 205
   Met Asp Gln Thr Glu Pro Leu Asp Arg
       210                 215
<210> 63
   <211> 206
   <212> PRT
   <213> Oryza sativa
<220>
   <223> rice Japonica Group, cultivar Nipponbare, hypothetical protein Os06g0528300, GenBank Accession No. NP_001057772.1
<400> 63
   Met Asn Gly Val Gly Gly Ala Gly Gly Ala Ala Ala Gly Lys Leu Pro
    1               5                  10                  15
   Met Val Ser His Arg Arg Val Gln Trp Arg Leu Ala Asp Glu Arg Cys
               20                  25                  30
   Glu Leu Arg Glu Glu Glu Met Glu Tyr Ile Arg Arg Phe His Arg His
           35                  40                  45
   Glu Pro Ser Ser Asn Gln Cys Thr Ser Phe Ala Ala Lys His Ile Lys
       50                  55                  60
   Ala Pro Leu His Thr Val Trp Ser Leu Val Arg Arg Phe Asp Gln Pro
   65                  70                  75                  80
   Gln Leu Phe Lys Pro Phe Val Arg Asn Cys Val Met Arg Glu Asn Ile
                   85                  90                  95
   Ile Ala Thr Gly Cys Ile Arg Glu Val Asn Val Gln Ser Gly Leu Pro
               100                 105                 110
   Ala Thr Arg Ser Thr Glu Arg Leu Glu Leu Leu Asp Asp Asn Glu His
           115                 120                 125
   Ile Leu Lys Val Asn Phe Ile Gly Gly Asp His Met Leu Lys Asn Tyr
       130                 135                 140
   Ser Ser Ile Leu Thr Val His Ser Glu Val Ile Asp Gly Gln Leu Gly
   145                 150                 155                 160
   Thr Leu Val Val Glu Ser Phe Ile Val Asp Val Pro Glu Gly Asn Thr
                   165                 170                 175
   Lys Asp Asp Ile Ser Tyr Phe Ile Glu Asn Val Leu Arg Cys Asn Leu
               180                 185                 190
   Arg Thr Leu Ala Asp Val Ser Glu Glu Arg Leu Ala Asn Pro
           195                 200                 205
<210> 64
   <211> 206
   <212> PRT
   <213> Oryza sativa
<220>
   <223> rice Indica Group, cultivar 93-11, hypothetical protein OsI_23215, GenBank Accession No. EAZ01188.1
<400> 64
   Met Asn Gly Ala Gly Gly Ala Gly Gly Ala Ala Ala Gly Lys Leu Pro
    1               5                  10                  15
   Met Val Ser His Arg Gln Val Gln Trp Arg Leu Ala Asp Glu Arg Cys
               20                  25                  30
   Glu Leu Arg Glu Glu Glu Met Glu Tyr Ile Arg Gln Phe His Arg His
           35                  40                  45
   Glu Pro Ser Ser Asn Gln Cys Thr Ser Phe Val Ala Lys His Ile Lys
       50                  55                  60
   Ala Pro Leu Gln Thr Val Trp Ser Leu Val Arg Arg Phe Asp Gln Pro
   65                  70                  75                  80
   Gln Leu Phe Lys Pro Phe Val Arg Lys Cys Val Met Arg Glu Asn Ile
                   85                  90                  95
   Ile Ala Thr Gly Cys Val Arg Glu Val Asn Val Gln Ser Gly Leu Pro
               100                 105                 110
   Ala Thr Arg Ser Thr Glu Arg Leu Glu Leu Leu Asp Asp Asn Glu His
           115                 120                 125
   Ile Leu Lys Val Lys Phe Ile Gly Gly Asp His Met Leu Lys Asn Tyr
       130                 135                 140
   Ser Ser Ile Leu Thr Ile His Ser Glu Val Ile Asp Gly Gln Leu Gly
   145                 150                 155                 160
   Thr Leu Val Val Glu Ser Phe Val Val Asp Ile Pro Glu Gly Asn Thr
                   165                 170                 175
   Lys Asp Asp Ile Cys Tyr Phe Ile Glu Asn Ile Leu Arg Cys Asn Leu
               180                 185                 190
   Met Thr Leu Ala Asp Val Ser Glu Glu Arg Leu Ala Asn Pro
           195                 200                 205
<210> 65
   <211> 205
   <212> PRT
   <213> Oryza sativa
<220>
   <223> rice Japonica Group, cultivar Nipponbare, hypothetical protein OsJ_06125, GenBank Accession No. EAZ22456.1
<400> 65
   Met Val Glu Val Gly Gly Gly Ala Ala Glu Ala Ala Ala Gly Arg Arg
    1               5                  10                  15
   Trp Arg Leu Ala Asp Glu Arg Cys Asp Leu Arg Ala Ala Glu Thr Glu
               20                  25                  30
   Tyr Val Arg Arg Phe His Arg His Glu Pro Arg Asp His Gln Cys Ser
           35                  40                  45
   Ser Ala Val Ala Lys His Ile Lys Ala Pro Val His Leu Val Trp Ser
       50                  55                  60
   Leu Val Arg Arg Phe Asp Gln Pro Gln Leu Phe Lys Pro Phe Val Ser
   65                  70                  75                  80
   Arg Cys Glu Met Lys Gly Asn Ile Glu Ile Gly Ser Val Arg Glu Val
                   85                  90                  95
   Asn Val Lys Ser Gly Leu Pro Ala Thr Arg Ser Thr Glu Arg Leu Glu
               100                 105                 110
   Leu Leu Asp Asp Asn Glu His Ile Leu Ser Val Arg Phe Val Gly Gly
           115                 120                 125
   Asp His Arg Leu Lys Asn Tyr Ser Ser Ile Leu Thr Val His Pro Glu
       130                 135                 140
   Val Ile Asp Gly Arg Pro Gly Thr Leu Val Ile Glu Ser Phe Val Val
   145                 150                 155                 160
   Asp Val Pro Glu Gly Asn Thr Lys Asp Glu Thr Cys Tyr Phe Val Glu
                   165                 170                 175
   Ala Leu Leu Lys Cys Asn Leu Lys Ser Leu Ala Glu Val Ser Glu Arg
               180                 185                 190
   Leu Val Cys Gln Gly Pro Asn Arg Ala Pro Ser Thr Arg
           195                 200                 205
<210> 66
   <211> 204
   <212> PRT
   <213> Oryza sativa
<220>
   <223> rice Japonica Group, cultivar Nipponbare, hypothetical protein Os02g0255500, similar to extensin (fragment), GenBank Accession No. NP_001046464.1
<400> 66
   Met Val Glu Val Gly Gly Gly Ala Ala Glu Ala Ala Ala Gly Arg Arg
    1               5                   10                  15
   Trp Arg Leu Ala Asp Glu Arg Cys Asp Leu Arg Ala Ala Glu Thr Glu
               20                  25                  30
   Tyr Val Arg Arg Phe His Arg His Glu Pro Arg Asp His Gln Cys Ser
           35                  40                  45
   Ser Ala Val Ala Lys His Ile Lys Ala Pro Val His Leu Val Trp Ser
       50                  55                  60
   Leu Val Arg Arg Phe Asp Gln Pro Gln Leu Phe Lys Pro Phe Val Ser
   65                  70                  75                  80
   Arg Cys Glu Met Lys Gly Asn Ile Glu Ile Gly Ser Val Arg Glu Val
                   85                  90                  95
   Asn Val Lys Ser Gly Leu Pro Ala Thr Arg Ser Thr Glu Arg Leu Glu
               100                 105                 110
   Leu Leu Asp Asp Asn Glu His Ile Leu Ser Val Arg Phe Val Gly Gly
           115                 120                 125
   Asp His Arg Leu Lys Asn Tyr Ser Ser Ile Leu Thr Val His Pro Glu
       130                 135                 140
   Val Ile Asp Gly Arg Pro Gly Thr Leu Val Ile Glu Ser Phe Val Val
   145                 150                 155                 160
   Asp Val Pro Glu Gly Asn Thr Lys Asp Glu Thr Cys Tyr Phe Val Glu
                   165                 170                 175
   Ala Leu Leu Lys Cys Asn Leu Lys Ser Leu Ala Glu Val Ser Glu Arg
               180                 185                 190
   Leu Val Val Lys Asp Gln Thr Glu Pro Leu Asp Arg
           195                 200
<210> 67
   <211> 199
   <212> PRT
   <213> Medicago truncatula
<220>
   <223> barrel medic unknown protein, clone MTYFP_FQ_FR_FS1G-G-11, GenBank Accession No. ACJ86004.1
<400> 67
   Met Glu Lys Met Asn Gly Thr Glu Asn Asn Gly Val Phe Asn Ser Thr
    1               5                  10                  15
   Glu Met Glu Tyr Ile Arg Arg His His Asn Gln Gln Pro Gly Glu Asn
               20                  25                  30
   Gln Cys Ser Ser Ala Leu Val Lys His Ile Arg Ala Pro Val Pro Leu
           35                  40                  45
   Val Trp Ser Leu Val Arg Arg Phe Asp Gln Pro Gln Lys Tyr Lys Pro
       50                  55                  60
   Phe Val Ser Arg Cys Val Val Arg Gly Asn Leu Glu Ile Gly Ser Leu
   65                  70                  75                  80
   Arg Glu Val Asp Val Lys Ser Gly Leu Pro Ala Thr Thr Ser Thr Glu
                   85                  90                  95
   Arg Leu Glu Val Leu Asp Asp Asn Glu His Ile Leu Ser Ile Arg Ile
               100                 105                 110
   Ile Gly Gly Asp His Arg Leu Arg Asn Tyr Ser Ser Ile Met Ser Leu
           115                 120                 125
   His Pro Glu Ile Ile Asp Gly Arg Pro Gly Thr Leu Val Ile Glu Ser
       130                 135                 140
   Phe Val Val Asp Val Pro Glu Gly Asn Thr Lys Asp Glu Thr Cys Tyr
   145                 150                 155                 160
   Phe Val Glu Ala Leu Ile Lys Cys Asn Leu Lys Ser Leu Ser Asp Val
                   165                 170                 175
   Ser Glu Gly His Ala Val Gln Asp Leu Thr Glu Pro Leu Asp Arg Val
               180                 185                 190
   His Glu Leu Leu Ile Ser Gly
           195
<210> 68
   <211> 199
   <212> PRT
   <213> Medicago truncatula
<220>
   <223> barrel medic unknown protein, clone MTYF1_F2_F3_FY1G-K-4, GenBank Accession No. ACJ83958.1
<400> 68
   Met Glu Lys Met Asn Gly Thr Glu Asn Asn Gly Val Phe Asn Ser Thr
    1               5                  10                  15
   Glu Met Glu Tyr Ile Arg Arg His His Asn Gln Gln Pro Gly Glu Asn
               20                  25                  30
   Gln Cys Ser Ser Ala Leu Val Lys His Ile Arg Ala Pro Val Pro Leu
           35                  40                  45
   Val Trp Ser Leu Val Arg Arg Phe Asp Gln Pro Gln Lys Tyr Lys Pro
       50                  55                  60
   Phe Val Ser Arg Cys Val Val Arg Gly Asn Leu Glu Ile Gly Ser Leu
   65                  70                  75                  80
   Arg Glu Val Asp Val Lys Ser Gly Leu Pro Ala Thr Thr Ser Thr Glu
                   85                  90                  95
   Arg Leu Glu Val Leu Asp Asp Asn Glu His Ile Leu Ser Ile Arg Ile
               100                 105                 110
   Ile Gly Gly Asp His Arg Leu Arg Asn Tyr Ser Ser Ile Met Ser Leu
           115                 120                 125
   His Pro Glu Ile Ile Asp Gly Arg Pro Gly Thr Leu Val Ile Glu Ser
       130                 135                 140
   Phe Val Val Asp Val Pro Glu Gly Asn Thr Lys Asp Glu Thr Cys Tyr
   145                 150                 155                 160
   Phe Val Glu Ala Leu Ile Lys Cys Asn Leu Lys Ser Leu Ser Asp Val
                   165                 170                 175
   Ser Glu Gly His Ala Ala Gln Asp Leu Thr Glu Pro Leu Asp Arg Met
               180                 185                 190
   His Glu Leu Leu Ile Ser Gly
           195
<210> 69
   <211> 197
   <212> PRT
   <213> Zea mays
<220>
   <223> maize CAPIP1 protein, clone 244179, GenBank Accession No. ACG34726.1
<400> 69
   Met Val Gly Leu Val Gly Gly Ser Thr Ala Arg Ala Glu His Val Val
    1               5                  10                  15
   Ala Asn Ala Gly Gly Glu Ala Glu Tyr Val Arg Arg Met His Arg His
               20                  25                  30
   Ala Pro Thr Glu His Gln Cys Thr Ser Thr Leu Val Lys His Ile Lys
           35                  40                  45
   Ala Pro Val His Leu Val Trp Gln Leu Val Arg Arg Phe Asp Gln Pro
       50                  55                  60
   Gln Arg Tyr Lys Pro Phe Val Arg Asn Cys Val Val Arg Gly Asp Gln
   65                  70                  75                  80
   Leu Glu Val Gly Ser Leu Arg Asp Val Asn Val Lys Thr Gly Leu Pro
                   85                  90                  95
   Ala Thr Thr Ser Thr Glu Arg Leu Glu Gln Leu Asp Asp Asp Leu His
               100                 105                 110
   Ile Leu Gly Val Lys Phe Val Gly Gly Asp His Arg Leu Gln Asn Tyr
           115                 120                 125
   Ser Ser Ile Ile Thr Val His Pro Glu Ser Ile Asp Gly Arg Pro Gly
       130                 135                 140
   Thr Leu Val Ile Glu Ser Phe Val Val Asp Val Pro Asp Gly Asn Thr
   145                 150                 155                 160
   Lys Asp Glu Thr Cys Tyr Phe Val Glu Ala Val Ile Lys Cys Asn Leu
                           165                 170                 175
   Asn Ser Leu Ala Glu Val Ser Glu Gln Leu Ala Val Glu Ser Pro Thr
               180                 185                 190
   Ser Leu Ile Asp Gln
           195
<210> 70
   <211> 197
   <212> PRT
   <213> Zea mays
<220>
   <223> maize CAPIP1 protein, clone 1448906, GenBank Accession No. ACG26022.1
<400> 70
   Met Val Gly Leu Val Gly Gly Ser Thr Ala Arg Ala Glu His Val Val
    1               5                  10                  15
   Ala Asn Ala Gly Gly Glu Ala Glu Tyr Val Arg Arg Met His Arg His
               20                  25                  30
   Ala Pro Thr Glu His Gln Cys Thr Ser Thr Leu Val Lys His Ile Lys
           35                  40                  45
   Ala Pro Val His Leu Val Trp Glu Leu Val Arg Arg Phe Asp Gln Pro
       50                  55                  60
   Gln Arg Tyr Lys Pro Phe Val Arg Asn Cys Val Val Arg Gly Asp Gln
   65                  70                  75                  80
   Leu Glu Val Gly Ser Leu Arg Asp Val Asn Val Lys Thr Gly Leu Pro
                   85                  90                  95
   Ala Thr Thr Ser Thr Glu Arg Leu Glu Gln Leu Asp Asp Asp Leu His
               100                 105                 110
   Ile Leu Gly Val Lys Phe Val Gly Gly Asp His Arg Leu Gln Asn Tyr
           115                 120                 125
   Ser Ser Ile Ile Thr Val His Pro Glu Ser Ile Asp Gly Arg Pro Gly
       130                 135                 140
   Thr Leu Val Ile Glu Ser Phe Val Val Asp Val Pro Asp Gly Asn Thr
   145                 150                 155                 160
   Lys Asp Glu Thr Cys Tyr Phe Val Glu Ala Val Ile Lys Cys Asn Leu
                   165                 170                 175
   Asn Ser Leu Ala Glu Val Ser Glu Gln Leu Ala Val Glu Ser Pro Thr
               180                 185                 190
   Ser Leu Ile Asp Gln
           195
<210> 71
   <211> 212
   <212> PRT
   <213> Zea mays
<220>
   <223> maize strain B73 unknown protein, clone ZM_BFc0183D21, GenBank Accession No. ACF86162.1
<400> 71
   Met Val Met Val Glu Met Asp Gly Gly Val Gly Gly Gly Gly Gly Gly
   1               5                   10                  15
   Gly Gln Thr Pro Ala Pro Arg Arg Trp Arg Leu Ala Asp Glu Arg Cys
               20                  25                  30
   Asp Leu Arg Ala Met Glu Thr Asp Tyr Val Arg Arg Phe His Arg His
           35                  40                  45
   Glu Pro Arg Glu His Gln Cys Ser Ser Ala Val Ala Lys His Ile Lys
       50                  55                  60
   Ala Pro Val His Leu Val Trp Ser Leu Val Arg Arg Phe Asp Gln Pro
   65                  70                  75                  80
   Gln Leu Phe Lys Pro Phe Val Ser Arg Cys Glu Met Lys Gly Asn Ile
                   85                  90                  95
   Glu Ile Gly Ser Val Arg Glu Val Asn Val Lys Ser Gly Leu Pro Ala
               100                 105                 110
   Thr Arg Ser Thr Glu Arg Leu Glu Leu Leu Asp Asp Asn Glu His Ile
           115                 120                 125
   Leu Ser Val Arg Phe Val Gly Gly Asp His Arg Leu Gln Asn Tyr Ser
       130                 135                 140
   Ser Ile Leu Thr Val His Pro Glu Val Ile Asp Gly Arg Pro Gly Thr
   145                 150                 155                 160
   Leu Val Ile Glu Ser Phe Val Val Asp Val Pro Asp Gly Asn Thr Lys
                   165                 170                 175
   Asp Glu Thr Cys Tyr Phe Val Glu Ala Leu Leu Lys Cys Asn Leu Lys
               180                 185                 190
   Ser Leu Ala Glu Val Ser Glu Arg Gln Val Val Lys Asp Gln Thr Glu
           195                 200                 205
   Pro Leu Asp Arg
       210
<210> 72
   <211> 205
   <212> PRT
   <213> Oryza sativa
<220>
   <223> rice Japonica Group, cultivar Nipponbare, conserved hypothetical protein Os06g0527800, GenBank Accession No. NP_001057771.1
<400> 72
   Met Asn Gly Ala Gly Gly Ala Gly Gly Ala Ala Ala Gly Lys Leu Pro
    1               5                  10                  15
   Met Val Ser His Arg Arg Val Gln Cys Arg Leu Ala Asp Lys Arg Cys
               20                  25                  30
   Glu Leu Arg Glu Glu Glu Met Glu Tyr Ile Arg Gln Phe His Arg His
           35                  40                  45
   Glu Pro Ser Ser Asn Gln Cys Thr Ser Phe Val Ala Lys His Ile Lys
       50                  55                  60
   Ala Pro Leu Gln Thr Val Trp Ser Leu Val Arg Arg Phe Asp Gln Pro
   65                  70                  75                  80
   Gln Leu Phe Lys Pro Phe Val Arg Lys Cys Val Met Arg Glu Asn Ile
                   85                  90                  95
   Ile Val Thr Gly Cys Val Arg Glu Val Asn Val Gln Ser Gly Leu Pro
               100                 105                 110
   Ala Thr Arg Ser Thr Glu Arg Leu Glu Leu Leu Asp Asp Asn Glu His
           115                 120                 125
   Ile Leu Lys Val Lys Phe Ile Gly Gly Asp His Met Leu Lys Asn Tyr
       130                 135                 140
   Ser Ser Ile Leu Thr Ile His Ser Glu Val Ile Asp Gly Gln Leu Gly
   145                 150                 155                 160
   Thr Leu Val Val Glu Ser Phe Val Val Asp Ile Pro Asp Gly Asn Thr
                   165                 170                 175
   Lys Asp Asp Ile Cys Tyr Phe Ile Glu Asn Val Leu Arg Cys Asn Leu
               180                 185                 190
   Met Thr Leu Ala Asp Val Ser Glu Glu Arg Leu Ala Asn
           195                 200                 205
<210> 73
   <211> 197
   <212> PRT
   <213> Zea mays
<220>
   <223> maize strain B73 unknown protein, clone ZM_BFc0063E17, GenBank Accession No. ACF85073.1
<400> 73
   Met Val Gly Leu Val Gly Gly Ser Thr Ala Arg Ala Glu His Val Val
    1               5                   10                  15
   Ala Asn Ala Gly Gly Glu Thr Glu Tyr Val Arg Arg Leu His Arg His
               20                  25                  30
   Ala Pro Ala Glu His Gln Cys Thr Ser Thr Leu Val Lys His Ile Lys
           35                  40                  45
   Ala Pro Val His Leu Val Trp Glu Leu Val Arg Ser Phe Asp Gln Pro
       50                  55                  60
   Gln Arg Tyr Lys Pro Phe Val Arg Asn Cys Val Val Arg Gly Asp Gln
   65                  70                  75                  80
   Leu Glu Val Gly Ser Leu Arg Asp Val Asn Val Lys Thr Gly Leu Pro
                   85                  90                  95
   Ala Thr Thr Ser Thr Glu Arg Leu Glu Gln Leu Asp Asp Asp Leu His
               100                 105                 110
   Ile Leu Gly Val Lys Phe Val Gly Gly Asp His Arg Leu Gln Asn Tyr
           115                 120                 125
   Ser Ser Ile Ile Thr Val His Pro Glu Ser Ile Asp Gly Arg Pro Gly
       130                 135                 140
   Thr Leu Val Ile Glu Ser Phe Val Val Asp Val Pro Asp Gly Asn Thr
   145                 150                 155                 160
   Lys Asp Glu Thr Cys Tyr Phe Val Glu Ala Val Ile Lys Cys Asn Leu
                   165                 170                 175
   Lys Ser Leu Ala Glu Val Ser Glu Gln Leu Ala Val Glu Ser Pro Thr
               180                 185                 190
   Ser Pro Ile Asp Gln
           195
<210> 74
   <211> 206
   <212> PRT
   <213> Oryza sativa
<220>
   <223> rice Indica Group, cultivar 93-11, hypothetical protein OsI_23218, GenBank Accession No. EAZ01191.1
<400> 74
   Met Asn Gly Val Gly Gly Ala Gly Gly Ala Ala Ala Gly Lys Leu Pro
    1               5                   10                  15
   Met Val Ser His Arg Arg Val Gln Trp Arg Leu Ala Asp Glu Arg Cys
               20                  25                  30
   Glu Leu Arg Glu Glu Glu Met Glu Tyr Ile Arg Arg Phe His Arg His
           35                  40                  45
   Glu Pro Ser Ser Asn Gln Cys Thr Ser Phe Ala Ala Lys His Ile Lys
       50                  55                  60
   Ala Pro Leu His Thr Val Trp Ser Leu Val Arg Arg Phe Asp Gln Pro
   65                  70                  75                  80
   Gln Leu Phe Lys Pro Phe Val Arg Asn Cys Val Met Arg Glu Asn Ile
                   85                  90                  95
   Ile Ala Thr Gly Cys Ile Arg Glu Val Asn Val Gln Ser Gly Leu Pro
               100                 105                 110
   Ala Thr Arg Ser Thr Glu Arg Leu Glu Leu Leu Asp Asp Asn Glu His
           115                 120                 125
   Ile Leu Lys Val Lys Phe Ile Gly Gly Asp His Met Leu Lys Asn Tyr
       130                 135                 140
   Ser Ser Ile Leu Thr Val His Ser Glu Val Ile Asp Gly Gln Leu Gly
   145                 150                 155                 160
   Thr Leu Val Val Glu Ser Phe Ile Val Asp Val Leu Glu Gly Asn Thr
                   165                 170                 175
   Lys Asp Asp Ile Ser Tyr Phe Ile Glu Asn Val Leu Arg Cys Asn Leu
                   180                 185                 190
   Arg Thr Leu Ala Asp Val Ser Glu Glu Arg Leu Ala Asn Pro
           195                 200                 205
<210> 75
   <211> 209
   <212> PRT
   <213> Oryza sativa
<220>
   <223> rice Japonica Group, cultivar Nipponbare, conserved hypothetical protein Os05g0213500, GenBank Accession No. NP_001054923.1
<400> 75
   Met Val Gly Leu Val Gly Gly Gly Gly Trp Arg Val Gly Asp Asp Ala
    1               5                  10                  15
   Ala Gly Gly Gly Gly Gly Gly Ala Val Ala Ala Gly Ala Ala Ala Ala
               20                  25                  30
   Ala Glu Ala Glu His Met Arg Arg Leu His Ser His Ala Pro Gly Glu
           35                  40                  45
   His Gln Cys Ser Ser Ala Leu Val Lys His Ile Lys Ala Pro Val His
       50                  55                  60
   Leu Val Trp Ser Leu Val Arg Ser Phe Asp Gln Pro Gln Arg Tyr Lys
   65                  70                  75                  80
   Pro Phe Val Ser Arg Cys Val Val Arg Gly Gly Asp Leu Glu Ile Gly
                   85                  90                  95
   Ser Val Arg Glu Val Asn Val Lys Thr Gly Leu Pro Ala Thr Thr Ser
               100                 105                 110
   Thr Glu Arg Leu Glu Leu Leu Asp Asp Asp Glu His Ile Leu Ser Val
           115                 120                 125
   Lys Phe Val Gly Gly Asp His Arg Leu Arg Asn Tyr Ser Ser Ile Val
       130                 135                 140
   Thr Val His Pro Glu Ser Ile Asp Gly Arg Pro Gly Thr Leu Val Ile
   145                 150                 155                 160
   Glu Ser Phe Val Val Asp Val Pro Asp Gly Asn Thr Lys Asp Glu Thr
                   165                 170                 175
   Cys Tyr Phe Val Glu Ala Val Ile Lys Cys Asn Leu Thr Ser Leu Ala
               180                 185                 190
   Glu Val Ser Glu Arg Leu Ala Val Gln Ser Pro Thr Ser Pro Leu Glu
           195                 200                 205
   Gln
<210> 76
   <211> 180
   <212> PRT
   <213> Oryza sativa
<220>
   <223> rice Japonica Group, cultivar Nipponbare, Bet v I allergen-like protein, clone OSJNBa0052K15, gene OSJNBa0052K15.17, GenBank Accession No. BAD29692.1
<400> 76
   Met Val Glu Met Asp Ala Gly Gly Arg Pro Glu Pro Ser Pro Pro Ser
    1               5                  10                  15
   Gly Gln Cys Ser Ser Ala Val Thr Met Arg Ile Asn Ala Pro Val His
               20                  25                  30
   Leu Val Trp Ser Ile Val Arg Arg Phe Glu Glu Pro His Ile Phe Gln
           35                  40                  45
   Pro Phe Val Arg Gly Cys Thr Met Arg Gly Ser Thr Ser Leu Ala Val
       50                  55                  60
   Gly Cys Val Arg Glu Val Asp Phe Lys Ser Gly Phe Pro Ala Lys Ser
   65                  70                  75                  80
   Ser Val Glu Arg Leu Glu Ile Leu Asp Asp Lys Glu His Val Phe Gly
                   85                  90                  95
   Val Arg Ile Ile Gly Gly Asp His Arg Leu Lys Asn Tyr Ser Ser Val
               100                 105                 110
   Leu Thr Ala Lys Pro Glu Val Ile Asp Gly Glu Pro Ala Thr Leu Val
           115                 120                 125
   Ser Glu Ser Phe Val Val Asp Val Pro Glu Gly Asn Thr Ala Asp Glu
       130                 135                 140
   Thr Arg His Phe Val Glu Phe Leu Ile Arg Cys Asn Leu Arg Ser Leu
   145                 150                 155                 160
   Ala Met Val Ser Gln Arg Leu Leu Leu Ala Gln Gly Asp Leu Ala Glu
                   165                 170                 175
   Pro Pro Ala Gln
               180
<210> 77
   <211> 176
   <212> PRT
   <213> Vitis vinifera
<220>
   <223> wine grape cultivar Pinot Noir hypothetical protein, clone ENTAV 115, locus tag VITISV_029498, GenBank Accession No. CAN64668.1
<400> 77
   Met Asn Gly Asn Gly Leu Ser Ser Met Glu Ser Glu Tyr Ile Arg Arg
    1               5                  10                  15
   His His Arg His Glu Pro Ala Glu Asn Gln Cys Ser Ser Ala Leu Val
               20                  25                  30
   Lys His Ile Lys Ala Pro Val Pro Leu Val Trp Ser Leu Val Arg Arg
           35                  40                  45
   Phe Asp Gln Pro Gln Lys Tyr Lys Pro Phe Ile Ser Arg Cys Val Val
       50                  55                  60
   Gln Gly Asn Leu Glu Ile Gly Ser Leu Arg Glu Val Asp Val Lys Ser
   65                  70                  75                  80
   Gly Leu Pro Ala Thr Thr Ser Thr Glu Arg Leu Glu Leu Leu Asp Asp
                   85                  90                  95
   Asp Glu His Ile Leu Ser Met Arg Ile Ile Gly Gly Asp His Arg Leu
               100                 105                 110
   Arg Asn Tyr Ser Ser Ile Ile Ser Leu His Pro Glu Ile Ile Asp Gly
           115                 120                 125
   Arg Pro Gly Thr Met Val Ile Glu Ser Tyr Val Val Asp Val Pro Glu
       130                 135                 140
   Gly Asn Thr Lys Asp Glu Thr Cys Tyr Phe Ser Leu Ala Asp Val Ser
   145                 150                 155                 160
   Glu Arg Leu Ala Val Ala Gly Thr Val Thr Glu Pro Ile Asp Arg Met
                   165                 170                 175
<210> 78
   <211> 180
   <212> PRT
   <213> Oryza sativa
<220>
   <223> rice Indica Group, cultivar 93-11, hypothetical protein, locus tag OsI_06615, GenBank Accession No. EEC72859.1
<400> 78
   Met Val Glu Met Asp Ala Gly Gly Arg Pro Glu Pro Ser Pro Pro Ser
    1               5                  10                  15
   Gly Gln Cys Ser Ser Ala Val Thr Met Arg Ile Asn Ala Pro Val His
               20                  25                  30
   Leu Val Trp Ser Ile Val Arg Arg Phe Glu Glu Pro His Ile Phe Gln
           35                  40                  45
   Pro Phe Val Arg Gly Cys Thr Met Arg Gly Ser Thr Ser Leu Ala Val
       50                  55                  60
   Gly Cys Val Arg Glu Val Asp Phe Lys Ser Gly Phe Ser Ala Lys Ser
   65                  70                  75                  80
   Ser Val Glu Arg Leu Glu Ile Leu Asp Asp Lys Glu His Val Phe Gly
                   85                  90                  95
   Val Arg Ile Ile Gly Gly Asp His Arg Leu Lys Asn Tyr Ser Ser Val
               100                 105                 110
   Leu Thr Ala Lys Pro Glu Val Ile Asp Gly Glu Pro Ala Thr Leu Val
           115                 120                 125
   Ser Glu Ser Phe Val Ile Asp Val Pro Glu Gly Asn Thr Ala Asp Glu
       130                 135                 140
   Thr Arg His Phe Val Glu Phe Leu Ile Arg Cys Asn Leu Arg Ser Leu
   145                 150                 155                 160
   Ala Met Val Ser Gln Arg Leu Leu Leu Ala Gln Gly Asp Leu Ala Glu
                   165                 170                 175
   Pro Pro Ala Gln
               180
<210> 79
   <211> 215
   <212> PRT
   <213> Oryza sativa
<220>
   <223> rice Japonica Group, cultivar Nipponbare, hypothetical protein, locus tag OsJ_10498, GenBank Accession No. EAZ26598.1
<400> 79
   Met Pro Cys Ile Pro Ala Ser Ser Pro Gly Ile Pro His Gln His Gln
    1               5                  10                  15
   His Gln His His Arg Ala Leu Ala Gly Val Gly Met Ala Val Gly Cys
               20                  25                  30
   Ala Ala Glu Ala Ala Val Ala Ala Ala Gly Val Ala Gly Thr Arg Cys
           35                  40                  45
   Gly Ala His Asp Gly Glu Val Pro Met Glu Val Ala Arg His His Glu
       50                  55                  60
   His Ala Glu Pro Gly Ser Gly Arg Cys Cys Ser Ala Val Val Gln His
   65                  70                  75                  80
   Val Ala Ala Pro Ala Ala Ala Val Trp Ser Val Val Arg Arg Phe Asp
                   85                  90                  95
   Gln Pro Gln Ala Tyr Lys Arg Phe Val Arg Ser Cys Ala Leu Leu Ala
               100                 105                 110
   Gly Asp Gly Gly Leu Gly Lys Val Arg Glu Arg Leu Glu Ile Leu Asp
           115                 120                 125
   Asp Glu Ser His Val Leu Ser Phe Arg Val Val Gly Gly Glu His Arg
       130                 135                 140
   Leu Lys Asn Tyr Leu Ser Val Thr Thr Val His Pro Ser Pro Ser Ala
   145                 150                 155                 160
   Pro Thr Ala Ala Thr Val Val Val Glu Ser Tyr Val Val Asp Val Pro
                   165                 170                 175
   Pro Gly Asn Thr Pro Glu Asp Thr Arg Val Phe Val Asp Thr Ile Val
               180                 185                 190
   Lys Cys Asn Leu Gln Ser Leu Ala Lys Thr Ala Glu Lys Leu Ala Ala
           195                 200                 205
   Gly Ala Arg Ala Ala Gly Ser
       210                 215
<210> 80
   <211> 186
   <212> PRT
   <213> Rheum australe
<220>
   <223> Himalayan rhubarb pathogen-induced protein-like protein, GenBank Accession No. ACH63237.1
<400> 80
   Met Asn Gly Asp Gly Tyr Gly Gly Ser Glu Glu Glu Phe Val Lys Arg
    1               5                  10                  15
   Tyr His Glu His Val Leu Ala Asp His Gln Cys Ser Ser Val Leu Val
               20                  25                  30
   Glu His Ile Asn Ala Pro Leu His Leu Val Trp Ser Leu Val Arg Ser
           35                  40                  45
   Phe Asp Gln Pro Gln Lys Tyr Lys Pro Phe Val Ser Arg Cys Val Val
       50                  55                  60
   Gln Gly Gly Asp Leu Glu Ile Gly Ser Val Arg Glu Val Asp Val Lys
   65                  70                  75                  80
   Ser Gly Leu Pro Ala Thr Thr Ser Met Glu Glu Leu Glu Leu Leu Asp
                   85                  90                  95
   Asp Lys Glu His Val Leu Arg Val Lys Phe Val Gly Gly Asp His Arg
               100                 105                 110
   Leu Lys Asn Tyr Ser Ser Ile Val Ser Leu His Pro Glu Ile Ile Gly
           115                 120                 125
   Gly Arg Ser Gly Thr Met Val Ile Glu Ser Phe Ile Val Asp Ile Ala
       130                 135                 140
   Asp Gly Asn Thr Lys Glu Glu Thr Cys Tyr Phe Ile Glu Ser Leu Ile
   145                 150                 155                 160
   Asn Cys Asn Leu Lys Ser Leu Ser Cys Val Ser Glu Arg Leu Ala Val
                   165                 170                 175
   Glu Asp Ile Ala Glu Arg Ile Ala Gln Met
               180                 185
<210> 81
   <211> 254
   <212> PRT
   <213> Oryza sativa
<220>
   <223> rice Japonica Group, cultivar Nipponbare, hypothetical protein, locus tag OsJ_016770, GenBank Accession No. EAZ33287.1
<400> 81
   Met Val Gly Leu Val Gly Gly Gly Gly Trp Arg Val Gly Asp Asp Ala
    1               5                  10                  15
   Ala Gly Gly Gly Gly Gly Gly Ala Val Ala Ala Gly Ala Ala Ala Ala
               20                  25                  30
   Ala Glu Ala Glu His Met Arg Arg Leu His Ser Gln Gly Pro Arg Arg
           35                  40                  45
   Ala Pro Val Gln Leu Arg Ala Arg Gln Ala His Gln Gly Ser Cys Ser
       50                  55                  60
   Pro Pro Arg Ile Glu Cys Ala Asn Phe Ala Val Phe Leu Ala Ala Arg
   65                  70                  75                  80
   Asp Pro Lys Ile Val Trp Ser Leu Val Arg Ser Phe Asp Gln Pro Gln
                   85                  90                  95
   Arg Tyr Lys Pro Phe Val Ser Arg Cys Val Val Arg Gly Gly Asp Leu
               100                 105                 110
   Glu Ile Gly Ser Val Arg Glu Val Asn Val Lys Thr Gly Leu Pro Ala
           115                 120                 125
   Thr Thr Ser Thr Glu Arg Leu Glu Leu Leu Asp Asp Asp Glu His Ile
       130                 135                 140
   Leu Ser Val Lys Phe Val Gly Gly Asp His Arg Leu Arg Asn Tyr Ser
   145                 150                 155                 160
   Ser Ile Val Thr Val His Pro Glu Ser Ile Asp Gly Arg Pro Gly Thr
                   165                 170                 175
   Leu Val Ile Glu Ser Phe Val Val Asp Val Pro Asp Gly Asn Thr Lys
               180                 185                 190
   Asp Glu Thr Cys Tyr Phe Val Glu Ala Val Ile Lys Cys Asn Leu Thr
           195                 200                 205
   Ser Leu Ala Glu Met Val Arg Met Ile Ser Leu Val Leu Pro Phe Met
       210                 215                 220
   Leu Val Asp Arg Met Ser Gly Ile Thr Cys Glu Ser His Leu Glu Thr
   225                 230                 235                 240
   Thr Leu Val Arg Cys Gly Glu Tyr Ala Val Leu Ala His Val
                   245                 250
<210> 82
   <211> 186
   <212> PRT
   <213> Oryza sativa
<220>
   <223> rice Japonica Group, cultivar Nipponbare, hypothetical protein, locus tag OsJ_005784, GenBank Accession No. EAZ22301.1
<400> 82
   Met Glu Pro His Met Glu Arg Ala Leu Arg Glu Ala Val Ala Ser Glu
    1               5                  10                  15
   Ala Glu Arg Arg Glu Leu Glu Gly Val Val Arg Ala His His Thr Gly
               20                  25                  30
   Trp Asn Ala Pro Leu Ala Ala Val Trp Pro His Arg Ala Arg Val Arg
           35                  40                  45
   Pro Thr Arg Ser Gly Thr Ser Thr Ser Ser Ser Arg Ala Ser Ser Pro
       50                  55                  60
   Pro Gly Asp Gly Ala Thr Val Gly Ser Val Arg Glu Val Ala Val Val
   65                  70                  75                  80
   Ser Gly Leu Pro Ala Ser Thr Ser Thr Glu Arg Leu Glu Ile Leu Asp
                   85                  90                  95
   Asp Asp Arg His Val Leu Ser Phe Arg Val Val Gly Gly Asp His Arg
               100                 105                 110
   Leu Arg Asn Tyr Arg Ser Val Thr Ser Val Thr Glu Phe Ser Ser Pro
           115                 120                 125
   Ser Ser Pro Pro Arg Pro Tyr Cys Val Val Val Glu Ser Tyr Val Val
       130                 135                 140
   Asp Val Pro Glu Gly Asn Thr Glu Glu Asp Thr Arg Met Phe Thr Asp
   145                 150                 155                 160
   Thr Val Val Lys Leu Asn Leu Gln Lys Leu Ala Ala Val Ala Thr Ser
                   165                 170                 175
   Ser Ser Pro Pro Ala Ala Gly Asn His His
               180                 185
<210> 83
   <211> 150
   <212> PRT
   <213> Oryza sativa
<220>
   <223> rice Japonica Group, cultivar Nipponbare, hypothetical protein, locus tag OsJ_005938, GenBank Accession No. EAZ22455.1
<400> 83
   Met Glu Val Val Trp Ser Ile Val Arg Arg Phe Glu Glu Pro His Ile
    1               5                  10                  15
   Phe Gln Pro Phe Val Arg Gly Cys Thr Met Arg Gly Ser Thr Ser Leu
               20                  25                  30
   Ala Val Gly Cys Val Arg Glu Val Asp Phe Lys Ser Gly Phe Pro Ala
           35                  40                  45
   Lys Ser Ser Val Glu Arg Leu Glu Ile Leu Asp Asp Lys Glu His Val
       50                  55                  60
   Phe Gly Val Arg Ile Ile Gly Gly Asp His Arg Leu Lys Asn Tyr Ser
   65                  70                  75                  80
   Ser Val Leu Thr Ala Lys Pro Glu Val Ile Asp Gly Glu Pro Ala Thr
                   85                  90                  95
   Leu Val Ser Glu Ser Phe Val Val Asp Val Pro Glu Gly Asn Thr Ala
               100                 105                 110
   Asp Glu Thr Arg His Phe Val Glu Phe Leu Ile Arg Cys Asn Leu Arg
           115                 120                 125
   Ser Leu Ala Met Val Ser Gln Arg Leu Leu Leu Ala Gln Gly Asp Leu
       130                 135                 140
   Ala Glu Pro Pro Gly Gln
   145                 150
<210> 84
   <211> 206
   <212> PRT
   <213> Oryza sativa
<220>
   <223> rice Japonica Group, cultivar Nipponbare, hypothetical protein, locus tag OsJ_018129, GenBank Accession No. EAZ34646.1
<400> 84
   Met Pro Tyr Thr Ala Pro Arg Pro Ser Pro Pro Gln His Ser Arg Ile
    1               5                  10                  15
   Gly Gly Cys Gly Gly Gly Gly Val Leu Lys Ala Ala Gly Ala Ala Gly
               20                  25                  30
   His Ala Ala Ser Cys Val Ala Val Pro Ala Glu Val Ala Arg His His
           35                  40                  45
   Glu His Ala Ala Gly Val Gly Gln Cys Cys Ser Ala Val Val Gln Ala
       50                  55                  60
   Ile Ala Ala Pro Val Asp Ala Val Trp Arg Thr Ser Thr Ser Ser Gly
   65                  70                  75                  80
   Ala Ala Ala Ser Trp Thr Ala Thr Ala Thr Ala Gly Pro Leu Pro Val
                   85                  90                  95
   Gly Ser Val Arg Glu Phe Arg Val Leu Ser Gly Leu Pro Gly Thr Ser
               100                 105                 110
   Ser Arg Glu Arg Leu Glu Ile Leu Asp Asp Glu Arg Arg Val Leu Ser
           115                 120                 125
   Phe Arg Val Val Gly Gly Glu His Arg Leu Ser Asn Tyr Arg Ser Val
       130                 135                 140
   Thr Thr Val His Glu Thr Ala Ala Gly Ala Ala Ala Ala Val Val Val
   145                 150                 155                 160
   Glu Ser Tyr Val Val Asp Val Pro His Gly Asn Thr Ala Asp Glu Thr
                   165                 170                 175
   Arg Met Phe Val Asp Thr Ile Val Arg Cys Asn Leu Gln Ser Leu Ala
               180                 185                 190
   Arg Thr Ala Glu Gln Leu Ala Leu Ala Ala Pro Arg Ala Ala
           195                 200                 205
<210> 85
   <211> 396
   <212> PRT
   <213> Vitis vinifera
<220>
   <223> wine grape cultivar Pinot Noir hypothetical protein, clone ENTAV 115, locus tag VITISV_001710, GenBank Accession No. CAN76441.1
<220>
   <221> VARIANT
   <222> (1)...(396)
   <223> Xaa = any amino acid
<400> 85
   Met Pro Ile Ser Ser Leu Pro Phe Ser Leu Tyr Thr Val Thr Pro Asn
    1               5                  10                  15
   Pro Leu Lys Leu Ile Thr Thr His Ala His Ala Phe Thr Pro His Thr
               20                  25                  30
   His Ile Phe Thr Leu Lys Phe Met Ser His Thr Tyr Cys Pro His Ile
           35                  40                  45
   His His Ile Thr Ser Ile His Tyr Thr His Leu Leu Xaa Pro Ile Pro
       50                  55                  60
   His Met Pro Leu Gln Pro Pro Leu Pro Pro His Pro Ile Leu Pro Ser
   65                  70                  75                  80
   Met Pro Ala Phe Gln His Leu Tyr Ser Thr Asn Gln His Leu Gln Val
                   85                  90                  95
   Ala Leu Phe Ser Ala Arg Gly Pro Asn Ile Arg Asp Phe Asn Phe Gln
               100                 105                 110
   Asp Ala Asp Leu Leu Lys Leu Asp Ile Leu Ala Pro Gly Ser Leu Ile
           115                 120                 125
   Trp Ala Ala Trp Ser Pro Asn Gly Thr Asp Glu Ala Asn Tyr Val Gly
       130                 135                 140
   Glu Gly Ser Pro Thr Val Ala Met Ile Ala Lys Arg Gly Pro Arg His
   145                 150                 155                 160
   Gly Lys Tyr Met Ala Phe Cys Xaa Met Tyr Arg Asp Asn Val Ala Pro
                   165                 170                 175
   Lys Gly Val Asn Xaa Ala Val Ala Thr Val Lys Thr Lys Arg Thr Ile
               180                 185                 190
   Gln Leu Lys Thr Ser Leu Glu Ile Ala Cys His Tyr Ala Gly Ile Asn
           195                 200                 205
   Ile Ser Gly Ile Asn Gly Glu Val Met Pro Gly Gln Trp Glu Tyr Gln
       210                 215                 220
   Val Gly Pro Gly Gln Cys Ser Ser Leu Leu Ala Gln Arg Val His Val
   225                 230                 235                 240
   Pro Leu Ser Ala Val Gly Ser Val Val His Arg Phe Asp Lys Pro Gln
                   245                 250                 255
   Arg Tyr Gln His Val Ile Lys Ser Cys Arg Ile Glu Asp Gly Phe Glu
               260                 265                 270
   Met Arg Met Gly Xaa Leu Arg Asp Val Asn Ile Ile Ser Gly Leu Pro
           275                 280                 285
   Thr Ala Thr Asn Thr Gly Arg Leu Asp Met Gln Asp Asp Glu Arg His
       290                 295                 300
   Val Thr Arg Cys Pro His Gln Arg Gln Ser Glu Ser Lys Tyr Thr Glu
   305                 310                 315                 320
   Asn Asn Asn Ser Asp Ala Ser Ser Ile Lys Ser Pro Ile Asn Gly Pro
                   325                 330                 335
   Ser Glu His Leu Lys Thr Ala Ala Ser Pro Lys Thr Glu Ser Ile Ile
               340                 345                 350
   Val Ile Asp Thr Ser Lys Phe Leu Asn Glu Glu Asp Phe Glu Gly Lys
           355                 360                 365
   Asp Glu Thr Ser Ser Ser Asn Gln Val Gln Ile Glu Asp Glu Asn Trp
       370                 375                 380
   Glu Thr Arg Phe Pro Asn Thr Asp Ala Gly Ile Trp
   385                 390                 395
<210> 86
   <211> 443
   <212> PRT
   <213> Vitis vinifera
<220>
   <223> wine grape cultivar Pinot Noir hypothetical protein, clone ENTAV 115, locus tag VITISV_014403, GenBank Accession No. CAN9881.1
<220>
   <221> VARIANT
   <222> (1)...(443)
   <223> Xaa = any amino acid
<400> 86
   Met Pro Ser Ala Xaa Lys Ser Ser Thr Val Pro Leu Ser Leu Xaa Gln
    1               5                  10                  15
   Phe Lys Leu Gly Leu Arg His Gly His Arg Val Ile Pro Trp Gly Asp
               20                  25                  30
   Leu Asp Ser Leu Ala Met Leu Gln Arg Gln Leu Asp Val Asp Ile Leu
           35                  40                  45
   Val Thr Gly His Thr His Arg Phe Thr Ala Tyr Lys His Glu Gly Gly
       50                  55                  60
   Val Val Ile Asn Pro Gly Ser Ala Thr Gly Ala Phe Gly Ser Ile Thr
   65                  70                  75                  80
   Tyr Asp Val Asn Pro Ser Phe Val Leu Met Asp Ile Asp Gly Leu Arg
                   85                  90                  95
   Val Val Val Cys Val Tyr Glu Leu Ile Asp Glu Thr Ala Asn Ile Ile
               100                 105                 110
   Lys Glu Leu His Ala Arg Lys Ile Ser Phe Gly Thr Lys Ser Met Ile
           115                 120                 125
   Xaa Cys Leu Leu Leu Lys Arg Arg Ser Thr Pro Lys Phe Arg Arg Lys
       130                 135                 140
   Lys Leu Phe Leu Phe Gln Cys Arg Val Gln Met Thr Leu Thr Leu Thr
   145                 150                 155                 160
   Asn Leu Ala Val Ser Gly Ile Ala Gln Thr Leu Gln Val Asp Gln Trp
                   165                 170                 175
   Thr Val Cys Ala Leu Ile Phe Met Thr Arg Arg Asp Ile His Leu Asp
               180                 185                 190
   Lys Ala Arg Phe Leu Asp Phe Lys Asp Met Gly Lys Leu Leu Ala Asp
           195                 200                 205
   Ala Ser Gly Leu Arg Lys Ala Leu Ser Gly Gly Xaa Val Thr Ala Gly
       210                 215                 220
   Met Ala Ile Phe Asp Thr Met Arg His Ile Arg Pro Asp Val Pro Thr
   225                 230                 235                 240
   Val Cys Val Gly Leu Ala Ala Val Ala Met Ile Ala Lys Arg Gly Pro
                   245                 250                 255
   Arg His Gly Lys Tyr Met Ala Phe Cys Pro Met Tyr Arg Asp Asn Val
               260                 265                 270
   Ala Pro Lys Gly Val Asn Val Ala Val Val Thr Val Lys Thr Lys Arg
           275                 280                 285
   Thr Ile Gln Leu Lys Thr Ser Leu Glu Ile Ala Cys His Tyr Ala Gly
       290                 295                 300
   Ile Asn Ile Ser Gly Ile Asn Gly Glu Val Met Pro Gly Gln Trp Glu
   305                 310                 315                 320
   Tyr Gln Val Gly Pro Gly Gln Cys Ser Ser Leu Leu Ala Gln Arg Val
                   325                 330                 335
   His Val Pro Leu Ser Ala Val Gly Ser Val Val His Arg Phe Asp Lys
               340                 345                 350
   Pro Gln Arg Tyr Gln His Val Ile Lys Ser Cys Arg Ile Glu Asp Gly
           355                 360                 365
   Phe Glu Met Arg Met Gly Arg Leu Arg Asp Val Asn Ile Ile Ser Gly
       370                 375                 380
   Leu Pro Thr Ala Thr Asn Thr Gly Arg Leu Asp Met Gln Asp Asp Glu
   385                 390                 395                 400
   Xaa His Val Thr Arg Cys Pro His Gln Arg Gln Ser Glu Ser Lys Tyr
                   405                 410                 415
   Thr Glu Asn Asn Asn Ser Asp Ala Ser Ser Val Lys Ser Pro Ile Asn
               420                 425                 430
   Gly Pro Ser Glu His Leu Lys Thr Ala Ala Xaa
           435                 440
<210> 87
   <211> 95
   <212> PRT
   <213> Oryza sativa
<220>
   <223> rice Indica Group, cultivar Pokkali, capip1 protein, clone OSR-385-428-D5, GenBank Accession No. ABR25904.1
<400> 87
   Glu Ile Gly Ser Val Arg Glu Val Asn Val Lys Thr Gly Leu Pro Ala
    1               5                  10                  15
   Thr Thr Ser Thr Glu Arg Leu Glu Leu Leu Asp Asp Asp Glu His Ile
               20                  25                  30
   Leu Ser Val Lys Phe Val Gly Gly Asp His Arg Leu Arg Asn Tyr Ser
           35                  40                  45
   Ser Ile Val Thr Val His Pro Glu Ser Ile Asp Gly Arg Pro Gly Thr
       50                  55                  60
   Leu Val Ile Glu Ser Phe Val Val Asp Val Pro Asp Gly Asn Thr Lys
   65                  70                  75                  80
   Asp Glu Thr Cys Tyr Phe Val Glu Ala Val Ile Lys Cys Asn Leu
                   85                  90                  95
<210> 88
   <211> 191
   <212> PRT
   <213> Zea mays
<220>
   <223> maize strain B73 unknown protein, clone ZM_BFc0034007, GenBank Accession No. ACF84624.1
<400> 88
   Met Val Val Glu Met Asp Gly Gly Val Gly Val Ala Ala Ala Gly Gly
    1               5                  10                  15
   Gly Gly Ala Gln Thr Pro Ala Pro Pro Pro Pro Arg Arg Trp Arg Leu
               20                  25                  30
   Ala Asp Glu Arg Cys Asp Leu Arg Ala Met Glu Thr Asp Tyr Val Arg
           35                  40                  45
   Arg Phe His Arg His Glu Pro Arg Asp His Gln Cys Ser Ser Ala Val
       50                  55                  60
   Ala Lys His Ile Lys Ala Pro Val His Leu Val Trp Ser Leu Val Arg
   65                  70                  75                  80
   Arg Phe Asp Gln Pro Gln Leu Phe Lys Pro Phe Val Ser Arg Cys Glu
                   85                  90                  95
   Met Lys Gly Asn Ile Glu Ile Gly Ser Val Arg Glu Val Asn Val Lys
               100                 105                 110
   Ser Gly Leu Pro Ala Thr Arg Ser Thr Glu Arg Leu Glu Leu Leu Asp
           115                 120                 125
   Asp Asp Glu Arg Ile Leu Ser Val Arg Phe Val Gly Gly Asp His Arg
       130                 135                 140
   Leu Gln Val Cys Ser Val Leu His Leu Ser Ile Phe Cys Ala Ala His
   145                 150                 155                 160
   Ala Arg Tyr Phe Ala His His Leu Lys Cys Val Leu Glu Phe Leu Cys
                   165                 170                 175
   Gln Met His Leu Asp Val Leu Pro Cys Asp Asp Ala Ile Leu Glu
               180                 185                 190
<210> 89
   <211> 239
   <212> PRT
   <213> Oryza sativa
<220>
   <223> rice Japonica Group, cultivar Nipponbare, hypothetical protein, locus tag OsJ_020681, GenBank Accession No. EAZ37198.1
<400> 89
   Met Asn Gly Cys Thr Gly Gly Ala Gly Gly Val Ala Ala Gly Arg Leu
    1               5                  10                  15
   Pro Ala Val Ser Leu Gln Gln Ala Gln Trp Lys Leu Val Asp Glu Arg
               20                  25                  30
   Cys Glu Leu Arg Glu Glu Glu Met Glu Tyr Val Arg Arg Phe His Arg
           35                  40                  45
   His Glu Ile Gly Ser Asn Gln Cys Asn Ser Phe Ile Ala Lys His Val
       50                  55                  60
   Arg Ala Pro Leu Gln Asn Val Trp Ser Leu Val Arg Arg Phe Asp Gln
   65                  70                  75                  80
   Pro Gln Ile Tyr Lys Pro Phe Val Arg Lys Cys Val Met Arg Gly Asn
                   85                  90                  95
   Val Glu Thr Gly Ser Val Arg Glu Ile Ile Val Gln Ser Gly Leu Pro
               100                 105                 110
   Ala Thr Arg Ser Ile Glu Arg Leu Glu Phe Leu Asp Asp Asn Glu Tyr
           115                 120                 125
   Ile Leu Arg Val Lys Phe Ile Gly Gly Asp His Met Leu Lys Lys Arg
       130                 135                 140
   Ile Pro Lys Lys Thr Tyr Ala Ile Ser Ser Arg Thr Cys Ser Asp Ser
   145                 150                 155                 160
   Ala Ile Ile Ala Val Gly Gln Ser Asn Cys Ala Pro Glu Ile Thr Ala
                   165                 170                 175
   Met Asn Gly Gly Val Ser Ile Gln Pro Trp Leu Ile Leu Leu Ala Phe
               180                 185                 190
   Phe Ser Ser Pro Ser Asn Gln Thr Asn Pro Asp Ser Leu Arg Asp Met
           195                 200                 205
   His Pro Gly Ser Trp Phe Gln Ile Leu Leu Val Leu Ala Met Phe Thr
       210                 215                 220
   Cys Ser Lys Gly Ser Val Leu Pro Pro Ser Glu Lys Val Asn Val
   225                 230                 235
<210> 90
   <211> 188
   <212> PRT
   <213> Zea mays
<220>
   <223> maize GRMZM2G154987_P01 protein
<400> 90
   Met Glu Pro His Met Glu Ser Ala Leu Arg Gln Gly Leu Ser Glu Ala
    1               5                  10                  15
   Glu Gln Arg Glu Leu Glu Gly Val Val Arg Ala His His Thr Phe Pro
               20                  25                  30
   Gly Arg Ala Pro Gly Thr Cys Thr Ser Leu Val Thr Gln Arg Val Asp
           35                  40                  45
   Ala Pro Leu Ala Ala Val Trp Pro Ile Val Arg Gly Phe Gly Ser Pro
       50                  55                  60
   Gln Arg Tyr Lys His Phe Ile Lys Ser Cys Asp Leu Lys Ala Gly Asp
   65                  70                  75                  80
   Gly Ala Thr Val Gly Ser Val Arg Glu Val Thr Val Val Ser Gly Leu
                   85                  90                  95
   Pro Ala Ser Thr Ser Thr Glu Arg Leu Glu Ile Leu Asp Asp His Arg
               100                 105                 110
   His Ile Leu Ser Phe Arg Val Val Gly Gly Asp His Arg Leu Arg Asn
           115                 120                 125
   Tyr Arg Ser Val Thr Ser Val Thr Glu Phe Gln Pro Gly Pro Tyr Cys
       130                 135                 140
   Val Val Leu Glu Ser Tyr Val Val Asp Val Pro Asp Gly Asn Thr Glu
   145                 150                 155                 160
   Glu Asp Thr Arg Met Phe Thr Asp Thr Val Val Lys Leu Asn Leu Gln
                   165                 170                 175
   Lys Leu Ala Ala Ile Ala Thr Ser Ser Ser Ala Asn
               180                 185
<210> 91
   <211> 205
   <212> PRT
   <213> Zea mays
<220>
   <223> maize GRMZM2G134731_P01 protein
<400> 91
   Met Asp Gln Gln Gly Ala Gly Gly Asp Val Glu Val Pro Ala Gly Leu
    1               5                  10                  15
   Gly Leu Thr Ala Ala Glu Tyr Glu Gln Leu Arg Pro Thr Val Asp Ala
               20                  25                  30
   His His Arg Tyr Ala Val Gly Glu Gly Gln Cys Ser Ser Leu Leu Ala
           35                  40                  45
   Gln Arg Ile His Ala Pro Pro Ala Ala Val Trp Ala Ile Val Arg Arg
       50                  55                  60
   Phe Asp Cys Pro Gln Val Tyr Lys His Phe Ile Arg Ser Cys Ala Val
   65                  70                  75                  80
   Arg Pro Asp Pro Asp Ala Gly Asp Ala Leu Arg Pro Gly Arg Leu Arg
                   85                  90                  95
   Glu Val Cys Val Ile Ser Gly Leu Pro Ala Ser Thr Ser Thr Glu Arg
               100                 105                 110
   Leu Asp His Leu Asp Asp Ala Ala Arg Val Phe Gly Phe Ser Ile Thr
           115                 120                 125
   Gly Gly Glu His Arg Leu Arg Asn Tyr Arg Ser Val Thr Thr Val Ser
       130                 135                 140
   Glu Leu Ala Gly Pro Gly Ile Cys Thr Val Val Leu Glu Ser Tyr Ala
   145                 150                 155                 160
   Val Asp Val Pro Asp Gly Asn Thr Glu Asp Asp Thr Arg Leu Phe Ala
                   165                 170                 175
   Asp Thr Val Ile Arg Leu Asn Leu Gln Lys Leu Lys Ser Val Ala Glu
               180                 185                 190
   Ala Ser Thr Ser Ser Ser Ala Pro Pro Pro Pro Ser Glu
           195                 200                 205
<210> 92
   <211> 220
   <212> PRT
   <213> Zea mays
<220>
   <223> maize GRMZM2G144224_P01 protein
<400> 92
   Met Pro Cys Ile Gln Ala Ser Ser Pro Gly Gly Met Pro His Gln His
    1               5                  10                  15
   Gly Arg Gly Arg Val Leu Gly Gly Gly Val Gly Cys Ala Ala Glu Val
               20                  25                  30
   Ala Ala Ala Val Ala Ala Ser Ala Gly Gly Met Arg Cys Gly Ala His
           35                  40                  45
   Asp Gly Glu Val Pro Ala Glu Ala Ala Arg His His Glu His Ala Ala
       50                  55                  60
   Ala Gly Pro Gly Arg Cys Cys Ser Ala Val Val Gln His Val Ala Ala
   65                  70                  75                  80
   Pro Ala Ala Ala Val Trp Ser Val Val Arg Arg Phe Asp Gln Pro Gln
                   85                  90                  95
   Val Tyr Lys Arg Phe Val Arg Ser Cys Ala Leu Leu Ala Gly Asp Gly
               100                 105                 110
   Gly Val Gly Thr Leu Arg Glu Val Arg Val Val Ser Gly Leu Pro Ala
           115                 120                 125
   Ala Ser Ser Arg Glu Arg Leu Glu Val Leu Asp Asp Glu Ser His Val
       130                 135                 140
   Leu Ser Phe Arg Val Val Gly Gly Glu His Arg Leu Arg Asn Tyr Leu
   145                 150                 155                 160
   Ser Val Thr Thr Val His Pro Ser Pro Ala Ala Pro Asp Ala Ala Thr
                   165                 170                 175
   Val Val Val Glu Ser Tyr Val Val Asp Val Pro Pro Gly Asn Thr Pro
               180                 185                 190
   Glu Asp Thr Arg Val Phe Val Asp Thr Ile Val Lys Cys Asn Leu Gln
           195                 200                 205
   Ser Leu Ala Thr Thr Ala Glu Lys Leu Ala Ala Val
       210                 215                 220
<210> 93
   <211> 221
   <212> PRT
   <213> Glycine max
<220>
   <223> soybean Glyma01g02290.1 protein
<400> 93
   Met Glu Lys Ala Glu Ser Ser Ala Ser Thr Ser Glu Pro Asp Ser Asp
    1              5                  10                  15
   Glu Asn His His Arg His Pro Thr Asn His His Ile Asn Pro Pro Ser
               20                  25                  30
   Gly Leu Thr Pro Leu Glu Phe Ala Ser Leu Ile Pro Ser Val Ala Glu
           35                  40                  45
   His His Ser Tyr Leu Val Gly Ser Gly Gln Cys Ser Ser Leu Leu Ala
       50                  55                  60
   Gln Arg Val Gln Ala Pro Pro Asp Ala Val Trp Ser Val Val Arg Arg
   65                  70                  75                  80
   Phe Asp Lys Pro Gln Thr Tyr Lys His Phe Ile Lys Ser Cys Ala Val
                   85                  90                  95
   Lys Glu Pro Phe His Met Ala Val Gly Val Thr Arg Asp Val Asn Val
               100                 105                 110
   Ile Ser Gly Leu Pro Ala Ala Thr Ser Thr Glu Arg Leu Asp Leu Leu
           115                 120                 125
   Asp Asp Ile Arg Cys Val Thr Gly Phe Ser Ile Ile Gly Gly Glu His
       130                 135                 140
   Arg Leu Arg Asn Tyr Arg Ser Val Thr Thr Val His Ser Phe Glu Asp
   145                 150                 155                 160
   Asp Ala Asp Asp Gly Lys Ile Tyr Thr Val Val Leu Glu Ser Tyr Val
                   165                 170                 175
   Val Asp Val Pro Asp Gly Asn Thr Glu Glu Asp Thr Arg Leu Phe Ala
               180                 185                 190
   Asp Thr Val Val Lys Leu Asn Leu Gln Lys Leu Ala Ser Val Thr Glu
           195                 200                 205
   Gly Thr Asn Arg Asp Gly Asp Gly Lys Ser His Ser Arg
       210                 215                 220
<210> 94
   <211> 214
   <212> PRT
   <213> Glycine max
<220>
   <223> soybean Glyma01g12970.1 protein
<400> 94
   Met Glu Lys Thr His Ser Ser Ser Ala Glu Glu Gln Asp Pro Thr Arg
    1               5                  10                  15
   Arg His Leu Asp Pro Pro Pro Gly Leu Thr Ala Glu Glu Phe Glu Asp
               20                  25                  30
   Leu Lys Pro Ser Val Leu Glu His His Thr Tyr Ser Val Thr Pro Thr
           35                  40                  45
   Arg Gln Ser Ser Ser Leu Leu Ala Gln Arg Ile His Ala Pro Pro His
       50                  55                  60
   Ala Val Trp Ser Val Val Arg Cys Phe Asp Asn Pro Gln Ala Tyr Lys
   65                  70                  75                  80
   His Phe Ile Lys Ser Cys His Val Lys Glu Gly Phe Gln Leu Ala Val
                   85                  90                  95
   Gly Ser Thr Arg Asp Val His Val Ile Ser Gly Leu Pro Ala Ala Thr
               100                 105                 110
   Ser Thr Glu Arg Leu Asp Leu Leu Asp Asp Asp Arg His Val Ile Gly
           115                 120                 125
   Phe Thr Ile Val Gly Gly Asp His Arg Leu Arg Asn Tyr Arg Ser Val
       130                 135                 140
   Thr Ser Val His Gly Phe Glu Cys Asp Gly Lys Ile Trp Thr Val Val
   145                 150                 155                 160
   Leu Glu Ser Tyr Val Val Asp Val Pro Glu Gly Asn Thr Glu Glu Asp
                   165                 170                 175
   Thr Arg Leu Phe Ala Asp Thr Val Val Lys Leu Asn Leu Gln Lys Leu
               180                 185                 190
   Ala Ser Val Ser Glu Gly Met Cys Gly Asp Gly Asp Gly Asp Gly Asp
           195                 200                 205
   Gly Lys Gly Asn Lys Ser
       210
<210> 95
   <211> 216
   <212> PRT
   <213> Glycine max
<220>
   <223> soybean Glyma01g31320.1 protein
<400> 95
   Met Leu Gln Asn Ser Ser Met Ser Ser Leu Leu Leu His Arg Ile Asn
    1               5                  10                  15
   Gly Gly Gly Gly Ala Thr Thr Ala Thr Asn Cys His Asp Thr Val Phe
               20                  25                  30
   Met Thr Val Pro Asp Gly Val Ala Arg Tyr His Thr His Ala Val Ala
           35                  40                  45
   Pro Asn Gln Cys Cys Ser Ser Val Ala Gln Glu Ile Gly Ala Ser Val
       50                  55                  60
   Ala Thr Val Trp Ser Val Leu Arg Arg Phe Asp Asn Pro Gln Ala Tyr
   65                  70                  75                  80
   Lys His Phe Val Lys Ser Cys His Val Ile Gly Gly Asp Gly Asp Val
                   85                  90                  95
   Gly Thr Leu Arg Glu Val His Val Ile Ser Gly Leu Pro Ala Ala Arg
               100                 105                 110
   Ser Thr Glu Arg Leu Glu Ile Leu Asp Asp Glu Arg His Val Ile Ser
           115                 120                 125
   Phe Ser Val Val Gly Gly Asp His Arg Leu Ala Asn Tyr Arg Ser Val
       130                 135                 140
   Thr Thr Leu His Pro Thr Ala Ser Ser Ala Ser Gly Gly Cys Ser Gly
   145                 150                 155                 160
   Thr Val Val Val Glu Ser Tyr Val Val Asp Val Pro Pro Gly Asn Thr
                   165                 170                 175
   Arg Glu Asp Thr Arg Val Phe Val Asp Thr Ile Val Lys Cys Asn Leu
               180                 185                 190
   Gln Ser Leu Ala Gln Thr Ala Glu Asn Leu Thr Leu Arg Lys Asn Asn
           195                 200                 205
   Asn Asn Asp Tyr Lys Cys Cys Ser
       210                 215
<210> 96
   <211> 208
   <212> PRT
   <213> Glycine max
<220>
   <223> soybean Glyma02g42990.1 protein
<400> 96
   Met Thr Ser Leu Gln Phe His Arg Phe Asn Pro Ala Thr Asp Thr Ser
    1               5                  10                  15
   Thr Ala Ile Ala Asn Gly Val Asn Cys Pro Lys Pro Pro Ser Thr Leu
               20                  25                  30
   Arg Leu Leu Ala Lys Val Ser Leu Ser Val Pro Glu Thr Val Ala Arg
           35                  40                  45
   His His Ala His Pro Val Gly Pro Asn Gln Cys Cys Ser Val Val Ile
       50                  55                  60
   Gln Ala Ile Asp Ala Pro Val Ser Ala Val Trp Pro Val Val Arg Arg
   65                  70                  75                  80
   Phe Asp Asn Pro Gln Ala Tyr Lys His Phe Val Lys Ser Cys His Val
                   85                  90                  95
   Val Ala Ala Ala Gly Gly Gly Glu Asp Gly Ile Arg Val Gly Ala Leu
               100                 105                 110
   Arg Glu Val Arg Val Val Ser Gly Leu Pro Ala Val Ser Ser Thr Glu
           115                 120                 125
   Arg Leu Glu Ile Leu Asp Asp Glu Arg His Val Met Ser Phe Ser Val
       130                 135                 140
   Val Gly Gly Asp His Arg Leu Arg Asn Tyr Arg Ser Val Thr Thr Leu
   145                 150                 155                 160
   His Gly Asp Gly Asn Gly Gly Thr Val Val Ile Glu Ser Tyr Val Val
                   165                 170                 175
   Asp Val Pro Pro Gly Asn Thr Lys Glu Glu Thr Cys Val Phe Val Asp
               180                 185                 190
   Thr Ile Val Arg Cys Asn Leu Gln Ser Leu Ala Gln Ile Ala Glu Thr
           195                 200                 205
<210> 97
   <211> 176
   <212> PRT
   <213> Glycine max
<220>
   <223> soybean Glyma04g05380.1 protein
<400> 97
   Ala Tyr Pro Val Leu Gly Leu Thr Pro Glu Glu Phe Ser Glu Leu Glu
    1               5                  10                  15
   Ser Ile Ile Asn Thr His His Lys Phe Glu Pro Ser Pro Glu Ile Cys
               20                  25                  30
   Ser Ser Ile Ile Ala Gln Arg Ile Asp Ala Pro Ala His Thr Val Trp
           35                  40                  45
   Pro Leu Val Arg Ser Phe Glu Asn Pro Gln Lys Tyr Lys His Phe Val
       50                  55                  60
   Lys Ser Cys Asn Met Arg Ser Gly Asp Gly Gly Val Gly Ser Ile Arg
   65                  70                  75                  80
   Glu Val Thr Val Val Ser Gly Leu Pro Ala Ser Thr Ser Thr Glu Arg
                   85                  90                  95
   Leu Glu Ile Leu Asp Asp Asp Lys His Leu Leu Ser Phe Arg Val Val
               100                 105                 110
   Gly Gly Glu His Arg Leu His Asn Tyr Arg Ser Val Thr Ser Val Asn
           115                 120                 125
   Glu Phe Lys Asn Pro Asp Asn Gly Lys Val Tyr Thr Ile Val Leu Glu
       130                 135                 140
   Ser Tyr Val Val Asp Ile Pro Glu Gly Asn Thr Gly Val Asp Thr Lys
   145                 150                 155                 160
   Met Phe Val Asp Thr Val Val Lys Leu Asn Leu Gln Lys Leu Gly Glu
                   165                 170                 175
<210> 98
   <211> 172
   <212> PRT
   <213> Glycine max
<220>
   <223> soybean Glyma06g05440.1 protein
<400> 98
   Glu Phe Thr Glu Leu Glu Ser Thr Ile Asn Thr His His Lys Phe Glu
    1               5                  10                  15
   Ala Ser Pro Glu Ile Cys Ser Ser Ile Ile Ala Gln Arg Ile Asp Ala
               20                  25                  30
   Pro Ala His Thr Val Trp Pro Leu Val Arg Ser Phe Glu Asn Pro Gln
           35                  40                  45
   Lys Tyr Lys His Phe Val Lys Ser Cys Asn Met Arg Ser Gly Asp Gly
       50                  55                  60
   Gly Val Gly Ser Ile Arg Glu Val Thr Val Val Ser Gly Leu Pro Ala
   65                  70                  75                  80
   Ser Thr Ser Thr Glu Arg Leu Glu Ile Leu Asp Asp Asp Asn His Leu
                   85                  90                  95
   Leu Ser Phe Arg Val Val Gly Gly Glu His Arg Leu His Asn Tyr Arg
               100                 105                 110
   Ser Val Thr Ser Val Asn Glu Phe Lys Arg Pro Asp Asn Gly Lys Val
           115                 120                 125
   Tyr Thr Ile Val Leu Glu Ser Tyr Val Val Asp Ile Pro Glu Gly Asn
       130                 135                 140
   Thr Gly Val Asp Thr Lys Met Phe Val Asp Thr Val Val Lys Leu Asn
   145                 150                 155                 160
   Leu Gln Lys Leu Gly Glu Val Ala Met Ala Thr Asn
                   165                 170
<210> 99
   <211> 191
   <212> PRT
   <213> Glycine max
<220>
   <223> soybean Glyma06g13150.1 protein
<400> 99
   Met Thr Glu Leu Ser Ser Arg Glu Val Glu Tyr Ile Arg Arg His His
    1               5                  10                  15
   Ser Lys Ala Ala Glu Asp Asn Gln Cys Ala Ser Ala Leu Val Lys His
               20                  25                  30
   Ile Arg Ala Pro Leu Pro Leu Val Trp Ser Leu Val Arg Arg Phe Asp
           35                  40                  45
   Glu Pro Gln Lys Tyr Lys Pro Phe Val Ser Arg Cys Val Val Arg Gly
       50                  55                  60
   Asn Leu Glu Ile Gly Ser Leu Arg Glu Val Asp Val Lys Ser Gly Leu
   65                  70                  75                  80
   Pro Ala Thr Thr Ser Thr Glu Arg Leu Glu Ile Leu Asp Asp Asn His
                   85                  90                  95
   His Ile Leu Ser Val Arg Ile Ile Gly Gly Asp His Arg Leu Arg Asn
               100                 105                 110
   Tyr Ser Ser Ile Met Ser Leu His Pro Glu Ile Val Asp Gly Arg Pro
           115                 120                 125
   Gly Thr Leu Val Ile Glu Ser Phe Val Val Asp Ile Pro Glu Gly Asn
       130                 135                 140
   Thr Lys Asp Glu Thr Cys Tyr Phe Val Glu Ala Leu Ile Lys Cys Asn
   145                 150                 155                 160
   Leu Lys Ser Leu Ala Asp Val Ser Glu Gly Leu Thr Leu Gln Asp His
                   165                 170                 175
   Thr Glu Pro Ile Asp Arg Lys Tyr Glu Leu Leu Ile Thr Arg Gly
               180                 185                 190
<210> 100
   <211> 185
   <212> PRT
   <213> Glycine max
<220>
   <223> soybean Glyma07g06270.1 protein
<400> 100
   Met Asn Gly Gly Glu Ser Tyr Gly Ala Ile Glu Thr Gln Tyr Ile Arg
    1               5                  10                  15
   Arg His His Lys His Glu Pro Arg Glu Asn Gln Cys Thr Ser Ala Leu
               20                  25                  30
   Val Lys His Ile Arg Ala Pro Val His Leu Val Trp Ser Leu Val Arg
           35                  40                  45
   Arg Phe Asp Gln Pro Gln Lys Tyr Lys Pro Phe Val Ser Arg Cys Ile
       50                  55                  60
   Met Gln Gly Asp Leu Gly Ile Gly Ser Val Arg Glu Val Asn Val Lys
   65                  70                  75                  80
   Ser Gly Leu Pro Ala Thr Thr Ser Thr Glu Arg Leu Glu Gln Leu Asp
                   85                  90                  95
   Asp Glu Glu His Ile Leu Gly Ile Arg Ile Val Gly Gly Asp His Arg
               100                 105                 110
   Leu Arg Asn Tyr Ser Ser Ile Ile Thr Val His Pro Glu Val Ile Asp
           115                 120                 125
   Gly Arg Pro Gly Thr Met Val Ile Glu Ser Phe Val Val Asp Val Pro
       130                 135                 140
   Asp Gly Asn Thr Arg Asp Glu Thr Cys Tyr Phe Val Glu Ala Leu Ile
   145                 150                 155                 160
   Arg Cys Asn Leu Ser Ser Leu Ala Asp Val Ser Glu Arg Met Ala Val
                   165                 170                 175
   Gln Gly Arg Thr Asn Pro Ile Asn His
               180                 185
<210> 101
   <211> 178
   <212> PRT
   <213> Glycine max
<220>
   <223> soybean Glyma07g19120.1 protein
<400> 101
   Met Ser Pro Asn Asn Pro Ser Thr Ile Val Ser Asp Ala Val Ala Arg
    1               5                  10                  15
   His His Thr His Val Val Ser Pro His Gln Cys Cys Ser Ala Val Val
               20                  25                  30
   Gln Glu Ile Ala Ala Pro Val Ser Thr Val Trp Ser Val Val Arg Arg
           35                  40                  45
   Phe Asp Asn Pro Gln Ala Tyr Lys His Phe Val Lys Ser Cys His Val
       50                  55                  60
   Ile Leu Gly Asp Gly Asp Val Gly Thr Leu Arg Glu Val Arg Val Ile
   65                  70                  75                  80
   Ser Gly Leu Pro Ala Ala Val Ser Thr Glu Arg Leu Asp Val Leu Asp
                   85                  90                  95
   Asp Glu Arg His Val Ile Gly Phe Ser Met Val Gly Gly Asp His Arg
               100                 105                 110
   Leu Ser Asn Tyr Arg Ser Val Thr Ile Leu His Pro Arg Ser Ala Thr
           115                 120                 125
   Asp Thr Val Val Val Glu Ser Tyr Val Val Asp Val Pro Ala Gly Asn
       130                 135                 140
   Thr Thr Glu Asp Thr Arg Val Phe Val Asp Thr Ile Leu Arg Cys Asn
   145                 150                 155                 160
   Leu Gln Ser Leu Ala Lys Phe Ala Glu Asn Leu Thr Asn Lys Leu His
                   165                 170                 175
   Gln Arg
<210> 102
   <211> 246
   <212> PRT
   <213> Glycine max
<220>
   <223> soybean Glyma08g36770.1 protein
<400> 102
   Met Ser Arg Ser His Asn Lys Arg Lys Pro Phe Ser Phe Ile Phe Lys
    1               5                  10                  15
   Ile Thr Leu Leu Glu Leu Leu Ser Ser Leu Leu Ser Ser Ser Leu Arg
               20                  25                  30
   Phe Ala Met Asp Lys Thr His Ser Gly Glu Glu Gln Asp Pro Asn Pro
           35                  40                  45
   Thr His Pro Thr Arg Asn His Leu Asp Pro Pro Pro Gly Leu Thr Pro
       50                  55                  60
   Glu Glu Phe Glu Asp Leu Lys Pro Ser Val Leu Glu His His Thr Tyr
   65                  70                  75                  80
   Ser Val Thr Pro Thr Arg Gln Cys Ser Ser Leu Leu Ala Gln Arg Ile
                   85                  90                  95
   His Ala Pro Pro His Thr Val Trp Thr Val Val Arg Cys Phe Asp Asn
               100                 105                 110
   Pro Gln Ala Tyr Lys His Phe Ile Lys Ser Cys His Val Lys Glu Gly
           115                 120                 125
   Phe Gln Leu Ala Val Gly Ser Thr Arg Asp Val His Val Ile Ser Gly
       130                 135                 140
   Leu Pro Ala Ala Thr Ser Thr Glu Arg Leu Asp Leu Leu Asp Asp Asp
   145                 150                 155                 160
   Arg His Val Ile Gly Phe Thr Ile Val Gly Gly Asp His Arg Leu Arg
                   165                 170                 175
   Asn Tyr Arg Ser Val Thr Ser Val His Gly Phe Glu Arg Asp Gly Lys
               180                 185                 190
   Ile Trp Thr Val Val Leu Glu Ser Tyr Val Val Asp Val Pro Glu Gly
           195                 200                 205
   Asn Thr Glu Glu Asp Thr Arg Leu Phe Ala Asp Thr Val Val Lys Leu
       210                 215                 220
   Asn Leu Gln Lys Leu Ala Ser Val Thr Glu Gly Met Cys Gly Asp Ser
   225                 230                 235                 240
   Asp Gly Lys Gly Asn Asn
                   245
<210> 103
   <211> 223
   <212> PRT
   <213> Glycine max
<220>
   <223> soybean Glyma09g33700.1 protein
<400> 103
   Met Glu Lys Ala Glu Ser Ser Ala Ser Thr Ser Glu Pro Asp Ser Asp
   1 5 10 15
   Asp Asn His His Arg His Pro Thr Asn His His Leu Asn Pro Pro Ser
   20 25 30
   Gly Leu Thr Pro Leu Glu Phe Ala Ser Leu Val Pro Ser Val Ala Glu
   35 40 45
   His His Ser Tyr Leu Val Gly Pro Gly Gln Cys Ser Ser Leu Leu Ala
   50 55 60
   Gln Arg Val His Ala Pro Pro Asp Ala Val Trp Ser Phe Val Arg Arg
   65 70 75 80
   Phe Asp Lys Pro Gln Thr Tyr Lys His Phe Ile Lys Ser Cys Ala Val
   85 90 95
   Lys Glu Pro Phe His Met Ala Val Gly Val Thr Arg Asp Val Asn Val
   100 105 110
   Ile Ser Gly Leu Pro Ala Ala Thr Ser Thr Glu Arg Leu Asp Phe Leu
   115 120 125
   Asp Asp Val Arg Arg Val Thr Gly Phe Ser Ile Ile Gly Gly Glu His
   130 135 140
   Arg Leu Arg Asn Tyr Arg Ser Val Thr Thr Val His Ser Phe Asp Asp
   145 150 155 160
   Asp Asn Ala Ser Ala Asp Gly Lys Ile Tyr Thr Val Val Leu Glu Ser
   165 170 175
   Tyr Val Val Asp Val Pro Asp Gly Asn Thr Glu Glu Asp Thr Arg Leu
   180 185 190
   Phe Ala Asp Thr Val Val Lys Leu Asn Leu Gln Lys Leu Ala Ser Val
   195 200 205
   Thr Glu Gly Thr Asn Gly Asp Gly Asp Gly Lys Pro His Ser Arg
   210 215 220
<210> 104
   <211> 229
   <212> PRT
   <213> Glycine max
<220>
   <223> soybean Glyma11g35670.1 protein
<400> 104
   Met Pro Ser Ser Leu His Phe Asp Arg Phe Asn Pro Ile Thr His Ala
   1 5 10 15
   Ala Thr Thr Val Ala Ile Ala Asn Gly Val Asn Cys Pro Lys Gln Pro
   20 25 30
   Gln Ala Pro Pro Ser Ser Thr Ala Ala Arg Arg Leu Val Val Pro Ser
   35 40 45
   Leu Ser Ser Gly Arg Gly Ile Ala Ala Pro Asp Thr Val Ala Leu His
   50 55 60
   His Ala His Val Val Asp Pro Asn Gln Cys Cys Ser Ile Val Thr Gln
   65 70 75 80
   His Ile Asn Ala Pro Val Ser Ala Val Trp Ala Val Val Arg Arg Phe
   85 90 95
   Asp Asn Pro Gln Gly Tyr Lys Asn Phe Val Arg Ser Cys His Val Ile
   100 105 110
   Thr Gly Asp Gly Ile Arg Val Gly Ala Val Arg Glu Val Arg Val Val
           115                 120                 125
   Ser Gly Leu Pro Ala Glu Thr Ser Thr Glu Arg Leu Glu Ile Leu Asp
       130                 135                 140
   Asp Glu Arg His Val Ile Ser Phe Ser Met Val Gly Gly Asp His Arg
   145                 150                 155                 160
   Leu Arg Asn Tyr Gln Ser Val Thr Thr Leu His Ala Asn Gly Asn Gly
                   165                 170                 175
   Thr Leu Val Ile Glu Ser Tyr Val Val Asp Val Pro Gln Gly Asn Thr
               180                 185                 190
   Lys Glu Glu Thr Cys Val Phe Val Asp Thr Ile Val Arg Cys Asn Leu
           195                 200                 205
   Gln Ser Leu Ala Gln Ile Ala Glu Asn Arg Thr Asn Asn Cys Glu His
       210                 215                 220
   Thr Ala Gln His Cys
   225
<210> 105
   <211> 191
   <212> PRT
   <213> Glycine max
<220>
   <223> soybean Glyma13g08120.1 protein
<400> 105
   Met Asn Gly Ile Gly Asn Asp Gly Gly Gly Gly Leu Ser Asn Val Glu
    1               5                  10                  15
   Met Glu Tyr Ile Arg Arg His His Arg His Glu Pro Gly Glu Asn Gln
               20                  25                  30
   Cys Gly Ser Ala Leu Val Lys His Ile Arg Ala Pro Val Pro Gln Val
           35                  40                  45
   Trp Ser Leu Val Arg Arg Phe Asp Gln Pro Gln Lys Tyr Lys Pro Phe
       50                  55                  60
   Val Ser Arg Cys Val Val Arg Gly Asn Leu Glu Ile Gly Ser Leu Arg
   65                  70                  75                  80
   Glu Val Asp Val Lys Ser Gly Leu Pro Ala Thr Thr Ser Thr Glu Arg
                   85                  90                  95
   Leu Glu Leu Leu Asp Asp Asn Glu His Leu Leu Ser Ile Arg Ile Ile
               100                 105                 110
   Gly Gly Asp His Arg Leu Arg Asn Tyr Ser Ser Ile Met Ser Leu His
           115                 120                 125
   Pro Glu Ile Ile Asp Gly Arg Pro Gly Thr Leu Val Ile Glu Ser Phe
       130                 135                 140
   Val Val Asp Val Pro Glu Gly Asn Thr Lys Asp Glu Thr Cys Tyr Phe
   145                 150                 155                 160
   Val Glu Ala Leu Ile Lys Cys Asn Leu Lys Ser Leu Ala Asp Val Ser
                   165                 170                 175
   Glu Gly Ile Ala Val Gln Asp Arg Thr Glu Pro Ile Asp Arg Ile
               180                 185                 190
<210> 106
   <211> 169
   <212> PRT
   <213> Glycine max
<220>
   <223> soybean Glyma14g06100.1 protein
<400> 106
   Met Val Ala Arg His His Ala His Ala Val Gly Pro Asn Gln Cys Cys
    1               5                  10                  15
   Ser Phe Val Ile Gln Ala Ile Asp Ala Pro Val Ser Ala Val Trp Pro
               20                  25                  30
   Val Val Arg Arg Phe Asp Asn Pro Gln Ala Tyr Lys His Phe Val Lys
           35                  40                  45
   Ser Cys His Val Val Ala Ala Gly Gly Ala Gly Gly Asp Gly Gly Ile
       50                  55                  60
   His Val Gly Ala Leu Arg Glu Val Arg Val Val Ser Gly Leu Pro Ala
   65                  70                  75                  80
   Val Ser Ser Thr Glu Arg Leu Glu Ile Leu Asp Asp Glu Arg His Val
                   85                  90                  95
   Met Ser Phe Ser Val Val Gly Gly Asp His Arg Leu Arg Asn Tyr Arg
               100                 105                 110
   Ser Val Thr Thr Leu His Gly Asp Gly Ser Asn Gly Gly Thr Val Val
           115                 120                 125
   Ile Glu Ser Tyr Val Val Asp Ile Pro Ala Gly Asn Thr Lys Glu Glu
       130                 135                 140
   Thr Cys Val Phe Val Asp Thr Ile Val Arg Cys Asn Leu Gln Ser Leu
   145                 150                 155                 160
   Ala Gln Met Ala Glu Asn Met Gly Ser
                   165
<210> 107
   <211> 210
   <212> PRT
   <213> Glycine max
<220>
   <223> soybean Glyma14g10730.1 protein
<400> 107
   Met Thr Ile Leu Pro His Ser Asn Asn Lys Ser Ser Asn His Lys Phe
    1               5                  10                  15
   Ile Ala His Gln Asn Tyr Met Ala Ser Glu Thr His His His Val Gln
               20                  25                  30
   Gly Leu Thr Pro Glu Glu Leu Thr Lys Leu Glu Pro Ile Ile Lys Lys
           35                  40                  45
   Tyr His Leu Phe Glu Gln Ser Pro Asn Thr Cys Phe Ser Ile Ile Thr
       50                  55                  60
   Tyr Arg Ile Glu Ala Pro Ala Lys Ala Val Trp Pro Phe Val Arg Ser
   65                  70                  75                  80
   Phe Asp Asn Pro Gln Lys Tyr Lys His Phe Ile Lys Gly Cys Asn Met
                   85                  90                  95
   Arg Gly Asp Gly Gly Val Gly Ser Ile Arg Glu Val Thr Val Val Ser
               100                 105                 110
   Gly Leu Pro Ala Ser Thr Ser Thr Glu Arg Leu Glu Ile Leu Asp Asp
           115                 120                 125
   Asp Lys His Val Leu Ser Phe Arg Val Val Gly Gly Glu His Arg Leu
       130                 135                 140
   Lys Asn Tyr Arg Ser Val Thr Ser Val Asn Glu Phe Asn Lys Glu Gly
   145                 150                 155                 160
   Lys Val Tyr Thr Ile Val Leu Glu Ser Tyr Ile Val Asp Ile Pro Glu
                   165                 170                 175
   Gly Asn Thr Glu Glu Asp Thr Lys Met Phe Val Asp Thr Val Val Lys
               180                 185                 190
   Leu Asn Leu Gln Lys Leu Gly Val Val Ala Met Ala Ser Ser Met His
           195                 200                 205
   Gly Gln
       210
<210> 108
   <211> 193
   <212> PRT
   <213> Glycine max
<220>
   <223> soybean Glyma14g30260.1 protein
<400> 108
   Met Asn Arg Ile Gly Asn Gly Gly Gly Gly Gly Gly Gly Leu Ser Asn
    1               5                  10                  15
   Val Glu Met Glu Tyr Ile Arg Arg His His Arg His Glu Pro Gly Glu
               20                  25                  30
   Asn Gln Cys Gly Ser Ala Leu Val Lys His Ile Arg Ala Pro Val Pro
           35                  40                  45
   Gln Val Trp Ser Leu Val Arg Arg Phe Asp Gln Pro Gln Lys Tyr Lys
       50                  55                  60
   Pro Phe Ile Ser Arg Cys Val Val Arg Gly Asn Leu Glu Ile Gly Ser
   65                  70                  75                  80
   Leu Arg Glu Val Asp Val Lys Ser Gly Leu Pro Ala Thr Thr Ser Thr
                   85                  90                  95
   Glu Arg Leu Glu Leu Leu Asp Asp Asn Glu His Ile Leu Ser Ile Arg
               100                 105                 110
   Ile Ile Gly Gly Asp His Arg Leu Arg Asn Tyr Ser Ser Ile Met Ser
           115                 120                 125
   Leu His Pro Glu Ile Ile Asp Gly Arg Pro Gly Thr Leu Val Ile Glu
       130                 135                 140
   Ser Phe Val Val Asp Val Pro Glu Gly Asn Thr Lys Asp Glu Thr Cys
   145                 150                 155                 160
   Tyr Phe Val Glu Ala Leu Ile Lys Cys Asn Leu Lys Ser Leu Ala Asp
                   165                 170                 175
   Val Ser Glu Gly Leu Ala Val Gln Asp Cys Thr Glu Pro Ile Asp Arg
               180                 185                 190
   Ile
<210> 109
   <211> 188
   <212> PRT
   <213> Glycine max
<220>
   <223> soybean Glyma17g34800.1 protein
<400> 109
   Met Ala Ser Glu Thr His His His Val Gln Gly Leu Thr Pro Glu Glu
    1               5                  10                  15
   Leu Thr Gln Leu Glu Pro Ile Ile Lys Lys Tyr His Leu Phe Glu Ala
               20                  25                  30
   Ser Ser Asn Lys Cys Phe Ser Ile Ile Thr His Arg Ile Glu Ala Pro
           35                  40                  45
   Ala Ser Ser Val Trp Pro Leu Val Arg Asn Phe Asp Asn Pro Gln Lys
       50                  55                  60
   Tyr Lys His Phe Ile Lys Gly Cys Asn Met Lys Gly Asp Gly Ser Val
   65                  70                  75                  80
   Gly Ser Ile Arg Glu Val Thr Val Val Ser Gly Leu Pro Ala Ser Thr
                   85                  90                  95
   Ser Thr Glu Arg Leu Glu Ile Leu Asp Asp Asp Lys His Val Leu Ser
               100                 105                 110
   Phe Arg Val Val Gly Gly Glu His Arg Leu Gln Asn Tyr Arg Ser Val
           115                 120                 125
   Thr Ser Val Asn Glu Phe His Lys Glu Gly Lys Val Tyr Thr Ile Val
       130                 135                 140
   Leu Glu Ser Tyr Ile Val Asp Ile Pro Glu Gly Asn Thr Glu Glu Asp
   145                 150                 155                 160
   Thr Lys Met Phe Val Asp Thr Val Val Lys Leu Asn Leu Gln Lys Leu
                   165                 170                 175
   Gly Val Val Ala Met Ala Ser Ser Met Asn Gly Arg
               180                 185
<210> 110
   <211> 177
   <212> PRT
   <213> Glycine max
<220>
   <223> soybean Glyma18g43680.1 protein
<400> 110
   Met Leu Pro Asn Asn Pro Ser Thr Ile Val Pro Asp Ala Val Ala Arg
    1               5                  10                  15
   His His Thr His Val Val Ser Pro Gln Gln Cys Cys Ser Ala Val Val
               20                  25                  30
   Gln Glu Ile Ala Ala Pro Val Ser Thr Val Trp Ser Val Val Arg Arg
           35                  40                  45
   Phe Asp Asn Pro Gln Ala Tyr Lys His Phe Val Lys Ser Cys His Val
       50                  55                  60
   Ile Leu Gly Asp Gly Asp Val Gly Thr Leu Arg Glu Val His Val Ile
   65                  70                  75                  80
   Ser Gly Leu Pro Ala Ala Val Ser Thr Glu Arg Leu Asp Val Leu Asp
                   85                  90                  95
   Asp Glu Arg His Val Ile Gly Phe Ser Met Val Gly Gly Asp His Arg
               100                 105                 110
   Leu Phe Asn Tyr Arg Ser Val Thr Thr Leu His Pro Arg Ser Ala Ala
           115                 120                 125
   Gly Thr Val Val Val Glu Ser Tyr Val Val Asp Val Pro Pro Gly Asn
       130                 135                 140
   Thr Thr Glu Asp Thr Arg Val Phe Val Asp Thr Ile Leu Arg Cys Asn
   145                 150                 155                 160
   Leu Gln Ser Leu Ala Lys Phe Ala Glu Asn Leu Thr Lys Leu His Gln
                   165                 170                 175
   Arg
<210> 111
   <211> 185
   <212> PRT
   <213> Glycine max
<220>
   <223> soybean Glyma07g06270.2 protein
<400> 111
   Met Asn Gly Gly Glu Ser Tyr Gly Ala Ile Glu Thr Gln Tyr Ile Arg
   1 5 10 15
   Arg His His Lys His Glu Pro Arg Glu Asn Gln Cys Thr Ser Ala Leu
   20 25 30
   Val Lys His Ile Arg Ala Pro Val His Leu Val Trp Ser Leu Val Arg
   35 40 45
   Arg Phe Asp Gln Pro Gln Lys Tyr Lys Pro Phe Val Ser Arg Cys Ile
   50 55 60
   Met Gln Gly Asp Leu Gly Ile Gly Ser Val Arg Glu Val Asn Val Lys
   65 70 75 80
   Ser Gly Leu Pro Ala Thr Thr Ser Thr Glu Arg Leu Glu Gln Leu Asp
   85 90 95
   Asp Glu Glu His Ile Leu Gly Ile Arg Ile Val Gly Gly Asp His Arg
   100 105 110
   Leu Arg Asn Tyr Ser Ser Ile Ile Thr Val His Pro Glu Val Ile Asp
   115 120 125
   Gly Arg Pro Gly Thr Met Val Ile Glu Ser Phe Val Val Asp Val Pro
   130 135 140
   Asp Gly Asn Thr Arg Asp Glu Thr Cys Tyr Phe Val Glu Ala Leu Ile
   145 150 155 160
   Arg Cys Asn Leu Ser Ser Leu Ala Asp Val Ser Glu Arg Met Ala Val
   165 170 175
   Gln Gly Arg Thr Asn Pro Ile Asn His
   180 185
<210> 112
   <211> 191
   <212> PRT
   <213> Glycine max
<220>
   <223> soybean Glyma16g02910.1 protein
<400> 112
   Met Gly Ile Thr Ile Gly Ile Gln Cys Leu Glu Ile Glu Glu Ile Ser
   1 5 10 15
   Ile Cys Asp Gly Met Phe Cys Tyr Leu Val Asp Phe Val Asp Val Lys
   20 25 30
   Glu Lys Met Asn Tyr Cys Leu Met Trp Phe Gly Tyr Phe Pro Ser Gln
   35 40 45
   Val Trp Ser Leu Val Arg Arg Phe Asp Gln Pro Gln Lys Tyr Lys Pro
   50 55 60
   Phe Val Ser Arg Cys Ile Met Gln Gly Asp Leu Gly Ile Gly Ser Val
   65 70 75 80
   Arg Glu Val Asn Val Lys Ser Gly Leu Pro Ala Thr Thr Ser Thr Glu
   85 90 95
   Arg Leu Glu Gln Leu Asp Asp Glu Glu His Ile Leu Gly Ile Arg Ile
   100 105 110
   Val Gly Gly Asp His Arg Leu Arg Asn Tyr Ser Ser Ile Ile Thr Val
   115 120 125
   His Pro Glu Val Ile Asp Gly Arg Pro Ser Thr Met Val Ile Glu Ser
   130 135 140
   Phe Val Val Asp Val Pro Asp Gly Asn Thr Arg Asp Glu Thr Cys Tyr
   145 150 155 160
   Phe Val Glu Ala Leu Ile Arg Cys Asn Leu Ser Ser Leu Ala Asp Val
   165 170 175
   Ser Glu Arg Met Ala Val Gln Gly Arg Thr Asp Pro Ile Asn His
   180 185 190
<210> 113
   <211> 185
   <212> PRT
   <213> Artificial Sequence
<220>
   <223> synthetic PYR/PYL receptor protein
<400> 113
   Met Asn Gly Gly Glu Ser Tyr Gly Ala Ile Glu Thr Gln Tyr Ile Arg
    1               5                  10                  15
   Arg His His Lys His Glu Pro Arg Glu Asn Gln Cys Thr Ser Ala Leu
               20                  25                  30
   Val Lys His Ile Arg Ala Pro Val His Leu Val Trp Ser Leu Val Arg
           35                  40                  45
   Arg Phe Asp Gln Pro Gln Lys Tyr Lys Pro Phe Val Ser Arg Cys Ile
       50                  55                  60
   Met Gln Gly Asp Leu Gly Ile Gly Ser Val Arg Glu Val Asn Val Lys
   65                  70                  75                  80
   Ser Gly Leu Pro Ala Thr Thr Ser Thr Glu Arg Leu Glu Gln Leu Asp
                   85                  90                  95
   Asp Glu Glu His Ile Leu Gly Ile Arg Ile Val Gly Gly Asp His Arg
               100                 105                 110
   Leu Arg Asn Tyr Ser Ser Ile Ile Thr Val His Pro Glu Val Ile Asp
           115                 120                 125
   Gly Arg Pro Ser Thr Met Val Ile Glu Ser Phe Val Val Asp Val Pro
       130                 135                 140
   Asp Gly Asn Thr Arg Asp Glu Thr Cys Tyr Phe Val Glu Ala Leu Ile
   145                 150                 155                 160
   Arg Cys Asn Leu Ser Ser Leu Ala Asp Val Ser Glu Arg Met Ala Val
                   165                 170                 175
   Gln Gly Arg Thr Asp Pro Ile Asn His
               180                 185
<210> 114
   <211> 204
   <212> PRT
   <213> Sorghum bicolor
<220>
   <223> sorghum Sb10g022200 protein
<400> 114
   Met Glu Thr His Val Glu Arg Ala Leu Arg Ala Thr Leu Thr Glu Ala
   1 5 10 15
   Glu Val Arg Ala Leu Glu Pro Ala Val Arg Glu His His Thr Phe Pro
   20 25 30
   Ala Gly Arg Val Ala Ala Gly Thr Thr Thr Pro Thr Pro Thr Thr Cys
   35 40 45
   Thr Ser Leu Val Ala Gln Arg Val Ser Ala Pro Val Arg Ala Val Trp
   50 55 60
   Pro Ile Val Arg Ser Phe Gly Asn Pro Gln Arg Tyr Lys His Phe Val
   65 70 75 80
   Arg Thr Cys Ala Leu Ala Ala Gly Asp Gly Ala Ser Val Gly Ser Val
   85 90 95
   Arg Glu Val Thr Val Val Ser Gly Leu Pro Ala Ser Ser Ser Thr Glu
   100 105 110
   Arg Leu Glu Val Leu Asp Asp Asp Arg His Ile Leu Ser Phe Arg Val
   115 120 125
   Val Gly Gly Asp His Arg Leu Arg Asn Tyr Arg Ser Val Thr Ser Val
   130 135 140
   Thr Glu Phe Gln Pro Gly Pro Tyr Cys Val Val Val Glu Ser Tyr Ala
   145 150 155 160
   Val Asp Val Pro Glu Gly Asn Thr Ala Glu Asp Thr Arg Met Phe Thr
   165 170 175
   Asp Thr Val Val Arg Leu Asn Leu Gln Lys Leu Ala Ala Val Ala Glu
   180 185 190
   Glu Ser Ala Ala Ala Ala Ala Ala Gly Asn Arg Arg
   195 200
<210> 115
   <211> 204
   <212> PRT
   <213> Sorghum bicolor
<220>
   <223> sorghum Sb04g008040 protein
<400> 115
   Met Glu Pro His Met Glu Thr Ala Leu Arg Gln Gly Gly Leu Ser Glu
   1 5 10 15
   Leu Glu Gln Arg Glu Leu Glu Pro Val Val Arg Ala His His Thr Phe
   20 25 30
   Pro Gly Arg Ser Pro Gly Thr Thr Cys Thr Ser Leu Val Thr Gln Arg
   35 40 45
   Val Asp Ala Pro Leu Ser Ala Val Trp Pro Ile Val Arg Gly Phe Ala
   50 55 60
   Ala Pro Gln Arg Tyr Lys His Phe Ile Lys Ser Cys Asp Leu Arg Ser
   65 70 75 80
   Gly Asp Gly Ala Thr Val Gly Ser Val Arg Glu Val Thr Val Val Ser
                   85                  90                  95
   Gly Leu Pro Ala Ser Thr Ser Thr Glu Arg Leu Glu Ile Leu Asp Asp
               100                 105                 110
   Asp Arg His Ile Leu Ser Phe Arg Val Val Gly Gly Asp His Arg Leu
           115                 120                 125
   Arg Asn Tyr Arg Ser Val Thr Ser Val Thr Glu Phe His His His His
       130                 135                 140
   Gln Ala Ala Ala Gly Arg Pro Tyr Cys Val Val Val Glu Ser Tyr Val
   145                 150                 155                 160
   Val Asp Val Pro Glu Gly Asn Thr Glu Glu Asp Thr Arg Met Phe Thr
                   165                 170                 175
   Asp Thr Val Val Lys Leu Asn Leu Gln Lys Leu Ala Ala Ile Ala Thr
               180                 185                 190
   Ser Ser Ala Ala Ala Ala Ala Ser Asn Ser Ser Thr
           195                 200
<210> 116
   <211> 258
   <212> PRT
   <213> Sorghum bicolor
<220>
   <223> sorghum Sb01g028330 protein
<400> 116
   Met Val Glu Ser Pro Asn Pro Asn Ser Pro Ser Arg Pro Leu Cys Ile
    1               5                  10                  15
   Lys Tyr Thr Arg Ala Pro Ala Arg His Phe Ser Pro Pro Leu Pro Phe
               20                  25                  30
   Ser Ser Leu Ile Ile Ser Ala Asn Pro Ile Glu Pro Lys Ala Met Asp
           35                  40                  45
   Lys Gln Gly Ala Gly Gly Asp Val Glu Val Pro Ala Gly Leu Gly Leu
       50                  55                  60
   Thr Ala Ala Glu Tyr Glu Gln Leu Arg Ser Thr Val Asp Ala His His
   65                  70                  75                  80
   Arg Tyr Ala Val Gly Glu Gly Gln Cys Ser Ser Leu Leu Ala Gln Arg
                   85                  90                  95
   Ile Gln Ala Pro Pro Ala Ala Val Trp Ala Ile Val Arg Arg Phe Asp
               100                 105                 110
   Cys Pro Gln Val Tyr Lys His Phe Ile Arg Ser Cys Ala Leu Arg Pro
           115                 120                 125
   Asp Pro Glu Ala Gly Asp Ala Leu Arg Pro Gly Arg Leu Arg Glu Val
       130                 135                 140
   Ser Val Ile Ser Gly Leu Pro Ala Ser Thr Ser Thr Glu Arg Leu Asp
   145                 150                 155                 160
   Leu Leu Asp Asp Ala Ala Arg Val Phe Gly Phe Ser Ile Thr Gly Gly
                   165                 170                 175
   Glu His Arg Leu Arg Asn Tyr Arg Ser Val Thr Thr Val Ser Glu Leu
               180                 185                 190
   Ala Asp Pro Gly Ile Cys Thr Val Val Leu Glu Ser Tyr Val Val Asp
           195                 200                 205
   Val Pro Asp Gly Asn Thr Glu Asp Asp Thr Arg Leu Phe Ala Asp Thr
       210                 215                 220
   Val Ile Arg Leu Asn Leu Gln Lys Leu Lys Ser Val Ala Glu Ala Asn
   225                 230                 235                 240
   Ala Ala Ala Ala Ala Ser Phe Val Ser Val Val Pro Pro Pro Glu Pro
                   245                 250                 255
   Glu Glu
<210> 117
   <211> 222
   <212> PRT
   <213> Sorghum bicolor
<220>
   <223> sorghum Sb01g038150 protein
<400> 117
   Met Pro Cys Leu Gln Ala Ser Ser Ser Pro Gly Ser Met Pro His Gln
    1               5                  10                  15
   His His Gly Arg Val Leu Ala Gly Val Gly Cys Ala Ala Glu Val Ala
               20                  25                  30
   Ala Ala Ala Val Ala Ala Thr Ser Pro Ala Ala Gly Met Arg Cys Gly
           35                  40                  45
   Ala His Asp Gly Glu Val Pro Ala Glu Ala Ala Arg His His Glu His
       50                  55                  60
   Ala Ala Pro Gly Pro Gly Arg Cys Cys Ser Ala Val Val Gln His Val
   65                  70                  75                  80
   Ala Ala Pro Ala Ser Ala Val Trp Ser Val Val Arg Arg Phe Asp Gln
                   85                  90                  95
   Pro Gln Ala Tyr Lys Arg Phe Val Arg Ser Cys Ala Leu Leu Ala Gly
               100                 105                 110
   Asp Gly Gly Val Gly Thr Leu Arg Glu Val Arg Val Val Ser Gly Leu
           115                 120                 125
   Pro Ala Ala Ser Ser Arg Glu Arg Leu Glu Val Leu Asp Asp Glu Ser
       130                 135                 140
   His Val Leu Ser Phe Arg Val Val Gly Gly Glu His Arg Leu Gln Asn
   145                 150                 155                 160
   Tyr Leu Ser Val Thr Thr Val His Pro Ser Pro Ala Ala Pro Asp Ala
                   165                 170                 175
   Ala Thr Val Val Val Glu Ser Tyr Val Val Asp Val Pro Pro Gly Asn
               180                 185                 190
   Thr Pro Glu Asp Thr Arg Val Phe Val Asp Thr Ile Val Lys Cys Asn
           195                 200                 205
   Leu Gln Ser Leu Ala Thr Thr Ala Glu Lys Leu Ala Ala Val
       210                 215                 220
<210> 118
   <211> 211
   <212> PRT
   <213> Sorghum bicolor
<220>
   <223> sorghum Sb04g009280 protein
<400> 118
   Met Val Glu Met Asp Gly Gly Val Gly Val Val Gly Gly Gly Gln Gln
   1               5                   10                  15
   Thr Pro Ala Pro Arg Arg Trp Arg Leu Ala Asp Glu Leu Arg Cys Asp
               20                  25                  30
   Leu Arg Ala Met Glu Thr Asp Tyr Val Arg Arg Phe His Arg His Glu
           35                  40                  45
   Pro Arg Asp His Gln Cys Ser Ser Ala Val Ala Lys His Ile Lys Ala
       50                  55                  60
   Pro Val His Leu Val Trp Ser Leu Val Arg Arg Phe Asp Gln Pro Gln
   65                  70                  75                  80
   Leu Phe Lys Pro Phe Val Ser Arg Cys Glu Met Lys Gly Asn Ile Glu
                   85                  90                  95
   Ile Gly Ser Val Arg Glu Val Asn Val Lys Ser Gly Leu Pro Ala Thr
               100                 105                 110
   Arg Ser Thr Glu Arg Leu Glu Leu Leu Asp Asp Asn Glu His Ile Leu
           115                 120                 125
   Ser Val Lys Phe Val Gly Gly Asp His Arg Leu Gln Asn Tyr Ser Ser
       130                 135                 140
   Ile Leu Thr Val His Pro Glu Val Ile Asp Gly Arg Pro Gly Thr Leu
   145                 150                 155                 160
   Val Ile Glu Ser Phe Val Val Asp Val Pro Asp Gly Asn Thr Lys Asp
                   165                 170                 175
   Glu Thr Cys Tyr Phe Val Glu Ala Leu Leu Lys Cys Asn Leu Lys Ser
               180                 185                 190
   Leu Ala Glu Val Ser Glu Arg Gln Val Ile Lys Asp Gln Thr Glu Pro
           195                 200                 205
   Leu Asp Arg
       210
<210> 119
   <211> 216
   <212> PRT
   <213> Sorghum bicolor
<220>
   <223> sorghum Sb09g023180 protein
<400> 119
   Met Pro Tyr Thr Ala Pro Arg Pro Ser Pro Gln Gln His Ser Arg Val
    1               5                  10                  15
   Thr Gly Gly Gly Ala Lys Ala Ala Ile Val Ala Ala Ser His Gly Ala
               20                  25                  30
   Ser Cys Ala Ala Val Pro Ala Glu Val Ala Arg His His Glu His Ala
           35                  40                  45
   Ala Arg Ala Gly Gln Cys Cys Ser Ala Val Val Gln Ala Ile Ala Ala
       50                  55                  60
   Pro Val Gly Ala Val Trp Ser Val Val Arg Arg Phe Asp Arg Pro Gln
   65                  70                  75                  80
   Ala Tyr Lys His Phe Ile Arg Ser Cys Arg Leu Val Asp Asp Gly Gly
                   85                  90                  95
   Gly Gly Ala Gly Ala Gly Ala Gly Ala Thr Val Ala Val Gly Ser Val
               100                 105                 110
   Arg Glu Val Arg Val Val Ser Gly Leu Pro Ala Thr Ser Ser Arg Glu
           115                 120                 125
   Arg Leu Glu Ile Leu Asp Asp Glu Arg Arg Val Leu Ser Phe Arg Val
       130                 135                 140
   Val Gly Gly Glu His Arg Leu Ala Asn Tyr Arg Ser Val Thr Thr Val
   145                 150                 155                 160
   His Glu Ala Glu Ala Gly Ala Gly Gly Thr Val Val Val Glu Ser Tyr
                   165                 170                 175
   Val Val Asp Val Pro Pro Gly Asn Thr Ala Asp Glu Thr Arg Val Phe
               180                 185                 190
   Val Asp Thr Ile Val Arg Cys Asn Leu Gln Ser Leu Ala Arg Thr Ala
           195                 200                 205
   Glu Arg Leu Ala Leu Ala Leu Ala
       210                 215
<210> 120
   <211> 36
   <212> PRT
   <213> Artificial Sequence
<220>
   <223> synthetic consensus sequence, amino acid residues 30-65 of PYR1
<220>
   <221> VARIANT
   <222> (1)...(36)
   <223> Xaa = any amino acid
<400> 120
   Cys Xaa Ser Xaa Xaa Xaa Xaa Xaa Xaa Xaa Ala Pro Xaa Xaa Xaa Xaa
    1               5                  10                  15
   Trp Xaa Xaa Xaa Xaa Xaa Phe Xaa Xaa Pro Xaa Xaa Xaa Xaa Xaa Phe
               20                  25                  30
   Xaa Xaa Xaa Cys
           35
<210> 121
   <211> 25
   <212> PRT
   <213> Artificial Sequence
<220>
   <223> synthetic consensus sequence, amino acid residues 76-100 of PYR1
<220>
   <221> VARIANT
   <222> (1)...(25)
   <223> Xaa = any amino acid
<400> 121
   Gly Xaa Xaa Arg Xaa Val Xaa Xaa Xaa Ser Xaa Xaa Pro Ala Xaa Xaa
    1               5                  10                  15
   Ser Xaa Glu Xaa Leu Xaa Xaa Xaa Asp
               20                  25
<210> 122
   <211> 11
   <212> PRT
   <213> Artificial Sequence
<220>
   <223> synthetic consensus sequence, amino acid residues 112-122 of PYR1
<220>
   <221> VARIANT
   <222> (1)...(11)
   <223> Xaa = any amino acid
<400> 122
   Gly Gly Xaa His Arg Leu Xaa Asn Tyr Xaa Ser
    1               5                  10
<210> 123
   <211> 31
   <212> PRT
   <213> Artificial Sequence
<220>
   <223> synthetic consensus sequence, amino acid residues 141-171 of PYR1
<220>
   <221> VARIANT
   <222> (1)...(31)
   <223> Xaa = any amino acid
<400> 123
   Glu Ser Xaa Xaa Val Asp Xaa Pro Xaa Gly Xaa Xaa Xaa Xaa Xaa Thr
    1               5                  10                  15
   Xaa Xaa Phe Xaa Xaa Xaa Xaa Xaa Xaa Xaa Asn Leu Xaa Xaa Leu
               20                  25                  30
<210> 124
   <211> 191
   <212> PRT
   <213> Artificial Sequence
<220>
   <223> synthetic fenhexamid-responsive PYR/PYL receptor mutant clone #27-18
<400> 124
   Met Pro Ser Glu Leu Thr Pro Glu Glu Arg Ser Glu Leu Lys Asn Ser
    1               5                  10                  15
   Ile Ala Glu Phe His Thr Tyr Gln Leu Asp Pro Gly Ser Cys Ser Ser
               20                  25                  30
   Leu His Ala Gln Arg Ile His Ala Pro Pro Glu Leu Val Trp Pro Ile
           35                  40                  45
   Val Arg Arg Phe Asp Lys Pro Gln Thr Tyr Arg His Phe Ile Lys Ser
       50                  55                  60
   Cys Ser Val Glu Gln Asn Phe Glu Met Arg Val Gly Cys Thr Arg Asp
   65                  70                  75                  80
   Val Ile Val Ile Ser Gly Leu Pro Ala Asn Thr Ser Thr Glu Arg Leu
                   85                  90                  95
   Asp Ile Leu Asp Asp Glu Arg Arg Val Thr Gly Phe Ser Ile Ile Gly
               100                 105                 110
   Gly Glu His Arg Leu Thr Asn His Lys Ser Val Thr Thr Val His Arg
           115                 120                 125
   Phe Glu Lys Glu Asn Arg Ile Trp Thr Val Val Leu Glu Ser Tyr Val
       130                 135                 140
   Val Asp Met Pro Glu Gly Asn Ser Glu Asp Asp Thr Arg Met Phe Ala
   145                 150                 155                 160
   Asp Thr Val Val Lys Leu Asn Leu Gln Lys Leu Ala Thr Val Ala Glu
                   165                 170                 175
   Ala Met Ala Arg Asn Ser Gly Asp Gly Ser Gly Ser Gln Val Thr
               180                 185                 190
<210> 125
   <211> 191
   <212> PRT
   <213> Artificial Sequence
<220>
   <223> synthetic fenhexamid-responsive PYR/PYL receptor mutant clone #27-24
<400> 125
   Met Pro Ser Glu Leu Thr Pro Glu Glu Arg Ser Glu Leu Lys Asn Ser
    1               5                  10                  15
   Ile Ala Glu Phe His Thr Tyr Gln Leu Asp Pro Gly Ser Cys Ser Ser
               20                  25                  30
   Leu His Ala Gln Arg Ile His Ala Pro Pro Glu Leu Val Trp Ser Ile
           35                  40                  45
   Val Arg Arg Phe Asp Lys Pro Gln Thr Tyr Arg His Phe Ile Lys Ser
       50                  55                  60
   Cys Ser Val Glu Gln Asn Phe Glu Met Arg Val Gly Cys Thr Arg Asp
   65                  70                  75                  80
   Val Ile Val Ile Ser Gly Leu Pro Ala Asn Thr Ser Thr Glu Arg Leu
                   85                  90                  95
   Asp Ile Leu Asp Asp Glu Arg Arg Val Thr Gly Phe Ser Ile Ile Gly
               100                 105                 110
   Gly Glu His Arg Leu Thr Asn His Lys Ser Val Thr Thr Val His Arg
           115                 120                 125
   Phe Glu Lys Glu Asn Arg Ile Trp Thr Val Val Leu Glu Ser Tyr Ala
       130                 135                 140
   Val Asp Met Pro Glu Gly Asn Ser Glu Asp Asp Thr Arg Met Phe Ala
   145                 150                 155                 160
   Asp Thr Val Val Lys Leu Asn Leu Gln Lys Leu Ala Thr Val Ala Glu
                   165                 170                 175
   Ala Met Ala Arg Asn Ser Gly Asp Gly Ser Gly Ser Gln Val Thr
               180                 185                 190
<210> 126
   <211> 191
   <212> PRT
   <213> Artificial Sequence
<220>
   <223> synthetic fenhexamid-responsive PYR/PYL receptor mutant clone #27-31
<400> 126
   Met Pro Ser Glu Leu Thr Pro Glu Glu Arg Ser Glu Leu Lys Asn Ser
    1               5                  10                  15
   Ile Ala Glu Phe His Thr Tyr Gln Leu Asp Pro Gly Ser Cys Ser Ser
               20                  25                  30
   Leu His Ala Gln Arg Ile His Ala Pro Ser Glu Leu Val Trp Ser Ile
           35                  40                  45
   Val Arg Arg Phe Asp Lys Pro Gln Thr Tyr Arg His Phe Ile Lys Ser
       50                  55                  60
   Cys Ser Val Glu Gln Asn Phe Glu Met Arg Val Gly Cys Thr Arg Asp
   65                  70                  75                  80
   Val Ile Val Ile Ser Gly Leu Pro Ala Asn Thr Ser Thr Glu Arg Leu
                   85                  90                  95
   Asp Ile Leu Asp Asp Glu Arg Arg Val Thr Gly Phe Ser Ile Ile Gly
               100                 105                 110
   Gly Glu His Arg Leu Thr Asn Cys Lys Ser Val Thr Thr Val His Arg
           115                 120                 125
   Phe Glu Lys Glu Asn Arg Ile Trp Thr Val Val Leu Glu Ser Tyr Val
       130                 135                 140
   Val Asp Met Pro Glu Gly Asn Ser Glu Asp Asp Thr Arg Met Phe Ala
   145                 150                 155                 160
   Asp Thr Val Val Lys Leu Asn Leu Gln Lys Leu Ala Thr Val Ala Glu
                   165                 170                 175
   Ala Met Ala Arg Asn Ser Gly Asp Gly Ser Gly Ser Gln Val Thr
               180                 185                 190
<210> 127
   <211> 191
   <212> PRT
   <213> Artificial Sequence
<220>
   <223> synthetic fenhexamid-responsive PYR/PYL receptor mutant clone #27-28
<400> 127
   Met Pro Ser Glu Leu Thr Pro Glu Glu Arg Ser Glu Leu Lys Asn Ser
   1 5 10 15
   Ile Ala Glu Phe His Thr Tyr Gln Leu Asp Pro Gly Ser Cys Ser Ser
   20 25 30
   Leu His Ala Gln Arg Ile His Ala Pro Ser Glu Leu Val Trp Ser Ile
   35 40 45
   Val Arg Arg Phe Asp Lys Pro Gln Thr Tyr Arg His Phe Ile Lys Ser
   50 55 60
   Cys Ser Val Glu Gln Asn Phe Glu Met Arg Val Gly Cys Thr Arg Asp
   65 70 75 80
   Val Ile Val Ile Ser Gly Leu Pro Ala Asn Thr Ser Thr Glu Arg Leu
   85 90 95
   Asp Ile Leu Asp Asp Glu Arg Arg Val Thr Gly Phe Ser Ile Ile Gly
   100 105 110
   Gly Glu His Arg Leu Thr Asn Cys Lys Ser Val Met Thr Val His Arg
   115 120 125
   Phe Glu Lys Glu Asn Arg Ile Trp Thr Val Val Leu Glu Ser Tyr Val
   130 135 140
   Val Asp Met Pro Glu Gly Asn Ser Glu Asp Asp Thr Arg Met Phe Ala
   145 150 155 160
   Asp Thr Val Val Lys Leu Asn Leu Gln Lys Leu Ala Thr Val Ala Glu
   165 170 175
   Ala Met Ala Arg Asn Ser Gly Asp Gly Ser Gly Ser Gln Val Thr
   180 185 190
<210> 128
   <211> 191
   <212> PRT
   <213> Artificial Sequence
<220>
   <223> synthetic fenhexamid-responsive PYR/PYL receptor mutant clone #27-9
<400> 128
   Met Pro Ser Glu Leu Thr Pro Glu Glu Arg Ser Glu Leu Lys Asn Ser
   1 5 10 15
   Ile Ala Glu Phe His Thr Tyr Gln Leu Asp Pro Gly Ser Cys Ser Ser
   20 25 30
   Leu His Ala Gln Arg Ile His Ala Pro Ser Glu Leu Val Trp Ser Ile
   35 40 45
   Val Arg Arg Phe Asp Lys Pro Gln Thr Tyr Arg His Phe Ile Lys Ser
   50 55 60
   Cys Ser Val Glu Gln Asn Phe Glu Met Arg Val Gly Cys Thr Arg Asp
   65 70 75 80
   Val Ile Val Ile Ser Gly Leu Pro Ala Asn Thr Ser Thr Glu Arg Leu
   85 90 95
   Asp Ile Leu Asp Asp Glu Arg Arg Val Thr Gly Phe Ser Ile Ile Gly
   100 105 110
   Gly Glu His Arg Leu Thr Asn Cys Lys Ser Val Thr Thr Val His Arg
   115 120 125
   Phe Glu Lys Glu Asn Arg Ile Trp Thr Val Val Leu Glu Ser Tyr Val
   130 135 140
   Val Asp Met Pro Glu Gly Asn Ser Gly Asp Asp Thr Arg Met Phe Ala
   145 150 155 160
   Asp Thr Val Val Lys Leu Asn Leu Gln Lys Leu Ala Thr Val Ala Glu
   165 170 175
   Ala Met Ala Arg Asn Ser Gly Asp Gly Ser Gly Ser Gln Val Thr
   180 185 190
<210> 129
   <211> 191
   <212> PRT
   <213> Artificial Sequence
<220>
   <223> synthetic fenhexamid-responsive PYR/PYL receptor mutant clone #27-36
<400> 129
   Met Pro Ser Glu Leu Thr Pro Glu Glu Arg Ser Gly Leu Lys Asn Ser
   1               5                   10                  15
   Ile Ala Glu Phe His Thr Tyr Gln Leu Asp Pro Gly Ser Cys Ser Ser
               20                  25                  30
   Leu His Ala Gln Arg Ile His Ala Pro Pro Gly Leu Val Trp Ser Ile
           35                  40                  45
   Val Arg Arg Phe Asp Lys Pro Gln Thr Tyr Arg His Phe Ile Lys Ser
       50                  55                  60
   Cys Ser Val Glu Gln Asn Phe Glu Met Arg Val Gly Cys Thr Arg Asp
   65                  70                  75                  80
   Val Ile Val Ile Ser Gly Leu Pro Ala Asn Thr Ser Thr Glu Arg Leu
                   85                  90                  95
   Asp Ile Leu Asp Asp Glu Arg Arg Val Thr Gly Phe Ser Ser Ile Gly
               100                 105                 110
   Gly Glu His Arg Leu Thr Asn Tyr Lys Ser Val Thr Thr Val His Arg
           115                 120                 125
   Phe Glu Lys Glu Asp Arg Ile Trp Thr Val Val Leu Glu Ser Tyr Val
       130                 135                 140
   Val Asp Met Pro Glu Gly Asn Ser Glu Asp Asp Thr Arg Met Phe Ala
   145                 150                 155                 160
   Asp Thr Val Val Lys Leu Asn Leu Gln Lys Leu Ala Thr Val Ala Glu
                   165                 170                 175
   Ala Met Ala Arg Asn Ser Gly Asp Gly Ser Gly Ser Gln Val Thr
               180                 185                 190
<210> 130
   <211> 191
   <212> PRT
   <213> Artificial Sequence
<220>
   <223> synthetic fenhexamid-responsive PYR/PYL receptor mutant clone #27-14
<400> 130
   Met Pro Ser Glu Leu Thr Pro Glu Glu Arg Ser Gly Leu Lys Asn Ser
    1               5                  10                  15
   Ile Ala Glu Phe His Thr Tyr Gln Arg Asp Pro Gly Ser Cys Ser Ser
               20                  25                  30
   Leu His Ala Gln Arg Ile His Ala Pro Pro Gly Leu Val Trp Ser Ile
           35                  40                  45
   Val Arg Arg Phe Asp Lys Pro Gln Thr Tyr Arg His Phe Ile Lys Ser
       50                  55                  60
   Cys Ser Val Glu Gln Asn Phe Glu Met Arg Val Gly Cys Thr Arg Asp
   65                  70                  75                  80
   Val Ile Val Ile Ser Gly Leu Pro Ala Asn Thr Ser Thr Glu Arg Leu
                   85                  90                  95
   Asp Ile Leu Asp Asp Glu Arg Arg Val Thr Gly Phe Ser Ser Ile Gly
               100                 105                 110
   Gly Glu His Arg Leu Thr Asn Tyr Lys Ser Val Thr Thr Val His Arg
           115                 120                 125
   Phe Glu Lys Glu Asp Arg Ile Trp Thr Val Val Leu Glu Ser Tyr Val
       130                 135                 140
   Val Asp Met Pro Glu Gly Asn Ser Glu Asp Asp Thr Arg Met Phe Ala
   145                 150                 155                 160
   Asp Thr Val Val Lys Leu Asn Leu Gln Lys Leu Ala Thr Val Ala Glu
                   165                 170                 175
   Ala Met Ala Arg Asn Ser Gly Asp Gly Ser Gly Ser Gln Val Thr
               180                 185                 190
<210> 131
   <211> 191
   <212> PRT
   <213> Artificial Sequence
<220>
   <223> synthetic bromoxynil-responsive PYR/PYL receptor mutant clone #74A-12
<400> 131
   Met Pro Ser Glu Leu Thr Pro Glu Glu Arg Ser Glu Leu Lys Asn Ser
    1               5                  10                  15
   Ile Ala Glu Phe His Thr Tyr Gln Leu Asp Pro Gly Ser Cys Ser Ser
               20                  25                  30
   Leu His Ala Gln Arg Ile His Ala Pro Pro Glu Leu Val Trp Ser Ile
           35                  40                  45
   Val Arg Arg Phe Asp Lys Pro Gln Ala Tyr Arg His Phe Ile Lys Ser
       50                  55                  60
   Cys Ser Val Glu Gln Asn Phe Glu Met Arg Val Gly Cys Thr Arg Asp
   65                  70                  75                  80
   Val Ile Val Ile Ser Gly Leu Pro Ala Asn Thr Ser Thr Glu Arg Leu
                   85                  90                  95
   Asp Ile Leu Asp Asp Glu Arg Arg Val Thr Gly Phe Ser Ile Ile Gly
               100                 105                 110
   Gly Glu His Arg Leu Thr Asn Tyr Lys Ser Val Thr Thr Val His Arg
           115                 120                 125
   Phe Glu Lys Glu Asn Arg Ile Trp Thr Val Val Leu Gly Ser Tyr Val
       130                 135                 140
   Val Asp Met Pro Glu Gly Asn Ser Glu Asp Asp Thr Arg Met Phe Ala
   145                 150                 155                 160
   Asp Thr Val Val Lys Leu Asn Leu Gln Lys Leu Ala Thr Val Ala Glu
                   165                 170                 175
   Ala Met Ala Arg Asn Ser Gly Asp Gly Ser Gly Ser Gln Val Thr
               180                 185                 190
<210> 132
   <211> 191
   <212> PRT
   <213> Artificial Sequence
<220>
   <223> synthetic bromoxynil-responsive PYR/PYL receptor mutant clone #74A-24
<400> 132
   Met Pro Ser Glu Leu Thr Pro Glu Glu Arg Ser Glu Leu Lys Asn Ser
    1               5                  10                  15
   Ile Ala Glu Phe His Thr Tyr Gln Leu Asp Pro Gly Ser Cys Ser Ser
               20                  25                  30
   Leu His Ala Gln Arg Ile His Ala Pro Pro Glu Leu Val Trp Ser Ile
           35                  40                  45
   Val Gly Arg Phe Asp Lys Pro Gln Thr Tyr Arg His Phe Ile Lys Ser
       50                  55                  60
   Cys Ser Val Glu Gln Asn Phe Glu Met Arg Val Gly Cys Thr Arg Asp
   65                  70                  75                  80
   Val Ile Val Ile Ser Gly Leu Pro Ala Asn Thr Ser Thr Glu Arg Leu
                   85                  90                  95
   Asp Ile Leu Asp Asp Glu Arg Arg Val Thr Gly Phe Ser Ile Ile Gly
               100                 105                 110
   Gly Glu His Arg Leu Thr Asn Tyr Lys Ser Val Thr Thr Val His Arg
           115                 120                 125
   Phe Glu Lys Glu Asn Arg Ile Trp Thr Val Val Leu Gln Ser Tyr Val
       130                 135                 140
   Val Asp Met Pro Glu Gly Asn Ser Glu Asp Asp Thr Arg Met Phe Ala
   145                 150                 155                 160
   Asp Thr Val Val Lys Leu Asn Leu Gln Lys Leu Ala Thr Val Ala Glu
                   165                 170                 175
   Ala Met Ala Arg Asn Ser Gly Asp Gly Ser Gly Ser Gln Val Thr
               180                 185                 190
<210> 133
   <211> 191
   <212> PRT
   <213> Artificial Sequence
<220>
   <223> synthetic bromoxynil-responsive PYR/PYL receptor mutant clone #74A-1
<400> 133
   Met Pro Ser Glu Leu Thr Pro Glu Glu Arg Ser Glu Leu Lys Asn Ser
    1               5                  10                  15
   Ile Ala Glu Phe His Thr Tyr Gln Leu Asp Pro Gly Ser Cys Ser Ser
               20                  25                  30
   Leu His Ala Gln Arg Ile His Ala Pro Pro Glu Leu Val Trp Ser Ile
           35                  40                  45
   Val Arg Arg Phe Asp Lys Pro Gln Thr Tyr Arg Arg Phe Ile Lys Ser
       50                  55                  60
   Cys Ser Val Glu Gln Asn Phe Glu Met Arg Val Gly Cys Thr Arg Asp
   65                  70                  75                  80
   Val Ile Val Ile Ser Gly Leu Pro Ala Asn Thr Ser Thr Glu Arg Leu
                   85                  90                  95
   Asp Ile Leu Asp Asp Glu Arg Arg Val Thr Gly Phe Ser Ile Ile Gly
               100                 105                 110
   Gly Glu His Arg Leu Thr Asn Tyr Lys Ser Val Thr Thr Val His Arg
           115                 120                 125
   Phe Glu Lys Glu Asn Arg Ile Trp Thr Val Val Leu Glu Ser Tyr Val
       130                 135                 140
   Val Asp Met Pro Glu Gly Asp Ser Glu Asp Asp Thr Arg Met Phe Ala
   145                 150                 155                 160
   Asp Thr Val Val Lys Leu Asn Leu Gln Lys Leu Ala Thr Val Ala Glu
                   165                 170                 175
   Ala Met Ala Arg Asn Ser Gly Asp Gly Ser Gly Ser Gln Val Thr
               180                 185                 190
<210> 134
   <211> 191
   <212> PRT
   <213> Artificial Sequence
<220>
   <223> synthetic bromoxynil-responsive PYR/PYL receptor mutant clone #74A-4
<400> 134
   Met Pro Ser Glu Leu Thr Pro Glu Glu Arg Ser Glu Leu Lys Asn Ser
    1               5                  10                  15
   Ile Ala Glu Phe His Thr Tyr Gln Leu Asp Pro Gly Ser Cys Ser Ser
               20                  25                  30
   Leu His Ala Gln Arg Ile His Ala Pro Pro Glu Leu Val Trp Ser Ile
           35                  40                  45
   Val Arg Arg Phe Asp Lys Pro Gln Thr Tyr Arg Arg Phe Ile Lys Ser
       50                  55                  60
   Cys Ser Val Glu Gln Asn Phe Glu Met Arg Val Gly Cys Thr Arg Asp
   65                  70                  75                  80
   Val Ile Val Ile Ser Gly Leu Pro Ala Asn Thr Ser Thr Glu Arg Leu
                   85                  90                  95
   Asp Ile Leu Asp Asp Gly Arg Arg Val Thr Gly Phe Ser Ile Ile Gly
               100                 105                 110
   Gly Glu His Arg Leu Thr Asn Tyr Lys Ser Val Thr Ala Val His Arg
           115                 120                 125
   Phe Glu Lys Glu Asn Arg Ile Trp Thr Val Val Leu Asp Ser Tyr Val
       130                 135                 140
   Val Asp Met Pro Glu Gly Asn Ser Glu Asp Asp Thr Arg Met Phe Ala
   145                 150                 155                 160
   Asp Thr Val Val Lys Leu Asn Leu Gln Lys Leu Ala Thr Val Ala Glu
                   165                 170                 175
   Ala Met Ala Arg Asn Ser Gly Asp Gly Ser Gly Ser Gln Val Thr
               180                 185                 190
<210> 135
   <211> 191
   <212> PRT
   <213> Artificial Sequence
<220>
   <223> synthetic bromoxynil-responsive PYR/PYL receptor mutant clone #74A-13
<400> 135
   Met Pro Ser Glu Leu Thr Pro Glu Glu Arg Ser Glu Leu Lys Asn Ser
    1               5                  10                  15
   Ile Ala Glu Phe His Thr Tyr Gln Leu Asp Pro Gly Ser Cys Ser Ser
               20                  25                  30
   Leu His Ala Gln Arg Ile His Ala Pro Pro Glu Leu Val Trp Ser Ile
           35                  40                  45
   Val Arg Arg Phe Asp Lys Pro Gln Thr Tyr Arg His Phe Ile Lys Ser
       50                  55                  60
   Cys Ser Val Glu Gln Asn Phe Glu Met Arg Val Gly Cys Thr Arg Asp
   65                  70                  75                  80
   Val Asn Val Ile Ser Gly Leu Pro Ala Asn Thr Ser Thr Glu Arg Leu
                   85                  90                  95
   Asp Ile Leu Asp Asp Glu Arg Arg Val Thr Gly Phe Ser Ile Ile Gly
               100                 105                 110
   Gly Glu His Arg Leu Thr Asn Tyr Lys Ser Val Thr Thr Val His Arg
           115                 120                 125
   Phe Glu Lys Glu Asn Arg Ile Trp Thr Val Val Leu Glu Ser Tyr Val
       130                 135                 140
   Val Asp Met Pro Glu Gly Asn Ser Glu Asp Asp Thr Arg Met Phe Ala
   145                 150                 155                 160
   Asp Thr Val Val Lys Leu Asn Leu Gln Lys Leu Ala Thr Val Ala Glu
                   165                 170                 175
   Ala Met Ala Arg Asn Ser Gly Asp Gly Ser Gly Ser Gln Val Thr
               180                 185                 190
<210> 136
   <211> 191
   <212> PRT
   <213> Artificial Sequence
<220>
   <223> synthetic bromoxynil-responsive PYR/PYL receptor mutant clone #74A-2
<400> 136
   Met Pro Ser Glu Leu Thr Pro Glu Glu Arg Ser Glu Leu Lys Asn Ser
    1               5                  10                  15
   Ile Ala Glu Phe His Thr Tyr Gln Leu Asp Pro Gly Ser Cys Ser Ser
               20                  25                  30
   Leu His Ala Gln Arg Ile His Ala Pro Pro Glu Leu Val Trp Ser Ile
           35                  40                  45
   Val Arg Arg Phe Asp Lys Pro Gln Thr Tyr Arg His Phe Ile Lys Ser
       50                  55                  60
   Cys Ser Val Glu Gln Asn Phe Glu Met Arg Val Gly Cys Thr Arg Asp
   65                  70                  75                  80
   Val Ile Val Ile Ser Gly Leu Pro Ala Asn Thr Thr Thr Glu Arg Leu
                   85                  90                  95
   Asp Ile Leu Asp Asp Glu Arg Arg Val Thr Gly Phe Ser Ile Ile Gly
               100                 105                 110
   Gly Glu His Arg Leu Thr Asn Tyr Lys Ser Val Thr Thr Val His Arg
           115                 120                 125
   Phe Glu Lys Glu Asn Arg Ile Trp Thr Val Val Leu Glu Ser Tyr Val
       130                 135                 140
   Val Asp Met Pro Glu Gly Asn Ser Glu Asp Asp Thr Arg Met Phe Ala
   145                 150                 155                 160
   Asp Thr Val Val Lys Leu Asn Leu Gln Lys Leu Ala Thr Val Ala Glu
                   165                 170                 175
   Ala Met Ala Arg Asn Ser Gly Asp Gly Ser Gly Ser Gln Val Thr
               180                 185                 190
<210> 137
   <211> 191
   <212> PRT
   <213> Artificial Sequence
<220>
   <223> synthetic bromoxynil-responsive PYR/PYL receptor mutant clone #74A-15
<400> 137
   Met Pro Ser Glu Leu Thr Pro Glu Glu Arg Ser Glu Leu Lys Asn Ser
    1              5                   10                  15
   Ile Ala Glu Phe His Thr Tyr Gln Leu Asp Pro Gly Ser Cys Ser Ser
               20                  25                  30
   Leu His Ala Gln Arg Ile His Ala Pro Pro Glu Leu Val Trp Ser Ile
           35                  40                  45
   Val Arg Arg Phe Asp Lys Pro Gln Thr Tyr Arg His Phe Ile Lys Ser
       50                  55                  60
   Cys Ser Val Glu Gln Asn Phe Glu Met Arg Val Gly Cys Thr Arg Asp
   65                  70                  75                  80
   Val Ile Val Ile Ser Gly Leu Pro Ala Asn Thr Thr Thr Glu Arg Leu
                   85                  90                  95
   Asp Ile Leu Asp Asp Glu Arg Arg Val Thr Gly Phe Ser Ile Ile Gly
               100                 105                 110
   Gly Glu His Arg Leu Thr Asn Tyr Lys Ser Val Thr Thr Val His Arg
           115                 120                 125
   Phe Glu Lys Glu Asn Arg Ile Trp Thr Val Val Leu Glu Ser Tyr Val
       130                 135                 140
   Val Asp Met Pro Glu Gly Asn Ser Glu Asp Asp Thr Arg Met Phe Ala
   145                 150                 155                 160
   Asp Thr Val Val Lys Leu Asn Leu Gln Lys Leu Ala Thr Val Ala Glu
                   165                 170                 175
   Ala Met Ala Arg Asn Ser Gly Asp Gly Ser Gly Ser Gln Val Thr
               180                 185                 190
<210> 138
   <211> 191
   <212> PRT
   <213> Artificial Sequence
<220>
   <223> synthetic bromoxynil-responsive PYR/PYL receptor mutant clone #74B-1
<400> 138
   Met Pro Ser Glu Leu Thr Pro Glu Glu Arg Ser Glu Leu Lys Asn Ser
    1               5                  10                  15
   Ile Ala Glu Phe Tyr Thr Tyr Gln Leu Asp Pro Gly Ser Cys Ser Ser
               20                  25                  30
   Leu His Ala Gln Arg Ile His Ala Pro Pro Glu Leu Val Trp Ser Ile
           35                  40                  45
   Val Arg Arg Phe Asp Lys Pro Gln Thr Tyr Arg Arg Phe Ile Lys Ser
       50                  55                  60
   Cys Ser Val Glu Gln Asn Phe Glu Met Arg Val Gly Cys Thr Arg Asp
   65                  70                  75                  80
   Val Ile Val Ile Ser Gly Leu Pro Ala Asn Thr Thr Thr Glu Arg Leu
                   85                  90                  95
   Asp Ile Leu Asp Asp Glu Arg Arg Val Thr Gly Phe Ser Ile Ile Gly
               100                 105                 110
   Gly Glu His Lys Leu Thr Asn Tyr Lys Ser Val Thr Thr Val His Arg
           115                 120                 125
   Phe Glu Lys Glu Asn Arg Ile Trp Thr Val Val Leu Glu Ser Tyr Val
       130                 135                 140
   Val Asp Met Pro Glu Gly Asn Ser Glu Asp Asp Thr Arg Met Phe Ala
   145                 150                 155                 160
   Asp Thr Val Val Lys Leu Asn Leu Gln Lys Leu Ala Thr Val Ala Glu
                   165                 170                 175
   Ala Met Ala Arg Asn Ser Gly Asp Gly Ser Gly Ser Gln Val Thr
               180                 185                 190
<210> 139
   <211> 191
   <212> PRT
   <213> Artificial Sequence
<220>
   <223> synthetic bromoxynil-responsive PYR/PYL receptor mutant clone #74B-7
<400> 139
   Met Pro Ser Glu Leu Thr Pro Glu Glu Arg Ser Glu Leu Lys Asn Ser
    1               5                  10                  15
   Ile Ala Glu Phe His Thr Tyr Gln Leu Asp Pro Gly Ser Cys Ser Ser
               20                  25                  30
   Leu His Ala Gln Arg Ile His Ala Leu Pro Glu Leu Val Trp Ser Ile
           35                  40                  45
   Val Arg Arg Phe Asp Lys Pro Gln Thr Tyr Arg Arg Phe Ile Lys Ser
       50                  55                  60
   Cys Ser Val Glu Gln Asn Phe Glu Met Arg Val Gly Cys Thr Arg Asp
   65                  70                  75                  80
   Val Ile Val Ile Ser Gly Leu Pro Ala Asn Thr Ser Thr Glu Arg Leu
                   85                  90                  95
   Asp Ile Leu Asp Asp Glu Arg Arg Val Thr Gly Phe Ser Ile Ile Gly
               100                 105                 110
   Gly Glu His Arg Leu Thr Asn Tyr Lys Ser Val Thr Thr Val His Arg
           115                 120                 125
   Phe Glu Lys Glu Asn Arg Ile Trp Thr Val Val Leu Glu Ser Tyr Val
       130                 135                 140
   Val Asp Met Pro Glu Gly Asn Ser Glu Asp Asp Thr Arg Met Phe Ala
   145                 150                 155                 160
   Asp Thr Val Val Lys Leu Asn Leu Gln Lys Leu Ala Thr Val Ala Glu
                   165                 170                 175
   Ala Met Ala Arg Asn Ser Gly Asp Gly Ser Gly Ser Gln Val Thr
               180                 185                 190
<210> 140
   <211> 191
   <212> PRT
   <213> Artificial Sequence
<220>
   <223> synthetic dichlobenil-responsive PYR/PYL receptor mutant clone #68-1
<400> 140
   Met Pro Ser Glu Leu Thr Pro Glu Glu Arg Ser Glu Leu Lys Asn Ser
    1               5                  10                  15
   Ile Ala Glu Phe His Thr Tyr Gln Leu Asp Pro Gly Ser Cys Ser Ser
               20                  25                  30
   Leu His Ala Gln Arg Ile His Ala Pro Pro Glu Leu Val Trp Ser Ile
           35                  40                  45
   Val Arg Arg Phe Asp Lys Pro Gln Thr Tyr Lys His Phe Ile Lys Ser
       50                  55                  60
   Cys Ser Val Glu Gln Asn Phe Glu Met Arg Val Gly Cys Thr Arg Asp
   65                  70                  75                  80
   Val Ile Val Ile Ser Gly Leu Pro Ala Asn Thr Ser Thr Asp Arg Leu
                   85                  90                  95
   Asp Ile Leu Asp Asp Glu Arg Arg Val Thr Gly Phe Ser Ile Ile Gly
               100                 105                 110
   Gly Glu His Arg Leu Thr Asn Tyr Lys Ser Val Thr Thr Val His Arg
           115                 120                 125
   Phe Glu Lys Glu Asn Arg Ile Trp Thr Val Val Leu Glu Ser Tyr Val
       130                 135                 140
   Val Asp Met Pro Glu Gly Asn Ser Glu Asp Asp Thr Arg Met Phe Ala
   145                 150                 155                 160
   Asp Thr Val Val Lys Leu Asn Leu Gln Lys Leu Ala Thr Val Ala Glu
                   165                 170                 175
   Ala Met Ala Arg Asn Ser Gly Asp Gly Ser Gly Ser Gln Val Thr
               180                 185                 190
<210> 141
   <211> 191
   <212> PRT
   <213> Artificial Sequence
<220>
   <223> synthetic dichlobenil-responsive PYR/PYL receptor mutant clone #68-2
<400> 141
   Met Pro Ser Glu Leu Thr Pro Glu Glu Arg Ser Glu Leu Lys Asn Ser
    1               5                  10                  15
   Ile Ala Glu Phe His Thr Tyr Gln Leu Asp Pro Gly Ser Cys Ser Ser
               20                  25                  30
   Leu His Ala Gln Gln Ile His Ala Pro Pro Glu Leu Val Trp Ser Ile
           35                  40                  45
   Val Arg Arg Phe Asp Lys Pro Gln Thr Tyr Lys His Phe Ile Lys Ser
       50                  55                  60
   Cys Ser Val Glu Gln Asn Ser Glu Met Arg Val Gly Cys Thr Arg Asp
   65                  70                  75                  80
   Val Ile Val Ile Ser Gly Leu Pro Ala Asn Thr Ser Thr Glu Arg Leu
                   85                  90                  95
   Asp Ile Leu Asp Asp Glu Arg Arg Val Thr Gly Phe Ser Ile Ile Gly
               100                 105                 110
   Gly Glu His Arg Leu Thr Asn Tyr Lys Ser Val Thr Thr Val His Arg
           115                 120                 125
   Phe Glu Lys Glu Asn Arg Ile Trp Thr Val Val Leu Glu Ser Tyr Val
       130                 135                 140
   Val Asp Met Pro Glu Gly Asn Ser Glu Asp Asp Thr Arg Met Phe Ala
   145                 150                 155                 160
   Asp Thr Val Val Lys Leu Asn Leu Gln Lys Leu Ala Thr Val Ala Glu
                   165                 170                 175
   Ala Met Ala Arg Asn Ser Gly Asp Gly Ser Gly Ser Gln Val Thr
               180                 185                 190
<210> 142
   <211> 191
   <212> PRT
   <213> Artificial Sequence
<220>
   <223> synthetic dichlobenil-responsive PYR/PYL receptor mutant clone #68-8
<400> 142
   Met Pro Ser Glu Leu Thr Pro Glu Glu Arg Ser Glu Leu Lys Asn Ser
    1               5                  10                  15
   Ile Ala Glu Phe His Thr Tyr Gln Leu Gly Pro Gly Ser Cys Ser Ser
               20                  25                  30
   Leu His Ala Gln Arg Ile His Ala Pro Pro Glu Leu Val Trp Ser Ile
           35                  40                  45
   Val Arg Arg Phe Asp Lys Pro Gln Thr Tyr Lys His Phe Ile Lys Ser
       50                  55                  60
   Cys Ser Val Glu Gln Asn Phe Glu Met Arg Val Gly Cys Thr Arg Asp
   65                  70                  75                  80
   Val Ile Val Ile Ser Gly Leu Pro Ala Asn Thr Ser Thr Glu Arg Leu
                   85                  90                  95
   Asp Ile Leu Asp Asp Glu Arg Arg Val Thr Gly Phe Ser Ile Ile Gly
               100                 105                 110
   Gly Glu His Arg Leu Thr Asn Tyr Lys Ser Val Thr Thr Val His Arg
           115                 120                 125
   Phe Glu Lys Glu Asn Arg Ile Trp Thr Val Val Leu Glu Ser Tyr Val
       130                 135                 140
   Val Asp Met Pro Glu Gly Asn Ser Glu Asp Asp Thr Arg Met Phe Ala
   145                 150                 155                 160
   Asp Thr Val Val Lys Leu Asn Leu Gln Lys Leu Ala Thr Val Ala Glu
                   165                 170                 175
   Ala Met Ala Arg Asn Ser Gly Asp Gly Ser Gly Ser Gln Val Thr
               180                 185                 190
<210> 143
   <211> 191
   <212> PRT
   <213> Artificial Sequence
<220>
   <223> synthetic dichlobenil-responsive PYR/PYL receptor mutant clone #68-3
<400> 143
   Met Pro Ser Glu Leu Thr Pro Glu Glu Arg Ser Glu Leu Lys Asn Ser
    1               5                  10                  15
   Ile Ala Glu Phe His Thr Tyr Gln Leu Asp Leu Gly Ser Cys Ser Ser
               20                  25                  30
   Leu His Ala Gln Arg Ile His Ala Pro Pro Glu Leu Val Trp Ser Ile
           35                  40                  45
   Val Arg Arg Phe Asp Lys Pro Gln Thr Tyr Arg His Phe Ile Asn Ser
       50                  55                  60
   Cys Ser Val Glu Gln Asn Phe Glu Met Arg Val Gly Cys Thr Arg Asp
   65                  70                  75                  80
   Val Ile Val Ile Ser Gly Leu Pro Ala Asn Thr Ser Thr Glu Arg Leu
                   85                  90                  95
   Asp Ile Leu Asp Asp Glu Arg Arg Val Thr Gly Phe Ser Ile Ile Gly
               100                 105                 110
   Gly Glu His Arg Leu Thr Asn Tyr Lys Ser Val Thr Thr Val His Arg
           115                 120                 125
   Phe Glu Lys Glu Asn Arg Ile Trp Thr Val Val Leu Glu Ser Tyr Val
       130                 135                 140
   Val Asp Met Pro Glu Gly Asn Ser Glu Asp Asp Thr Arg Met Phe Ala
   145                 150                 155                 160
   Asp Thr Val Val Lys Leu Asn Leu Gln Lys Leu Ala Thr Val Ala Glu
                   165                 170                 175
   Ala Met Ala Arg Asn Ser Gly Asp Gly Ser Gly Ser Gln Val Thr
               180                 185                 190
<210> 144
   <211> 191
   <212> PRT
   <213> Artificial Sequence
<220>
   <223> synthetic dichlobenil-responsive PYR/PYL receptor mutant clone #68-17
<400> 144
   Met Pro Ser Glu Leu Thr Pro Glu Glu Arg Ser Glu Leu Lys Asn Ser
    1               5                  10                  15
   Ile Ala Glu Phe His Thr Tyr Gln Leu Asp Pro Gly Ser Cys Ser Ser
               20                  25                  30
   Leu His Ala Gln Arg Ile His Ala Pro Pro Glu Leu Val Trp Ser Ile
           35                  40                  45
   Val Arg Arg Phe Asp Lys Pro Gln Thr Tyr Arg His Phe Ile Lys Ser
       50                  55                  60
   Cys Ser Val Glu Gln Asn Phe Glu Met Arg Val Gly Cys Thr Arg Asp
   65                  70                  75                  80
   Val Ile Val Ile Ser Gly Leu Pro Ala Asn Thr Ser Thr Glu Arg Leu
                   85                  90                  95
   Asp Ile Leu Asp Asp Glu Arg Arg Val Thr Gly Phe Ser Ile Ile Gly
               100                 105                 110
   Gly Glu His Arg Leu Thr Asn Tyr Lys Ser Val Thr Thr Val His Arg
           115                 120                 125
   Phe Glu Lys Glu Asn Arg Ile Trp Thr Val Val Leu Glu Ser Tyr Val
       130                 135                 140
   Val Asp Met Pro Glu Gly Asn Ser Glu Asp Asp Thr Arg Met Phe Ala
   145                 150                 155                 160
   Asp Thr Val Val Lys Leu Asn Leu Gln Lys Leu Ala Thr Val Ala Glu
                   165                 170                 175
   Ala Met Ala Arg Asn Ser Gly Asp Gly Ser Gly Ser Gln Val Thr
               180                 185                 190
<210> 145
   <211> 191
   <212> PRT
   <213> Artificial Sequence
<220>
   <223> synthetic benoxacor-responsive PYR/PYL receptor mutant clone #129-2
<400> 145
   Met Pro Ser Glu Leu Thr Pro Glu Glu Arg Ser Glu Leu Lys Asn Ser
    1               5                   10                  15
   Ile Ala Glu Phe His Thr Tyr Gln Leu Asp Pro Gly Ser Cys Ser Ser
               20                  25                  30
   Leu His Ala Gln Arg Ile His Ala Pro Pro Glu Leu Val Trp Ser Ile
           35                  40                  45
   Val Arg Arg Phe Asp Lys Pro Gln Thr Tyr Arg His Phe Ile Lys Ser
       50                  55                  60
   Cys Ser Val Glu Gln Asn Phe Glu Met Arg Val Gly Cys Thr Arg Asp
   65                  70                  75                  80
   Val Ile Val Ile Ser Gly Leu Pro Ala Asn Thr Ser Thr Glu Arg Leu
                   85                  90                  95
   Asp Ile Leu Asp Asp Glu Arg Arg Val Thr Gly Phe Ser Ile Ile Gly
               100                 105                 110
   Gly Glu His Arg Leu Thr Tyr Tyr Lys Ser Val Thr Thr Val His Arg
           115                 120                 125
   Phe Glu Lys Glu Asn Arg Ile Trp Thr Val Val Leu Glu Ser Tyr Val
       130                 135                 140
   Val Asp Met Pro Glu Gly Asn Ser Glu Asp Asp Thr Arg Met Phe Ala
   145                 150                 155                 160
   Asp Thr Val Val Lys Leu Asn Leu Gln Lys Leu Ala Thr Val Ala Glu
                   165                 170                 175
   Ala Met Ala Arg Asn Ser Gly Asp Gly Ser Gly Ser Gln Val Thr
               180                 185                 190
<210> 146
   <211> 191
   <212> PRT
   <213> Artificial Sequence
<220>
   <223> synthetic benoxacor-responsive PYR/PYL receptor mutant clone #127-1
<400> 146
   Met Pro Ser Glu Leu Thr Pro Glu Glu Arg Ser Glu Leu Lys Asn Ser
   1               5                   10                  15
   Ile Ala Glu Phe His Thr Tyr Gln Leu Asp Pro Gly Ser Cys Ser Ser
               20                  25                  30
   Leu His Ala Gln Arg Ile His Ala Pro Pro Glu Leu Val Trp Ser Ile
           35                  40                  45
   Val Arg Arg Phe Asp Lys Pro Gln Thr Tyr Lys His Phe Ile Lys Ser
       50                  55                  60
   Cys Ser Val Glu Gln Asn Phe Glu Met Arg Val Gly Cys Thr Arg Asp
   65                  70                  75                  80
   Val Ile Val Ile Ser Gly Leu Pro Ala Asn Thr Ser Thr Glu Arg Leu
                   85                  90                  95
   Asp Ile Leu Asp Asp Glu Arg Arg Val Thr Gly Phe Ser Thr Ile Gly
               100                 105                 110
   Gly Asp His Arg Leu Thr Asn Tyr Lys Ser Val Thr Thr Val His Arg
           115                 120                 125
   Phe Glu Lys Glu Asn Arg Ile Trp Thr Met Val Leu Glu Ser Tyr Val
       130                 135                 140
   Val Asp Met Pro Glu Gly Asn Ser Glu Asp Asp Thr Arg Met Phe Ala
   145                 150                 155                 160
   Asp Thr Val Val Lys Leu Asn Leu Gln Lys Leu Ala Thr Val Ala Glu
                   165                 170                 175
   Ala Met Ala Arg Asn Ser Gly Asp Gly Ser Gly Ser Gln Val Thr
               180                 185                 190
<210> 147
   <211> 191
   <212> PRT
   <213> Artificial Sequence
<220>
   <223> synthetic acibenzolar-S-methyl (BTH)-responsive PYR/PYL receptor mutant clone #BTH-1
<400> 147
   Met Pro Ser Glu Leu Thr Pro Glu Glu Arg Ser Glu Leu Lys Asn Ser
   1               5                   10                  15
   Ile Ala Glu Phe His Thr Tyr Gln Leu Asp Pro Gly Ser Cys Ser Ser
                   20                  25                  30
   Leu His Ala Gln Arg Ile His Ala Pro Pro Glu Leu Val Trp Ser Ile
           35                  40                  45
   Val Arg Arg Phe Asp Lys Pro Gln Thr Tyr Lys His Phe Ile Lys Ser
       50                  55                  60
   Cys Ser Val Glu Gln Asn Phe Glu Met Arg Val Gly Cys Thr Arg Asp
   65                  70                  75                  80
   Val Ile Val Ile Ser Gly Leu Pro Ala Asn Thr Ser Thr Glu Arg Leu
                   85                  90                  95
   Asp Ile Leu Asp Asp Glu Arg Arg Val Thr Gly Phe Ser Ile Ile Gly
               100                 105                 110
   Gly Glu Tyr Arg Leu Thr Asn Tyr Lys Ser Val Thr Thr Val His Arg
           115                 120                 125
   Phe Glu Lys Glu Asn Arg Ile Trp Thr Val Val Leu Glu Ser Tyr Val
       130                 135                 140
   Val Asp Met Pro Glu Gly Asn Ser Glu Asp Asp Thr Arg Met Ser Ala
   145                 150                 155                 160
   Asp Thr Val Val Lys Leu Asn Leu Gln Lys Leu Ala Thr Val Ala Glu
                   165                 170                 175
   Ala Met Ala Arg Asn Ser Gly Asp Gly Ser Gly Ser Gln Val Thr
               180                 185                 190
<210> 148
   <211> 191
   <212> PRT
   <213> Artificial Sequence
<220>
   <223> synthetic acibenzolar-S-methyl (BTH)-responsive PYR/PYL receptor mutant clone #BTH-9
<400> 148
   Met Pro Ser Glu Leu Thr Pro Glu Glu Arg Ser Glu Leu Lys Asn Ser
    1               5                  10                  15
   Ile Ala Glu Phe His Thr Tyr Gln Leu Asp Pro Gly Ser Cys Ser Ser
               20                  25                  30
   Leu His Ala Gln Arg Ile His Ala Pro Pro Glu Leu Val Trp Ser Ile
           35                  40                  45
   Val Arg Arg Phe Asp Lys Pro Gln Thr Tyr Lys His Phe Ile Lys Ser
       50                  55                  60
   Cys Ser Val Glu Gln Asn Phe Glu Met Arg Val Gly Cys Thr Arg Asp
   65                  70                  75                  80
   Val Ile Val Ile Ser Gly Leu Pro Ala Asn Thr Ser Thr Glu Arg Leu
                   85                  90                  95
   Asp Ile Leu Asp Asp Glu Arg Arg Val Thr Gly Phe Ser Ile Ile Gly
               100                 105                 110
   Gly Glu His Arg Leu Thr Asn Tyr Lys Ser Val Thr Thr Val His Arg
           115                 120                 125
   Phe Glu Lys Glu Asn Arg Ile Trp Thr Val Val Leu Glu Ser Tyr Val
       130                 135                 140
   Val Asp Met Pro Glu Gly Asn Ser Glu Asp Asp Thr Arg Met Leu Ala
   145                 150                 155                 160
   Asp Thr Val Val Lys Leu Asn Leu Gln Lys Leu Ala Thr Val Ala Glu
                   165                 170                 175
   Ala Met Ala Arg Asn Ser Gly Asp Gly Ser Gly Ser Gln Val Thr
               180                 185                 190
<210> 149
   <211> 36
   <212> PRT
   <213> Artificial Sequence
<220>
   <223> synthetic modified PYR/PYL receptor consensus sequence with K59 mutation
<220>
   <221> VARIANT
   <222> (1)...(29)
   <223> Xaa = any amino acid
<220>
   <221> VARIANT
   <222> (30)...(30)
   <223> Xaa = Ala, Cys, Asp, Glu, Phe, Gly, His, Leu, Met, Asn, Gln, Arg, Ser, Thr, Val, Tyr or Trp
<220>
   <221> VARIANT
   <222> (31)...(36)
   <223> Xaa = any amino acid
<400> 149
   Cys Xaa Ser Xaa Xaa Xaa Xaa Xaa Xaa Xaa Ala Pro Xaa Xaa Xaa Xaa
    1               5                  10                  15
   Trp Xaa Xaa Xaa Xaa Xaa Phe Xaa Xaa Pro Xaa Xaa Xaa Xaa Xaa Phe
               20                  25                  30
   Xaa Xaa Xaa Cys
           35
<210> 150
   <211> 11
   <212> PRT
   <213> Artificial Sequence
<220>
   <223> synthetic modified PYR/PYL receptor consensus sequence with Y120 mutation
<220>
   <221> VARIANT
   <222> (1)...(8)
   <223> Xaa = any amiono acid
<220>
   <221> VARIANT
   <222> (9)...(9)
   <223> Xaa = His or Cys
<220>
   <221> VARIANT
   <222> (10)...(11)
   <223> Xaa = any amino acid
<400> 150
   Gly Gly Xaa His Arg Leu Xaa Asn Xaa Xaa Ser
    1               5                  10
<210> 151
   <211> 13
   <212> PRT
   <213> Artificial Sequence
<220>
   <223> synthetic modified PYR/PYL receptor consensus sequence with I110 mutation
<220>
   <221> VARIANT
   <222> (1)...(1)
   <223> Xaa = Ser, Thr, Cys, Ala, Tyr or Trp
<220>
   <221> VARIANT
   <222> (2)...(13)
   <223> Xaa = any amino acid
<400> 151
   Xaa Xaa Gly Gly Xaa His Arg Leu Xaa Asn Tyr Xaa Ser
    1               5                  10
<210> 152
   <211> 36
   <212> PRT
   <213> Artificial Sequence
<220>
   <223> synthetic modified PYR/PYL receptor consensus sequence with P42 mutation
<220>
   <221> VARIANT
   <222> (1)...(12)
   <223> Xaa - any amino acid
<220>
   <221> VARIANT
   <222> (13)...(13)
   <223> Xaa = Ser or Thr
<220>
   <221> VARIANT
   <222> (14)...(36)
   <223> Xaa = any amino acid
<400> 152
   Cys Xaa Ser Xaa Xaa Xaa Xaa Xaa Xaa Xaa Ala Pro Xaa Xaa Xaa Xaa
    1               5                  10                  15
   Trp Xaa Xaa Xaa Xaa Xaa Phe Xaa Xaa Pro Xaa Xaa Xaa Xaa Xaa Phe
               20                  25                  30
   Xaa Xaa Xaa Cys
           35
<210> 153
   <211> 36
   <212> PRT
   <213> Artificial Sequence
<220>
   <223> synthetic modified PYR/PYL receptor consensus sequence with S47 mutation
<220>
   <221> VARIANT
   <222> (1)...(17)
   <223> Xaa = any amino acid
<220>
   <221> VARIANT
   <222> (18)...(18)
   <223> Xaa = Pro, Arg or Ala
<220>
   <221> VARIANT
   <222> (19)...(36)
   <223> Xaa = any amino acid
<400> 153
   Cys Xaa Ser Xaa Xaa Xaa Xaa Xaa Xaa Xaa Ala Pro Xaa Xaa Xaa Xaa
    1               5                  10                  15
   Trp Xaa Xaa Xaa Xaa Xaa Phe Xaa Xaa Pro Xaa Xaa Xaa Xaa Xaa Phe
               20                  25                  30
   Xaa Xaa Xaa Cys
           35
<210> 154
   <211> 36
   <212> PRT
   <213> Artificial Sequence
<220>
   <223> synthetic modified PYR/PYL receptor consensus sequence with K59 and I110 mutations
<220>
   <221> VARIANT
   <222> (1)...(12)
   <223> Xaa = any amino acid
<220>
   <221> VARIANT
   <222> (13)...(13)
   <223> Xaa = Ser or Thr
<220>
   <221> VARIANT
   <222> (14)...(29)
   <223> Xaa = any amino acid
<220>
   <221> VARIANT
   <222> (30)...(30)
   <223> Xaa = Ala, Cys, Asp, Glu, Phe, Gly, His, Leu, Met, Asn, Gln, Arg, Ser, Thr, Val, Tyr or Trp
<220>
   <221> VARIANT
   <222> (31)...(36)
   <223> Xaa = any amino acid
<400> 154
   Cys Xaa Ser Xaa Xaa Xaa Xaa Xaa Xaa Xaa Ala Pro Xaa Xaa Xaa Xaa
    1               5                   10                  15
   Trp Xaa Xaa Xaa Xaa Xaa Phe Xaa Xaa Pro Xaa Xaa Xaa Xaa Xaa Phe
               20                  25                  30
   Xaa Xaa Xaa Cys
           35
<210> 155
   <211> 36
   <212> PRT
   <213> Artificial Sequence
<220>
   <223> synthetic modified PYR/PYL receptor consensus sequence with K59 and S47 mutations
<220>
   <221> VARIANT
   <222> (1)...(17)
   <223> Xaa = any amino acid
<220>
   <221> VARIANT
   <222> (18)...(18)
   <223> Xaa = Pro, Arg or Ala
<220>
   <221> VARIANT
   <222> (19)...(29)
   <223> Xaa = any amino acid
<220>
   <221> VARIANT
   <222> (30)...(30)
   <223> Xaa = Ala, Cys, Asp, Glu, Phe, Gly, His, Leu, Met, Asn, Gln, Arg, Ser, Thr, Val, Tyr or Trp
<220>
   <221> VARIANT
   <222> (31)...(36)
   <223> Xaa = any amino acid
<400> 155
   Cys Xaa Ser Xaa Xaa Xaa Xaa Xaa Xaa Xaa Ala Pro Xaa Xaa Xaa Xaa
    1               5                   10                  15
   Trp Xaa Xaa Xaa Xaa Xaa Phe Xaa Xaa Pro Xaa Xaa Xaa Xaa Xaa Phe
               20                  25                  30
   Xaa Xaa Xaa Cys
           35
<210> 156
   <211> 36
   <212> PRT
   <213> Artificial Sequence
<220>
   <223> synthetic modified PYR/PYL receptor consensus sequence with H60 mutation
<220>
   <221> VARIANT
   <222> (1)...(36)
   <223> Xaa = any amino acid
<400> 156
   Cys Xaa Ser Xaa Xaa Xaa Xaa Xaa Xaa Xaa Ala Pro Xaa Xaa Xaa Xaa
    1               5                  10                  15
   Trp Xaa Xaa Xaa Xaa Xaa Phe Xaa Xaa Pro Xaa Xaa Xaa Xaa Arg Phe
               20                  25                  30
   Xaa Xaa Xaa Cys
           35
<210> 157
   <211> 25
   <212> PRT
   <213> Artificial Sequence
<220>
   <223> synthetic modified PYR/PYL receptor consensus sequence with S92 mutation
<220>
   <221> VARIANT
   <222> (1)...(25)
   <223> Xaa = any amino acid
<400> 157
   Gly Xaa Xaa Arg Xaa Val Xaa Xaa Xaa Ser Xaa Xaa Pro Ala Xaa Xaa
    1               5                  10                  15
   Thr Xaa Glu Xaa Leu Xaa Xaa Xaa Asp
               20                  25
<210> 158
   <211> 31
   <212> PRT
   <213> Artificial Sequence
<220>
   <223> synthetic modified PYR/PYL receptor consensus sequence with E140 mutation
<220>
   <221> VARIANT
   <222> (1)...(1)
   <223> Xaa = Gly, Gln or Asp
<220>
   <221> VARIANT
   <222> (2)...(31)
   <223> Xaa = any amino acid
<400> 158
   Xaa Ser Xaa Xaa Val Asp Xaa Pro Xaa Gly Asn Xaa Xaa Xaa Xaa Thr
    1              5                   10                  15
   Xaa Xaa Phe Xaa Xaa Xaa Xaa Xaa Xaa Xaa Asn Leu Xaa Xaa Leu
               20                  25                  30
<210> 159
   <211> 36
   <212> PRT
   <213> Artificial Sequence
<220>
   <223> synthetic modified PYR/PYL receptor consensus sequence with K59 and H60 mutations
<220>
   <221> VARIANT
   <222> (1)...(29)
   <223> Xaa = any amino acid
<220>
   <221> VARIANT
   <222> (30)...(30)
   <223> Xaa = Ala, Cys, Asp, Glu, Phe, Gly, His, Leu, Met, Asn, Gln, Arg, Ser, Thr, Val, Tyr or Trp
<220>
   <221> VARIANT
   <222> (31)...(36)
   <223> Xaa = any amino acid
<400> 159
   Cys Xaa Ser Xaa Xaa Xaa Xaa Xaa Xaa Xaa Ala Pro Xaa Xaa Xaa Xaa
    1              5                   10                  15
   Trp Xaa Xaa Xaa Xaa Xaa Phe Xaa Xaa Pro Xaa Xaa Xaa Xaa Arg Phe
               20                  25                  30
   Xaa Xaa Xaa Cys
           35
<210> 160
   <211> 25
   <212> PRT
   <213> Artificial Sequence
<220>
   <223> synthetic modified PYR/PYL receptor consensus sequence with E94 mutation
<220>
   <221> VARIANT
   <222> (1)...(25)
   <223> Xaa = any amino acid
<400> 160
   Gly Xaa Xaa Arg Xaa Val Xaa Xaa Xaa Ser Xaa Xaa Pro Ala Xaa Xaa
    1              5                   10                  15
   Ser Xaa Asp Xaa Leu Xaa Xaa Xaa Asp
               20                  25
<210> 161
   <211> 11
   <212> PRT
   <213> Artificial Sequence
<220>
   <223> synthetic modified PYR/PYL receptor consensus sequence with K59 and N119 mutations
<220>
   <221> VARIANT
   <222> (1)...(11)
   <223> Xaa = any amino acid
<400> 161
   Gly Gly Xaa His Arg Leu Xaa Tyr Tyr Xaa Ser
    1               5                  10
<210> 162
   <211> 11
   <212> PRT
   <213> Artificial Sequence
<220>
   <223> synthetic modified PYR/PYL receptor consensus sequence with H115 mutation
<220>
   <221> VARIANT
   <222> (1)...(11)
   <223> Xaa = any amino acid
<400> 162
   Gly Gly Xaa Tyr Arg Leu Xaa Asn Tyr Xaa Ser
    1               5                  10
<210> 163
   <211> 31
   <212> PRT
   <213> Artificial Sequence
<220>
   <223> synthetic modified PYR/PYL receptor consensus sequence with F159 mutation
<220>
   <221> VARIANT
   <222> (1)...(18)
   <223> Xaa = any amino acid
<220>
   <221> VARIANT
   <222> (19)...(19)
   <223> Xaa = Ser or Leu
<220>
   <221> VARIANT
   <222> (20)...(31)
   <223> Xaa = any amino acid
<400> 163
   Glu Ser Xaa Xaa Val Asp Xaa Pro Xaa Gly Asn Xaa Xaa Xaa Xaa Thr
    1               5                  10                  15
   Xaa Xaa Xaa Xaa Xaa Xaa Xaa Xaa Xaa Xaa Asn Leu Xaa Xaa Leu
               20                  25                  30
<210> 164
   <211> 191
   <212> PRT
   <213> Artificial Sequence
<220>
   <223> synthetic acibenzolar-S-methyl (BTH)-responsive PYR/PYL receptor mutant clone #BTH-An7
<400> 164
   Met Pro Ser Glu Leu Thr Pro Glu Glu Arg Ser Glu Leu Lys Asn Ser
    1               5                  10                  15
   Ile Ala Glu Phe His Thr Tyr Arg Leu Asp Pro Gly Ser Cys Ser Ser
               20                  25                  30
   Leu His Ala Gln Arg Ile His Ala Pro Pro Glu Leu Val Trp Ser Ile
           35                  40                  45
   Val Arg Arg Phe Asp Lys Pro Gln Thr Tyr Arg His Phe Ile Lys Ser
       50                  55                  60
   Cys Ser Val Glu Gln Asn Phe Glu Met Arg Val Gly Cys Thr Arg Asp
   65                  70                  75                  80
   Val Thr Val Ile Ser Gly Leu Pro Ala Asn Thr Ser Thr Glu Arg Leu
                   85                  90                  95
   Asp Ile Leu Asp Asp Glu Arg Arg Val Thr Gly Phe Ser Ile Ile Gly
               100                 105                 110
   Gly Glu His Arg Leu Thr Asn Tyr Lys Ser Val Thr Thr Val His Arg
           115                 120                 125
   Phe Glu Lys Glu Asn Arg Ile Trp Thr Val Val Leu Glu Ser Tyr Val
       130                 135                 140
   Val Asp Met Pro Glu Gly Asn Ser Glu Asp Asp Thr Arg Met Leu Ala
   145                 150                 155                 160
   Gly Thr Val Val Lys Leu Asn Leu Gln Lys Leu Ala Thr Val Ala Glu
                   165                 170                 175
   Ala Met Ala Arg Asn Ser Gly Asp Gly Ser Gly Ser Gln Val Thr
               180                 185                 190
<210> 165
   <211> 191
   <212> PRT
   <213> Artificial Sequence
<220>
   <223> synthetic fenhexamid-responsive PYR/PYL receptor mutant clones #27B-1 and #27B-8
<400> 165
   Met Pro Ser Glu Leu Thr Pro Glu Glu Arg Ser Glu Leu Lys Asn Ser
    1               5                  10                  15
   Ile Ala Glu Phe His Thr Tyr Gln Leu Asp Pro Gly Ser Cys Ser Ser
               20                  25                  30
   Leu His Ala Gln Arg Ile His Ala Pro Ser Glu Leu Val Trp Ser Ile
           35                  40                  45
   Val Arg Arg Phe Asp Lys Pro Gln Thr Tyr Arg His Phe Ile Lys Ser
       50                  55                  60
   Cys Ser Val Glu Gln Asn Phe Glu Met Arg Val Gly Cys Thr Arg Asp
   65                  70                  75                  80
   Val Ile Val Ile Ser Gly Leu Pro Ala Asn Thr Ser Thr Glu Arg Leu
                   85                  90                  95
   Asn Ile Leu Asp Asp Glu Arg Arg Val Thr Gly Phe Ser Ile Ile Gly
               100                 105                 110
   Gly Glu His Arg Leu Thr Asn His Lys Ser Val Thr Thr Val His Arg
           115                 120                 125
   Phe Glu Lys Glu Asn Arg Ile Trp Thr Val Val Leu Glu Ser Tyr Val
       130                 135                 140
   Val Asp Met Pro Glu Gly Asn Ser Glu Asp Asp Thr Arg Met Phe Ala
   145                 150                 155                 160
   Asp Thr Ile Val Lys Leu Asn Leu Gln Lys Leu Thr Thr Val Ala Glu
                   165                 170                 175
   Ala Met Ala Arg Asn Ser Gly Asp Gly Ser Gly Ser Gln Val Thr
               180                 185                 190
<210> 166
   <211> 191
   <212> PRT
   <213> Artificial Sequence
<220>
   <223> synthetic fenhexamid-responsive PYR/PYL receptor mutant clone #27B-2
<400> 166
   Met Pro Ser Glu Leu Thr Pro Glu Glu Arg Ser Glu Leu Lys Asn Ser
    1               5                  10                  15
   Ile Ala Glu Phe His Thr Tyr Gln Leu Asp Pro Gly Ser Cys Ser Ser
               20                  25                  30
   Leu His Ala Gln Arg Ile His Ala Pro Ser Glu Phe Val Trp Ser Ile
           35                  40                  45
   Val Arg Arg Phe Asp Lys Pro Gln Thr Tyr Arg His Phe Ile Lys Ser
       50                  55                  60
   Cys Ser Val Glu Gln Asn Phe Glu Met Arg Val Gly Cys Thr Arg Asp
   65                  70                  75                  80
   Val Ile Val Ile Ser Gly Leu Pro Ala Asn Thr Ser Thr Glu Arg Leu
                   85                  90                  95
   Asp Ile Leu Asp Asp Glu Arg Arg Val Thr Gly Phe Ser Ile Ile Gly
               100                 105                 110
   Gly Glu His Arg Leu Thr Asn His Lys Ser Val Thr Thr Val His Arg
           115                 120                 125
   Phe Glu Lys Glu Asn Arg Ile Trp Thr Met Val Leu Glu Ser Tyr Val
       130                 135                 140
   Val Asp Met Pro Glu Gly Asn Ser Glu Asp Asp Thr Arg Ile Phe Ala
   145                 150                 155                 160
   Asp Thr Val Val Lys Leu Asn Leu Gln Lys Leu Ala Thr Val Ala Glu
                   165                 170                 175
   Ala Met Ala Arg Asn Ser Gly Asp Gly Ser Gly Ser Gln Val Thr
               180                 185                 190
<210> 167
   <211> 191
   <212> PRT
   <213> Artificial Sequence
<220>
   <223> synthetic fenhexamid-responsive PYR/PYL receptor mutant clone #27B-3
<400> 167
   Met Pro Ser Glu Leu Thr Pro Glu Glu Arg Ser Glu Leu Lys Asn Ser
    1               5                  10                  15
   Ile Ala Glu Phe His Thr Tyr Gln Leu Asp Pro Gly Ser Cys Ser Ser
               20                  25                  30
   Leu His Ala Gln Arg Ile His Ala Pro Ser Glu Leu Val Trp Ser Ile
           35                  40                  45
   Val Arg Arg Phe Asp Lys Pro Gln Thr Tyr Arg His Phe Ile Lys Ser
       50                  55                  60
   Cys Ser Val Glu Gln Asn Phe Glu Met Arg Val Gly Cys Thr Arg Asp
   65                  70                  75                  80
   Val Ile Val Ile Ser Gly Leu Pro Ala Asn Thr Ser Thr Glu Arg Leu
                   85                  90                  95
   Asp Ile Leu Asp Asp Glu Arg Arg Val Thr Gly Phe Ser Ile Ile Gly
               100                 105                 110
   Gly Glu His Arg Leu Thr Asn His Lys Ser Ile Thr Thr Val His Arg
           115                 120                 125
   Phe Glu Lys Glu Asn Arg Ile Trp Thr Val Ile Leu Glu Ser Tyr Val
       130                 135                 140
   Val Asp Met Pro Glu Gly Asn Ser Glu Asp Asp Thr Arg Ile Phe Ala
   145                 150                 155                 160
   Asp Thr Val Val Lys Leu Asn Leu Gln Lys Leu Ala Thr Val Ala Glu
                   165                 170                 175
   Ala Met Ala Arg Asn Ser Gly Asp Gly Ser Gly Ser Gln Val Thr
               180                 185                 190
<210> 168
   <211> 191
   <212> PRT
   <213> Artificial Sequence
<220>
   <223> synthetic fenhexamid-responsive PYR/PYL receptor mutant clone #27B-4
<400> 168
   Met Pro Ser Glu Leu Thr Pro Glu Glu Arg Ser Glu Leu Lys Asn Ser
    1               5                  10                  15
   Ile Ala Glu Phe His Thr Tyr Gln Leu Asp Pro Gly Ser Cys Ser Ser
               20                  25                  30
   Leu His Ala Gln Arg Ile His Ala Pro Pro Glu Leu Val Trp Pro Ile
           35                  40                  45
   Ile Arg Arg Phe Asp Lys Pro Gln Thr Tyr Arg His Phe Ile Lys Ser
       50                  55                  60
   Cys Ser Val Glu Gln Asn Phe Glu Met Arg Val Gly Cys Thr Arg Asp
   65                  70                  75                  80
   Val Ile Val Ile Ser Gly Leu Pro Ala Asn Thr Ser Thr Glu Arg Leu
                   85                  90                  95
   Asp Ile Leu Asp Asp Glu Arg Arg Val Thr Gly Phe Ser Ile Ile Gly
               100                 105                 110
   Gly Glu His Arg Leu Thr Asn His Lys Ser Val Thr Thr Val His Arg
           115                 120                 125
   Phe Glu Lys Glu Asn Arg Ile Trp Thr Val Val Leu Glu Ser Tyr Val
       130                 135                 140
   Val Asp Met Pro Glu Gly Asn Ser Glu Asp Asp Thr Arg Ile Phe Ala
   145                 150                 155                 160
   Asp Thr Val Val Lys Leu Asn Leu Gln Lys Leu Ala Thr Val Ala Glu
                   165                 170                 175
   Thr Met Ala Arg Asn Ser Gly Asp Gly Ser Gly Ser Gln Val Thr
               180                 185                 190
<210> 169
   <211> 191
   <212> PRT
   <213> Artificial Sequence
<220>
   <223> synthetic fenhexamid-responsive PYR/PYL receptor mutant clone #27B-7
<400> 169
   Met Pro Ser Glu Leu Thr Pro Glu Glu Arg Ser Glu Leu Lys Asn Ser
    1               5                  10                  15
   Ile Ala Glu Phe His Thr Tyr Gln Leu Asp Pro Gly Ser Cys Ser Ser
               20                  25                  30
   Leu His Ala Gln Arg Ile His Ala Pro Pro Glu Leu Val Trp Ser Ile
           35                  40                  45
   Val Arg Arg Phe Asp Lys Pro Gln Thr Tyr Arg His Phe Ile Lys Ser
       50                  55                  60
   Cys Ser Val Glu Gln Asn Phe Glu Met Arg Val Gly Cys Thr Arg Asp
   65                  70                  75                  80
   Met Ile Val Ile Ser Gly Leu Pro Ala Asn Thr Ser Thr Glu Arg Leu
                   85                  90                  95
   Asp Ile Leu Asp Asp Glu Arg Arg Val Thr Gly Phe Ser Ile Ile Gly
               100                 105                 110
   Gly Glu His Arg Leu Thr Asn Cys Lys Ser Val Thr Thr Val His Arg
           115                 120                 125
   Phe Glu Lys Glu Asn Arg Ile Trp Thr Val Val Leu Glu Ser Tyr Val
       130                 135                 140
   Val Asp Met Pro Glu Gly Asn Ser Glu Asp Asp Thr Arg Ile Phe Ala
   145                 150                 155                 160
   Asp Thr Ile Val Lys Leu Asn Leu Gln Lys Leu Ala Thr Val Ala Glu
                   165                 170                 175
   Ala Met Ala Arg Asn Ser Gly Asp Gly Ser Gly Ser Gln Val Thr
               180                 185                 190
<210> 170
   <211> 191
   <212> PRT
   <213> Artificial Sequence
<220>
   <223> synthetic fenhexamid-responsive PYR/PYL receptor mutant clone #27C-1
<400> 170
   Met Pro Ser Glu Leu Thr Pro Glu Glu Arg Ser Glu Leu Lys Asn Ser
    1               5                  10                  15
   Ile Ala Glu Phe His Thr Tyr Gln Leu Asp Leu Gly Ser Cys Ser Ser
               20                  25                  30
   Leu His Ala Gln Arg Ile His Ala Pro Ser Glu Leu Val Trp Ser Ile
           35                  40                  45
   Val Arg Arg Phe Asp Lys Pro Gln Thr Tyr Arg His Phe Ile Lys Ser
       50                  55                  60
   Cys Ser Val Glu Gln Asn Phe Glu Met Arg Val Gly Cys Thr Arg Asp
   65                  70                  75                  80
   Val Ile Val Ile Ser Gly Leu Pro Ala Asn Thr Ser Thr Glu Arg Leu
                   85                  90                  95
   Asn Ile Leu Asp Asp Glu Arg Arg Val Thr Gly Phe Ser Ile Ile Gly
               100                 105                 110
   Gly Glu His Arg Leu Thr Asn His Lys Ser Val Thr Thr Val His Arg
           115                 120                 125
   Phe Glu Lys Glu Asn Arg Ile Trp Thr Val Val Leu Glu Ser Tyr Val
       130                 135                 140
   Val Asp Met Pro Glu Gly Asn Ser Glu Asp Asp Thr Arg Ile Phe Ala
   145                 150                 155                 160
   Asp Thr Val Val Lys Leu Asn Leu Gln Lys Leu Ala Ala Val Ala Glu
                   165                 170                 175
   Ala Met Ala Arg Asn Ser Gly Asp Gly Ser Gly Ser Gln Val Thr
               180                 185                 190
<210> 171
   <211> 191
   <212> PRT
   <213> Artificial Sequence
<220>
   <223> synthetic fenhexamid-responsive PYR/PYL receptor mutant clone #27C-2
<400> 171
   Met Pro Ser Glu Leu Thr Pro Glu Glu Arg Ser Glu Leu Lys Asn Ser
    1               5                  10                  15
   Ile Ala Glu Phe His Thr Tyr Gln Leu Asp Pro Gly Ser Cys Ser Ser
               20                  25                  30
   Leu His Ala Gln Arg Ile His Ala Pro Ser Glu Leu Val Trp Ser Ile
           35                  40                  45
   Val Arg Arg Phe Asp Lys Pro Gln Thr Tyr Arg His Phe Ile Lys Ser
       50                  55                  60
   Cys Ser Val Glu Gln Asn Phe Glu Met Cys Val Gly Cys Thr Arg Asp
   65                  70                  75                  80
   Val Ile Val Ile Ser Gly Leu Pro Ala Asn Thr Ser Thr Glu Arg Leu
                   85                  90                  95
   Asp Ile Leu Asp Asp Glu Arg Arg Val Thr Gly Phe Ser Ile Ile Gly
               100                 105                 110
   Gly Glu His Arg Leu Thr Asn His Lys Ser Val Thr Thr Val His Arg
           115                 120                 125
   Phe Glu Lys Glu Asn Arg Ile Trp Thr Val Val Leu Glu Ser Tyr Val
       130                 135                 140
   Val Asp Met Pro Glu Gly Asn Ser Glu Asp Asp Thr Arg Ile Phe Ala
   145                 150                 155                 160
   Asp Thr Val Val Lys Leu Asn Leu Gln Lys Leu Ala Thr Val Ala Glu
                   165                 170                 175
   Ala Met Ala Arg Asn Ser Gly Asp Gly Ser Gly Ser Gln Val Thr
               180                 185                 190
<210> 172
   <211> 191
   <212> PRT
   <213> Artificial Sequence
<220>
   <223> synthetic fenhexamid-responsive PYR/PYL receptor mutant clone #27C-3
<400> 172
   Met Pro Ser Glu Leu Thr Pro Glu Glu Arg Ser Glu Leu Lys Asn Ser
   1               5                   10                  15
   Ile Ala Glu Phe His Thr Tyr Gln Leu Asp Pro Gly Asn Cys Ser Ser
               20                  25                  30
   Leu His Ala Gln Arg Ile His Ala Pro Pro Glu Leu Val Trp Ser Ile
           35                  40                  45
   Val Arg Arg Phe Asp Lys Pro Gln Thr Tyr Arg His Phe Ile Lys Ser
       50                  55                  60
   Cys Ser Val Glu Gln Asn Phe Glu Met Arg Val Gly Cys Thr Arg Asp
   65                  70                  75                  80
   Val Ile Val Ile Ser Gly Leu Pro Ala Asn Thr Ser Thr Glu Arg Leu
                   85                  90                  95
   Asn Ile Leu Asp Asp Glu Arg Arg Val Thr Gly Phe Ser Ile Ile Gly
               100                 105                 110
   Gly Glu His Arg Leu Thr Asn His Lys Ser Val Thr Thr Val His Arg
           115                 120                 125
   Phe Glu Lys Glu Asn Arg Ile Trp Thr Val Val Leu Glu Ser Tyr Val
       130                 135                 140
   Val Asp Met Pro Glu Gly Asn Ser Glu Asp Asp Thr Arg Met Phe Ala
   145                 150                 155                 160
   Asp Thr Ile Val Lys Leu Asn Leu Gln Lys Leu Thr Thr Val Ala Glu
                   165                 170                 175
   Ala Met Ala Arg Asn Ser Gly Asp Gly Ser Gly Ser Gln Val Thr
               180                 185                 190
<210> 173
   <211> 191
   <212> PRT
   <213> Artificial Sequence
<220>
   <223> synthetic fenhexamid-responsive PYR/PYL receptor mutant clone #27C-5
<400> 173
   Met Pro Ser Glu Leu Thr Pro Glu Glu Arg Ser Glu Leu Lys Asn Ser
   1               5                   10                  15
   Ile Ala Glu Phe His Thr Tyr Gln Leu Asp Pro Gly Ser Cys Ser Ser
               20                  25                  30
   Leu His Ala Gln Arg Ile His Ala Pro Ser Glu Leu Val Trp Ser Ile
           35                  40                  45
   Val Arg Arg Phe Asp Lys Pro Gln Thr Tyr Arg His Phe Ile Lys Ser
       50                  55                  60
   Cys Ser Val Glu Gln Asn Phe Glu Met Arg Val Gly Cys Thr Arg Asp
   65                  70                  75                  80
   Val Ile Val Ile Ser Gly Leu Pro Ala Asn Thr Ser Thr Glu Arg Leu
                   85                  90                  95
   Asp Ile Leu Asp Asp Glu Arg Arg Val Thr Gly Phe Ser Ile Ile Gly
               100                 105                 110
   Gly Glu His Arg Leu Thr Asn His Lys Ser Ile Thr Thr Val His Arg
           115                 120                 125
   Phe Glu Lys Glu Asn Arg Ile Trp Thr Val Ile Leu Glu Ser Tyr Val
       130                 135                 140
   Val Asp Met Pro Glu Gly Asn Ser Glu Asp Asp Thr Arg Ile Phe Ala
   145                 150                 155                 160
   Asp Thr Ile Val Lys Leu Asn Leu Gln Lys Leu Ala Thr Val Ala Glu
                   165                 170                 175
   Ala Met Ala Arg Asn Ser Gly Asp Gly Ser Gly Ser Gln Val Thr
               180                 185                 190
<210> 174
   <211> 191
   <212> PRT
   <213> Artificial Sequence
<220>
   <223> synthetic fenhexamid-responsive PYR/PYL receptor mutant clone #27C-16
<400> 174
   Met Pro Ser Glu Leu Thr Pro Glu Glu Arg Ser Glu Leu Lys Asn Ser
    1               5                  10                  15
   Ile Ala Glu Phe His Thr Tyr Gln Leu Asp Pro Gly Ser Cys Ser Ser
               20                  25                  30
   Leu His Ala Gln Arg Ile His Ala Pro Pro Glu Leu Val Trp Ser Ile
           35                  40                  45
   Val Arg Arg Phe Asp Lys Pro Gln Thr Tyr Arg His Phe Ile Lys Ser
       50                  55                  60
   Cys Ser Val Glu Gln Asn Phe Glu Met Arg Val Gly Cys Thr Arg Asp
   65                  70                  75                  80
   Met Ile Val Ile Ser Gly Leu Pro Ala Asn Thr Ser Thr Glu Arg Leu
                   85                  90                  95
   Asp Ile Leu Asp Asp Glu Arg Arg Val Thr Gly Phe Ser Ile Ile Gly
               100                 105                 110
   Gly Glu His Arg Leu Thr Asn His Lys Ser Val Thr Thr Val His Arg
           115                 120                 125
   Phe Glu Lys Glu Asn Arg Ile Trp Thr Val Val Leu Glu Ser Tyr Val
       130                 135                 140
   Val Asp Met Pro Glu Gly Asn Ser Glu Asp Asp Thr Arg Ile Phe Ala
   145                 150                 155                 160
   Asp Thr Ile Val Lys Leu Asn Leu Gln Lys Leu Ala Thr Val Ala Glu
                   165                 170                 175
   Ala Met Ala Arg Asn Ser Gly Asp Gly Ser Gly Ser Gln Val Thr
               180                 185                 190
<210> 175
   <211> 191
   <212> PRT
   <213> Artificial Sequence
<220>
   <223> synthetic fenhexamid-responsive PYR/PYL receptor mutant clone #27C-18
<400> 175
   Met Pro Ser Glu Leu Thr Pro Glu Glu Arg Ser Lys Leu Lys Asn Ser
    1               5                  10                  15
   Ile Ala Glu Phe His Thr Tyr Gln Leu Asp Pro Gly Ser Cys Ser Ser
               20                  25                  30
   Leu His Ala Gln Arg Ile His Ala Pro Pro Glu Leu Val Trp Ser Ile
           35                  40                  45
   Val Arg Arg Phe Asp Lys Pro Gln Thr Tyr Arg His Phe Ile Lys Ser
       50                  55                  60
   Cys Ser Val Glu Gln Asn Phe Glu Met Arg Ile Gly Cys Thr Arg Asp
   65                  70                  75                  80
   Val Ile Val Ile Ser Gly Leu Pro Ala Asn Thr Ser Thr Glu Arg Leu
                   85                  90                  95
   Asn Ile Leu Asp Asp Glu Arg Arg Val Thr Gly Phe Ser Ile Ile Gly
               100                 105                 110
   Gly Glu His Arg Leu Thr Asn His Lys Ser Val Thr Thr Val His Arg
           115                 120                 125
   Phe Glu Lys Glu Asn Arg Ile Trp Thr Val Val Leu Glu Ser Tyr Val
       130                 135                 140
   Val Asp Met Pro Glu Gly Asn Ser Glu Asp Asp Thr Arg Met Phe Ala
   145                 150                 155                 160
   Asp Thr Ile Val Lys Leu Asn Leu Gln Lys Leu Thr Thr Val Ala Glu
                   165                 170                 175
   Ala Met Ala Arg Asn Ser Gly Asp Gly Ser Gly Ser Gln Val Thr
               180                 185                 190
<210> 176
   <211> 191
   <212> PRT
   <213> Artificial Sequence
<220>
   <223> synthetic fenhexamid-responsive PYR/PYL receptor mutant clone #27C-19
<400> 176
   Met Pro Ser Glu Leu Thr Pro Glu Glu Arg Ser Glu Leu Lys Asn Ser
    1               5                  10                  15
   Ile Ala Glu Phe His Thr Tyr Gln Leu Asp Pro Gly Ser Cys Ser Ser
               20                  25                  30
   Phe His Ala Gln Arg Ile His Ala Pro Ser Glu Leu Val Trp Ser Ile
           35                  40                  45
   Val Arg Arg Phe Asp Lys Pro Gln Thr Tyr Arg His Phe Ile Lys Ser
       50                  55                  60
   Cys Ser Val Glu Gln Asn Phe Glu Met Arg Val Gly Cys Thr Arg Asp
   65                  70                  75                  80
   Val Ile Val Ile Ser Gly Leu Pro Ala Asn Thr Ser Thr Glu Arg Leu
                   85                  90                  95
   Asp Ile Leu Asp Asp Glu Arg Arg Val Thr Gly Phe Ser Ile Ile Gly
               100                 105                 110
   Gly Glu His Arg Leu Thr Asn His Lys Ser Ile Thr Thr Val His Arg
           115                 120                 125
   Phe Glu Lys Glu Asn Arg Ile Trp Thr Val Val Leu Glu Ser Tyr Val
       130                 135                 140
   Val Asp Met Pro Glu Gly Asn Ser Glu Asp Asp Thr Arg Ile Phe Ala
   145                 150                 155                 160
   Asp Thr Val Val Lys Leu Asn Leu Gln Lys Leu Ala Thr Val Ala Glu
                   165                 170                 175
   Ala Met Ala Arg Asn Ser Gly Asp Gly Ser Gly Ser Gln Val Thr
               180                 185                 190
<210> 177
   <211> 191
   <212> PRT
   <213> Artificial Sequence
<220>
   <223> synthetic fenhexamid-responsive PYR/PYL receptor mutant clone #27C-20
<400> 177
   Met Pro Ser Glu Leu Thr Pro Glu Glu Arg Ser Glu Leu Lys Asn Ser
    1               5                  10                  15
   Ile Ala Glu Phe His Thr Tyr Gln Leu Asp Pro Gly Ser Cys Ser Ser
                   20                  25                  30
   Leu His Ala Gln Arg Ile His Ala Pro Ser Glu Leu Val Trp Ser Ile
           35                  40                  45
   Val Arg Arg Phe Asp Lys Pro Gln Thr Tyr Arg His Phe Ile Lys Ser
       50                  55                  60
   Cys Ser Val Glu Gln Asn Phe Glu Met Arg Val Gly Cys Thr Arg Asp
   65                  70                  75                  80
   Val Ile Val Ile Ser Gly Leu Pro Ala Asn Thr Ser Thr Glu Arg Leu
                   85                  90                  95
   Asp Ile Leu Asp Asp Glu Arg Arg Val Thr Gly Phe Ser Ile Ile Gly
               100                 105                 110
   Gly Glu His Arg Leu Thr Asn His Lys Ser Val Thr Thr Val His Arg
           115                 120                 125
   Phe Glu Lys Glu Asn Arg Ile Trp Thr Val Val Leu Glu Ser Tyr Val
       130                 135                 140
   Val Asp Met Pro Glu Gly Asn Ser Glu Asp Asp Thr Arg Ile Phe Ala
   145                 150                 155                 160
   Asp Thr Ile Val Lys Leu Asn Leu Gln Lys Leu Ala Thr Ile Ala Glu
                   165                 170                 175
   Ala Met Ala Arg Asn Ser Gly Asp Gly Ser Gly Ser Gln Val Thr
               180                 185                 190
<210> 178
   <211> 191
   <212> PRT
   <213> Artificial Sequence
<220>
   <223> synthetic fenhexamid-responsive PYR/PYL receptor mutant clone #27C-21
<400> 178
   Met Pro Ser Glu Leu Thr Pro Glu Glu Gln Ser Glu Leu Lys Asn Ser
    1               5                  10                  15
   Ile Ala Glu Phe His Thr Tyr Gln Leu Asp Pro Gly Ser Cys Ser Ser
               20                  25                  30
   Leu His Ala Gln Arg Ile His Ala Pro Ser Glu Leu Val Trp Ser Ile
           35                  40                  45
   Val Arg Arg Phe Asp Lys Pro Gln Thr Tyr Arg His Phe Ile Lys Ser
       50                  55                  60
   Cys Ser Val Glu Gln Asn Phe Glu Met Arg Val Gly Cys Thr Arg Asp
   65                  70                  75                  80
   Val Ile Val Ile Ser Gly Leu Pro Ala Asn Thr Ser Thr Glu Arg Leu
                   85                  90                  95
   Asn Ile Leu Asp Asp Glu Arg Arg Val Thr Gly Phe Ser Ile Ile Gly
               100                 105                 110
   Gly Glu His Arg Leu Thr Asn His Lys Ser Val Thr Thr Val His Arg
           115                 120                 125
   Phe Glu Lys Glu Asn Arg Ile Trp Thr Val Val Leu Glu Ser Tyr Val
       130                 135                 140
   Val Asp Met Pro Glu Gly Asn Ser Glu Asp Asp Thr Arg Met Phe Ala
   145                 150                 155                 160
   Asp Thr Ile Val Lys Leu Asn Leu Gln Lys Leu Thr Thr Val Ala Glu
                   165                 170                 175
   Ala Met Ala Arg Asn Ser Gly Asp Gly Ser Gly Ser Gln Val Thr
               180                 185                 190
<210> 179
   <211> 6
   <212> PRT
   <213> Artificial Sequence
<220>
   <223> synthetic N-terminus 6X-histidine tag
<400> 179
   His His His His His His
    1               5

## Description

### BACKGROUND OF THE INVENTION

Rising temperatures and lessening fresh water supplies are two forms of environmental stress, also called abiotic stress, that lower the amount of food produced by agriculture. A key regulator of abiotic stress tolerance is the plant hormone abscisic acid (ABA), which is synthesized by plants in response to various abiotic stresses and orchestrates adaptive responses that enhance plant survival (Cutler, S. et al., Annual Review of Plant Biology (2009); Nambara, E. et al., Annual Review of Plant Biology 56:165-185 (2005)). Crop plants engineered to have increased ABA sensitivity show improved yield under conditions of drought (Wang, Y. et al., Plant J 43:413-424 (2005)). Moreover, the direct application of ABA or ABA analogs to plants in the field has been shown to improve water use efficiency (Hawkins, A.F. et al., Plant Growth Regulators for Agricultural and Amenity Use (British Crop Protection Council) (1987); Kreeb, K.H. et al., Structural and Functional Responses to Environmental Stresses (Balogh Scientific Books) (1989)); however, ABA has not been successfully commercialized for this use given its complicated production routes and high cost.

Interestingly, numerous fungicides and insecticides have shown stress-tolerance "side-effects" of unknown mechanism and have been commercialized for stress-tolerance uses, which demonstrates the strong interest in, and recognized need for chemical methods to control stress tolerance (Asrar, J. et al., In US 2009/0270254 A1 (USA, Monsanto Technology) (2003); Beckers, G.J.M. et al., Current Opinion in Plant Biology 10:425-431 (2007); Schulz, A. et al., In US 2007/0124839 A1 (USA, Bayer Crop Sciences) (2006)). An important driver of this interest has been the realization that the dramatic increases in corn yield achieved over last 100 years can be attributed largely to improvements in abiotic stress tolerance of new high-yielding corn varieties (Duvick, D.N. et al., Crop Science 39:1622-1630 (1999); Tollenaar, M. et al., Field Crops Research 75:161-169 (2002); Tollenaar, M. et al., Crop Sci 39:1597-1604 (1999)). Because ABA is recognized as the critical hormonal regulator of plant stress physiology, there is intense interest in modulating the ABA pathway in crops. One possible point at which to control the ABA signaling pathway is receptor proteins, which in principle would allow both chemical and genetic modulation of ABA signaling and stress tolerance.

Recently a new family of ABA receptors, the Pyrabactin resistance/PYR-like ("PYR/PYL") family, was identified as a modulator of ABA signaling (Park, S.Y. et al., Science 324:1068-1071 (2009)). The over-expression of the ABA receptor PYL5 confers drought tolerance on Arabidopsis plants (Santiago, J. et al., The Plant Journal 9999 (2009)), validating this new receptor family as a key target for control of plant stress tolerance. However, gene over-expression can have adverse yield consequences, which are referred to as "yield drag". Yield drag is thought to occur because the unregulated activation of stress tolerance pathways, which is associated with slowed growth, occurs under normal conditions (*i. e.* in the absence of drought or other stressors). One way to gain regulated control of ABA signaling is to develop chemical agents that activate ABA receptors (*i.e.* agonists). These can be applied to plants once drought or other stress conditions have ensued, which allows for selective protection in adverse conditions. This allows the benefits of stress tolerance to be realized without lowering yield under ideal growth conditions.

In principle, ABA could be used as an agonist to realize these advantages. However, it is a natural product that is costly to make and rapidly degraded by both UV photo-isomerization and metabolic inactivation. It also has physiological effects in mammals that could conceivably affect its suitability for use as an agrochemical (Guri, A.J. et al., Clin Nutr. (2010)). Therefore, a cheap, environmentally stable and non-toxic molecule would be an ideal reagent with which to control ABA signaling.

### BRIEF SUMMARY OF THE INVENTION

The present invention provides for plants (or a plant cell, seed, flower, leaf or fruit) comprising a heterologous expression cassette, the expression cassette comprising a promoter operably linked to a polynucleotide encoding a mutated PYR/PYL receptor polypeptide, wherein the mutated PYR/PYL receptor polypeptide comprises a mutation at an amino acid residue corresponding to position K59 of PYR1 (SEQ ID NO:1), wherein the amino acid residue is mutated to alanine, cysteine, phenylalanine, glycine, histidine, leucine, methionine, arginine, serine, threonine, tyrosine, valine, asparagine, or tryptophan, wherein the mutated PYR/PYL receptor polypeptide is agonized by a chemical when the chemical is contacted to the mutated PYR/PYL receptor polypeptide and wherein the chemical does not significantly agonize a wild-type PYR/PYL receptor polypeptide when the chemical is contacted to the wild-type PYR/PYL receptor polypeptide.

In some embodiments, the chemical comprises a fungicide, an herbicide, a pesticide, a nematicide, a plant activator, a synergist, an herbicide safener, a plant growth regulator, an insect repellant, or a fertilizer.

In some embodiments, the chemical is selected from the group consisting of bromoxynil, chloroxynil, ioxynil, coumatetralyl, dichlobenil, fenhexamid, benoxacor, and BTH (acibenzolar-s-methyl).

In some instances described herein, the amino acid of the mutated PYR/PYL receptor polypeptide corresponding to position K59 of SEQ ID NO:1 is X, wherein X is alanine, cysteine, aspartic acid, glutamic acid, phenylalanine, glycine, histidine, leucine, methionine, glutamine, arginine, serine, threonine, valine, tyrosine, asparagine, or tryptophan.

In some instances described herein, the amino acid of the mutated PYR/PYL receptor polypeptide corresponding to position K59 of SEQ ID NO:1 is X, wherein X is alanine, cysteine, aspartic acid, glutamic acid, phenylalanine, glycine, histidine, leucine, methionine, glutamine, arginine, serine, threonine, valine, tyrosine, asparagine, or tryptophan, and the chemical is bromoxynil, chloroxynil, ioxynil, dichlobenil, benoxacor, or fenhexamid.

In some instances described herein, wherein the mutated PYR/PYL receptor polypeptide is agonized by bromoxynil, chloroxynil, or ioxynil when the bromoxynil, chloroxynil, or ioxynil is contacted to the mutated PYR/PYL receptor polypeptide, the amino acid of the mutated PYR/PYL receptor polypeptide corresponding to position K59 of SEQ ID NO:1 is X, wherein X is alanine, cysteine, aspartic acid, glutamic acid, phenylalanine, glycine, histidine, leucine, methionine, glutamine, arginine, serine, threonine, valine, tyrosine, asparagine, or tryptophan, and the mutated PYR/PYL receptor polypeptide further comprises at least one additional mutation at an amino acid corresponding to positions 21, 41, 50, 57, 60, 82, 92, 102, 116, 125, 141, and/or 151 in PYR1 (SEQ ID NO:1) wherein the mutation is selected from H21Y, P41L, R50G, T57A, H60R, I82N, S92T, E102G, R116K, T125A, E141Q, E141D, N151D, or combinations thereof.

In some instances described herein, wherein the mutated PYR/PYL receptor polypeptide is agonized by fenhexamid when the fenhexamid is contacted to the mutated PYR/PYL receptor polypeptide, the amino acid of the mutated PYR/PYL receptor polypeptide corresponding to position K59 of SEQ ID NO:1 is X, wherein X is alanine, cysteine, aspartic acid, glutamic acid, phenylalanine, glycine, histidine, leucine, methionine, glutamine, arginine, serine, threonine, valine, tyrosine, asparagine, or tryptophan, and the mutated PYR/PYL receptor polypeptide further comprises at least one additional mutation at an amino acid corresponding to positions 10, 12, 25, 27, 29, 33, 42, 43, 44, 47, 49, 74, 75, 81, 97, 110, 120, 123, 124, 133, 138, 139, 144, 154, 158, 163, 172, 173, 174, and/or 177 in PYR1 (SEQ ID NO:1) wherein the mutation is selected from R10Q, E12G, E12K, L25R, P27L, S29N, L33F, P42S, E43G, L44F, S47P, V49I, R74C, V75I, V81M, D97N, I110S, Y120C, Y120H, V123I, T124M, N133D, V138M, V139I, V144A, E154G, M158I, V163I, A172T, T173A, V174I, A177T or combinations thereof.

In some embodiments, wherein the mutated PYR/PYL receptor polypeptide is agonized by fenhexamid when the fenhexamid is contacted to the mutated PYR/PYL receptor polypeptide, the mutated PYR/PYL receptor polypeptide comprises mutations at amino acids corresponding to positions 59, 120, and 158 in PYR1 (SEQ ID NO:1) wherein the mutations are K59R, Y120H, and M158I. In some embodiments, the mutated PYR/PYL receptor polypeptide further comprises isoleucine residues at the amino acid positions corresponding to positions 62 and 110 in PYR1 (SEQ ID NO:1).

In some instances described herein, wherein the mutated PYR/PYL receptor polypeptide is agonized by dichlobenil when the dichlobenil is contacted to the mutated PYR/PYL receptor polypeptide, the amino acid of the mutated PYR/PYL receptor polypeptide corresponding to position K59 of SEQ ID NO:1 is X, wherein X is alanine, cysteine, aspartic acid, glutamic acid, phenylalanine, glycine, histidine, leucine, methionine, glutamine, arginine, serine, threonine, valine, tyrosine, asparagine, or tryptophan, and the mutated PYR/PYL receptor polypeptide further comprises at least one additional mutation at an amino acid corresponding to positions 27 and/or 63 in PYR1 (SEQ ID NO:1) wherein the mutation is selected from P27L, K63N, or combinations thereof.

In some instances, wherein the mutated PYR/PYL receptor polypeptide is agonized by dichlobenil when the dichlobenil is contacted to the mutated PYR/PYL receptor polypeptide, the mutated PYR/PYL receptor polypeptide comprises at least one mutation at an amino acid corresponding to positions 26, 37, 71, and/or 94 in PYR1 (SEQ ID NO:1) wherein the mutation is selected from D26G, R37Q, F71S, E94D, or combinations thereof.

In some instances, wherein the mutated PYR/PYL receptor polypeptide is agonized by benoxacor when the benoxacor is contacted to the mutated PYR/PYL receptor polypeptide, the amino acid of the mutated PYR/PYL receptor polypeptide corresponding to position K59 of SEQ ID NO:1 is X, wherein X is alanine, cysteine, aspartic acid, glutamic acid, phenylalanine, glycine, histidine, leucine, methionine, glutamine, arginine, serine, threonine, valine, tyrosine, asparagine, or tryptophan, and the mutated PYR/PYL receptor polypeptide further comprises a mutation at an amino acid corresponding to position 119 in PYR1 (SEQ ID NO:1) wherein the mutation is N119Y.

In some instances, wherein the mutated PYR/PYL receptor polypeptide is agonized by benoxacor when the benoxacor is contacted to the mutated PYR/PYL receptor polypeptide, the mutated PYR/PYL receptor polypeptide comprises at least one mutation at an amino acid corresponding to positions 110, 114, and/or 138 in PYR1 (SEQ ID NO:1) wherein the mutation is selected from I110T, E114D V138M, or combinations thereof.

In some instances, wherein the mutated PYR/PYL receptor polypeptide is agonized by BTH when the BTH is contacted to the mutated PYR/PYL receptor polypeptide, the amino acid of the mutated PYR/PYL receptor polypeptide corresponding to position K59 of SEQ ID NO:1 is X, wherein X is alanine, cysteine, aspartic acid, glutamic acid, phenylalanine, glycine, histidine, leucine, methionine, glutamine, arginine, serine, threonine, valine, tyrosine, asparagine, or tryptophan, and the mutated PYR/PYL receptor polypeptide further comprises at least one mutation at an amino acid corresponding to positions 24, 82, 159, and/or 161 in PYR1 (SEQ ID NO:1) wherein the mutation is selected from Q24R, I82T, F159L, D161G, or combinations thereof.

In some instances, wherein the mutated PYR/PYL receptor polypeptide is agonized by BTH when the BTH is contacted to the mutated PYR/PYL receptor polypeptide, the mutated PYR/PYL receptor polypeptide comprises at least one mutation at an amino acid corresponding to positions 115 and/or 159 in PYR1 (SEQ ID NO:1) wherein the mutation is selected from H115Y, F159S, F159L, or combinations thereof.

In some instances, the mutated PYR/PYL receptor polypeptide is substantially identical to *(e.g.,* at least 70%, at least 75%, at least 80%, at least 85%, at least 90%, at least 91%, at least 92%, at least 93%, at least 94%, at least 95%, at least 96%, at least 97%, at least 98%, or at least 99% identical to) any of SEQ ID NO:1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, or 119 and comprises one or more mutations as described herein.

In some instances, the mutated PYR/PYL receptor polypeptide has the sequence of, or is substantially identical to *(e.g.,* at least 70%, at least 75%, at least 80%, at least 85%, at least 90%, at least 91%, at least 92%, at least 93%, at least 94%, at least 95%, at least 96%, at least 97%, at least 98%, or at least 99% identical to) any of SEQ ID NOs:131-139 *(i.e.,* any of SEQ ID NO:131, 132, 133, 134, 135, 136, 137, 138, or 139) and the mutated PYR/PYL receptor polypeptide is agonized by bromoxynil, chloroxynil, or ioxynil when the bromoxynil, chloroxynil, or ioxynil is contacted to the mutated PYR/PYL receptor polypeptide.

In some instances, the mutated PYR/PYL receptor polypeptide has the sequence of, or is substantially identical to *(e.g.,* at least 70%, at least 75%, at least 80%, at least 85%, at least 90%, at least 91%, at least 92%, at least 93%, at least 94%, at least 95%, at least 96%, at least 97%, at least 98%, or at least 99% identical to) any of SEQ ID NOs:124-130 or 165-178 *(i.e.,* any of SEQ ID NO:124, 125, 126, 127, 128, 129, 130, 165, 166, 167, 168, 169, 170, 171, 172, 173, 174, 175, 176, 177, or 178) and the mutated PYR/PYL receptor polypeptide is agonized by fenhexamid when the fenhexamid is contacted to the mutated PYR/PYL receptor polypeptide.

In some instances, the mutated PYR/PYL receptor polypeptide has the sequence of, or is substantially identical to *(e.g.,* at least 70%, at least 75%, at least 80%, at least 85%, at least 90%, at least 91%, at least 92%, at least 93%, at least 94%, at least 95%, at least 96%, at least 97%, at least 98%, or at least 99% identical to) any of SEQ ID NOs:140-144 *(i.e.,* any of SEQ ID NO:140, 141, 142, 143, or 144) and the mutated PYR/PYL receptor polypeptide is agonized by dichlobenil when the dichlobenil is contacted to the mutated PYR/PYL receptor polypeptide.

In some instances, the mutated PYR/PYL receptor polypeptide has the sequence of, or is substantially identical to *(e.g.,* at least 70%, at least 75%, at least 80%, at least 85%, at least 90%, at least 91%, at least 92%, at least 93%, at least 94%, at least 95%, at least 96%, at least 97%, at least 98%, or at least 99% identical to) any of SEQ ID NOs:145 or 146 and the mutated PYR/PYL receptor polypeptide is agonized by benoxacor when the benoxacor is contacted to the mutated PYR/PYL receptor polypeptide.

In some instances, the mutated PYR/PYL receptor polypeptide has the sequence of, or is substantially identical to *(e.g.,* at least 70%, at least 75%, at least 80%, at least 85%, at least 90%, at least 91%, at least 92%, at least 93%, at least 94%, at least 95%, at least 96%, at least 97%, at least 98%, or at least 99% identical to) any of SEQ ID NOs:147, 148 or 164, and the mutated PYR/PYL receptor polypeptide is agonized by BTH (acibenzolar-s-methyl) when the BTH is contacted to the mutated PYR/PYL receptor polypeptide.

In some instances, the mutated PYR/PYL receptor polypeptide comprises at least one mutation at an amino acid residue comprising the ligand-binding pocket of the PYR/PYL receptor polypeptide.

In some embodiments, the plant has improved abiotic stress tolerance when contacted with the chemical as compared to a plant lacking the expression cassette.

The present invention further provides a plant cell, seed, flower, leaf or fruit of a plant of the invention, comprising the heterologous expression cassette.

The present invention also provides for methods of improving abiotic stress tolerance in such plants of the invention by contacting the plant with a chemical selected from the group consisting of bromoxynil, chloroxynil, ioxynil, coumatetralyl, dichlobenil, fenhexamid, benoxacor, and BTH (acibenzolar-s-methyl).

The present disclosure also provides for polypeptides comprising the mutated PYR/PYL receptor polypeptides as described herein. In some instances a polypeptide comprises a mutated PYR/PYL receptor polypeptide that has the sequence of, or is substantially identical to *(e.g.,* at least 70%, at least 75%, at least 80%, at least 85%, at least 90%, at least 91%, at least 92%, at least 93%, at least 94%, at least 95%, at least 96%, at least 97%, at least 98%, or at least 99% identical to) any of SEQ ID NOs:124-148 or 164-178 (*i.e*., any of SEQ ID NO:124, 125, 126, 127, 128, 129, 130, 131, 132, 133, 134, 135, 136, 137, 138, 139, 140, 141, 142, 143, 144, 145, 146, 147, 148, 164, 165, 166, 167, 168, 169, 170, 171, 172, 173, 174, 175, 176, 177, or 178).

The present disclosure further provides for polynucleotides encoding one or more of the mutated PYR/PYL receptor polypeptides as described herein. In some instances, a polynucleotide encodes one or more mutated PYR/PYL receptor polypeptides that has the sequence of, or is substantially identical to *(e.g.,* at least 70%, at least 75%, at least 80%, at least 85%, at least 90%, at least 91%, at least 92%, at least 93%, at least 94%, at least 95%, at least 96%, at least 97%, at least 98%, or at least 99% identical to) any of SEQ ID NOs:124-148 or 164-178 (*i.e*., any of SEQ ID NO:124, 125, 126, 127, 128, 129, 130, 131, 132, 133, 134, 135, 136, 137, 138, 139, 140, 141, 142, 143, 144, 145, 146, 147, 148, 164, 165, 166, 167, 168, 169, 170, 171, 172, 173, 174, 175, 176, 177, or 178). The present disclosure further provides for isolated nucleic acids comprising a polynucleotide sequence encoding one or more of the mutated PYR/PYL receptor polypeptides as described herein.

The invention provides a polypeptide comprising a mutated PYR/PYL receptor polypeptide that:
(i) has at least 70% identity to any of SEQ ID NOs:124-139, 143, 144, 146 or 164-178 and comprises a mutation at an amino acid residue corresponding to position K59 of PYR1 (SEQ ID NO:1), wherein the amino acid residue is mutated to alanine, cysteine, phenylalanine, glycine, histidine, leucine, methionine, arginine, serine, threonine, tyrosine, valine, asparagine, or tryptophan, and/or
(ii) has at least 70% sequence identity to any of SEQ ID NO:147, 148, 164 and comprises a mutation at an amino acid residue corresponding to position F159, wherein the amino acid residue is mutated to leucine or serine;
wherein the mutated PYR/PYL receptor polypeptide is agonized by a chemical when the chemical is contacted to the mutated PYR/PYL receptor polypeptide, wherein the chemical does not significantly agonize a wild-type PYR/PYL receptor polypeptide when the chemical is contacted to the wild-type PYR/PYL receptor polypeptide;
or a polynucleotide encoding said polypeptide.

The present invention also provides for methods of making a mutated PYR/PYL receptor polypeptide that is agonized by a chemical when the chemical is contacted to the mutated PYR/PYL receptor polypeptide, wherein the chemical does not significantly agonize a wild-type PYR/PYL receptor polypeptide when the chemical is contacted to the wild-type PYR/PYL receptor polypeptide, the method comprising
(a) mutagenizing the wild-type PYR/PYL receptor polypeptide;
(b) contacting one or more mutated PYR/PYL receptor polypeptides with the chemical; and
(c) determining whether the chemical activates the one or more mutated PYR/PYL receptor polypeptides, wherein activation identifies the one or more mutated PYR/PYL receptor polypeptides as being agonized by the chemical.

In some embodiments, the method further comprises, prior to step (b), screening the chemical to determine whether the chemical binds to the wild-type PYR/PYL receptor polypeptide prior to contacting the one or more mutated PYR/PYL receptor polypeptides with the chemical.

In some instances, determining step (c) of the method comprises contacting the chemical to a cell comprising a two-hybrid system, wherein the two-hybrid system detects interaction of the mutated PYR/PYL receptor polypeptide with a type 2 protein phosphatase (PP2C), and wherein chemical-specific interaction of the mutated PYR/PYL receptor polypeptide with the PP2C identifies the mutated PYR/PYL receptor polypeptide as being agonized by the chemical.

In some instances, the chemical of the method comprises a fungicide, an herbicide, a pesticide, a nematicide, a plant activator, a synergist, an herbicide safener, a plant growth regulator, an insect repellant, or a fertilizer.

In some instances, the chemical of the method is selected from the group consisting of bromoxynil, chloroxynil, ioxynil, coumatetralyl, dichlobenil, fenhexamid, benoxacor, and BTH (acibenzolar-s-methyl).

The present disclosure also provides for expression cassettes comprising a promoter operably linked to a polynucleotide encoding a mutated PYR/PYL receptor polypeptide, wherein the mutated PYR/PYL receptor polypeptide is agonized by a chemical when the chemical is contacted to the mutated PYR/PYL receptor polypeptide and wherein the chemical does not significantly agonize a wild-type PYR/PYL receptor polypeptide when the chemical is contacted to the wild-type PYR/PYL receptor polypeptide.

Specifically, the present invention provides an expression cassette comprising a promoter operably linked to a polynucleotide encoding a mutated PYR/PYL receptor polypeptide, wherein the mutated PYR/PYL receptor polypeptide comprises a mutation at an amino acid residue corresponding to position K59 of PYR1 (SEQ ID NO:1), wherein the amino acid residue is mutated to alanine, cysteine, phenylalanine, glycine, histidine, leucine, methionine, arginine, serine, threonine, tyrosine, valine, asparagine, or tryptophan, wherein the mutated PYR/PYL receptor polypeptide is agonized by a chemical when the chemical is contacted to the mutated PYR/PYL receptor polypeptide and wherein the chemical does not significantly agonize a wild-type PYR/PYL receptor polypeptide when the chemical is contacted to the wild-type PYR/PYL receptor polypeptide;
or an expression vector comprising said expression cassette.

In some instances, the expression cassette comprises a promoter operably linked to a polynucleotide encoding a mutated PYR/PYL receptor polypeptide, wherein the amino acid of the mutated PYR/PYL receptor polypeptide corresponding to position K59 of SEQ ID NO:1 is X, wherein X is alanine, cysteine, aspartic acid, glutamic acid, phenylalanine, glycine, histidine, leucine, methionine, glutamine, arginine, serine, threonine, valine, tyrosine, asparagine, or tryptophan. In some instances, the amino acid of the mutated PYR/PYL receptor polypeptide corresponding to position K59 of SEQ ID NO:1 is X, wherein X is alanine, cysteine, aspartic acid, glutamic acid, phenylalanine, glycine, histidine, leucine, methionine, glutamine, arginine, serine, threonine, valine, tyrosine, asparagine, or tryptophan, and the chemical is bromoxynil, chloroxynil, ioxynil, dichlobenil, benoxacor, or fenhexamid.

In some instances, the expression cassette comprises a promoter operably linked to a polynucleotide encoding a mutated PYR/PYL receptor polypeptide, wherein the mutated PYR/PYL receptor polypeptide is agonized by bromoxynil, chloroxynil, or ioxynil when the bromoxynil, chloroxynil, or ioxynil is contacted to the mutated PYR/PYL receptor polypeptide, and the amino acid of the mutated PYR/PYL receptor polypeptide corresponding to position K59 of SEQ ID NO:1 is X, wherein X is alanine, cysteine, aspartic acid, glutamic acid, phenylalanine, glycine, histidine, leucine, methionine, glutamine, arginine, serine, threonine, valine, tyrosine, asparagine, or tryptophan, and the mutated PYR/PYL receptor polypeptide further comprises at least one additional mutation at an amino acid corresponding to positions 21, 41, 50, 57, 60, 82, 92, 102, 116, 125, 141, and/or 151 in PYR1 (SEQ ID NO:1) wherein the mutation is selected from H21Y, P41L, R50G, T57A, H60R, I82N, S92T, E102G, R116K, T125A, E141Q, E141D, N151D, or combinations thereof.

In some instances, the expression cassette comprises a promoter operably linked to a polynucleotide encoding a mutated PYR/PYL receptor polypeptide, wherein the mutated PYR/PYL receptor polypeptide is agonized by fenhexamid when the fenhexamid is contacted to the mutated PYR/PYL receptor polypeptide, and the amino acid of the mutated PYR/PYL receptor polypeptide corresponding to position K59 of SEQ ID NO:1 is X, wherein X is alanine, cysteine, aspartic acid, glutamic acid, phenylalanine, glycine, histidine, leucine, methionine, glutamine, arginine, serine, threonine, valine, tyrosine, asparagine, or tryptophan, and the mutated PYR/PYL receptor polypeptide further comprises at least one additional mutation at an amino acid corresponding to positions 10, 12, 25, 27, 29, 33, 42, 43, 44, 47, 49, 74, 75, 81, 97, 110, 120, 123, 124, 133, 138, 139, 144, 154, 158, 163, 172, 173, 174, and/or 177 in PYR1 (SEQ ID NO:1) wherein the mutation is selected from R10Q, E12G, E12K, L25R, P27L, S29N, L33F, P42S, E43G, L44F, S47P, V49I, R74C, V75I, V81M, D97N, I110S, Y120C, Y120H, V123I, T124M, N133D, V138M, V139I, V144A, E154G, M158I, V163I, A172T, T173A, V174I, A177T or combinations thereof.

In some instances, the expression cassette comprises a promoter operably linked to a polynucleotide encoding a mutated PYR/PYL receptor polypeptide, wherein the mutated PYR/PYL receptor polypeptide is agonized by fenhexamid when the fenhexamid is contacted to the mutated PYR/PYL receptor polypeptide, and the mutated PYR/PYL receptor polypeptide comprises mutations at amino acids corresponding to positions 59, 120, and 158 in PYR1 (SEQ ID NO:1) wherein the mutations are K59R, Y120H, and M158I. In some instances, the mutated PYR/PYL receptor polypeptide further comprises isoleucine residues at the amino acid positions corresponding to positions 62 and 110 in PYR1 (SEQ ID NO:1).

In some instances, the expression cassette comprises a promoter operably linked to a polynucleotide encoding a mutated PYR/PYL receptor polypeptide, wherein the mutated PYR/PYL receptor polypeptide is agonized by dichlobenil when the dichlobenil is contacted to the mutated PYR/PYL receptor polypeptide, and the amino acid of the mutated PYR/PYL receptor polypeptide corresponding to position K59 of SEQ ID NO:1 is X, wherein X is alanine, cysteine, aspartic acid, glutamic acid, phenylalanine, glycine, histidine, leucine, methionine, glutamine, arginine, serine, threonine, valine, tyrosine, asparagine, or tryptophan, and the mutated PYR/PYL receptor polypeptide further comprises at least one additional mutation at an amino acid corresponding to positions 27 and/or 63 in PYR1 (SEQ ID NO:1) wherein the mutation is selected from P27L, K63N, or combinations thereof.

In some instances, the expression cassette comprises a promoter operably linked to a polynucleotide encoding a mutated PYR/PYL receptor polypeptide, wherein the mutated PYR/PYL receptor polypeptide is agonized by dichlobenil when the dichlobenil is contacted to the mutated PYR/PYL receptor polypeptide, and the mutated PYR/PYL receptor polypeptide comprises at least one mutation at an amino acid corresponding to positions 26, 37, 71, and/or 94 in PYR1 (SEQ ID NO:1) wherein the mutation is selected from D26G, R37Q, F71S, E94D, or combinations thereof.

In some instances, the expression cassette comprises a promoter operably linked to a polynucleotide encoding a mutated PYR/PYL receptor polypeptide, wherein the mutated PYR/PYL receptor polypeptide is agonized by benoxacor when the benoxacor is contacted to the mutated PYR/PYL receptor polypeptide, and the amino acid of the mutated PYR/PYL receptor polypeptide corresponding to position K59 of SEQ ID NO:1 is X, wherein X is alanine, cysteine, aspartic acid, glutamic acid, phenylalanine, glycine, histidine, leucine, methionine, glutamine, arginine, serine, threonine, valine, tyrosine, asparagine, or tryptophan, and the mutated PYR/PYL receptor polypeptide further comprises a mutation at an amino acid corresponding to position 119 in PYR1 (SEQ ID NO:1) wherein the mutation is N1119Y.

In some instances, the expression cassette comprises a promoter operably linked to a polynucleotide encoding a mutated PYR/PYL receptor polypeptide, wherein the mutated PYR/PYL receptor polypeptide is agonized by benoxacor when the benoxacor is contacted to the mutated PYR/PYL receptor polypeptide, and the mutated PYR/PYL receptor polypeptide comprises at least one mutation at an amino acid corresponding to positions 110, 114, and/or 138 in PYR1 (SEQ ID NO:1) wherein the mutation is selected from I110T, E114D, V138M, or combinations thereof.

In some instances, the expression cassette comprises a promoter operably linked to a polynucleotide encoding a mutated PYR/PYL receptor polypeptide, wherein the mutated PYR/PYL receptor polypeptide is agonized by BTH when the BTH is contacted to the mutated PYR/PYL receptor polypeptide, and the amino acid of the mutated PYR/PYL receptor polypeptide corresponding to position K59 of SEQ ID NO:1 is X, wherein X is alanine, cysteine, aspartic acid, glutamic acid, phenylalanine, glycine, histidine, leucine, methionine, glutamine, arginine, serine, threonine, valine, tyrosine, asparagine, or tryptophan, and the mutated PYR/PYL receptor polypeptide further comprises at least one mutation at an amino acid corresponding to positions 24, 82, 159, and/or 161 in PYR1 (SEQ ID NO:1) wherein the mutation is selected from Q24R, I82T, F159L, D161G, or combinations thereof.

In some instances, the expression cassette comprises a promoter operably linked to a polynucleotide encoding a mutated PYR/PYL receptor polypeptide, wherein the mutated PYR/PYL receptor polypeptide is agonized by BTH when the BTH is contacted to the mutated PYR/PYL receptor polypeptide, and the mutated PYR/PYL receptor polypeptide comprises at least one mutation at an amino acid corresponding to positions 115 and/or 159 in PYR1 (SEQ ID NO:1) wherein the mutation is selected from H115Y, F159S, F159L, or combinations thereof.

In some instances, the expression cassette comprises a promoter operably linked to a polynucleotide encoding a mutated PYR/PYL receptor polypeptide, wherein the mutated PYR/PYL receptor polypeptide has the sequence of, or is substantially identical to *(e.g.,* at least 70%, at least 75%, at least 80%, at least 85%, at least 90%, at least 91%, at least 92%, at least 93%, at least 94%, at least 95%, at least 96%, at least 97%, at least 98%, or at least 99% identical to) any of SEQ ID NOs:131-139 (*i.e*., any of SEQ ID NO:131, 132, 133, 134, 135, 136, 137, 138, or 139) and the mutated PYR/PYL receptor polypeptide is agonized by bromoxynil, chloroxynil, or ioxynil when the bromoxynil, chloroxynil, or ioxynil is contacted to the mutated PYR/PYL receptor polypeptide.

In some instances, the expression cassette comprises a promoter operably linked to a polynucleotide encoding a mutated PYR/PYL receptor polypeptide, wherein the mutated PYR/PYL receptor polypeptide has the sequence of, or is substantially identical to *(e.g.,* at least 70%, at least 75%, at least 80%, at least 85%, at least 90%, at least 91%, at least 92%, at least 93%, at least 94%, at least 95%, at least 96%, at least 97%, at least 98%, or at least 99% identical to) any of SEQ ID NOs:124-130 or 165-178 (*i.e*., any of SEQ ID NO:124, 125, 126, 127, 128, 129, 130, 165, 166, 167, 168, 169, 170, 171, 172, 173, 174, 175, 176, 177, or 178) and the mutated PYR/PYL receptor polypeptide is agonized by fenhexamid when the fenhexamid is contacted to the mutated PYR/PYL receptor polypeptide.

In some instances, the expression cassette comprises a promoter operably linked to a polynucleotide encoding a mutated PYR/PYL receptor polypeptide, wherein the mutated PYR/PYL receptor polypeptide has the sequence of, or is substantially identical to *(e.g.,* at least 70%, at least 75%, at least 80%, at least 85%, at least 90%, at least 91%, at least 92%, at least 93%, at least 94%, at least 95%, at least 96%, at least 97%, at least 98%, or at least 99% identical to) any of SEQ ID NOs:140-144 (*i.e*., any of SEQ ID NO:140, 141, 142, 143, or 144) and the mutated PYR/PYL receptor polypeptide is agonized by dichlobenil when the dichlobenil is contacted to the mutated PYR/PYL receptor polypeptide.

In some instances, the expression cassette comprises a promoter operably linked to a polynucleotide encoding a mutated PYR/PYL receptor polypeptide, wherein the mutated PYR/PYL receptor polypeptide has the sequence of, or is substantially identical to *(e.g.,* at least 70%, at least 75%, at least 80%, at least 85%, at least 90%, at least 91%, at least 92%, at least 93%, at least 94%, at least 95%, at least 96%, at least 97%, at least 98%, or at least 99% identical to) any of SEQ ID NOs:145 or 146 and the mutated PYR/PYL receptor polypeptide is agonized by benoxacor when the benoxacor is contacted to the mutated PYR/PYL receptor polypeptide.

In some instances, the expression cassette comprises a promoter operably linked to a polynucleotide encoding a mutated PYR/PYL receptor polypeptide, wherein the mutated PYR/PYL receptor polypeptide has the sequence of, or is substantially identical to *(e.g.,* at least 70%, at least 75%, at least 80%, at least 85%, at least 90%, at least 91%, at least 92%, at least 93%, at least 94%, at least 95%, at least 96%, at least 97%, at least 98%, or at least 99% identical to) any of SEQ ID NOs:147, 148, or 164, and the mutated PYR/PYL receptor polypeptide is agonized by BTH (acibenzolar-s-methyl) when the BTH is contacted to the mutated PYR/PYL receptor polypeptide.

In some instances, the expression cassette comprises a promoter operably linked to a polynucleotide encoding a mutated PYR/PYL receptor polypeptide, wherein the mutated PYR/PYL receptor polypeptide comprises at least one mutation at an amino acid residue comprising the ligand-binding pocket of the PYR/PYL receptor polypeptide.

The present invention also provides for expression vectors comprising an expression cassette of the invention.

The present disclosure further provides for polynucleotide sequences comprising an expression cassette of the invention (or as described herein).

The present disclosure further provides for methods of producing plants having increased stress tolerance. In some instances, the method comprises growing a transgenic plant comprising at least one polynucleotide sequence encoding a mutated PYR/PYL receptor polypeptide that has the sequence of, or is substantially identical to *(e.g.,* at least 70%, at least 75%, at least 80%, at least 85%, at least 90%, at least 91%, at least 92%, at least 93%, at least 94%, at least 95%, at least 96%, at least 97%, at least 98%, or at least 99% identical to) any of SEQ ID NOs:124-148 or 164-178 (*i.e*., any of SEQ ID NO:124, 125, 126, 127, 128, 129, 130, 131, 132, 133, 134, 135, 136, 137, 138, 139, 140, 141, 142, 143, 144, 145, 146, 147, 148, 164, 165, 166, 167, 168, 169, 170, 171, 172, 173, 174, 175, 176, 177, or 178), whereby the transgenic plant expresses the mutated PYR/PYL receptor polypeptide.

In some instances, the method comprises growing a transgenic plant comprising at least one polynucleotide sequence encoding a mutated PYR/PYL receptor polypeptide that has the sequence of, or is substantially identical to *(e.g.,* at least 70%, at least 75%, at least 80%, at least 85%, at least 90%, at least 91%, at least 92%, at least 93%, at least 94%, at least 95%, at least 96%, at least 97%, at least 98%, or at least 99% identical to) any of SEQ ID NOs:131-139, whereby the transgenic plant expresses the mutated PYR/PYL receptor polypeptide and the mutated PYR/PYL receptor polypeptide is agonized by bromoxynil, chloroxynil, or ioxynil when the bromoxynil, chloroxynil, or ioxynil is contacted to the mutated PYR/PYL receptor polypeptide.

In some instances, the method comprises growing a transgenic plant comprising at least one polynucleotide sequence encoding a mutated PYR/PYL receptor polypeptide that has the sequence of, or is substantially identical to *(e.g.,* at least 70%, at least 75%, at least 80%, at least 85%, at least 90%, at least 91%, at least 92%, at least 93%, at least 94%, at least 95%, at least 96%, at least 97%, at least 98%, or at least 99% identical to) any of SEQ ID NOs:124-130 or 165-178, whereby the transgenic plant expresses the mutated PYR/PYL receptor polypeptide and the mutated receptor polypeptide is agonized by fenhexamid when the fenhexamid is contacted to the mutated PYR/PYL receptor polypeptide.

In some instances, the method comprises growing a transgenic plant comprising at least one polynucleotide sequence encoding a mutated PYR/PYL receptor polypeptide that has the sequence of, or is substantially identical to *(e.g.,* at least 70%, at least 75%, at least 80%, at least 85%, at least 90%, at least 91%, at least 92%, at least 93%, at least 94%, at least 95%, at least 96%, at least 97%, at least 98%, or at least 99% identical to) any of SEQ ID NOs:140-144, whereby the transgenic plant expresses the mutated PYR/PYL receptor polypeptide and the mutated PYR/PYL receptor polypeptide is agonized by dichlobenil when the dichlobenil is contacted to the mutated PYR/PYL receptor polypeptide.

In some instances, the method comprises growing a transgenic plant comprising at least one polynucleotide sequence encoding a mutated PYR/PYL receptor polypeptide that has the sequence of, or is substantially identical to *(e.g.,* at least 70%, at least 75%, at least 80%, at least 85%, at least 90%, at least 91%, at least 92%, at least 93%, at least 94%, at least 95%, at least 96%, at least 97%, at least 98%, or at least 99% identical to) any of SEQ ID NOs:145-146, whereby the transgenic plant expresses the mutated PYR/PYL receptor polypeptide and the mutated PYR/PYL receptor polypeptide is agonized by benoxacor when the benoxacor is contacted to the mutated PYR/PYL receptor polypeptide.

In some instances, the method comprises growing a transgenic plant comprising at least one polynucleotide sequence encoding a mutated PYR/PYL receptor polypeptide that has the sequence of, or is substantially identical to *(e.g.,* at least 70%, at least 75%, at least 80%, at least 85%, at least 90%, at least 91%, at least 92%, at least 93%, at least 94%, at least 95%, at least 96%, at least 97%, at least 98%, or at least 99% identical to) any of SEQ ID NOs:147-148 or 164, whereby the transgenic plant expresses the mutated PYR/PYL receptor polypeptide and the mutated PYR/PYL receptor polypeptide is agonized by BTH (acibenzolar-s-methyl) when the BTH is contacted to the mutated PYR/PYL receptor polypeptide.

In some instances, the method comprises growing a transgenic plant comprising at least one polynucleotide sequence encoding a mutated PYR/PYL receptor polypeptide that is substantially identical to *(e.g.,* at least 70%, at least 75%, at least 80%, at least 85%, at least 90%, at least 91%, at least 92%, at least 93%, at least 94%, at least 95%, at least 96%, at least 97%, at least 98%, or at least 99% identical to) any of SEQ ID NOs:1-119 (*i.e*., any of SEQ ID NO:1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, or 119) and comprises an amino acid X at the position corresponding to position K59 of SEQ ID NO:1, wherein X is alanine, cysteine, aspartic acid, glutamic acid, phenylalanine, glycine, histidine, leucine, methionine, glutamine, arginine, serine, threonine, valie, tyrosine, asparagine, or tryptophan; whereby the transgenic plant expresses the mutated PYR/PYL receptor polypeptide. In some embodiments, the mutated PYR/PYL receptor polypeptide further comprises at least one additional mutation at an amino acid corresponding to positions 21, 41, 50, 57, 60, 82, 92, 102, 116, 125, 141, and/or 151 in SEQ ID NO:1 wherein the mutation is selected from H21Y, P41L, R50G, T57A, H60R, I82N, S92T, E102G, R116K, T125A, E141Q, E141D, N151D, or combinations thereof. In some instances, the mutated PYR/PYL receptor polypeptide further comprises at least one additional mutation at an amino acid corresponding to positions 10, 12, 25, 27, 29, 33, 42, 43, 44, 47, 49, 74, 75, 81, 97, 110, 120, 123, 124, 133, 138, 139, 144, 154, 158, 163, 172, 173, 174, and/or 177 in SEQ ID NO:1 wherein the mutation is selected from R10Q, E12G, E12K, L25R, P27L, S29N, L33F, P42S, E43G, L44F, S47P, V49I, R74C, V75I, V81M, D97N, I110S, Y120C, Y120H, V123I, T124M, N133D, V138M, V139I, V144A, E154G, M158I, V163I, A172T, T173A, V174I, A177T or combinations thereof. In some instances, the mutated PYR/PYL receptor polypeptide further comprises at least one additional mutation at an amino acid corresponding to positions 27 and/or 63 in SEQ ID NO:1 wherein the mutation is selected from P27L, K63N, or combinations thereof. In some instances, the mutated PYR/PYL receptor polypeptide further comprises a mutation at an amino acid corresponding to position 119 in SEQ ID NO:1 wherein the mutation is N1119Y. In some instances, the mutated PYR/PYL receptor polypeptide further comprises at least one mutation at an amino acid corresponding to positions 24, 82, 159, and/or 161 in SEQ ID NO:1 wherein the mutation is selected from Q24R, I82T, F159L, D161G, or combinations thereof.

In some instances, the method comprises growing a transgenic plant comprising at least one polynucleotide sequence encoding a mutated PYR/PYL receptor polypeptide that is substantially identical to *(e.g.,* at least 70%, at least 75%, at least 80%, at least 85%, at least 90%, at least 91%, at least 92%, at least 93%, at least 94%, at least 95%, at least 96%, at least 97%, at least 98%, or at least 99% identical to) any of SEQ ID NOs:1-119 and comprises at least one mutation at an amino acid corresponding to positions 26, 37, 71, and/or 94 in SEQ ID NO:1 wherein the mutation is selected from R37Q, F71S, E94D, or combinations thereof; whereby the transgenic plant expresses the mutated PYR/PYL receptor polypeptide.

In some instances, the method comprises growing a transgenic plant comprising at least one nucleotide sequence encoding a mutated PYR/PYL receptor polypeptide that is substantially identical to *(e.g.,* at least 70%, at least 75%, at least 80%, at least 85%, at least 90%, at least 91%, at least 92%, at least 93%, at least 94%, at least 95%, at least 96%, at least 97%, at least 98%, or at least 99% identical to) any of SEQ ID NOs:1-119 and comprises at least one mutation at an amino acid corresponding to positions 110, 114, and/or 138 in SEQ ID NO:1 wherein the mutation is selected from I110T, E114D, V138M, or combinations thereof; whereby the transgenic plant expresses the mutated PYR/PYL receptor polypeptide.

In some instances, the method comprises growing a transgenic plant comprising at least one polynucleotide sequence encoding a mutated PYR/PYL receptor polypeptide that is substantially identical to *(e.g.,* at least 70%, at least 75%, at least 80%, at least 85%, at least 90%, at least 91%, at least 92%, at least 93%, at least 94%, at least 95%, at least 96%, at least 97%, at least 98%, or at least 99% identical to) any of SEQ ID NOs:1-119 and the mutated PYR/PYL receptor polypeptide comprises at least one mutation at an amino acid corresponding to positions 115 and/or 159 in SEQ ID NO:1 wherein the mutation is selected from H115Y, F159S, F159L, or combinations thereof; whereby the transgenic plant expresses the mutated PYR/PYL receptor polypeptide.

In some instances, the method comprises growing a transgenic plant comprising at least one polynucleotide sequence encoding a mutated PYR/PYL receptor polypeptide that is substantially identical to *(e.g.,* at least 70%, at least 75%, at least 80%, at least 85%, at least 90%, at least 91%, at least 92%, at least 93%, at least 94%, at least 95%, at least 96%, at least 97%, at least 98%, or at least 99% identical to) any of SEQ ID NOs:1-119 and the mutated PYR/PYL receptor polypeptide comprises mutations at amino acid corresponding to positions 59, 120, and 158 in SEQ ID NO:1 wherein the mutations are K59R, Y120H, and M158I; whereby the transgenic plant expresses the mutated PYR/PYL receptor polypeptide. In some instances, the mutated PYR/PYL receptor polypeptide further comprises isoleucine residues at the amino acid positions corresponding to positions 62 and 110 in SEQ ID NO:1.

The invention specifically provides a method of producing a plant having increased stress tolerance, the method comprising growing a transgenic plant comprising at least one polynucleotide sequence encoding a mutated PYR/PYL receptor polypeptide that:
(i) has at least 70% sequence identity to any of SEQ ID NOs:124-139, 143, 144, 146, or 164-178 and comprises a mutation at an amino acid residue corresponding to position K59 of PYR1 (SEQ ID NO:1), wherein the amino acid residue is mutated to alanine, cysteine, phenylalanine, glycine, histidine, leucine, methionine, arginine, serine, threonine, tyrosine, valine, asparagine, or tryptophan, and/or
(ii) has at least 70% sequence identity to any of SEQ ID NO:147, 148, 164 and comprises a mutation at an amino acid residue corresponding to position F159, wherein the amino acid residue is mutated to leucine or serine;
wherein the mutated PYR/PYL receptor polypeptide is agonized by a chemical when the chemical is contacted to the mutated PYR/PYL receptor polypeptide, wherein the chemical does not significantly agonize a wild-type PYR/PYL receptor polypeptide when the chemical is contacted to the wild-type PYR/PYL receptor polypeptide, whereby the transgenic plant expresses the mutated PYR/PYL receptor polypeptide.

### BRIEF DESCRIPTION OF THE DRAWINGS

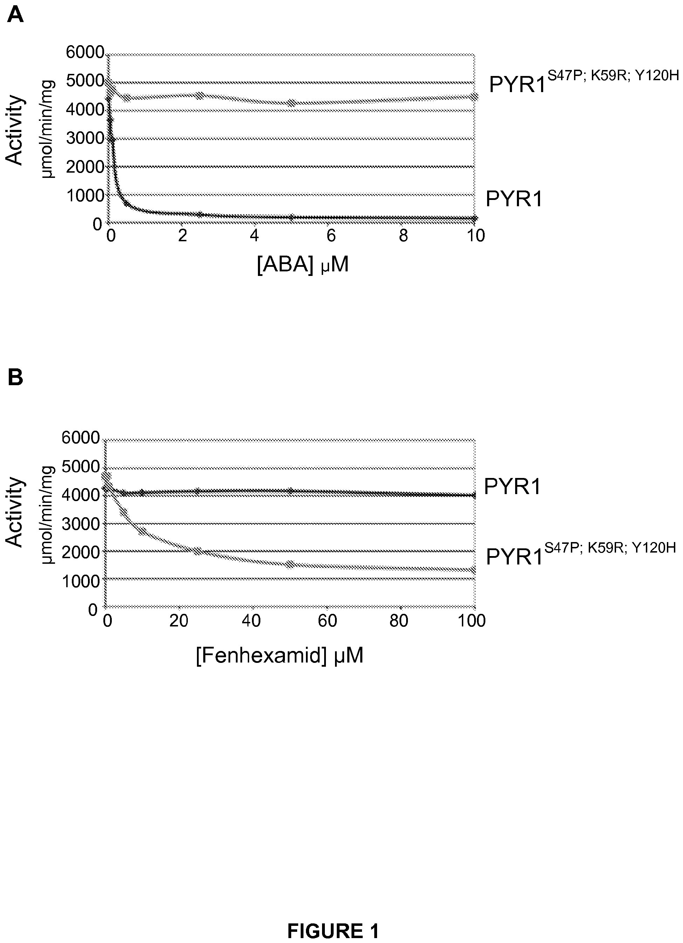

**Figure 1****. PYR1 mutant 27-18 confers responsiveness to fenhexamid but not ABA.** Representative PYR1 mutant 27-18 (comprising S47P, K59R, and Y120H substitution mutations) was tested for responsiveness to abscisic acid ("ABA") (A) and fenhexamid (B) at increasing concentrations as compared to a wild-type PYR1 control using an *in vitro* PP2C phosphatase activity assay.

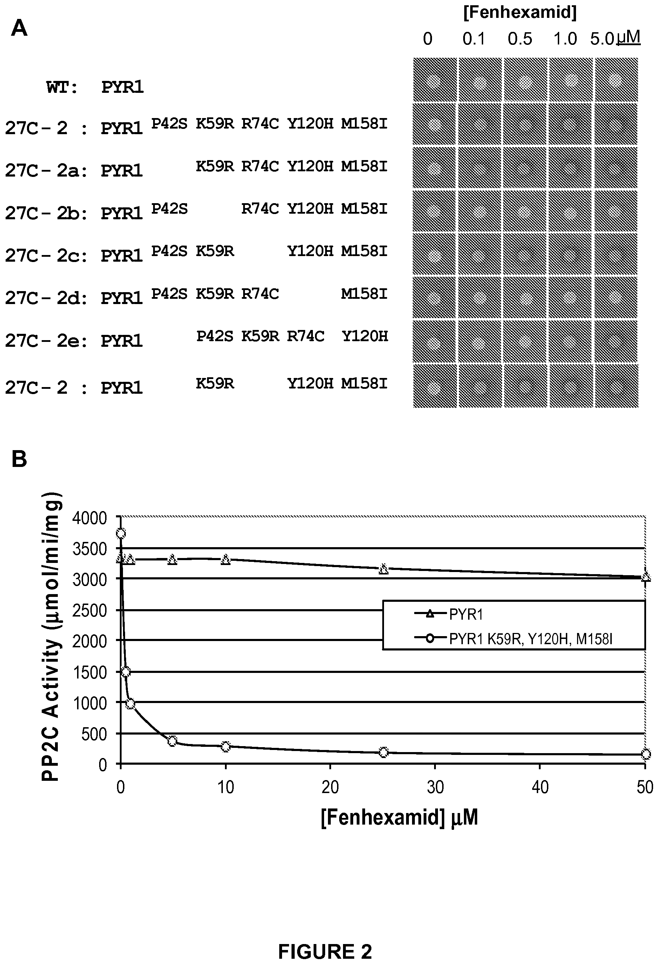

**Figure 2****. Identification of residues essential for fenhexamid responsiveness in the 27C-2 mutant.** A. The mutants (listed on left side of panel) were constructed in pBD-PYR1 and transformed into Y190 pAD-HAB1 yeast cells. The transformants were then grown on increasing concentrations of fenhexamid and scored for responsiveness using X-gal staining, performed as described in Park *et al*., 2009. As shown, the mutations K59R, Y120H, and M158I are necessary for fenhexamid response by 27C-2. B. *In vitro* characterization of the PYR1 K59R, Y120H, M158I mutant. Recombinant wild-type and mutant PYR1 proteins were produced as described previously and used in PP2C assays, as described previously (Park *et al*., 2009), with the exception that a 2:1 ratio of receptor to phosphatase was utilized.

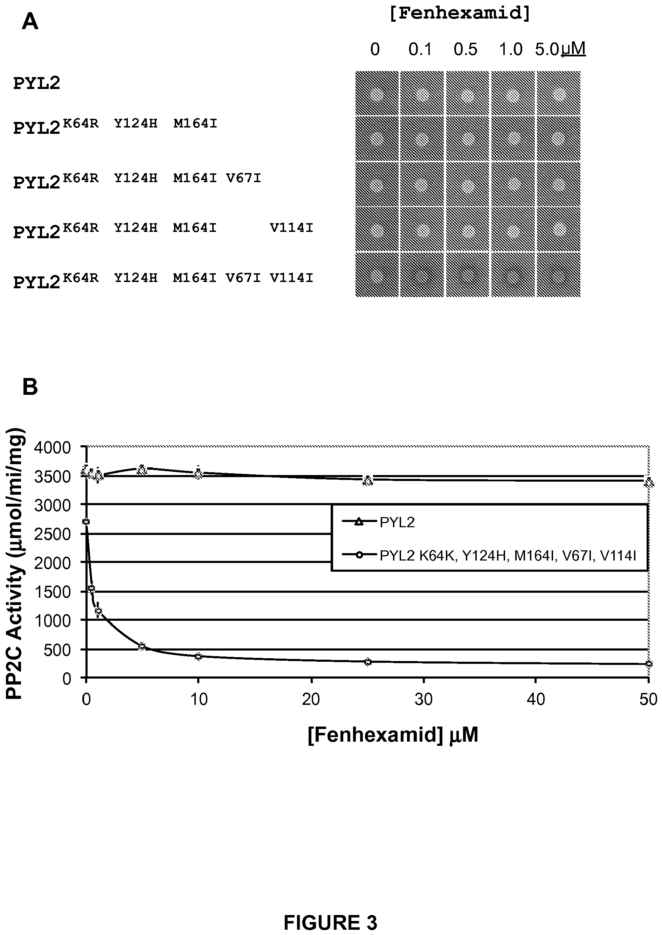

**Figure 3****. Engineering of fenhexamid responsiveness into PYL2**. A. Mutations homologous to those sufficient for fenhexamid responsiveness in PYR1 (K59R, Y120H, M158I) were introduced into PYL2 (K64R, Y124H, M164I). In addition, pocket residue mutations at V114I and/or V67I were introduced. The mutants (listed on left side of panel) were constructed in pBD-PYL2 and transformed into Y190 pAD-HAB1 yeast cells. The transformants were then grown on increasing concentrations of fenhexamid and scored for responsiveness using X-gal staining, performed as described in Park *et al*., 2009. As shown, fenhexamid responsiveness can be engineered by the combination of these mutations. B. *In vitro* characterization of PYL2 K64R, Y124H, M164I, V67I, V114I mutant. Recombinant wild-type and mutant PYL2 proteins were produced as described previously and used in PP2C assays, as described previously (Park *et al*., 2009), with the exception that a 2:1 ratio of receptor for phosphatase was used.

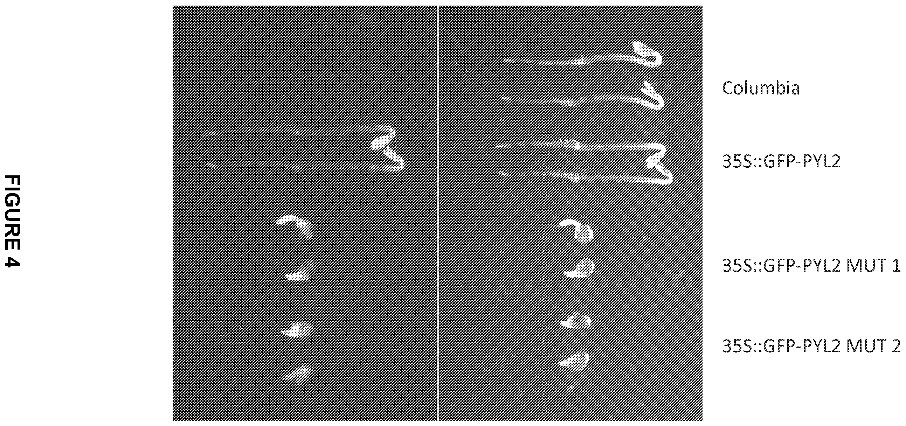

**Figure 4****. Characterization of seedling responses to fenhexamid in 35S::GFP-PYL2 K64R, Y124H, M164I, V67I, V114I transgenic plants**. To investigate the sensitivity of the fenhexamid responsive PYL2 receptor *in vivo,* transgenic plants expressing the wild-type and mutant receptor proteins were made by agrobacterium mediated transformation. Shown are segregating GFP-expressing T2 seedlings from primary transgenic plants (T1). Columbia is the wild-type background and is non-transgenic. Two independent PYL2 K64R, Y124H, M164I, V67I, V114I ("PYL2 MUT") transgenic lines were characterized in addition to a wild-type PYL2 overexpression line. Seeds from respective lines were germinated in the dark on media containing 100 µM fenhexamid and scored two days post-imbibition. As shown, the two transgenic lines show strong post-germination arrest while the control lines do not. The top panel shows transmitted light images of representative seedlings and the bottom panel shows epi-fluorescence confirming GFP expression of lines and showing that the expression levels between lines is similar. The data show that the PYL2 K64R, Y124H, M164I, V67I, V114I receptor protein enables activation of a well-characterized ABA response (growth inhibition) in response to fenhexamid.

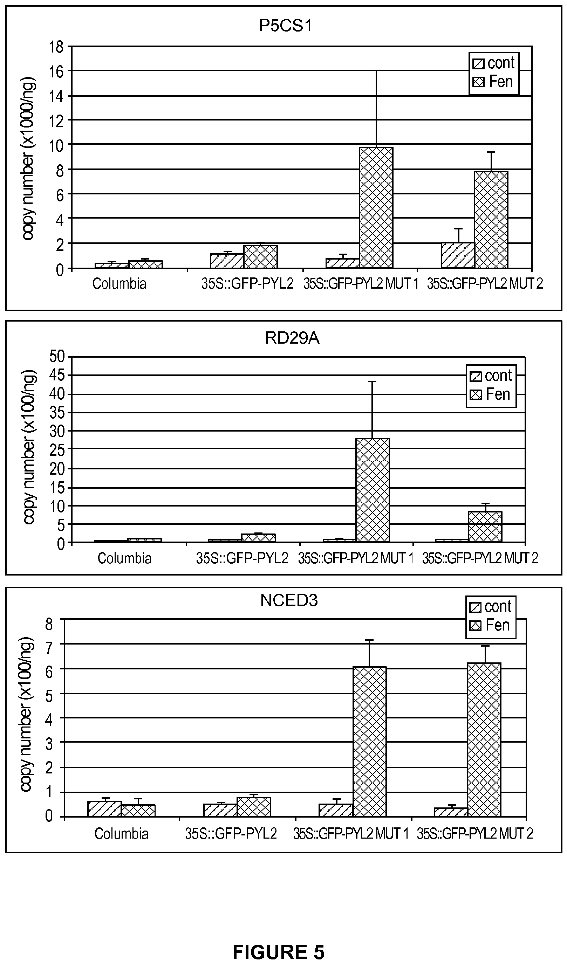

**Figure 5****. Characterization of seedling transcriptional responses to fenhexamid in 35S::GFP-PYL2 K64R, Y124H, M164I, V67I, V114I transgenic plants.** GFP-expressing T2 seedlings from respective lines were isolated after germination on petri plates and pre-screened by epi-fluorescence microscopy and subsequently grown in liquid media culture for 7 days, after which seedlings were treated with either 100 µM fenhexamid or control (0.1% DMSO) for 6 hours. RNA was subsequently isolated from the samples and utilized in quantitative RT-PCR experiments using three well-characterized ABA responsive transcripts (as described previously, Park *et al*., 2009). The primer sequences used for RT-PCR analysis were obtained from Roche and synthesized by Invitrogen. Quantitative RT-PCR was performed using SybrGreen on a BioRad CFX 96 real-time PCR instrument. mRNA copy number is expressed as copy number per nanogram total RNA. Data was normalized using an ACT2 gene. Copy number was established using a standard curve of known concentration of the target genes. The data obtained show that the PYL2 K64R, Y124H, M164I, V67I, V114I receptor protein enables activation of the ABA signaling pathway in response to fenhexamid. Data shown are the average of triplicate technical replicates and error bars show standard deviation.

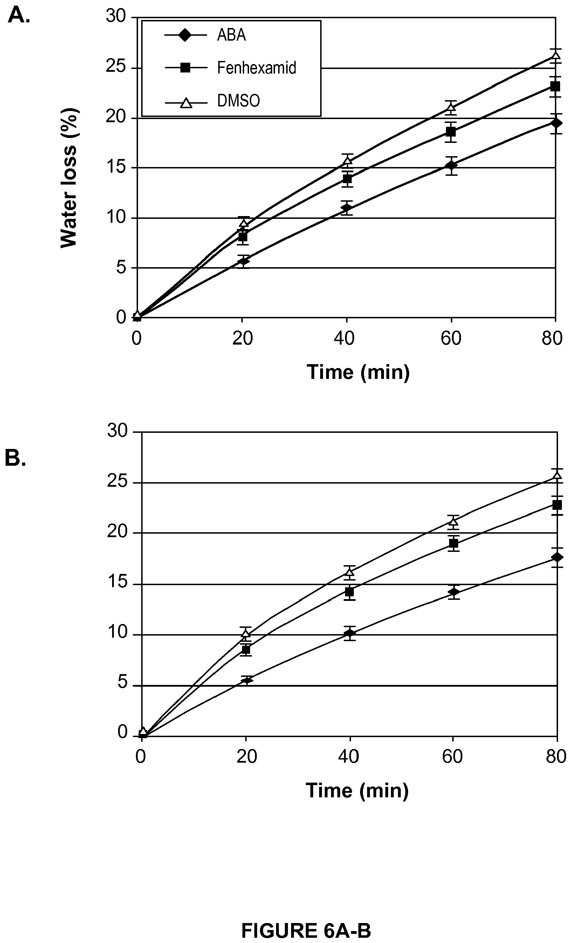

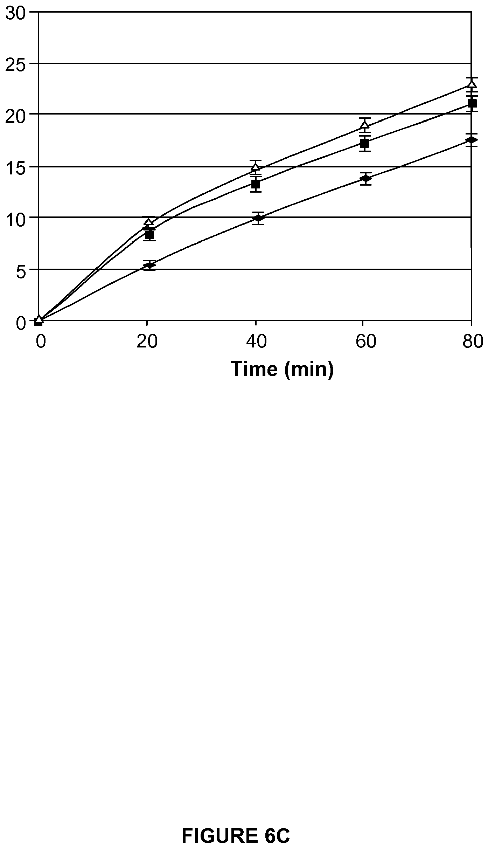

**Figure 6****. Fenhexamid reduces water loss in transgenic PYL2^{K64R, Y124H, M164I, V67I, V114I} expressing plants.** Shown are three independent water loss experiments (A-C) conducted on transgenic plants treated with control, 100 µM fenhexamid, or 100 µM ABA containing solutions. Plants were treated as described in the Examples section below and water loss from aerial rosettes was measured after detachment. In all experiments, the transgenic PYL2 line displayed reduced water loss in response to fenhexamid treatment.

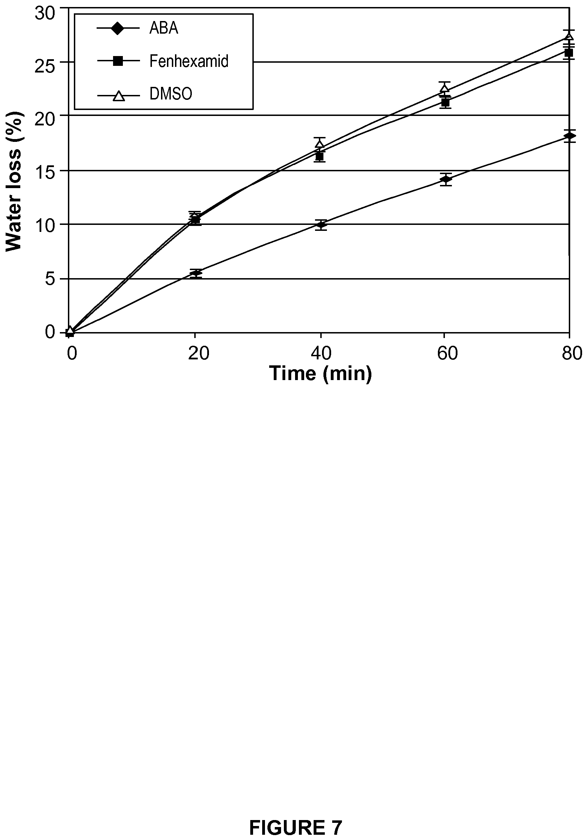

**Figure 7****. Fenhexamid pre-treatment does not reduce water loss in wild-type Columbia plants**. Columbia plants were treated with control, 100 µM fenhexamid, or 100 µM ABA containing solutions. Plants were treated as described in the Examples section below and water loss from aerial rosettes was measured after detachment.

### DEFINITIONS

The term "PYR/PYL receptor polypeptide" refers to a protein characterized in part by the presence of one or more or all of a polyketide cyclase domain 2 (PF10604), a polyketide cyclase domain 1 (PF03364), and a Bet V I domain (PF03364), which in wild-type form mediates abscisic acid (ABA) and ABA analog signaling. A wide variety of PYR/PYL receptor polypeptide sequences are known in the art. In some instances, a PYR/PYL receptor polypeptide comprises a polypeptide that is substantially identical to PYR1 (SEQ ID NO:1), PYL1 (SEQ ID NO:2), PYL2 (SEQ ID NO:3), PYL3 (SEQ ID NO:4), PYL4 (SEQ ID NO:5), PYL5 (SEQ ID NO:6), PYL6 (SEQ ID NO:7), PYL7 (SEQ ID NO:8), PYL8 (SEQ ID NO:9), PYL9 (SEQ ID NO:10), PYL10 (SEQ ID NO:11), PYL211 (SEQ ID NO:12), PYL12 (SEQ ID NO:13), or PYL13 (SEQ ID NO:14), or to any of SEQ ID NOs:15-119.

A "wild-type PYR/PYL receptor polypeptide" refers to a naturally occurring PYR/PYL receptor polypeptide that mediates abscisic acid (ABA) and ABA analog signaling.

A "mutated PYR/PYL receptor polypeptide" or "modified PYR/PYL receptor polypeptide" refers to a PYR/PYL receptor polypeptide that is a variant from a naturally-occurring (*i.e*., wild-type) PYR/PYL receptor polypeptide. As used herein, a mutated or modified PYR/PYL receptor polypeptide comprises one or more amino acid substitutions relative to a corresponding wild-type PYR/PYL receptor polypeptide. In this context, a "mutated" polypeptide or "modified" polypeptide can be generated by any method for generating non-wild type nucleotide sequences. A mutated PYR/PYL receptor polypeptide may or may not mediate abscisic acid (ABA) and ABA analog signaling.

An amino acid "corresponding to position [X] of [specific sequence]" refers to an amino acid in a polypeptide of interest that aligns with the equivalent amino acid of a specified sequence. Generally, as described herein, the amino acid corresponding to a position of a PYR/PYL receptor polypeptide can be determined using an alignment algorithm such as BLAST. Typically, "correspondence" of amino acid positions is determined by aligning to a region of the PYR/PYL receptor polypeptide comprising SEQ ID NO:1, as discussed further herein. When a PYR/PYL receptor polypeptide sequence differs from SEQ ID NO:1 (*e.g.,* by changes in amino acids or addition or deletion of amino acids), it may be that a particular mutation associated with agonization by a chemical that does not agonize wild-type PYR/PYL will not be in the same position number as it is in SEQ ID NO:1. For example, amino acid position K86 of PYL1 (SEQ ID NO:2) aligns with amino acid position K59 in SEQ ID NO:1, as can be readily illustrated in an alignment of the two sequences. In this example, a mutation at amino acid position 86 in SEQ ID NO:2 corresponds to position 59 in SEQ ID NO:1.

Two nucleic acid sequences or polypeptides are said to be "identical" if the sequence of nucleotides or amino acid residues, respectively, in the two sequences is the same when aligned for maximum correspondence as described below. The terms "identical" or percent "identity," in the context of two or more nucleic acids or polypeptide sequences, refer to two or more sequences or subsequences that are the same or have a specified percentage of amino acid residues or nucleotides that are the same, when compared and aligned for maximum correspondence over a comparison window, as measured using one of the following sequence comparison algorithms or by manual alignment and visual inspection. When percentage of sequence identity is used in reference to proteins or peptides, it is recognized that residue positions that are not identical often differ by conservative amino acid substitutions, where amino acids residues are substituted for other amino acid residues with similar chemical properties (*e.g*., charge or hydrophobicity) and therefore do not change the functional properties of the molecule. Where sequences differ in conservative substitutions, the percent sequence identity may be adjusted upwards to correct for the conservative nature of the substitution. Means for making this adjustment are well known to those of skill in the art. Typically this involves scoring a conservative substitution as a partial rather than a full mismatch, thereby increasing the percentage sequence identity. Thus, for example, where an identical amino acid is given a score of 1 and a non-conservative substitution is given a score of zero, a conservative substitution is given a score between zero and 1. The scoring of conservative substitutions is calculated according to, *e.g.,* the algorithm of Meyers & Miller, Computer Applic. Biol. Sci. 4:11-17 (1988) *e.g.,* as implemented in the program PC/GENE (Intelligenetics, Mountain View, California, USA).

The phrase "substantially identical," used in the context of two nucleic acids or polypeptides, refers to a sequence that has at least 60% sequence identity with a reference sequence. Alternatively, percent identity can be any integer from 60% to 100%. Some embodiments include at least: 60%, 65%, 70%, 75%, 80%, 85%, 90%, 91%, 92%, 93%, 94%, 95%, 96%, 97%, 98%, or 99%, compared to a reference sequence using the programs described herein; preferably BLAST using standard parameters, as described below. The present disclosure provides for nucleic acids encoding polypeptides that are substantially identical to any of SEQ ID NO:1-119.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters.

A "comparison window", as used herein, includes reference to a segment of any one of the number of contiguous positions selected from the group consisting of from 20 to 600, usually about 50 to about 200, more usually about 100 to about 150 in which a sequence may be compared to a reference sequence of the same number of contiguous positions after the two sequences are optimally aligned. Methods of alignment of sequences for comparison are well-known in the art. Optimal alignment of sequences for comparison can be conducted, e.g., by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Nat'l. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection.

Algorithms that are suitable for determining percent sequence identity and sequence similarity are the BLAST and BLAST 2.0 algorithms, which are described in Altschul et al. (1990) J. Mol. Biol. 215: 403-410 and Altschul et al. (1977) Nucleic Acids Res. 25: 3389-3402, respectively. Software for performing BLAST analyses is publicly available through the National Center for Biotechnology Information (NCBI) web site. The algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul *et al, supra*). These initial neighborhood word hits acts as seeds for initiating searches to find longer HSPs containing them. The word hits are then extended in both directions along each sequence for as far as the cumulative alignment score can be increased. Cumulative scores are calculated using, for nucleotide sequences, the parameters M (reward score for a pair of matching residues; always >0) and N (penalty score for mismatching residues; always <0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a word size (W) of 28, an expectation (E) of 10, M=1, N=-2, and a comparison of both strands. For amino acid sequences, the BLASTP program uses as defaults a word size (W) of 3, an expectation (E) of 10, and the BLOSUM62 scoring matrix *(see* Henikoff & Henikoff, Proc. Natl. Acad. Sci. USA 89:10915 (1989)).

The BLAST algorithm also performs a statistical analysis of the similarity between two sequences (*see, e.g.,* Karlin & Altschul, Proc. Nat'l. Acad. Sci. USA 90:5873-5787 (1993)). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.01, more preferably less than about 10⁻⁵, and most preferably less than about 10⁻²⁰.

"Conservatively modified variants" applies to both amino acid and nucleic acid sequences. With respect to particular nucleic acid sequences, conservatively modified variants refers to those nucleic acids which encode identical or essentially identical amino acid sequences, or where the nucleic acid does not encode an amino acid sequence, to essentially identical sequences. Because of the degeneracy of the genetic code, a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations," which are one species of conservatively modified variations. Every nucleic acid sequence herein which encodes a polypeptide also describes every possible silent variation of the nucleic acid. One of skill will recognize that each codon in a nucleic acid (except AUG, which is ordinarily the only codon for methionine) can be modified to yield a functionally identical molecule. Accordingly, each silent variation of a nucleic acid which encodes a polypeptide is implicit in each described sequence.

As to amino acid sequences, one of skill will recognize that individual substitutions, in a nucleic acid, peptide, polypeptide, or protein sequence which alters a single amino acid or a small percentage of amino acids in the encoded sequence is a "conservatively modified variant" where the alteration results in the substitution of an amino acid with a chemically similar amino acid. Conservative substitution tables providing functionally similar amino acids are well known in the art.

The following six groups each contain amino acids that are conservative substitutions for one another:
1) Alanine (A), Serine (S), Threonine (T);
2) Aspartic acid (D), Glutamic acid (E);
3) Asparagine (N), Glutamine (Q);
4) Arginine (R), Lysine (K);
5) Isoleucine (I), Leucine (L), Methionine (M), Valine (V); and
6) Phenylalanine (F), Tyrosine (Y), Tryptophan (W).
*(see, e.g.,* Creighton, Proteins (1984)).

It is contemplated that a substitution mutation in a mutated PYR/PYL receptor polypeptide includes not only those specific amino acid substitutions called out in the specification, *e.g*. in the Examples section or in any of the Figures or Tables of the specification, but also includes amino acids that are conservative substitutions for those specific amino acids, so long as the conservatively substituted amino acid is not the wild-type amino acid. As a non-limiting example, where a mutated PYR/PYL receptor polypeptide comprises a serine-to-threonine substitution, it is contemplated that the mutated PYR/PYL receptor polypeptide may alternatively comprise a serine-to-alanine substitution, as threonine and alanine are conservative substitutions for one another; but the mutated PYR/PYL receptor polypeptide would not comprise a serine-to-serine substitution, as serine is the amino acid that is present in the wild-type PYR/PYL polypeptide.

As used herein, the term "agonist" or "agonists" refers to a molecule identified using *in vitro* and *in vivo* assays for activity of a described target protein as described elsewhere herein. Agonists are agents that, e.g., induce or activate the expression of a described target protein or bind to, stimulate, increase, open, activate, facilitate, enhance activation, sensitize or up-regulate the activity of described target protein (or encoding polynucleotide). Agonists include naturally occurring and synthetic molecules. In some embodiments, the agonists are agrichemicals, *e.g*., fungicides, herbicides, pesticides, and/or fertilizers. Assays for determining whether an agonist "agonizes" or "does not agonize" a target protein include, *e.g*., contacting putative agonists to purified target protein(s) and then determining the functional effects on the described target protein activity, as described above, or contacting putative agonists to cells expressing the target protein(s) and then determining the functional effects on the described target protein activity, as described above. One of skill in the art will be able to determine whether an assay is suitable for determining whether an agonist agonizes or does not agonize a target protein. Samples or assays comprising described target protein that are treated with a putative agonist are compared to control samples without the agonist to examine the extent of effect. Control samples (untreated with agonists) are assigned a relative activity value of 100%. Agonism of the described target protein is achieved when the activity value relative to the control is 110%, optionally 150%, optionally 200%, 300%, 400%, 500%, or 1000-3000% or more higher.

As used herein, the term "orthogonal receptor" refers to a receptor that has been modified to selectively recognize new ligands ("orthogonal ligands"). As used herein, the term "orthogonal ligand" refers to an agent that agonizes a mutated or modified PYR/PYL receptor polypeptide but which does not agonize a wild-type PYR/PYL receptor polypeptide. In some embodiments, the orthogonal ligands are agrichemicals, *e.g*., fungicides, herbicides, pesticides, nematicides, plant activators, synergists, herbicide safeners, plant growth regulators, insect repellants, and/or fertilizers.

The term "plant" includes whole plants, shoot vegetative organs and/or structures *(e.g.,* leaves, stems and tubers), roots, flowers and floral organs *(e.g.,* bracts, sepals, petals, stamens, carpels, anthers), ovules (including egg and central cells), seed (including zygote, embryo, endosperm, and seed coat), fruit *(e.g.,* the mature ovary), seedlings, plant tissue *(e.g.,* vascular tissue, ground tissue, and the like), cells *(e.g.,* guard cells, egg cells, trichomes and the like), and progeny of same. The class of plants that can be used in the method of the invention is generally as broad as the class of higher and lower plants amenable to transformation techniques, including angiosperms (monocotyledonous and dicotyledonous plants), gymnosperms, ferns, and multicellular algae. It includes plants of a variety of ploidy levels, including aneuploid, polyploid, diploid, haploid, and hemizygous.

The term "promoter," as used herein, refers to a polynucleotide sequence capable of driving transcription of a coding sequence in a cell. Thus, promoters used in the polynucleotide constructs of the invention include cis-acting transcriptional control elements and regulatory sequences that are involved in regulating or modulating the timing and/or rate of transcription of a gene. For example, a promoter can be a cis-acting transcriptional control element, including an enhancer, a promoter, a transcription terminator, an origin of replication, a chromosomal integration sequence, 5' and 3' untranslated regions, or an intronic sequence, which are involved in transcriptional regulation. These cis-acting sequences typically interact with proteins or other biomolecules to carry out (turn on/off, regulate, modulate, *etc*.) gene transcription. A "plant promoter" is a promoter capable of initiating transcription in plant cells. A "constitutive promoter" is one that is capable of initiating transcription in nearly all tissue types, whereas a "tissue-specific promoter" initiates transcription only in one or a few particular tissue types.

A polynucleotide sequence is "heterologous" to an organism or a second polynucleotide sequence if it originates from a foreign species, or, if from the same species, is modified from its original form. For example, when a promoter is said to be operably linked to a heterologous coding sequence, it means that the coding sequence is derived from one species whereas the promoter sequence is derived another, different species; or, if both are derived from the same species, the coding sequence is not naturally associated with the promoter (*e.g*., is a genetically engineered coding sequence, e.g., from a different gene in the same species, or an allele from a different ecotype or variety).

An "expression cassette" refers to a nucleic acid construct that, when introduced into a host cell, results in transcription and/or translation of an RNA or polypeptide, respectively. Antisense or sense constructs that are not or cannot be translated are expressly included by this definition. In the case of both expression of transgenes and suppression of endogenous genes *(e.g.,* by antisense, or sense suppression) one of skill will recognize that the inserted polynucleotide sequence need not be identical, but may be only substantially identical to a sequence of the gene from which it was derived. As explained herein, these substantially identical variants are specifically covered by reference to a specific nucleic acid sequence.

As used herein, the terms "abiotic stress," "stress," or "stress condition" refer to the exposure of a plant, plant cell, or the like, to a non-living ("abiotic") physical or chemical agent that has an adverse effect on metabolism, growth, development, propagation, or survival of the plant (collectively, "growth"). A stress can be imposed on a plant due, for example, to an environmental factor such as water (*e.g*., flooding, drought, or dehydration), anaerobic conditions (*e.g*., a lower level of oxygen or high level of CO₂), abnormal osmotic conditions, salinity, or temperature (*e.g*., hot/heat, cold, freezing, or frost), a deficiency of nutrients or exposure to pollutants, or by a hormone, second messenger, or other molecule. Anaerobic stress, for example, is due to a reduction in oxygen levels (hypoxia or anoxia) sufficient to produce a stress response. A flooding stress can be due to prolonged or transient immersion of a plant, plant part, tissue, or isolated cell in a liquid medium such as occurs during monsoon, wet season, flash flooding, or excessive irrigation of plants, or the like. A cold stress or heat stress can occur due to a decrease or increase, respectively, in the temperature from the optimum range of growth temperatures for a particular plant species. Such optimum growth temperature ranges are readily determined or known to those skilled in the art. Dehydration stress can be induced by the loss of water, reduced turgor, or reduced water content of a cell, tissue, organ or whole plant. Drought stress can be induced by or associated with the deprivation of water or reduced supply of water to a cell, tissue, organ or organism. Salinity-induced stress (salt-stress) can be associated with or induced by a perturbation in the osmotic potential of the intracellular or extracellular environment of a cell. As used herein, the term "abiotic stress tolerance" or "stress tolerance" refers to a plant's increased resistance or tolerance to abiotic stress as compared to plants under normal conditions and the ability to perform in a relatively superior manner when under abiotic stress conditions. As used herein, the terms "drought resistance" and "drought tolerance" are used to refer to a plant's increased resistance or tolerance to stress induced by a reduction in water availability, as compared to normal circumstances, and the ability of the plant to function and survive in lower-water environments, and perform in a relatively superior manner.

### DETAILED DESCRIPTION

### I. Introduction

Surprisingly, proteins belonging to a family of abscisic acid (ABA) receptors, the PYR/PYL receptor family, can be mutated to bind and respond to chemicals other than ABA. It was found that certain agrochemicals, when contacted to wild-type PYR/PYL receptor polypeptides in the presence of ABA, lowered the level of PYR/PYL receptor activation by ABA. An additional surprising revelation was the discovery that the amino acid corresponding to residue K59 of PYR1, a conserved residue of the PYR/PYL ligand-binding pocket that contacts ABA in wild-type PYR/PYL receptors, can be mutated to many variants to enable the creation of orthogonal receptors for multiple orthogonal ligands.

When PYR/PYL receptor polypeptides were subsequently mutagenized and screened to establish whether PYR/PYL receptor binding to these new chemical agonists could be improved, it was unexpectedly discovered that certain mutations in PYR/PYL receptor polypeptides resulted in activation of the mutated PYR/PYL polypeptide by the non-natural ligands (orthogonal ligands). Moreover, in some cases, the mutations restructured the ligand-binding pockets of PYR/PYL and thus simultaneously abolished the ability of the natural ligand (ABA) to activate the mutated PYR/PYL polypeptide.

Thus, it is possible to alter ABA receptors such as PYR/PYL receptor polypeptides so that a compound other than ABA can be used to selectively activate them. Moreover, because the mutated PYR/PYL receptor (orthogonal receptor) can be selectively activated by applying an orthogonal ligand *(e.g.,* as part of a program to improve plant response to water deficit), the problem of "yield drag" can be avoided. Yield drag is traditionally associated with receptor over-expression, in which gene over-expression during normal or optimal growth conditions (*i. e.,* in the absence of drought or other stressors) is associated with slowed growth.

### II. Mutated PYR/PYL Receptor Polypeptides

The present disclosure provides for mutated PYR/PYL receptor polypeptides that are agonized by chemicals that do not agonize wild-type PYR/PYL receptor polypeptides, as well as polynucleotides encoding mutated PYR/PYL receptor polypeptides that are agonized by chemicals that do not agonize wild-type PYR/PYL receptor polypeptides; expression cassettes and expression vectors comprising polynucleotides encoding mutated PYR/PYL receptor polypeptides that are agonized by chemicals that do not agonize wild-type PYR/PYL receptor polypeptides; plants comprising mutated PYR/PYL receptor polypeptides that are agonized by chemicals that do not agonize wild-type PYR/PYL receptor polypeptides; methods of making plants comprising mutated PYR/PYL receptor polypeptides that are agonized by chemicals that do not agonize wild-type PYR/PYL receptor polypeptides; and methods of making mutated PYR/PYL receptor polypeptides.

In some instances, the mutated PYR/PYL receptor polypeptide, but not the wild-type PYR/PYL receptor polypeptide, is agonized by the chemical bromoxynil, chloroxynil, ioxynil, coumatetralyl, dichlobenil, fenhexamid, benoxacor, or BTH (acibenzolar-s-methyl) when the chemical is contacted to the PYR/PYL receptor polypeptide. In another instance, mutated PYR/PYL receptor polypeptides are agonized by chemicals that do not agonize wild-type PYR/PYL receptor polypeptides and also are agonized by ABA, a compound that does agonize wild-type PYR/PYL receptor polypeptides.

A wide variety of wild-type (naturally occurring) PYR/PYL polypeptide sequences are known in the art. Although PYR1 was originally identified as an abscisic acid (ABA) receptor in *Arabidopsis,* in fact PYR1 is a member of a group of at least 14 proteins (PYR/PYL proteins) in the same protein family in *Arabidopsis* that also mediate ABA signaling. This protein family is also present in other plants *(see, e.g.,* SEQUENCE LISTING) and is characterized in part by the presence of one or more or all of a polyketide cyclase domain 2 (PF10604), a polyketide cyclase domain 1 (PF03364), and a Bet V I domain (PF03364). START / Bet v 1 superfamily domain are described in, for example, Radauer, BMC Evol. Biol. 8:286 (2008). In some instances, a wild-type PYR/PYL receptor polypeptide comprises any of SEQ ID NOs:1-119. In some instances, a wild-type PYR/PYL receptor polypeptide is substantially identical to *(e.g.,* at least 70%, 75%, 80%, 85%, 90%, 91%, 92%, 93%, 94% 95%, 96%, 97%, 98%, or 99% identical to) any of SEQ ID NOs:1-119.

Mutated PYR/PYL receptor polypeptides are variants from naturally-occurring (*i. e.,* wild-type) PYR/PYL receptor polypeptides. Variants include, *e.g.,* fusion proteins, deletions, insertions, or mutations that retain activity. In some instances, a mutated PYR/PYL receptor polypeptide is substantially identical to *(e.g.,* at least 70%, 75%, 80%, 85%, 90%, 91%, 92%, 93%, 94% 95%, 96%, 97%, 98%, or 99% identical to) any of SEQ ID NO:1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, or 119 and comprises 1, 2, 3, 4, 5, 6, or more mutations as described herein relative to a corresponding wild-type PYR/PYL receptor polypeptide. In addition, in some instances, the mutated PYR/PYL receptor polypeptide further comprises an amino- and/or carboxyl terminal fusion with a heterologous amino acid sequence.

The inventors have found a number of mutations that affect response to chemicals. Some mutations occur across different chemicals tested and appear to allow in some cases for the modified PYR/PYL receptor protein to function in response to a diverse array of chemicals. This is not always the case; some mutations appear to be selective for one chemical and do not appear to promote activation of the modified PYR/PYL receptor protein by other chemicals. In some cases, a single mutation is sufficient for a modified PYR/PYL receptor protein to be agonized by a chemical agonist. In other cases, multiple mutations allow a modified PYR/PYL receptor protein to be agonized by a chemical agonist. In some cases, the modified PYR/PYL receptor protein contains two, three, four, five, six, or more mutations in order for the protein to be agonized by a chemical agonist.

The modified PYR/PYL receptor proteins may be mutated at any location in the PYR/PYL receptor polypeptide sequence. In some cases, and as discussed herein, the mutation may occur in the ligand-binding pocket of the PYR/PYL polypeptide. However, the mutation need not occur in the ligand-binding pocket of the PYR/PYL polypeptide in order to effectuate activation by an orthogonal ligand. Without being bound to a particular theory, it is hypothesized that mutations outside the ligand-binding pocket that result in activation of a PYR/PYL receptor protein by an orthogonal ligand have an indirect effect on ligand-binding architecture that results in the ability of the receptor to be activated by the orthogonal ligand. It is further hypothesized that specific mutations at the position corresponding to amino acid K59 in PYR1 (SEQ ID NO:1) enable PYR/PYL receptor polypeptides to be more easily activated by orthogonal ligands than wild-type PYR/PYL receptor polypeptides, which is a desirable property for engineering orthogonal receptors.

Any of the mutations described herein can be made in the polypeptides of any of SEQ ID NOs:1-119 or in polypeptides substantially identical to any of SEQ ID NOs:1-119. Alternatively, any of the mutations described above can be made in a polypeptide comprising any of the consensus sequences that identify PYR/PYL proteins, for example as set forth below.

### Consensus sequences

PYR/PYL receptor proteins can be described by reference to sequence alignments that identify conserved amino acid or motifs *(i.e.,* where alteration in sequences may alter protein function) and regions where variation occurs in alignment of sequences *(i.e.,* where variation of sequence is not likely to significantly affect protein activity). SEQ ID NOs:120-123 provide consensus sequences useful for identifying wild-type PYR/PYL receptor polypeptides. The consensus sequences of SEQ ID NOs:120-123 were generated by aligning all 14 members of the *Arabidopsis* PYR/PYL receptor protein family. In the consensus sequences of SEQ ID NOs:120-123, the capitalized letter represents an amino acid residue that is absolutely conserved among all 14 members of the *Arabidopsis* PYR/PYL receptor protein family, while "x" represents an amino acid residue that is not absolutely conserved among all 14 family members and which can be any amino acid. It will be appreciated that when selecting an amino acid to insert at a position marked by an "x" that in some embodiments, the amino acid is selected from those amino acids found at the corresponding position in a wild-type or mutated PYR/PYL protein.

### PYR1 to PYL13

CxSxxxxxxxAPxxxxWxxxxxFxxPxxxxxFxxxC (SEQ ID NO:120)
GxxRxVxxxSxxPAxxSxExLxxxD (SEQ ID NO:121)
GGxHRLxNYxS (SEQ ID NO:122)
ESxxVDxPxGxxxxxTxxFxxxxxxxNLxxL (SEQ ID NO:123)

Consensus sequence CxSxxxxxxxAPxxxxWxxxxxFxxPxxxxxFxxxC (SEQ ID NO:120) comprises the region corresponding to amino acids 30 to 65 of PYR1 (SEQ ID NO:1). Consensus sequence GxxRxVxxxSxxPAxxSxExLxxxD (SEQ ID NO:121) comprises the region corresponding to amino acids 76 to 100 of PYR1 (SEQ ID NO:1). Consensus sequence GGxHRLxNYxS (SEQ ID NO:122) comprises the region corresponding to amino acids 112 to 122 of PYR1 (SEQ ID NO:1). ESxxVDxPxGxxxxxTxxFxxxxxxxNLxxL (SEQ ID NO:123) comprises the region corresponding to amino acids 141 to 171 of PYR1 (SEQ ID NO:1).

In some cases, a PYR/PYL mutation occurs at a residue within a consensus sequence of SEQ ID NOs:120-123. In some cases, the mutation occurs at a residue that is absolutely conserved among all 14 members of the PYR/PYL receptor protein family. In some cases, the mutation occurs at a residue that is not absolutely conserved. As described herein, a substitution mutation at a residue within a consensus sequence is depicted as a bracket in place of an amino acid of the consensus sequence. Where more than one amino acid is enclosed by said bracket, it indicates that any of the amino acids enclosed by said bracket may be substituted for the wild-type amino acid at that residue of the consensus sequence.

Additionally, the modified PYR/PYL receptor protein may comprise more than one mutation within a consensus sequence, or may comprise at least two or more consensus sequences with each consensus sequence having at least one mutation.

K59. In some cases, the modified PYR/PYL receptor comprises the consensus sequence CxSxxxxxxxAPxxxxWxxxxxFxxPxxx[ACDEFGHLMNQRSTVYW]xFxxxC (SEQ ID NO:149).

**Y120.** In some cases, the modified PYR/PYL receptor comprises the consensus sequence GGxHRLxN[HC]xS (SEQ ID NO:150).

**I110.** In some cases, the modified PYR/PYL receptor comprises the consensus sequence [STCAYW]xGGxHRLxNYxS (SEQ ID NO:151).

**P42.** In some cases, the modified PYR/PYL receptor comprises the consensus sequence CxSxxxxxxxAP[ST]xxxWxxxxxFxxPxxxxxFxxxC (SEQ ID NO:152).

**S47.** In some cases, the modified PYR/PYL receptor comprises the consensus sequence CxSxxxxxxxAPxxxxW[PRA]xxxxFxxPxxxxxFxxxC (SEQ ID NO:153).

**K59 and Y120**. In some cases, the modified PYR/PYL receptor comprises the consensus sequences
CxSxxxxxxxAPxxxxWxxxxxFxxPxxx[ACDEFGHLMNQRSTVYW]xFxxxC (SEQ ID NO:149) and GGxHRLxN[HC]Xs (SEQ ID NO:150).

**K59 and I110.** In some cases, the modified PYR/PYL receptor comprises the consensus sequences
CxSxxxxxxxAPxxxxWxxxxxFxxPxxx[ACDEFGHLMNQRSTVYW]xFxxxC (SEQ ID NO:149) and [STCAYW]xGGxHRLxNYxS (SEQ ID NO:151).

**K59 and P42.** In some cases, the modified PYR/PYL receptor comprises the consensus sequence
CxSxxxxxxxAP[ST]xxxWxxxxxFxxPxxx[ACDEFGHLMNQRSTVYW]xFxxxC (SEQ ID NO:154).

**K59 and S47.** In some cases, the modified PYR/PYL receptor comprises the consensus sequence
CxSxxxxxxxAPxxxxW[PRA]xxxxFxxPxxx[ACDEFGHLMNQRSTVYW]xFxxxC (SEQ ID NO:155).

**H60.** In some cases, the modified PYR/PYL receptor comprises the consensus sequence CxSxxxxxxxAPxxxxWxxxxxFxxPxxxx[R]FxxxC (SEQ ID NO:156).

**S92.** In some cases, the modified PYR/PYL receptor comprises the consensus sequence GxxRxVxxxSxxPAxx[T]xExLxxxD (SEQ ID NO:157).

**E140.** In some cases, the modified PYR/PYL receptor comprises the consensus sequence [GQD]SxxVDxPxGNxxxxTxxFxxxxxxxNLxxL (SEQ ID NO:158).

**K59 and H60.** In some cases, the modified PYR/PYL receptor comprises the consensus sequence CxSxxxxxxxAPxxxxWxxxxxFxxPxxx[ACDEFGHLMNQRSTVYW][R]FxxxC (SEQ ID NO:159).

**K59 and S92.** In some cases, the modified PYR/PYL receptor comprises the consensus sequences
CxSxxxxxxxAPxxxxWxxxxxFxxPxxx[ACDEFGHLMNQRSTVYW]xFxxxC (SEQ ID NO:149) and GxxRxVxxxSxxPAxx[T]xExLxxxD (SEQ ID NO:157).

**K59 and E140.** In some cases, the modified PYR/PYL receptor comprises the consensus sequences
CxSxxxxxxxAPxxxxWxxxxxFxxPxxx[ACDEFGHLMNQRSTVYW]xFxxxC (SEQ ID NO:149) and [GQD]SxxVDxPxGNxxxxTxxFxxxxxxxNLxxL (SEQ ID NO:158).

**E94.** In some cases, the modified PYR/PYL receptor comprises the consensus sequence GxxRxVxxxSxxPAxxSx[D]xLxxxD (SEQ ID NO:160).

**K59 and E94.** In some cases, the modified PYR/PYL receptor comprises the consensus sequences
CxSxxxxxxxAPxxxxWxxxxxFxxPxxx[ACDEFGHLMNQRSTVYW]xFxxxC (SEQ ID NO:149) and GxxRxVxxxSxxPAxxSx[D]xLxxxD (SEQ ID NO:160).

**N119.** In some cases, the modified PYR/PYL receptor comprises the consensus sequence GGxHRLx[Y]YxS (SEQ ID NO:161).

**K59 and N119.** In some cases, the modified PYR/PYL receptor comprises the consensus sequences
CxSxxxxxxxAPxxxxWxxxxxFxxPxxx[ACDEFGHLMNQRSTVYW]xFxxxC (SEQ ID NO:149) and GGxHRLx[Y]YxS (SEQ ID NO:161).

**H115.** In some cases, the modified PYR/PYL receptor comprises the consensus sequence GGx[Y]RLxNYxS (SEQ ID NO:162).

**F159.** In some cases, the modified PYR/PYL receptor comprises the consensus sequence ESxxVDxPxGNxxxxTxx[SL]xxxxxxxNLxxL (SEQ ID NO:163).

**K59 and F159.** In some cases, the modified PYR/PYL receptor comprises the consensus sequences
CxSxxxxxxxAPxxxxWxxxxxFxxPxxx[ACDEFGHLMNQRSTVYW]xFxxxC (SEQ ID NO:149) and ESxxVDxPxGNxxxxTxx[SL]xxxxxxxNLxxL (SEQ ID NO:163).

Accordingly, in some instances, the mutated PYR/PYL receptor polypeptides comprise one or more of the above-described consensus sequences (SEQ ID NOs:149-163) or conservative variants thereof. In some instances, the present disclosure provides for polynucleotides encoding one or more mutated PYR/PYL receptor polypeptides comprising one or more of the above-described consensus sequences (SEQ ID NOs:149-163) or conservative variants thereof.

Modified PYR/PYL receptor proteins can alternatively be described by reference to sequence identifiers ("SEQ IDs") for PYR/PYL polypeptides. Modified PYR/PYL receptors may comprise a SEQ ID listed herein and further comprise at least one *(e.g.,* 1, 2, 3, 4, 5, 6, 7, 8, 9, 10, etc.) mutation at an amino acid position (*e.g.,* a substitution, deletion, or insertion mutation) as described herein. Thus, in some instances, a mutated PYR/PYL receptor polypeptide comprises any of SEQ ID NOs:1-119 and further comprises at least one mutation at any amino acid position.

Alternatively, modified PYR/PYL receptors may be substantially identical to a SEQ ID listed herein and further comprise at least one (*e.g.,* 1, 2, 3, 4, 5, 6, 7, 8, 9, 10, etc.) mutation at an amino acid position. In some instances, a mutated PYR/PYL receptor polypeptide is substantially identical to any of SEQ ID NOs:1-119 and further comprises at least one mutation at any amino acid position.

In some instances, the modified PYR/PYL receptor protein has been modified at the residue corresponding to amino acid position 59 in PYR1 (SEQ ID NO:1). A mutation at this residue, also called "K59," disrupts ABA responsiveness in the modified PYR/PYL receptor protein.

In some cases, a K59 mutation is not only sufficient to abolish ABA sensitivity in the modified PYR/PYL receptor protein, it is also sufficient to confer sensitivity to a new chemical agonist ("orthogonal ligand") on a modified PYR/PYL receptor protein. In some cases, the K59 mutation that results in receptor activation by an orthogonal ligand is a substitution of an alanine residue for the wild-type residue, a substitution of a cysteine residue for the wild-type residue, a substitution of an aspartic acid residue for the wild-type residue, a substitution of a glutamic acid residue for the wild-type residue, a substitution of a phenylalanine residue for the wild-type residue, a substitution of a glycine residue for the wild-type residue, a substitution of a histidine residue for the wild-type residue, a substitution of a leucine residue for the wild-type residue, a substitution of a methionine residue for the wild-type residue, a substitution of an asparagine residue for the wild-type residue, a substitution of a glutamine residue for the wild-type residue, a substitution of an arginine residue for the wild-type residue, a substitution of a serine residue for the wild-type residue, a substitution of a threonine residue for the wild-type residue, a substitution of a valine residue for the wild-type residue, a substitution of a tyrosine residue for the wild-type residue, or a substitution of a tryptophan residue for the wild-type residue.

Additionally, the modified PYR/PYL receptor protein may comprise at least one more mutation in addition to the K59 mutation. It was found that numerous chemical agonists activate modified PYR/PYL receptor proteins when the modifications include a K59 mutation and one, two, three, four, or five additional mutations at other residues in the protein, as shown below in Tables 1-5. A non-limiting list of exemplary combinations of mutations, for which K59 is one mutation site, and for which the modified PYR/PYL receptor is not agonized by ABA but is agonized by an orthogonal ligand, includes:
**S47, K59, and Y120**
**K59, Y120, and V144**
**P42, K59, and Y120**
**P42, K59, Y120, and T124**
**P42, K59, Y120, and E154**
**E12, E43, K59, I110, and N133**
**E12, L25, E43, K59, I110, and N133**
**P27, K59, and K63**
**T57 and K59**
**R50, K59, and E141**
**K59, H60, and N151**
**K59, H60, E102, T125, and E141**
**K59 and I82**
**K59 and S92**
**H21, K59, H60, S92, and R116**
**P41, K59, and H60**
**K59 and N119**
**Q24, K59, I82, F159, and D161**
**K59, Y120, and M158**
**P42, K59, Y120, and M158**
**P42, K59, D97,Y120, V163, and A172**
**P42, L44, K59, Y120, V138, and M158**
**P42, K59, Y120, V123, V139, and M158**
**S47**, **V49, K59, Y120, M158, and A177**
**K59, V81, Y120, M158, and V163**
**P27, P42, K59, D97, Y120, M158, and T173**
**P42, K59, R74, Y120, and M158**
**S29, K59, D97, Y120, V163, and A172**
**P42, K59, Y120, V123, V139, M158, and V163**
**K59, V81, Y120, M158, and V163**
**E12, K59, V75, D97, Y120, V163, and A172**
**L33, P42, K59, Y120, V123, and M158**
**P42, K59, Y120, M158, V163, and V174**
**R10, P42, K59, D97, Y120, V163, and A172**

Thus, in some instances, the amino acid of the mutated PYR/PYL receptor polypeptide corresponding to position K59 of SEQ ID NO:1 is X, wherein X is alanine, cysteine, aspartic acid, glutamic acid, phenylalanine, glycine, histidine, leucine, methionine, asparagine, glutamine, arginine, serine, threonine, valine, tyrosine, or tryptophan.

In some instances, the mutated PYR/PYL receptor polypeptide further comprises at least one additional mutation from a naturally-occurring residue to a non-naturally-occurring residue at an amino acid corresponding to positions 10, 12, 25, 27, 29, 33, 42, 43, 44, 47, 49, 74, 75, 81, 97, 110, 120, 123, 124, 133, 138, 139, 144, 154, 158, 163, 172, 173, 174, and/or 177 in PYR1 (SEQ ID NO:1). In some instances, the mutated PYR/PYL receptor polypeptide comprises at least one additional mutation from a naturally-occurring residue at an amino acid corresponding to positions 10, 12, 25, 27, 29, 33, 42, 43, 44, 47, 49, 74, 75, 81, 97, 110, 120, 123, 124, 133, 138, 139, 144, 154, 158, 163, 172, 173, 174, and/or 177 in PYR1 (SEQ ID NO:1) to an alanine residue, a cysteine residue, an aspartic acid residue, a glutamic acid residue, a phenylalanine residue, a glycine residue, a histidine residue, an isoleucine residue, a lysine residue, a leucine residue, a methionine residue, an asparagine residue, a proline residue, a glutamine residue, an arginine residue, a serine residue, a threonine residue, a valine residue, a tryptophan residue, or a tyrosine residue. In some instances, the mutated PYR/PYL receptor polypeptide comprises at least one additional mutation at an amino acid corresponding to positions 10, 12, 25, 27, 29, 33, 42, 43, 44, 47, 49, 74, 75, 81, 97, 110, 120, 123, 124, 133, 138, 139, 144, 154, 158, 163, 172, 173, 174, and/or 177 in PYR1 (SEQ ID NO:1) wherein the mutation is selected from R10Q, E12G, E12K, L25R, P27L, S29N, L33F, P42S, E43G, L44F, S47P, V49I, R74C, V75I, V81M, D97N, I110S, Y120C, Y120H, V123I, T124M, N133D, V138M, V139I, V144A, E154G, M158I, V163I, A172T, T173A, V174I, A177T or combinations thereof. In some instances, the mutated PYR/PYL receptor polypeptide still further comprises an isoleucine residue at one or more of the amino acid positions corresponding to positions 62 and 110 in PYR1 (SEQ ID NO:1). In some instances, the mutated PYR/PYL receptor polypeptide comprises a valine-to-isoleucine mutation at one or more of the amino acid positions corresponding to positions 62 and 110 in PYR1 (SEQ ID NO:1). In some instances, the present disclosure provides for a polynucleotide encoding one or more of said mutated PYR/PYL receptor polypeptides.

In some instances, the modified PYR/PYL receptor protein has been modified at a residue other than the K59 residue. A non-limiting exemplary list of mutations other than K59 which abolish ABA agonization but promote orthogonal ligand activation of a modified PYR/PYL receptor protein includes:
**D26**
**E94**
**F159**

Thus, in some instances, the mutated PYR/PYL receptor polypeptide comprises a mutation at an amino acid corresponding to amino acid position 26 in PYR1 (SEQ ID NO:1) wherein the mutation is D26G. In some instances, the mutated PYR/PYL receptor polypeptide comprises a mutation at an amino acid corresponding to amino acid position 94 in PYR1 (SEQ ID NO:1) wherein the mutation is E94D. In some instances, the mutated PYR/PYL receptor polypeptide comprises a mutation at an amino acid corresponding to amino acid position 159 in PYR1 (SEQ ID NO:1) wherein the mutation is F159L. In some instances, the present invention provides for a polynucleotide encoding one or more of said mutated PYR/PYL receptor polypeptides.

In some instances, the modified PYR/PYL receptor protein has been modified at at least two residues other than the K59 residue. A non-limiting list of exemplary combinations of mutations, which do not include a K59 mutation, and which abolish ABA agonization but promote orthogonal ligand activation of a modified PYR/PYL receptor protein, includes:
**R37 and F71**
**I110, E114, and V138**
**H115 and F159**

Thus, in some instances, the mutated PYR/PYL receptor polypeptide comprises at least two mutations at amino acids corresponding to positions 37, 71, 110, 114, 115, 138, and/or 159 in PYR1 (SEQ ID NO:1) wherein the mutations are selected from R37Q, F71S, I110T, E114D, H115Y, V138M, F159S, or combinations thereof. In some instances, the present invention provides for a polynucleotide encoding one or more of said mutated PYR/PYL receptor polypeptides.

In some embodiments, a mutated PYR/PYL receptor polypeptide comprises any of SEQ ID NOs:124-148 or 164-178.

### Ligand-binding pocket of PYR/PYL

PYR/PYL receptor proteins have a conserved START-domain ligand-binding pocket flanked by two loops called the "gate" and the "latch" (Melcher, K. et al., Nature 462 (2009)). ABA binds to a PYR/PYL receptor protein at the ligand-binding pocket and ABA binding induces closure of the loops to seal ABA inside the ligand-binding pocket. In wild-type PYR/PYL receptor proteins, residues comprising the ligand-binding pocket are those residues with side chains that are within 4 angstroms of ABA or the water molecules that accompany ABA when ABA binds in the pocket of the PYR/PYL receptor protein. For example, the residues comprising the ligand-binding pocket of PYR1 (SEQ ID NO:1) are: P55, K59, F61, I62, R79, V81, V83, P88, A89, S92, E94, F108, I110, H115, L117, Y120, E141, F159, V163, and N167.

In some instances, the PYR/PYL receptor polypeptides are mutated at at least one amino acid residue comprising the ligand-binding pocket of the PYR/PYL receptor protein. Accordingly, in some instances the mutated PYR/PYL receptor polypeptide comprises at least one mutation at an amino acid corresponding to positions 55, 59, 61, 62, 79, 81, 83, 88, 89, 92, 94, 108, 110, 115, 117, 120, 141, 159, 163, and/or 167 in PYR1 (SEQ ID NO:1) wherein the mutation is selected from P55, K59, F61, I62, R79, V81, V83, P88, A89, S92, E94, F108, I110, H115, L117, Y120, E141, F159, V163, N167, or combinations thereof. In some instances, the present invention provides for a polynucleotide encoding one or more of said mutated PYR/PYL receptor polypeptides.

Aspects of the present disclosure provide for use of the above proteins and/or nucleic acid sequences, encoding such polypeptides, in the methods and compositions (*e.g*., expression cassettes, plants, etc.) disclosed herein. The isolation of a polynucleotide sequence encoding a plant wild-type PYR/PYL receptor (*e.g*., from plants where PYR/PYL sequences have not yet been identified) may be accomplished by a number of techniques. For instance, oligonucleotide probes based on the PYR/PYL coding sequences disclosed *(e.g.,* as listed in the SEQUENCE LISTING) here can be used to identify the desired wild-type PYR/PYL gene in a cDNA or genomic DNA library. To construct genomic libraries, large segments of genomic DNA are generated by random fragmentation, *e.g*., using restriction endonucleases, and are ligated with vector DNA to form concatemers that can be packaged into the appropriate vector. To prepare a cDNA library, mRNA is isolated from the desired tissue, such as a leaf from a particular plant species, and a cDNA library containing the gene transcript of interest is prepared from the mRNA. Alternatively, cDNA may be prepared from mRNA extracted from other tissues in which PYR/PYL gene is expressed.

The cDNA or genomic library can then be screened using a probe based upon the sequence of a PYR/PYL gene disclosed here. Probes may be used to hybridize with genomic DNA or cDNA sequences to isolate homologous genes in the same or different plant species. Alternatively, antibodies raised against a polypeptide can be used to screen an mRNA expression library.

Alternatively, the nucleic acids encoding PYR/PYL can be amplified from nucleic acid samples using amplification techniques. For instance, polymerase chain reaction (PCR) technology can be used to amplify the coding sequences of PYR/PYL directly from genomic DNA, from cDNA, from genomic libraries or cDNA libraries. PCR and other *in vitro* amplification methods may also be useful, for example, to clone polynucleotide sequences encoding PYR/PYL to be expressed, to make nucleic acids to use as probes for detecting the presence of the desired mRNA in samples, for nucleic acid sequencing, or for other purposes. For a general overview of PCR see PCR Protocols: A Guide to Methods and Applications. (Innis, M., Gelfand, D., Sninsky, J. and White, T., eds.), Academic Press, San Diego (1990). Appropriate primers and probes for identifying sequences from plant tissues are generated from comparisons of the sequences provided here with other related genes.

In some instances, the partial or entire genome of a number of plants has been sequenced and open reading frames identified. By a BLAST search, one can identify the coding sequence for wild-type PYR/PYL in various plants.

### III. Chemical Agonists and Agonist Formulations

The present disclosure provides for agricultural chemical formulations formulated for contacting to mutated PYR/PYL receptor polypeptides and/or plants comprising mutated PYR/PYL receptor polypeptides, wherein the formulation comprises an agonist of a mutated PYR/PYL polypeptide described herein. In some instances, the agricultural chemical agonist comprises a fungicide, an herbicide, a pesticide, a nematicide, a plant activator, a synergist, an herbicide safener, a plant growth regulator, an insect repellant, or a fertilizer. In some instances, the agricultural chemical agonist is selected from the group consisting of bromoxynil, chloroxynil, ioxynil, coumatetralyl, dichlobenil, fenhexamid, benoxacor, and BTH (acibenzolar-s-methyl).

Agrochemicals are often prepared and applied to plants as esters or salts, which may improve uptake and efficacy. The action of ubiquitous cellular esterases can convert esters (or homologous compounds such as the S-methyl derivatives of acibenzolar) into free acids or alcohols, which are the bioactive forms. For example, bromoxynil butyrate, bromoxynil heptanoate, bromoxynil octanoate, and bromoxynil-potassium are alternate formulations of bromoxynil. After absorption these compounds form the same bioactive species (bromoxynil) in plants. Similar variants of chloroxynil and ioxynil can be made and are known (for example, ioxynil-lithium, ioxynil octanoate, ioxynil-sodium, etc.). BTH is an S-methyl derivative of acibenzolar, the active form of BTH in plants.

### Fenhexamid

It was found that mutating the amino acid corresponding to K59 in SEQ ID NO:1, along with mutating at least one more amino acid in the PYR/PYL receptor polypeptide, resulted in activation of the modified receptor by fenhexamid (Tables 1, 7, and 8). A non-limiting list of exemplary combinations of mutations that result in modified PYR/PYL receptor being agonized by fenhexamid includes:
**S47P, K59R, and Y120H**
**K59R, Y120H, and V144A**
**P42S, K59R, and Y120C**
**P42S, K59R, Y120C, and T124M**
**P42S, K59R, Y120C, and E154G**
**E12G, E43G, K59R, I110S, and N133D**
**E12G, L25R, E43G, K59R, I110S, and N133D**
**K59R, Y120H, and M158I**
**P42S, K59R, Y120H, and M158I**
**P42S, K59R, D97N, Y120H, V163I, and A172T**
**P42S, L44F, K59R, Y120H, V138M, and M158I**
**P42S, K59R, Y120H, V123I, V139I, and M158I**
**S47P, V49I, K59R, Y120H, M158I, and A177T**
**K59R, V81M, Y120C, M158I, and V163I**
**P27L, P42S, K59R, D97N, Y120H, M158I, and T173A**
**P42S, K59R, R74C, Y120H, and M158I**
**S29N, K59R, D97N, Y120H, V163I, and A172T**
**P42S, K59R, Y120H, V123I, V139I, M158I, and V163I**
**K59R, V81M, Y120H, M158I, and V163I**
**E12K, K59R, V75I, D97N, Y120H, V163I, and A172T**
**L33F, P42S, K59R, Y120H, V123I, and M158I**
**P42S, K59R, Y120H, M158I, V163I, and V174I**
**R10Q, P42S, K59R, D97N, Y120H, V163I, and A172T**

Thus, in some instances a modified PYR/PYL receptor is agonized by fenhexamid. In some instances, the modified PYR/PYL protein comprises one or more sets of mutations corresponding to those set forth above. In some instances, the modified PYR/PYL receptor protein comprising any of the sets of mutations corresponding to those set forth above further comprises an isoleucine residue at one or more of the amino acid positions corresponding to positions 62 and 110 in PYR1 (SEQ ID NO:1). In some instances, the modified PYR/PYL receptor protein comprises a valine-to-isoleucine mutation at one or more the amino acid positions corresponding to positions 62 and 110 in PYR1 (SEQ ID NO:1) (*e.g*., valine-to-isoleucine mutations at amino acid positions 64 and/or 114 in PYL2 (SEQ ID NO:3), which correspond to positions 62 and 110, respectively, in PYR1). In some instances, the modified PYR/PYL receptor proteins are substantially identical to any of SEQ ID NOs:1-119 and/or comprise one or more consensus sequence selected from SEQ ID NOs:149-163. In some instances, a polynucleotide encoding one or more of said modified PYR/PYL receptor polypeptides is provided.

Additionally, in some instances of the present invention, a method of screening for modified PYR/PYL receptor polypeptides includes screening the modified polypeptide to determine whether it is agonized by fenhexamid.

### Bromoxynil

It was also found that mutating the amino acid corresponding to K59 in SEQ ID NO:1, along with mutating at least one more amino acid in the PYR/PYL receptor polypeptide, resulted in activation of the modified receptor by bromoxynil (Table 2). A non-limiting list of exemplary combinations of mutations that result in modified PYR/PYL receptor being agonized by bromoxynil includes:
**T57A and K59R**
**R50G, K59R, and E141Q**
**K59, H60R, and N151D**
**K59R, H60R, E102G, T125A, and E141D**
**K59R and I82N**
**K59R and S92T**
**H21Y, K59R, H60R, S92T, and R116K**
**P41L, K59R, and H60R**

Thus, in some embodiments a modified PYR/PYL receptor of the present invention is agonized by bromoxynil or the bromoxynil analogs chloroxynil and ioxynil. In some embodiments, the modified PYR/PYL protein comprises one or more sets of mutations corresponding to those set forth above. In some instances, such proteins are substantially identical to any of SEQ ID NOs:1-119 and/or comprise one or more consensus sequence selected from SEQ ID NOs:149-163. In some instances, the present invention provides for a polynucleotide encoding one or more of said modified PYR/PYL receptor polypeptides.

Additionally, in some instances of the present invention, a method of screening for modified PYR/PYL receptor polypeptides includes screening the modified polypeptide to determine whether it is agonized by bromoxynil or the bromoxynil analogs chloroxynil and ioxynil.

### Dichlobenil

In some cases, mutating the amino acid corresponding to K59 in SEQ ID NO:1 alone results in activation of the modified receptor by dichlobenil (Table 3). In some cases, mutating the amino acid corresponding to K59 in SEQ ID NO:1, along with mutating at least one more amino acid in the PYR/PYL receptor polypeptide, results in activation of the modified receptor by dichlobenil. In some cases, the amino acid corresponding to K59 in SEQ ID NO:1 does not need be mutated in the modified PYR/PYL receptor polypeptide in order for dichlobenil to activate the receptor, so long as there is at least one mutation in another amino acid in the PYR/PYL receptor polypeptide. A non-limiting list of exemplary mutations and combinations of mutations that result in modified PYR/PYL receptor being agonized by dichlobenil includes:
**K59R**
**D26G**
**E94D**
**R37Q and F71S**
**P27L, K59R, and K63N**

Thus, in some instances a modified PYR/PYL receptor is agonized by dichlobenil. In some instances, the modified PYR/PYL protein comprises one or more sets of mutations corresponding to those set forth above. In some instances, such proteins are substantially identical to any of SEQ ID NOs:1-119 and/or comprise one or more consensus sequence selected from SEQ ID NOs:149-163. In some instances, a polynucleotide encoding one or more of said modified PYR/PYL receptor polypeptides is provided.

Additionally, in some instances, a method of screening for modified PYR/PYL receptor polypeptides includes screening the modified polypeptide to determine whether it is agonized by dichlobenil.

### Benoxacor

In some cases, mutating the amino acid corresponding to K59 in SEQ ID NO:1 alone results in activation of the modified receptor by benoxacor (Table 4). In some cases, the amino acid corresponding to K59 in SEQ ID NO:1 does not need be mutated in the modified PYR/PYL receptor polypeptide in order for benoxacor to activate the receptor, so long as there is at least one mutation in another amino acid in the PYR/PYL receptor polypeptide. A non-limiting list of exemplary combinations of mutations that result in modified PYR/PYL receptor being agonized by benoxacor includes:

### K59R and N119Y

### I110T, E114D, and V138M

Thus, in some instances a modified PYR/PYL receptor is agonized by benoxacor. In some instances, the modified PYR/PYL protein comprises one or more sets of mutations corresponding to those set forth above. In some instances, such proteins are substantially identical to any of SEQ ID NOs:1-119 and/or comprise one or more consensus sequence selected from SEQ ID NOs:49-163. In some instances a polynucleotide encoding one or more of said modified PYR/PYL receptor polypeptides is provided.

Additionally, in some instances, a method of screening for modified PYR/PYL receptor polypeptides includes screening the modified polypeptide to determine whether it is agonized by benoxacor.

### BTH

In some cases, mutating one amino acid in the PYR/PYL receptor polypeptide results in activation of the modified receptor by BTH (Table 5). In some cases, mutating two or more amino acids in the PYR/PYL receptor polypeptide results in activation of the modified receptor by BTH. A non-limiting list of exemplary mutations and combinations of mutations that result in modified PYR/PYL receptor being agonized by BTH includes:
**Q24R, K59R, I82T, F159L, and D161G**
**H115Y and F159S**
**F159L**

Thus, in some instances a modified PYR/PYL receptor is agonized by BTH. In some instances, the modified PYR/PYL protein comprises one or more sets of mutations corresponding to those set forth above. In some instances, such proteins are substantially identical to any of SEQ ID NOs:1-119 and/or comprise one or more consensus sequence selected from SEQ ID NOs:149-163. In some instances, a polynucleotide encoding one or more of said modified PYR/PYL receptor polypeptides is provided.

Additionally, in some instances of the present invention, a method of screening for modified PYR/PYL receptor polypeptides includes screening the modified polypeptide to determine whether it is agonized by BTH.

Chemical agonists can be prepared by a variety of methods known to one of skill in the art, for example, those described in Comprehensive Organic Transformations, 2d ed., Richard C. Larock, 1999. The starting materials for the methods described above are commercially available (Sigma-Aldrich) or can be prepared by methods known to one of skill in the art.

In some instances, the agricultural chemical formulations contemplated are formulated for contacting to plants. The formulations can be suitable for treating plants or plant propagation material, such as seeds, in accordance with the present invention, e.g., in a carrier. Suitable additives include buffering agents, wetting agents, coating agents, polysaccharides, and abrading agents. Exemplary carriers include water, aqueous solutions, slurries, solids and dry powders (*e.g*., peat, wheat, bran, vermiculite, clay, pasteurized soil, many forms of calcium carbonate, dolomite, various grades of gypsum, bentonite and other clay minerals, rock phosphates and other phosphorous compounds, titanium dioxide, humus, talc, alginate and activated charcoal). Any agriculturally suitable carrier known to one skilled in the art would be acceptable and is contemplated for use in the present invention. Optionally, the formulations can also include at least one surfactant, herbicide, fungicide, pesticide, or fertilizer.

Contacting the agricultural chemical formulation to the mutated PYR/PYL receptor polypeptide can be performed *in vitro* (*e.g*., wherein the mutated PYR/PYL receptor polypeptide exists in a purified form or is expressed in yeast cells) or *in vivo (e.g.,* wherein the mutated PYR/PYL receptor polypeptide is expressed by a plant). Contacting the agricultural chemical formulation to the mutated PYR/PYL receptor polypeptide *in vitro* can be performed using a variety of known methods, *e.g.,* by applying the formulation to protein binding assays, mammalian or yeast two-hybrid assays, competition assays, or cell-based assays using other organisms.

Contacting the agricultural chemical formulation to the mutated PYR/PYL receptor polypeptide *in vivo (e.g.,* to a plant) can be performed using a variety of known methods, *e.g*., by spraying, atomizing, dusting or scattering the compositions over the propagation material or brushing or pouring or otherwise contacting the compositions over the plant or, in the event of seed, by coating, encapsulating, or otherwise treating the seed. In an alternative to directly treating a plant or seed before planting, the formulations of the invention can also be introduced into the soil or other media into which the seed is to be planted. A carrier may also be used. The carrier can be solid or liquid, as noted above. In some instances peat is suspended in water as a carrier of the chemical agonist, and this mixture is sprayed into the soil or planting media and/or over the seed as it is planted.

### IV. Methods of Making Mutated PYR/PYL Receptor Polypeptides

Embodiments of the present invention provide for methods of making mutated PYR/PYL receptor polypeptides that are agonized by a chemical agonist that does not agonize a wild-type PYR/PYL receptor polypeptide. The method comprises mutagenizing the wild-type PYR/PYL receptor polypeptide, contacting one or more mutated PYR/PYL receptor polypeptides with the putative chemical agonist, and determining whether the chemical activates the one or more mutated PYR/PYL receptor polypeptides, wherein activation identifies the one or more mutated PYR/PYL receptor polypeptides as being agonized by the chemical.

Mutated PYR/PYL receptor polypeptides can be constructed by mutating the DNA sequences that encode the corresponding wild-type PYR/PYL receptor polypeptide *(e.g.,* a wild-type PYR/PYL polypeptide of any of SEQ ID NOs:1-119 or a corresponding variant from which the mutant PYR/PYL receptor polypeptide of the invention is derived), such as by using techniques commonly referred to as site-directed mutagenesis. Nucleic acid molecules encoding the wild-type PYR/PYL receptor polypeptide can be mutated by a variety of polymerase chain reaction (PCR) techniques well-known to one of ordinary skill in the art. *(See, e.g.,* PCR Strategies (M. A. Innis, D. H. Gelfand, and J. J. Sninsky eds., 1995, Academic Press, San Diego, CA) at Chapter 14; PCR Protocols : A Guide to Methods and Applications (M. A. Innis, D. H. Gelfand, J. J. Sninsky, and T. J. White eds., Academic Press, NY, 1990).

By way of non-limiting example, mutagenesis may be accomplished by means of error-prone PCR amplification (ePCR), which modifies PCR reaction conditions (*e.g*., using error-prone polymerases, varying magnesium or manganese concentration, or providing unbalanced dNTP ratios) in order to promote increased rates of error in DNA replication. Kits for ePCR mutagenesis are commercially available, such as the GeneMorph® PCR Mutagenesis kit (Stratagene) and Diversify® PCR Random Mutagenesis Kit (Clontech). Briefly, DNA polymerase (*e.g.,* Taq polymerase), salt (*e.g.,* MgCl2, MgSO4, or MnSO4), dNTPs in unbalanced ratios, reaction buffer, and DNA template are combined and subjected to standard PCR amplification according to manufacturer's instructions. Following ePCR amplification, the reaction products are cloned into a suitable vector to construct a mutagenized library, which can then be transformed into suitable cells (*e.g.,* yeast cells) for subsequent screening *(e.g.,* via a two-hybrid screen) as described below.

Alternatively, mutagenesis can be accomplished by recombination (*i.e*. DNA shuffling). Briefly, a shuffled mutant library is generated through DNA shuffling using in vitro homologous recombination by random fragmentation of a parent DNA followed by reassembly using PCR, resulting in randomly introduced point mutations. Methods of performing DNA shuffling are known in the art *(see, e.g.,* Stebel, S.C. et al., Methods Mol Biol 352:167-190 (2007)).

Optionally, multiple rounds of mutagenesis may be performed in order to improve the efficiency of mutant proteins isolated. Thus, PYR/PYL mutants isolated from ePCR and subsequent screening may be pooled and used as templates for later rounds of mutagenesis.

### V. Screening for Agonism of Mutated PYR/PYL Receptor Polypeptides

Methods of screening putative chemical agonists to determine whether the putative agonist agonizes a mutated PYR/PYL receptor polypeptide, but does not significantly agonize a wild-type PYR/PYL receptor polypeptide, when the putative agonist is contacted to the PYR/PYL receptor polypeptide are also provided. As used herein, an agent "agonizes" a PYR/PYL receptor protein if the presence of the agent results in activation or up-regulation of activity of the receptor, *e.g.,* to increase downstream signaling from the PYR/PYL receptor. For the present invention, an agent agonizes a PYR/PYL receptor if, when the agent is present at a concentration no greater than 200 µM, contacting the agent to the PYR/PYL receptor results in activation or up-regulation of the activity of the PYR/PYL receptor. If an agent does not induce activation or up-regulation of a PYR/PYL receptor protein's activity when the agent is present at a concentration no greater than 200 µM, then the agent does not significantly agonize the PYR/PYL receptor. As used herein, "activation" requires a minimum threshold of activity to be induced by the agent. Determining whether this minimum threshold of activity has been met can be accomplished, e.g., by using an enzymatic phosphatase assay that sets a minimum value for the level of enzymatic activity that must be induced, or by using an enzymatic phosphatase assay in the presence of a colorimetric detection reagent (*e.g*., para-nitrophenylphosphate) wherein the minimum threshold of activity has been met if a color change is observed.

A number of different screening protocols can be utilized to identify chemical agents that agonize a mutated PYR/PYL receptor polypeptide but not a wild-type PYR/PYL receptor polypeptide. Screening can take place using isolated, purified or partially purified reagents. Purified or partially purified PYR/PYL polypeptide can be used.

Alternatively, cell-based or plant-based methods of screening can be used. For example, cells that naturally express a wild-type PYR/PYL receptor polypeptide or that recombinantly express a wild-type or mutated PYR/PYL receptor polypeptide can be used. The cells used may be plant cells, animal cells, bacterial cells, fungal cells, including but not limited to yeast cells, insect cells, or mammalian cells. In general terms, the screening methods involve screening one or more chemical agents to identify an agent that agonizes the activity of a mutated PYR/PYL receptor polypeptide (*e.g*., activating the mutated PYR/PYL receptor polypeptide or increasing expression of the mutated PYR/PYL receptor polypeptide or of a transcript encoding a mutated PYR/PYL receptor polypeptide), but that does not agonize the activity of a wild-type PYR/PYL receptor polypeptide. Optionally, the screening method may involve two screening processes: first, screening a plurality of putative agonists to identify compounds that weakly interact with a wild-type PYR/PYL receptor polypeptide ("weak ligands"), then screening those weak ligands against wild-type PYR/PYL receptor polypeptide and a plurality of mutagenized PYR/PYL receptor polypeptides to determine which mutated PYR/PYL receptor polypeptides are agonized by weak ligands and which weak ligands selectively agonize only mutated PYR/PYL receptor polypeptides and not wild-type PYR/PYL receptor polypeptides.

### Binding assays

Optionally, preliminary screens can be conducted by screening for agents capable of binding to a wild-type PYR/PRL receptor polypeptide. Pre-selection of weak-binding ligands improves the frequency of isolating mutated PYR/PYL receptor polypeptides that are agonized by the agent, presumably because fewer alterations of the ligand binding site are required to achieve molecular recognition.

Binding assays can involve contacting a wild-type PYR/PYL receptor polypeptide with one or more chemical agents and allowing sufficient time for the protein and chemical agents to form a binding complex. Any binding complexes formed can be detected using any of a number of established analytical techniques. Protein binding assays include, but are not limited to, methods that measure co-precipitation or co-migration on non-denaturing SDS-polyacrylamide gels, and co-migration on Western blots (see, e.g., Bennet, J.P. and Yamamura, H.I. (1985) "Neurotransmitter, Hormone or Drug Receptor Binding Methods," in Neurotransmitter Receptor Binding (Yamamura, H. I., et al., eds.), pp. 61-89. Other binding assays involve the use of mass spectrometry or NMR techniques to identify molecules bound to the PYR/PYL polypeptide or displacement of labeled substrates (*e.g*., labeled agrochemical). The PYR/PYL polypeptide protein utilized in such assays can be naturally expressed, cloned or synthesized.

### Agonist assays

Agonist assays can involve screening putative chemical agonists (which may or may not have been pre-selected as weak binding ligands) to determine which putative agonists agonize at least one mutated PYR/PYL receptor polypeptides but not a wild-type PYR/PYL receptor polypeptide, and/or screening mutagenized PYR/PYL receptor polypeptides with putative chemical agonists (which may or may not have been pre-selected as weak binding ligands) to determine which mutagenized PYR/PYL receptor polypeptides are agonized by the putative agonist.

Any number of assays can be used to screen for agonists of mutated PYR/PYL receptor polypeptides. One activity assay involves testing whether a putative agonist can induce binding of a mutated PYR/PYL protein to a type 2 protein phosphatase (PP2C) polypeptide in an agonist-specific fashion. Mammalian or yeast two-hybrid approaches (*see, e.g.,* Bartel, P.L. et. al. Methods Enzymol, 254:241 (1995)) can be used to identify polypeptides or other molecules that interact or bind when expressed together in a cell. In some embodiments, agents that agonize a mutated PYR/PYL receptor polypeptide, but not a wild-type PYR/PYL receptor polypeptide, are identified in a two-hybrid assay between a PYR/PYL polypeptide and a type 2 protein phosphatase (PP2C) polypeptide, wherein an agonist is identified as an agent that activates or enables binding of the PYR/PYL polypeptide and the PP2C polypeptide. Thus, the two polypeptides bind in the presence, but not in the absence of the agent. Optionally, both positive and negative selection schemes can be utilized in the two-hybrid assay. For example, a yeast two-hybrid assay may utilize a URA3 reporter strain to conduct both positive and negative selection; growth of the URA strain in the absence of exogenously supplied uracil enables positive selection for mutants that improve agonist responsiveness (*i.e*. agonist-promoted protein-protein interaction), while growth on FOA (5-fluoro-orotic acid, which is metabolized by URA3 to a toxic metabolite) allows selection against mutants that promote agonist response (*e.g.* to remove mutants that lead to constitutive, *i.e.* unliganded, interactions).

Screening for a compound that increases the expression of a mutated PYR/PYL receptor polypeptide, but not a wild-type PYR/PYL receptor polypeptide, is also provided. Screening methods generally involve conducting cell-based or plant-based assays in which test compounds are contacted with one or more cells expressing PYR/PYL polypeptide, and then detecting an increase in PYR/PYL expression (either transcript or translation product). Assays can be performed with cells that naturally express wild-type PYR/PYL or in cells recombinantly altered to express mutated or wild-type PYR/PYL. Various controls can be conducted to ensure that an observed activity is authentic, including running parallel reactions with cells that lack the reporter construct or by not contacting a cell harboring the reporter construct with test compound.

Agents and mutated PYR/PYL receptor polypeptides that are initially identified by any of the foregoing screening methods can be further tested to validate the apparent activity and/or determine other biological effects of the agent and/or mutated PYR/PYL receptor polypeptide. In some cases, the identified agent and/or mutated PYR/PYL receptor polypeptide is tested for the ability to effect plant stress (*e.g*., drought tolerance), seed germination, or another phenotype affected by ABA. A number of such assays and phenotypes are known in the art and can be employed according to the methods described herein.

### VI. Recombinant Expression Vectors

Once a polynucleotide sequence encoding a mutated PYR/PYL receptor polypeptide is obtained, it can also be used to prepare an expression cassette for expressing the mutated PYR/PYL receptor polypeptide in a transgenic plant, directed by a heterologous promoter. Increased expression of mutated PYR/PYL polynucleotide is useful, for example, to produce plants that will be able to respond to a chemical agonist that does not agonize endogenous PYR/PYL receptor protein, thereby enhancing abiotic stress resistance.

Any of a number of means well known in the art can be used to drive mutated PYR/PYL activity or expression in plants. Any organ can be targeted, such as shoot vegetative organs/structures (*e.g*. leaves, stems and tubers), roots, flowers and floral organs/structures (*e.g*. bracts, sepals, petals, stamens, carpels, anthers and ovules), seed (including embryo, endosperm, and seed coat) and fruit. Alternatively, the mutated PYR/PYL polynucleotide can be expressed constitutively (*e.g*., using the CaMV 35S promoter).

To use a polynucleotide sequence for a mutated PYR/PYL receptor polypeptide in the above techniques, recombinant DNA vectors suitable for transformation of plant cells are prepared. Techniques for transforming a wide variety of higher plant species are well known and described in the technical and scientific literature. *See, e.g.,* Weising et al. Ann. Rev. Genet. 22:421-477 (1988). A DNA sequence coding for the mutated PYR/PYL receptor polypeptide preferably will be combined with transcriptional and translational initiation regulatory sequences which will direct the transcription of the sequence from the gene in the intended tissues of the transformed plant.

For example, a plant promoter fragment may be employed to direct expression of the mutated PYR/PYL polynucleotide in all tissues of a regenerated plant. Such promoters are referred to herein as "constitutive" promoters and are active under most environmental conditions and states of development or cell differentiation. Examples of constitutive promoters include the cauliflower mosaic virus (CaMV) 35S transcription initiation region, the 1'- or 2'- promoter derived from T-DNA of *Agrobacterium tumafaciens,* and other transcription initiation regions from various plant genes known to those of skill.

Alternatively, the plant promoter may direct expression of the mutated PYR/PYL receptor protein in a specific tissue (tissue-specific promoters) or may be otherwise under more precise environmental control (inducible promoters). Examples of tissue-specific promoters under developmental control include promoters that initiate transcription only in certain tissues, such as leaves or guard cells (including but not limited to those described in WO/2005/085449; U.S. Patent No. 6,653,535; Li et al., Sci China C Life Sci. 2005 Apr;48(2):181-6; Husebye, et al., Plant Physiol, April 2002, Vol. 128, pp. 1180-1188; and Plesch, et al., Gene, Volume 249, Number 1, 16 May 2000 , pp. 83-89(7)). Examples of environmental conditions that may affect transcription by inducible promoters include anaerobic conditions, elevated temperature, or the presence of light.

If proper protein expression is desired, a polyadenylation region at the 3'-end of the coding region should be included. The polyadenylation region can be derived from a naturally occurring PYR/PYL gene, from a variety of other plant genes, or from T-DNA.

The vector comprising the sequences (*e.g*., promoters or PYR/PYL coding regions) will typically comprise a marker gene that confers a selectable phenotype on plant cells. For example, the marker may encode biocide resistance, particularly antibiotic resistance, such as resistance to kanamycin, G418, bleomycin, hygromycin, or herbicide resistance, such as resistance to chlorosluforon or Basta.

In some embodiments, the mutated PYR/PYL nucleic acid sequence is expressed recombinantly in plant cells. A variety of different expression constructs, such as expression cassettes and vectors suitable for transformation of plant cells can be prepared. Techniques for transforming a wide variety of higher plant species are well known and described in the technical and scientific literature. See, *e.g.,* Weising et al. Ann. Rev. Genet. 22:421-477 (1988). A DNA sequence coding for a PYR/PYL protein can be combined with *cis*-acting (promoter) and *trans*-acting (enhancer) transcriptional regulatory sequences to direct the timing, tissue type and levels of transcription in the intended tissues of the transformed plant. Translational control elements can also be used.

Also described herein are a mutated PYR/PYL nucleic acid operably linked to a promoter which, in some embodiments, is capable of driving the transcription of the PYR/PYL coding sequence in plants. The promoter can be, *e.g.,* derived from plant or viral sources. The promoter can be, *e.g.*, constitutively active, inducible, or tissue specific. In construction of recombinant expression cassettes, vectors, transgenics, of the invention, a different promoters can be chosen and employed to differentially direct gene expression, *e.g.,* in some or all tissues of a plant or animal.

### Constitutive promoters

A promoter fragment can be employed to direct expression of a mutated PYR/PYL nucleic acid in all transformed cells or tissues, *e.g.*, as those of a regenerated plant. The term "constitutive regulatory element" means a regulatory element that confers a level of expression upon an operatively linked nucleic molecule that is relatively independent of the cell or tissue type in which the constitutive regulatory element is expressed. A constitutive regulatory element that is expressed in a plant generally is widely expressed in a large number of cell and tissue types. Promoters that drive expression continuously under physiological conditions are referred to as "constitutive" promoters and are active under most environmental conditions and states of development or cell differentiation.

A variety of constitutive regulatory elements useful for ectopic expression in a transgenic plant are well known in the art. The cauliflower mosaic virus 35S (CaMV 35S) promoter, for example, is a well-characterized constitutive regulatory element that produces a high level of expression in all plant tissues (Odell et al., Nature 313:810-812 (1985)). The CaMV 35S promoter can be particularly useful due to its activity in numerous diverse plant species (Benfey and Chua, Science 250:959-966 (1990); Futterer et al., Physiol. Plant 79:154 (1990); Odell *et al., supra,* 1985). A tandem 35S promoter, in which the intrinsic promoter element has been duplicated, confers higher expression levels in comparison to the unmodified 35S promoter (Kay et al., Science 236:1299 (1987)). Other useful constitutive regulatory elements include, for example, the cauliflower mosaic virus 19S promoter; the Figwort mosaic virus promoter; and the nopaline synthase (nos) gene promoter (Singer et al., Plant Mol. Biol. 14:433 (1990); An, Plant Physiol. 81:86 (1986)).

Additional constitutive regulatory elements including those for efficient expression in monocots also are known in the art, for example, the pEmu promoter and promoters based on the rice Actin-1 5' region (Last et al., Theor. Appl. Genet. 81:581 (1991); Mcelroy et al., Mol. Gen. Genet. 231:150 (1991); Mcelroy et al., Plant Cell 2:163 (1990)). Chimeric regulatory elements, which combine elements from different genes, also can be useful for ectopically expressing a nucleic acid molecule encoding a mutated PYR/PYL receptor protein (Comai et al., Plant Mol. Biol. 15:373 (1990)).

Other examples of constitutive promoters include the 1'- or 2'- promoter derived from T-DNA of Agrobacterium tumafaciens (see, *e.g.,* Mengiste (1997) *supra*; O'Grady (1995) Plant Mol. Biol. 29:99-108); actin promoters, such as the *Arabidopsis* actin gene promoter (see, *e.g.,* Huang (1997) Plant Mol. Biol. 1997 33:125-139); alcohol dehydrogenase (Adh) gene promoters (see, *e.g.,* Millar (1996) Plant Mol. Biol. 31:897-904); *ACT11* from *Arabidopsis* (Huang et al. Plant Mol. Biol. 33:125-139 (1996)), *Cat3* from *Arabidopsis* (GenBank No. U43147, Zhong et al., Mol. Gen. Genet. 251:196-203 (1996)), the gene encoding stearoyl-acyl carrier protein desaturase from *Brassica napus* (Genbank No. X74782, Solocombe et al. Plant Physiol. 104:1167-1176 (1994)), *GPc1* from maize (GenBank No. X15596, Martinez et al. J. Mol. Biol 208:551-565 (1989)), *Gpc2* from maize (GenBank No. U45855, Manjunath et al., Plant Mol. Biol. 33:97-112 (1997)), other transcription initiation regions from various plant genes known to those of skill. *See also* Holtorf Plant Mol. Biol. 29:637-646 (1995).

### Inducible promoters

Alternatively, a plant promoter may direct expression of the mutated PYR/PYL polynucleotide under the influence of changing environmental conditions or developmental conditions. Examples of environmental conditions that may effect transcription by inducible promoters include anaerobic conditions, elevated temperature, drought, or the presence of light. Such promoters are referred to herein as "inducible" promoters. For example, the invention can incorporate a drought-specific promoter such as a drought-inducible promoter of maize (*e.g.,* the maize rab17 drought-inducible promoter (Vilardell et al. (1991) Plant Mol. Biol. 17:985-993; Vilardell et al. (1994) Plant Mol. Biol. 24:561-569)); or alternatively a cold, drought, and high salt inducible promoter from potato (Kirch (1997) Plant Mol. Biol. 33:897-909).

Alternatively, plant promoters which are inducible upon exposure to plant hormones, such as auxins, are used to express the mutated PYR/PYL polynucleotide. For example, the invention can use the auxin-response elements E1 promoter fragment (AuxREs) in the soybean (Glycine max L.) (Liu (1997) Plant Physiol. 115:397-407); the auxin-responsive *Arabidopsis* GST6 promoter (also responsive to salicylic acid and hydrogen peroxide) (Chen (1996) Plant J. 10: 955-966); the auxin-inducible parC promoter from tobacco (Sakai (1996) 37:906-913); a plant biotin response element (Streit (1997) Mol. Plant Microbe Interact. 10:933-937); and, the promoter responsive to the stress hormone abscisic acid (Sheen (1996) Science 274:1900-1902).

Plant promoters inducible upon exposure to chemicals reagents that may be applied to the plant, such as herbicides or antibiotics, are also useful for expressing the mutated PYR/PYL polynucleotide. For example, the maize In2-2 promoter, activated by benzenesulfonamide herbicide safeners, can be used (De Veylder (1997) Plant Cell Physiol. 38:568-577); application of different herbicide safeners induces distinct gene expression patterns, including expression in the root, hydathodes, and the shoot apical meristem. A PYR/PYL coding sequence can also be under the control of, *e.g.,* a tetracycline-inducible promoter, *e.g*., as described with transgenic tobacco plants containing the *Avena sativa* L. (oat) arginine decarboxylase gene (Masgrau (1997) Plant J. 11:465-473); or, a salicylic acid-responsive element (Stange (1997) Plant J. 11:1315-1324; Uknes et al., Plant Cell 5:159-169 (1993); Bi et al., Plant J. 8:235-245 (1995)).

Examples of useful inducible regulatory elements include copper-inducible regulatory elements (Mett et al., Proc. Natl. Acad. Sci. USA 90:4567-4571 (1993); Furst et al., Cell 55:705-717 (1988)); tetracycline and chlor-tetracycline-inducible regulatory elements (Gatz et al., Plant J. 2:397-404 (1992); Röder et al., Mol. Gen. Genet. 243:32-38 (1994); Gatz, Meth. Cell Biol. 50:411-424 (1995)); ecdysone inducible regulatory elements (Christopherson et al., Proc. Natl. Acad. Sci. USA 89:6314-6318 (1992); Kreutzweiser et al., Ecotoxicol. Environ. Safety 28:14-24 (1994)); heat shock inducible regulatory elements (Takahashi et al., Plant Physiol. 99:383-390 (1992); Yabe et al., Plant Cell Physiol. 35:1207-1219 (1994); Ueda et al., Mol. Gen. Genet. 250:533-539 (1996)); and *lac* operon elements, which are used in combination with a constitutively expressed *lac* repressor to confer, for example, IPTG-inducible expression (Wilde et al., EMBO J. 11:1251-1259 (1992)). An inducible regulatory element useful in the transgenic plants of the invention also can be, for example, a nitrate-inducible promoter derived from the spinach nitrite reductase gene (Back et al., Plant Mol. Biol. 17:9 (1991)) or a light-inducible promoter, such as that associated with the small subunit of RuBP carboxylase or the LHCP gene families (Feinbaum et al., Mol. Gen. Genet. 226:449 (1991); Lam and Chua, Science 248:471 (1990)).

### Tissue-specific promoters

Alternatively, the plant promoter may direct expression of the mutated PYR/PYL polynucleotide in a specific tissue (tissue-specific promoters). Tissue specific promoters are transcriptional control elements that are only active in particular cells or tissues at specific times during plant development, such as in vegetative tissues or reproductive tissues.

Examples of tissue-specific promoters under developmental control include promoters that initiate transcription only (or primarily only) in certain tissues, such as vegetative tissues, *e.g*., roots or leaves, or reproductive tissues, such as fruit, ovules, seeds, pollen, pistols, flowers, or any embryonic tissue, or epidermis or mesophyll. Reproductive tissue-specific promoters may be, *e.g*., ovule-specific, embryo-specific, endosperm-specific, integument-specific, seed and seed coat-specific, pollen-specific, petal-specific, sepal-specific, or some combination thereof. The promoter may be cell-type specific, *e.g.,* guard cell-specific.

Other tissue-specific promoters include seed promoters. Suitable seed-specific promoters are derived from the following genes: *MAC1* from maize (Sheridan (1996) Genetics 142:1009-1020); *Cat3* from maize (GenBank No. L05934, Abler (1993) Plant Mol. Biol. 22:10131-1038); *vivparous-1* from *Arabidopsis* (GenbankNo. U93215); *atmyc1* from *Arabidopsis* (Urao (1996) Plant Mol. Biol. 32:571-57; Conceicao (1994) Plant 5:493-505); *napA* from *Brassica napus* (GenBank No. J02798, Josefsson (1987) JBL 26:12196-1301); and the napin gene family from *Brassica napus* (Sjodahl (1995) Planta 197:264-271).

A variety of promoters specifically active in vegetative tissues, such as leaves, stems, roots and tubers, can also be used to express polynucleotides encoding mutated PYR/PYL receptor polypeptides. For example, promoters controlling patatin, the major storage protein of the potato tuber, can be used, see, *e.g.,* Kim (1994) Plant Mol. Biol. 26:603-615; Martin (1997) Plant J. 11:53-62. The ORF13 promoter from *Agrobacterium rhizogenes* that exhibits high activity in roots can also be used (Hansen (1997) Mol. Gen. Genet. 254:337-343. Other useful vegetative tissue-specific promoters include: the tarin promoter of the gene encoding a globulin from a major taro (*Colocasia esculenta* L. Schott) corm protein family, tarin (Bezerra (1995) Plant Mol. Biol. 28:137-144); the curculin promoter active during taro corm development (de Castro (1992) Plant Cell 4:1549-1559) and the promoter for the tobacco root-specific gene TobRB7, whose expression is localized to root meristem and immature central cylinder regions (Yamamoto (1991) Plant Cell 3:371-382).

Leaf-specific promoters, such as the ribulose biphosphate carboxylase (RBCS) promoters can be used. For example, the tomato RBCS1, RBCS2 and RBCS3A genes are expressed in leaves and light-grown seedlings, only RBCS1 and RBCS2 are expressed in developing tomato fruits (Meier (1997) FEBS Lett. 415:91-95). A ribulose bisphosphate carboxylase promoters expressed almost exclusively in mesophyll cells in leaf blades and leaf sheaths at high levels, described by Matsuoka (1994) Plant J. 6:311-319, can be used. Another leaf-specific promoter is the light harvesting chlorophyll a/b binding protein gene promoter, see, *e.g.,* Shiina (1997) Plant Physiol. 115:477-483; Casal (1998) Plant Physiol. 116:1533-1538. The *Arabidopsis thaliana* myb-related gene promoter (Atmyb5) described by Li (1996) FEBS Lett. 379:117-121, is leaf-specific. The Atmyb5 promoter is expressed in developing leaf trichomes, stipules, and epidermal cells on the margins of young rosette and cauline leaves, and in immature seeds. Atmyb5 mRNA appears between fertilization and the 16 cell stage of embryo development and persists beyond the heart stage. A leaf promoter identified in maize by Busk (1997) Plant J. 11:1285-1295, can also be used.

Another class of useful vegetative tissue-specific promoters are meristematic (root tip and shoot apex) promoters. For example, the "*SHOOTMERISTEMLESS*" and "*SCARECROW*' promoters, which are active in the developing shoot or root apical meristems, described by Di Laurenzio (1996) Cell 86:423-433; and, Long (1996) Nature 379:66-69; can be used. Another useful promoter is that which controls the expression of 3-hydroxy-3- methylglutaryl coenzyme A reductase HMG2 gene, whose expression is restricted to meristematic and floral (secretory zone of the stigma, mature pollen grains, gynoecium vascular tissue, and fertilized ovules) tissues (see, *e.g.,* Enjuto (1995) Plant Cell. 7:517-527). Also useful are kn1-related genes from maize and other species which show meristem-specific expression, see, *e.g.,* Granger (1996) Plant Mol. Biol. 31:373-378; Kerstetter (1994) Plant Cell 6:1877-1887; Hake (1995) Philos. Trans. R. Soc. Lond. B. Biol. Sci. 350:45-51. For example, the *Arabidopsis thaliana* KNAT1 promoter (see, *e.g.,* Lincoln (1994) Plant Cell 6:1859-1876).

One of skill will recognize that a tissue-specific promoter may drive expression of operably linked sequences in tissues other than the target tissue. Thus, as used herein a tissue-specific promoter is one that drives expression preferentially in the target tissue, but may also lead to some expression in other tissues as well.

The mutated PYR/PYL polynucleotide may be expressed through a transposable element. This allows for constitutive, yet periodic and infrequent expression of the constitutively active polypeptide. The disclosure also provides for use of tissue-specific promoters derived from viruses including, *e.g*., the tobamovirus subgenomic promoter (Kumagai (1995) Proc. Natl. Acad. Sci. USA 92:1679-1683; the rice tungro bacilliform virus (RTBV), which replicates only in phloem cells in infected rice plants, with its promoter which drives strong phloem-specific reporter gene expression; the cassava vein mosaic virus (CVMV) promoter, with highest activity in vascular elements, in leaf mesophyll cells, and in root tips (Verdaguer (1996) Plant Mol. Biol. 31:1129-1139).

### VII. Production of Transgenic Plants

As detailed herein, embodiments of the present invention provide for transgenic plants comprising recombinant expression cassettes for expressing a mutant PYR/PYL receptor protein as described herein in a plant. A transgenic plant may be generated that contains a complete or partial sequence of a polynucleotide that is derived from a species other than the species of the transgenic plant. It should be recognized that transgenic plants encompass the plant or plant cell in which the expression cassette is introduced as well as progeny of such plants or plant cells that contain the expression cassette, including the progeny that have the expression cassette stably integrated in a chromosome.

A recombinant expression vector comprising a PYR/PYL coding sequence driven by a heterologous promoter may be introduced into the genome of the desired plant host by a variety of conventional techniques. For example, the DNA construct may be introduced directly into the genomic DNA of the plant cell using techniques such as electroporation and microinjection of plant cell protoplasts, or the DNA construct can be introduced directly to plant tissue using ballistic methods, such as DNA particle bombardment. Alternatively, the DNA construct may be combined with suitable T-DNA flanking regions and introduced into a conventional *Agrobacterium tumefaciens* host vector. The virulence functions of the *Agrobacterium tumefaciens* host will direct the insertion of the construct and adjacent marker into the plant cell DNA when the cell is infected by the bacteria. While transient expression of mutated PYR/PYL is contemplated, generally expression of the construction will be from insertion of expression cassettes into the plant genome, *e.g*., such that at least some plant offspring also contain the integrated expression cassette.

Microinjection techniques are also useful for this purpose. These techniques are well known in the art and thoroughly described in the literature. The introduction of DNA constructs using polyethylene glycol precipitation is described in Paszkowski et al. EMBO J. 3:2717-2722 (1984). Electroporation techniques are described in Fromm et al. Proc. Natl. Acad. Sci. USA 82:5824 (1985). Ballistic transformation techniques are described in Klein et al. Nature 327:70-73 (1987).

*Agrobacterium tumefaciens*-mediated transformation techniques, including disarming and use of binary vectors, are well described in the scientific literature. See, for example, Horsch et al. Science 233:496-498 (1984), and Fraley et al. Proc. Natl. Acad. Sci. USA 80:4803 (1983).

Transformed plant cells derived by any of the above transformation techniques can be cultured to regenerate a whole plant that possesses the transformed genotype and thus the desired phenotype such as enhanced abiotic stress resistance. Such regeneration techniques rely on manipulation of certain phytohormones in a tissue culture growth medium, typically relying on a biocide and/or herbicide marker which has been introduced together with the desired nucleotide sequences. Plant regeneration from cultured protoplasts is described in Evans et al., Protoplasts Isolation and Culture, Handbook of Plant Cell Culture, pp. 124-176, MacMillilan Publishing Company, New York, 1983; and Binding, Regeneration of Plants, Plant Protoplasts, pp. 21-73, CRC Press, Boca Raton, 1985. Regeneration can also be obtained from plant callus, explants, organs, or parts thereof. Such regeneration techniques are described generally in Klee et al. Ann. Rev. of Plant Phys. 38:467-486 (1987).

One of skill will recognize that after the expression cassette is stably incorporated in transgenic plants and confirmed to be operable, it can be introduced into other plants by sexual crossing. Any of a number of standard breeding techniques can be used, depending upon the species to be crossed.

The expression cassettes of the invention can be used to confer abiotic stress resistance on essentially any plant. Thus, the invention has use over a broad range of plants, including species from the genera *Asparagus, Atropa, Avena, Brassica, Citrus, Citrullus, Capsicum, Cucumis, Cucurbita, Daucus, Fragaria, Glycine, Gossypium, Helianthus, Heterocallis, Hordeum, Hyoscyamus, Lactuca, Linum, Lolium, Lycopersicon, Malus, Manihot, Majorana, Medicago, Nicotiana, Oryza, Panieum, Pannesetum, Persea, Pisum, Pyrus, Prunus, Raphanus, Secale, Senecio, Sinapis, Solanum, Sorghum, Trigonella, Triticum, Vitis, Vigna,* and, *Zea.* The plant may be selected from the group consisting of rice, maize, wheat, soybeans, cotton, canola, turfgrass, and alfalfa. In some instances, the plant is an ornamental plant. The plant may be a vegetable- or fruit-producing plant.

Those of skill will recognize that a number of plant species can be used as models to predict the phenotypic effects of transgene expression in other plants. For example, it is well recognized that both tobacco (*Nicotiana*) and *Arabidopsis* plants are useful models of transgene expression, particularly in other dicots.

In some embodiments, the plants of the invention have enhanced sensitivity to certain chemical agonists compared to plants are otherwise identical except for expression of the mutated PYR/PYL receptor polypeptide. Sensitivity to agonists that agonize the PYR/PYL family of ABA receptors can be monitored by observing or measuring any phenotype mediated by ABA. Those of skill in the art will recognize that ABA is a well-studied plant hormone and that ABA mediates many changes in characteristics, any of which can be monitored to determine whether ABA sensitivity has been modulated. Modulated ABA sensitivity may be manifested by altered timing of seed germination or altered stress (*e.g*., drought) tolerance.

Abiotic stress resistance can assayed according to any of a number of well-known techniques. For example, for drought tolerance, plants can be grown under conditions in which less than optimum water is provided to the plant. Drought resistance can be determined by any of a number of standard measures including turgor pressure, growth, yield, and the like.

### EXAMPLES

The following examples are offered to illustrate, but not to limit the claimed invention.

### Example 1: Isolation of PYR1 Orthogonal Receptors

For isolating mutated (orthogonal) PYR/PYL receptors, a suitable target ligand is first identified. Next, receptor mutagenesis and selection experiments are used to identify orthogonal receptors that respond to the target orthogonal ligand. In general, the higher the starting affinity of the target ligand for the receptor prior to mutagenesis, the fewer the number of mutations that will be needed to realize target recognition. Proteins with promiscuous ligand-binding pockets are inherently better starting points for engineering efforts than those with pockets that are highly selective, since they are likely to make weak contacts with a greater number of ligands than a highly selective binding pocket. Furthermore, a receptor protein whose function can be measured in a heterologous host such as *S. cerevisiae* is a preferred target for receptor engineering, because such assays allow large numbers of variant receptors to be screened rapidly.

### Methods and Results

To isolate PYR1 orthogonal receptors, a two-step process was used. First, a large collection of compounds was screened to identify compounds that weakly interact with receptors, defined as such by their ability to displace receptor-bound ABA in competition experiments. Once weak binders were identified, those with the most desirable properties from an end-use perspective were used as targets for iterative mutagenesis and selection schemes. Pre-selection of weak-binding ligands improves the frequency of isolating new receptor proteins, because fewer alterations of the ligand binding site are required to achieve molecular recognition. However, pre-selection is not a prerequisite for success.

**Pre-selection of ligands.** 74 structurally diverse agrochemicals were obtained, selected from a larger set of approximately -1500 commercially available agrochemicals. These 74 compounds were tested for their ability to reduce ABA-promoted PYR/PYL-PP2C protein-protein interactions in suitable yeast strains when added at 1000-fold excess relative to ABA (*i.e.* 100 nM ABA and 100 µM test chemical). This pilot screen revealed that the closely related herbicides bromoxynil and its chloro analog chloroxynil, the rodenticide coumatetralyl, the herbicide dicholbenil, and the fungicide fenhexamid all compete for ABA. This observed hit rate of 5 out of 74 screened compounds demonstrates that weak binding to the PYR/PYL ligand binding pocket is common, consistent with observations that START proteins have promiscuous ligand binding pockets (Mogensen, J.E. et al., Journal of Biological Chemistry 277:23684-23692 (2002)).

**PCR-based Mutagenesis of PYR1.** To identify PYR/PYL mutations that alter agonist responsiveness, the coding sequence for PYR1 was mutated by error-prone PCR using established protocols (Lin-Goerke et al., Biotechniques 23:409-412 (1997)) and cloned into the yeast two hybrid vector pBD-GAL4, yielding a library of ∼70,000 mutants (named ePCR1 library). This plasmid library was amplified in *E. coli* and then transformed into *S. cerevisiae* strain MAV99 co-transformed with a Gal4 activation domain (AD) - PP2C fusion protein encoded by the plasmid pAD-HAB1, as described previously (Park et al., Science 324:1068-1071 (2009)). MAV99 (Vidal et al., Proc Natl Acad Sci USA 93:10315-10320 (1996)) is a reverse two hybrid strain that contains a Gal4 activated URA3 reporter gene. This strain will not grow in the absence of exogenously supplied uracil. However, if the strain expresses a PYR1 mutant that enables a protein-protein interaction between PYR1 and HAB1, the URA3 reporter gene is activated, which enables strain growth and subsequent colony formation. Thus, co-expression of BD-PYR1 and AD-HAB1 in the MAV99 strain enables a positive selection scheme for PYR1 mutations that allow non-natural agonists to promote the PYR1-HAB1 interaction, which can be observed as agonist promoted growth in the absence of added uracil. In addition, inclusion of the compound 5-fluoro-orotic acid (FOA, which is metabolized by URA3 to a toxic metabolite) in growth media, instead of uracil, allows selection against mutations that confer BD-PYR1 - AD-HAB1 interactions in an agonist-independent fashion ("constitutive mutations"). Constitutive mutations are not desirable in this screening strategy because a target agrochemical molecule would not regulate the activity of a constitutive mutant. Because both regulated and constitutive PYR1 mutants will enable growth of the HAB1-AD expressing MAV99 strain in the absence of exogenously supplied uracil, it is beneficial to utilize both positive and negative selection schemes.

**Isolation of agrochemical responsive mutants.** To identify PYR1 mutations that confer responsiveness to non-natural agrochemical agonists, the ePCR1 library was transformed into the MAV99/pAD-HAB1 strain to create a library called "A". The A library was then grown on media containing 0.15% FOA. This created a mutant library called "A"', which is a library of PYR1 mutants with reduced numbers of constitutive mutations.

The A' library was then used for a variety of selection experiments using different agrochemicals. For example, for the isolation of fenhexamid responsive mutations ∼300,000 A' cells were plated onto growth media lacking uracil and containing 200 µM fenhexamid. After several days incubation, ∼80 colonies appeared and were individually collected and retested on growth media either containing or lacking fenhexamid. Colonies that grew on plates lacking fenhexamid contain constitutive mutations that were not eliminated during the preparation of the A' library and were discarded. The PYR1 coding sequences of the remaining -30 mutations were sequenced, which revealed that 7 distinct mutant sequences (Table 1) had been isolated:
**27-18:** S47P, K59R, Y120H
**27-24:** K59R, Y120H, V144A
**27-31:** P42S, K59R, Y120C
**27-28:** P42S, K59R, Y120C, T124M
**27-9:** P42S, K59R, Y120C, E154G
**27-36:** E12G, E43G, K59R, I110S, N133D
**27-14:** E12G, L25R, E43G, K59R, I110S, N133D

**Table 1. Fenhexamid-responsive PYR/PYL receptor polypeptide mutants**

| **Clone #** | **Mutations Present** | **Source** | **Growth* on [Fenhexamid]** | | **SEQ ID NO** |
|---|---|---|---|---|---|
| | | | 0 µM | 200 µM | |
| 27-18 | S47P, K59R, Y120H | ePCR1 | - | +++ | 124 |
| 27-24 | K59R, Y120H, V144A | ePCR1 | - | +++ | 125 |
| 27-31 | P42S, K59R, Y120C | ePCR1 | - | +++ | 126 |
| 27-28 | P42S, K59R, Y120C, T124M | ePCR1 | - | +++ | 127 |
| 27-9 | P42S, K59R, Y120C, E154G | ePCR1 | - | +++ | 128 |
| 27-36 | E12G, E43G, K59R, I110S, N133D | ePCR1 | - | +++ | 129 |
| -14 | E12G, L25R, E43G, K59R, I110S, N133D | ePCR1 | - | +++ | 130 |

| | | | | | |
|---|---|---|---|---|---|
| ePCR1=error prone PCR mutagenesis library of PYR1, 70,000 clones starting size. *Growth was measured on media lacking uracil and using the reporter strain MAV99, which only grows when an agonist-promoted protein-protein interaction between PYR1-GAL4 BD and HAB1-GAL4 AD reconstitutes GAL4 activity and enables expression of the strain's URA3 gene. | | | | | |

Fenhexamid responsiveness of the PYR1 mutants was confirmed by yeast two-hybid for representative mutants. Plasmids for three representative mutant clones (27-9, 27-18, and 27-36) were isolated from the primary yeast cells and transformed into a pAD-HAB1 expressing YRG-2 reporter strain (Park et al., Science 324:1068-1071 (2009)) in which Gal4 drives expression of a LacZ reporter gene to allow colorimetric indication of agonist response. Fenhexamid was applied at concentrations of 1, 5, 10, 25, and 50 µM. Mutant 27-18 strongly responded to fenhexamid at all the concentrations tested, as determined by the strong blue (LacZ-positive) staining. Mutant 27-9 strongly responded to fenhexamid at 5, 10, 25, and 50 µM concentrations, and was weakly positive at 1 µM. Mutant 27-36 was responsive to fenhexamid at 25 and 50 µM concentrations.

To probe orthogonal receptor-ligand interactions further, recombinant protein was expressed for the fenhexamid-responsive mutant 27-18, which has three mutations in PYR1. 6X-His-PYR1(27-18) protein was expressed and purified alongside wild-type PYR1 protein. Both proteins were tested for their ability to inhibit PP2C activity in response to increasing concentrations of either ABA or fenhexamid using phosphatase activity assays, in which activation of the PYR/PYL receptor is monitored by inhibition of PP2C activity (Figure 1). Phosphatase activity assays were performed as described in Park et al., Science 324:1068-1071 (2009), with the minor modification that the assay buffer utilized 10 mM Mn++ instead of Mg++; it was found that this modification enhanced HAB1's specific activity ∼10 fold. It was found that that ABA failed to activate mutant 27-18, but efficiently activated wild-type PYR1 protein (Figure 1A), therefore demonstrating that mutant 27-18 is insensitive to ABA. Furthermore, mutant 27-18 was activated by fenhexamid whereas wild-type PYR1 protein was not (Figure 1B), therefore demonstrating that mutant PYR/PYL receptor polypeptides can be used to control PP2C activity in response to fenhexamid.

Screens for bromoxynil (Table 2), dichlobenil (Table 3), and benoxacor (Table 4) responsive PYR1 mutants were conducted as described above for fenhexamid, each utilizing 200 µM test compound and the A' library, generated as described above. Yeast two-hybrid assay confirmed bromoxynil responsiveness for representative mutant clones 74A-1 and 74A-2. Bromoxynil was applied at concentrations of 5, 10, 25, and 50 µM. Both mutants 74A-1 and 74A-2 were responsive to bromoxynil at concentrations of 10, 25, and 50 µM.

**Table 2. Bromoxynil-responsive PYR/PYL receptor polypeptide mutants**

| **Clone #** | **Mutations Present** | **Source** | **Growth* on [Bromoxynil]** | | | **SEQ ID NO** |
|---|---|---|---|---|---|---|
| | | | 0 µM | 0.5 µM | 200 µM | |
| 74A-12 | T57A, K59R | ePCR1 | - | ND | ++ | 131 |
| 74A-24 | R50G, K59R, E141Q | ePCR1 | - | ND | ++ | 132 |
| 74A-1 | K59R, H60R, N151D | ePCR1 | - | ND | ++ | 133 |
| 74A-4 | K59R, H60R, E102G, T125A, E141D | ePCR1 | - | ND | ++ | 134 |
| 74A-13 | K59R, I82N | ePCR1 | - | ND | ++ | 135 |
| 74A-2 | K59R, S92T | ePCR1 | - | ND | ++ | 136 |
| 74A-15 | K59R, S92T | ePCR1 | - | ND | ++ | 137 |
| 74B-1 | H21Y, K59R, H60R, S92T, R116K | Shuffled First Round | - | ++ | ND | 138 |
| 74B-7 | P41L, K59R, H60R | Shuffled First Round | - | ++ | ND | 139 |

| | | | | | | |
|---|---|---|---|---|---|---|
| ePCR1=error prone PCR mutagenesis library of PYR1, 70,000 clones starting size; Shuffled First Round=PYR1 shuffled mutant library made using isolated ePCR mutants. *Growth was measured on media lacking uracil and using the reporter strain MAV99, which only grows when an agonist-promoted protein-protein interaction between PYR1-GAL4 BD and HAB1-GAL4 AD reconstitutes GAL4 activity and enables expression of the strain's URA3 gene. | | | | | | |

**Table 3. Dichlobenil-responsive PYR/PYL receptor polypeptide mutants**

| **Clone #** | **Mutations Present** | **Source** | **Growth* on [Dichlobenil]** | | **SEQ ID NO** |
|---|---|---|---|---|---|
| | | | 0 µM | 200 µM | |
| 68-1 | E94D | ePCR1 | - | ++ | 140 |
| 68-2 | R37Q, F71S | ePCR1 | - | ++ | 141 |
| 68-8 | D26G | ePCR1 | - | ++ | 142 |
| 68-3 | P27L, K59R, K63N | ePCR1 | - | ++ | 143 |
| 68-17 | K59R | ePCR1 | - | ++ | 144 |

| | | | | | |
|---|---|---|---|---|---|
| ePCR1=error prone PCR mutagenesis library of PYR1, 70,000 clones starting size. *Growth was measured on media lacking uracil and using the reporter strain MAV99, which only grows when an agonist-promoted protein-protein interaction between PYR1-GAL4 BD and HAB1-GAL4 AD reconstitutes GAL4 activity and enables expression of the strain's URA3 gene. | | | | | |

**Table 4. Benoxacor-responsive PYR/PYL receptor polypeptide mutants**

| **Clone #** | **Mutations Present** | **Source** | **Growth* on [Benoxacor]** | | **SEQ ID NO** |
|---|---|---|---|---|---|
| | | | 0 µM | 200 µM | |
| 129-2 | I110T, E114D, V138M | ePCR1 | - | + | 145 |
| 127-1 | K59R, N119Y | ePCR1 | - | + | 146 |

| | | | | | |
|---|---|---|---|---|---|
| ePCR1=error prone PCR mutagenesis library of PYR1, 70,000 clones starting size. *Growth was measured on media lacking uracil and using the reporter strain MAV99, which only grows when an agonist-promoted protein-protein interaction between PYR1-GAL4 BD and HAB1-GAL4 AD reconstitutes GAL4 activity and enables expression of the strain's URA3 gene. | | | | | |

**Improvement of primary mutants using DNA shuffling.** Recombining existing mutations or variants using DNA shuffling is a proven method to rapidly improve protein function and activity (Stemmer, Nature 370:389-391 (1994)). To improve the isolated bromoxynil responsive PYR1 mutants, plasmid DNA for the 7 mutant sequences identified by screening the A' library (74A-12, 74A-24, 74A-1, 74A-4, 74A-13, 74A-2 and 74A-15) were combined with an equimolar amount of plasmid for the original PYR1 ePCR1 mutant library. The addition of ePCR1 DNA to the shuffling reaction enabled new mutations to be introduced into existing mutations and therefore increases potential sequence diversity. The mixed DNAs were shuffled using an established protocol (Muller et al., Nucleic Acids Res 33:e117 (2005)) and the shuffled PCR product DNA was cloned into pBD-GAL4 to generate a ∼50,000 clones which were collected and named the "B" library, which amplified in E. coli and was introduced into yeast strain MAV99 co-transformed with pAD-HAB1. These cells were then grown on 0.15% FOA to remove constitutive mutants, yielding a "B"' library. The B' library was then grown on plates lacking added uracil and containing 0.5 µM bromoxynil. Two unique non-constitutive clones were isolated (74B-1 and 74B-7) (Table 2).

Given the plasticity of PYR1 indicated by these experiments, we sought to establish if an orthogonal receptor could be isolated for a target ligand that was not prescreened for weak binding using the yeast competition assays described above. The same screening methodology as described above was used to screen for mutants that respond to BTH (acibenzolar-s-methyl). This screen resulted in the successful isolation of three different PYR1 mutants that respond to BTH (Table 5). Yeast two-hybrid assay confirmed BTH responsiveness for representative mutant clone BTH-9. Bromoxynil was applied at concentrations of 25, 50, 100, and 200 µM, and mutant BTH-9 was responsive to BTH at concentrations of 50, 100, and 200 µM. Thus, the plasticity of PYR1 enables many orthogonal receptor variants to be isolated.

**Table 5. BTH-responsive PYR/PYL receptor polypeptide mutants**

| **Clone #** | **Mutations Present** | **Source** | **Growth* on [BTH]** | | **SEQ ID NO** |
|---|---|---|---|---|---|
| | | | 0 µM | 200 µM | |
| BTH-1 | H115Y,F159S | ePCR1 | - | + | 147 |
| BTH-9 | F159L | ePCR1 | - | + | 148 |
| BTH-An7 | Q24R, K59R, I82T, F159L, D161G | ePCR1 | - | + | 164 |

| | | | | | |
|---|---|---|---|---|---|
| ePCR1=error prone PCR mutagenesis library of PYR1, 70,000 clones starting size. *Growth was measured on media lacking uracil and using the reporter strain MAV99, which only grows when an agonist-promoted protein-protein interaction between PYR1-GAL4 BD and HAB1-GAL4 AD reconstitutes GAL4 activity and enables expression of the strain's URA3 gene. | | | | | |

### Example 2: Mutations at the K59 Position Sensitize PYR to Diverse Orthogonal Ligands

Inspection of the screening data in Tables 1-5 revealed that receptors containing a mutation at the K59 position were isolated at least once for all chemicals screened. In most cases, a K59R mutation was present in the majority of orthogonal receptors isolated for each chemical screened. This surprising observation suggests that K59 is a control point that can be targeted beneficially to engineer effective orthogonal receptors. Two plausible hypotheses for the frequent occurrence of a mutation at the position corresponding to amino acid K59 of PYR1 are the "brake" hypothesis and the "pocket shape" hypothesis. The brake hypothesis proposes that the K59 residue functions as part of a "braking" mechanism to help keep receptors in their "off" state in the absence of bound ABA; therefore, mutations at K59 may disrupt a control mechanism that keeps receptor activation linked to ABA binding and prevents receptors from being activated by non-natural ligands. The pocket shape hypothesis proposes that mutations at the position corresponding to K59 alters the PYR/PYL receptor's binding pocket to create a new pocket surface that facilitates interactions between PYR/PYL and orthogonal ligands to improve binding affinity.

To test these two hypotheses, a series of K59 mutants were constructed in PYR1 and examined for their sensitivity to the native ligand ABA and the orthogonal ligand dichlobenil (Table 6). Dichlobenil was chosen as a model orthogonal ligand for these studies because it was observed that it is a weak PYR/PYL agonist (*i.e.,* it can weakly activate receptors at concentrations of 200 µM or higher). Dichlobenil therefore provides a useful test molecule with which to probe a mutant's sensitivity to an orthogonal ligand.

**Table 6. Responsiveness of K59 mutations to the orthogonal ligand dichlobenil**

| **Mutation** | **ABA [10 µM]** | **Dichlobenil [50 µM]** |
|---|---|---|
| WT | + | - |
| K59A | - | + |
| K59C | - | + |
| K59F | + | + |
| K59G | - | + |
| K59H | - | + |
| K59I | - | - |
| K59L | - | + |
| K59M | - | + |
| K59P | - | - |
| K59R | - | + |
| K59S | - | + |
| K59T | - | + |
| K59V | - | + |
| K59Y | - | + |
| K59N | + | + |
| K59W | - | + |

As shown in Table 6, wild-type PYR1 was not activated by 50 µM dichlobenil. However, 14 of the 16 K59 substitution mutants constructed were activated by 50 µM dichlobenil. Thus, the majority of mutations at K59 enhance dichlobenil sensitivity. These results, coupled with the prevalence of K59R mutations isolated in the screens described herein (*e.g.,* in Example 1 and in Tables 1-5), suggest that many mutations in K59 are beneficial for constructing receptors that are activated by non-native (*i.e.,* orthogonal) ligands, and further suggest that K59 is more likely to be acting according to the brake hypothesis than the pocket shape hypothesis, as the pocket shape hypothesis predicts that an altered pocket surface that facilitates binding of one orthogonal ligand is unlikely to also be the shape that facilitates another orthogonal ligand's binding.

### Discussion

The receptor mutations isolated in the collection of mutants are preferentially located in residues whose side chains point into the ligand-binding pocket of PYR1. This is not surprising, as the surface of the ligand-binding pocket of PYR1 must be resculpted to make contact with new ligands. Additionally, mutations isolated in orthogonal receptors (*i.e.* K59R, S92T) mutate invariant positions within the PYR/PYL ABA receptor family. These positions are invariant because they are involved in ABA recognition and under strong natural selection for proper ABA-binding. Since mutations in conserved ABA-binding residues are known to reduce ABA responsiveness, orthogonal receptors can be expected to be insensitive to ABA when expressed in plant cells. An advantage of this feature is that over-expression of orthogonal receptors should not lead to activation of ABA signaling in the absence of the controlling orthogonal ligand.

The biochemical function of PYR1, and PYR/PYL proteins in general, is to inhibit PP2C activity. This can be measured in live cells using the yeast two hybrid or other cell-based methods. It can also be measured *in vitro* using enzymatic phosphatase assays in the presence of a colorimetric detection reagent (for example, para-nitrophenylphosphate). We note that the yeast-based assay used above provides an indirect indicator of ligand binding. It is possible that some compounds screened may reduce ABA responsiveness of yeast strains without directly binding in its central pocket. To address this potential limitation, one can use *in vitro* competition assays, or cell based assays using other organisms, as alternate approaches for identifying weak binding target compounds.

### Example 3: Improvement of Fenhexamid Receptor Sensitivity

### Generating fenexamid-responsive PYR1 variants

As detailed in Example 1, the screening of the ePCR1 mutant library for fenhexamid responsive mutants led to the isolation of several mutant PYR1 receptors that respond to fenhexamid (Table 1). To improve fenhexamid receptor sensitivity, DNA shuffling was employed using the same general experimental scheme outlined previously. Briefly, equimolar amounts of plasmid DNA for the fenhexamid receptors shown in Table 1 were pooled and combined with an equimolar amount of the ePCR1 library DNA. The pooled templates were utilized for DNA shuffling, which was conducted as described in Example 1. A library (named "27B") of ∼400,000 shuffled variants was prepared. The DNA for this library was introduced into the MAV99 pAD-HAB1 yeast strain as described above and the resulting yeast cells collected and grown on FOA-containing plates to reduce constitutive mutants in the library, yielding the 27B' library. The 27B' library was plated onto media lacking uracil but containing 20 µM fenhexamid. ∼50 positives were selected from these plates and subsequently retested on media lacking uracil to distinguish constitutive mutants (*i.e.,* false positives) from those mutants that grow specifically in response to fenhexamid (*i.e.,* true positives). The PYR1 coding sequences for the true positives were sequenced to yield the following series of fenhexamid-responsive PYR1 variants (Table 7):

**Table 7. Fenhexamid-responsive PYR/PYL receptor polypeptide mutants identified from second round of shuffling**

| **Clone #** | **Mutations Present** | **SEQ ID NO** |
|---|---|---|
| 27B-1 | P42S, K59R, D97N, Y120H, V163I, A172T | 165 |
| 27B-2 | P42S, L44F, K59R, Y120H, V138M, M158I | 166 |
| 27B-3 | P42S, K59R, Y120H, V123I, V139I, M158I | 167 |
| 27B-4 | S47P, V49I, K59R, Y120H, M158I, A177T | 168 |
| 27B-7 | K59R, V81M, Y120C, M158I, V163I | 169 |
| 27B-8 | P42S, K59R, D97N, Y120H, V163I, A172T | 165 |

A third round of shuffling was conducted by combining DNA for the "27B" mutants shown in Table 7 and ePCR1 library to create a library of ∼150,000 clones which was then transformed into MAV99 pAD-HAB1. Constitutive mutants were then depleted by growth on FOA after which fenhexamid sensitive mutants were selected by growth on media lacking uracil but containing 1.5 µM fenhexamid, using the methods described above. This effort yielded the following fenhexamid responsive variants (Table 8):

**Table 8. Fenhexamid-responsive PYR/PYL receptor polypeptide mutants identified from third round of shuffling**

| **Clone #** | **Mutations Present** | **SEQ ID NO** |
|---|---|---|
| 27C-1 | P27L, P42S, K59R, D97N, Y120H, M158I, T173A | 170 |
| 27C-2 | P42S, K59R, R74C, Y120H, M158I | 171 |
| 27C-3 | S29N, K59R, D97N, Y120H, V163I, A72T | 172 |
| 27C-5 | P42S, K59R, Y120H, V123I, V139I, M158I, V163I | 173 |
| 27C-16 | K59R, V81M, Y120H, M158I, V163I | 174 |
| 27C-18 | E12K, K59R, V75I, D97N, Y120H, V163I, A172T | 175 |
| 27C-19 | L33F, P42S, K59R, Y120H, V123I, M158I | 176 |
| 27C-20 | P42S, K59R, Y120H, M158I, V163I, V174I | 177 |
| 27C-21 | R10Q, P42S, K59R, D97N, Y120H, V163I, A172T | 178 |

### Establishes the role of specific mutations in fenhexamid-responsive mutant 27C-2

Mutagenesis protocols often introduce spurious mutations that do not affect the desired functionality. To establish the relevance of the residues identified by our screens, we focused on a highly sensitive fenhexamid-responsive mutant identified, 27C-2, which contains 5 mutations (P42S, K59R, R74C, Y120H, and M158I; SEQ ID NO:171; see Table 8) in comparison to the wild-type PYR1 sequence. The presence of K59R, Y120H, and M158I in many other isolated mutants suggested they were likely to be mutations contributing to fenhexamid sensitivity. To probe the role of the 5 mutations in fenhexamid response, each of these residues were reverted back to the wild-type residue using site-directed mutagenesis. The resulting clones were then transformed into the Y190 pAD-HAB1 yeast reporter strain and tested for fenhexamid responsiveness on a range of concentrations. As shown in Figure 2A, this effort defined K59R, Y120H, and M158I as being sufficient for fenhexamid sensitivity, and established that the mutations P42S and R74C are not sufficient to contribute to fenhexamid sensitivity in the 27C-2 clone. In addition, *in vitro* receptor assays show that the PYR1^{K59R,Y120H,M158I} triple mutant is sensitive to fenhexamid (IC₅₀ value ∼ 0.4 µM) (Figure 2B), which demonstrates that the observed sensitivity of the PYR1^{K59R,Y120H,M158I} triple mutant to fenhexamid is not an artifact of the yeast assay system used to identify the triple mutant.

### Engineering fenhexamid sensitivity into PYL2

PYR1 is a member of the PYR/PYL receptor protein family, which in Arabidopsis contains 14 members. Moreover, the mutations sufficient for fenhexamid responsiveness in 27C-2 are located in invariant or conserved residues within the ligand binding pocket (K59R, Y120H) or the PYR/PYL-PP2C interface (M158I). Given the conserved nature of these residues, we speculated that fenhexamid sensitivity could be engineered into other receptor family members by mutating homologous residues in other PYR/PYL receptors. To test this, we introduced the homologous mutations in PYL2 (K64R, corresponding to K59R in PYR1; Y124H, corresponding to Y120H in PYR1; and M164I, corresponding to M158I in PYR1), creating the mutant PYL2^{K64R,Y124H,M164I}. As shown in Figure 3A, this mutant does not show fenhexamid responsiveness when tested using the yeast two-hybrid assay (strain Y190 pAD-HAB1). Recent work (Peterson et al., Nat Struct Mol Biol 17:1109-1113 (2010)) has shown that subtle sequence variations between receptor family members affects a receptor's sensitivity to the selective agonist pyrabactin. In particular, PYR1 and PYL1 show strong responsiveness to pyrabactin, while PYL2 (and other family members) do not. Genetic, biochemical, and structural studies have shown that two key residues in the ligand-binding pocket of PYR1 determine the difference in pyrabactin agonist activity between PYR1 and PYL2. In PYR1, these residues are isoleucines I62 and I110 while in PYL2, the homologous residues (amino acid positions 64 and 114, respectively) are replaced by less bulky valines V67 and V 114. Based on the known role of these two residues in affecting differences in ligand responsiveness between receptors, we hypothesized that I62 and I110 may play important roles in fenhexamid response. We therefore introduced the V67I and V1141 mutations (alone or in combination) into the PYL2^{K64R,Y24H,M164I} receptor. The addition of the V67I and V114I mutations together enabled the final mutant receptor PYL2^{K64R,Y124H,M164I,V67I,V114I} to respond to fenhexamid in yeast assays (Figure 3A) and *in vitro* PP2C inhibition assays (Figure 3B).

### Efficacy of orthogonal receptors in plants

To investigate if the fenhexamid response observed in yeast and *in vitro* experiments would function in plants, transgenic 35S::GFP-PYL2 and 35S::GFP-PYL2^{K64R,Y124H,M164I,V67I,V114I} plants were constructed using standard molecular cloning methods into a modified version of pEGAD (Cutler et al., Proc Natl Acad Sci USA 97:3718-3723 (2000)) in which a 6X-histidine tag was added onto the N-terminus of GFP. Transgenic plants were made using the floral-dip method and primary transgenics (T1) identified by screening seedlings by epi-fluorescence using a Leica GFP dissecting microscope. The GFP⁺ transgenic plants were grown to maturity and T2 seed collected for further analyses.

To establish if the fenhexamid responsive PYL2^{K64R,Y124H,M164I,V67I,V114I} mutant functions *in planta,* T2 segregants and appropriate wild-type control were tested for their ability to grow on 100 µM fenhexamid, as activation of the receptors should inhibit germination. Plants expressing mutant receptors show a strong growth inhibition, suggesting that the ABA pathway is being activated by fenhexamid in plants expressing the mutant, but not the wild-type, receptor (Figure 4). To further investigate ABA-response activation, we next tested GFP⁺ transgenics (T2 segregants) and appropriate controls for activation of 3 ABA reporter genes (P5CS1, RD29A, and NCED3) in response to exposure to fenhexamid in liquid culture using quantitative RT-PCR methods. As shown in Figure 5, all three genes show substantial induction by fenhexamid in the two independent PYL2^{K64R,Y124H,M164I,V67I,V114I} transgenic lines. Thus, genes that are normally ABA regulated *in vivo* can be activated by fenhexamid using the PYL2^{K64R,Y124H,M164I,V67I,V114I} mutant receptor.

To further establish if the fenhexamid responsive PYL2^{K64R,Y124H,M164I,V67I,V114I} mutant receptor functions *in planta,* we examined the ability of fenhexamid to reduce water loss in detached leaves of wild-type plants or plants overexpressing PYL2^{K64R,Y124H,M164I,V67I,V114I}. Transgenic or control (wild-type) plants were grown under a 16 hr light / 8 hr dark cycle for 3 weeks and then treated with either 100 µM (+)-ABA, 100 µM fenhexamid, or control (containing 0.1 % Tween-20, 0.1 % DMSO). ABA treatments were conducted as a positive control. The plants were sprayed in the evening prior to conducting water loss experiments. The next morning, approximately 16 hours posttreatment, the above-ground rosettes of the experimental samples were collected and transferred to weighing dishes, 8 plants per measured sample, and maintained under ∼90-100 µEinstein/m² fluorescent light illumination. Four groups of eight plants were measured per time point at 20 min intervals. The experiments were repeated three times over the course of an eight-week interval. In all experiments, fenhexamid pretreatment was sufficient to reduce water loss from PYL2^{K64R,Y124H,M164I,V67I,V114I} transgenic plants (Figure 6A-C), albeit with less efficacy than ABA. It should be noted, however, that ABA can activate all of the PYR/PYL receptors in a plant genome (at least 13 in Arabidopsis), while fenhexamid is selectively activating a single engineered receptor, PYL2^{K64R,Y124H,M164I,V67I,V114I}. As a control for the effects of fenhexamid, we subjected wild-type plants to treatments with either 100 µM (+)-ABA, 100 µM fenhexamid, or control (containing 0.1% Tween-20, 0.1% DMSO) following the same protocol described above for transgenic plants. These experiments showed that fenhexamid does not affect water loss in wild-type plants (Figure 7). Thus, the expression of the fenhexamid responsive PYL2^{K64R,Y124H,M164I,V67I,V114I} mutant in transgenic plants enables fenhexamid to activate ABA signaling and physiological response.

## Claims

1. A plant comprising a heterologous expression cassette, the expression cassette comprising a promoter operably linked to a polynucleotide encoding a mutated PYR/PYL receptor polypeptide, wherein the mutated PYR/PYL receptor polypeptide comprises a mutation at an amino acid residue corresponding to position K59 of PYR1 (SEQ ID NO:1), wherein the amino acid residue is mutated to alanine, cysteine, phenylalanine, glycine, histidine, leucine, methionine, arginine, serine, threonine, tyrosine, valine, asparagine, or tryptophan, wherein the mutated PYR/PYL receptor polypeptide is agonized by a chemical when the chemical is contacted to the mutated PYR/PYL receptor polypeptide and wherein the chemical does not significantly agonize a wild-type PYR/PYL receptor polypeptide when the chemical is contacted to the wild-type PYR/PYL receptor polypeptide.

2. The plant of claim 1, wherein:
(i) the chemical comprises a pesticide, a fungicide, an herbicide, a nematicide, a plant activator, a synergist, an herbicide safener, a plant growth regulator, an insect repellant, or a fertilizer; or
(ii) the chemical is selected from the group consisting of fenhexamid, bromoxynil, chloroxynil, ioxynil, coumatetralyl, dichlobenil, benoxacor, and BTH (acibenzolar-s-methyl).

3. The plant of claim 1, wherein the amino acid residue corresponding to position K59 of SEQ ID NO:1 is mutated to arginine.

4. The plant of claim 1, wherein:
(i) the chemical is fenhexamid, bromoxynil, chloroxynil, ioxynil, dichlobenil or benoxacor; or
(ii) the mutated PYR/PYL receptor polypeptide further comprises at least one additional mutation at an amino acid corresponding to positions 21, 41, 50, 57, 60, 82, 92, 102, 116, 125, 141, and/or 151 in PYR1 (SEQ ID NO:1) wherein the mutation is selected from H21Y, P41L, R50G, T57A, H60R, I82N, S92T, E102G, R116K, T125A, E141Q, E141D, N151D, or combinations thereof, and wherein the mutated PYR/PYL receptor polypeptide is agonized by bromoxynil, chloroxynil, or ioxynil when the bromoxynil, chloroxynil, or ioxynil is contacted to the mutated PYR/PYL receptor polypeptide; or
(iii) the mutated PYR/PYL receptor polypeptide further comprises at least one additional mutation at an amino acid corresponding to positions 10, 12, 25, 27, 29, 33, 42, 43, 44, 47, 49, 74, 75, 81, 97, 110, 120, 123, 124, 133, 138, 139, 144, 154, 158, 163, 172, 173, 174, and/or 177 in PYR1 (SEQ ID NO:1) wherein the mutation is selected from R10Q, E12G, E12K, L25R, P27L, S29N, L33F, P42S, E43G, L44F, S47P, V49I, R74C, V75I, V81M, D97N, I110S, Y120C, Y120H, V123I, T124M, N133D, V138M, V139I, V144A, E154G, M158I, V163I, A172T, T173A, V174I, and/or A177T or combinations thereof, and wherein the mutated PYR/PYL receptor polypeptide is agonized by fenhexamid when the fenhexamid is contacted to the mutated PYR/PYL receptor polypeptide.

5. The plant of claim 1, wherein the mutated PYR/PYL receptor polypeptide comprises mutations at amino acids corresponding to positions 59, 120, and 158 in PYR1 (SEQ ID NO:1) wherein the mutations are K59R, Y120H, and M158I, and wherein the mutated PYR/PYL receptor polypeptide is agonized by fenhexamid when the fenhexamid is contacted to the mutated PYR/PYL receptor polypeptide;
preferably wherein the mutated PYR/PYL receptor polypeptide further comprises isoleucine residues at the amino acid positions corresponding to positions 62 and 110 in PYR1 (SEQ ID NO:1).

6. The plant of claim 1, wherein:
(i) the mutated PYR/PYL receptor polypeptide further comprises at least one additional mutation at an amino acid corresponding to positions 27 and/or 63 in PYR1 (SEQ ID NO:1) wherein the mutation is selected from P27L, K63N, or combinations thereof, and wherein the mutated PYR/PYL receptor polypeptide is agonized by dichlobenil when the dichlobenil is contacted to the mutated PYR/PYL receptor polypeptide; or
(ii) the mutated PYR/PYL receptor polypeptide further comprises a mutation at an amino acid corresponding to position 119 in PYR1 (SEQ ID NO:1) wherein the mutation is N119Y, and wherein the mutated PYR/PYL receptor polypeptide is agonized by benoxacor when the benoxacor is contacted to the mutated PYR/PYL receptor polypeptide; or
(iii) the mutated PYR/PYL receptor polypeptide further comprises at least one mutation at an amino acid corresponding to positions 24, 82, 159, and/or 161 in PYR1 (SEQ ID NO:1) wherein the mutation is selected from Q24R, I82T, F159L, D161G, or combinations thereof, and wherein the mutated PYR/PYL receptor polypeptide is agonized by BTH (acibenzolar-s-methyl) when the BTH is contacted to the mutated PYR/PYL receptor polypeptide.

7. The plant of claim 1, wherein the mutated PYR/PYL receptor polypeptide has at least 70% sequence identity to:
(i) any of SEQ ID NOs:131-139 and wherein the mutated PYR/PYL receptor polypeptide is agonized by bromoxynil, chloroxynil, or ioxynil when the bromoxynil, chloroxynil, or ioxynil is contacted to the mutated PYR/PYL receptor polypeptide; or
(ii) any of SEQ ID NOs:124-130 or 165-178 and wherein the mutated PYR/PYL receptor polypeptide is agonized by fenhexamid when the fenhexamid is contacted to the mutated PYR/PYL receptor polypeptide; or
(iii) any of SEQ ID NOs:143-144 and wherein the mutated PYR/PYL receptor polypeptide is agonized by dichlobenil when the dichlobenil is contacted to the mutated PYR/PYL receptor polypeptide; or
(iv) SEQ ID NO: 146 and wherein the mutated PYR/PYL receptor polypeptide is agonized by benoxacor when the benoxacor is contacted to the mutated PYR/PYL receptor polypeptide; or
(v) SEQ ID NO: 164, and wherein the mutated PYR/PYL receptor polypeptide is agonized by BTH (acibenzolar-s-methyl) when the BTH is contacted to the mutated PYR/PYL receptor polypeptide.

8. The plant of any of claims 1 to 7, wherein the plant has improved abiotic stress tolerance when contacted with the chemical as compared to a plant lacking the expression cassette.

9. A plant cell, seed, flower, leaf, or fruit from the plant of any of claims 1 to 7, wherein the plant cell, seed, flower, leaf, or fruit comprises the heterologous expression cassette.

10. A method of improving abiotic stress tolerance in the plant of any of claims 1 to 7 by contacting the plant with a chemical selected from the group consisting of fenhexamid, bromoxynil, chloroxynil, ioxynil, coumatetralyl, dichlobenil, benoxacor, and BTH (acibenzolar-s-methyl).

11. A method of making a mutated PYR/PYL receptor polypeptide that is agonized by a chemical when the chemical is contacted to the mutated PYR/PYL receptor polypeptide, wherein the chemical does not significantly agonize a wild-type PYR/PYL receptor polypeptide when the chemical is contacted to the wild-type PYR/PYL receptor polypeptide, the method comprising
(a) mutagenizing the wild-type PYR/PYL receptor polypeptide;
(b) contacting one or more mutated PYR/PYL receptor polypeptides with the chemical; and
(c) determining whether the chemical activates the one or more mutated PYR/PYL receptor polypeptides, wherein activation identifies the one or more mutated PYR/PYL receptor polypeptides as being agonized by the chemical;
preferably wherein the method further comprises, prior to step (b), screening the chemical to determine whether the chemical binds to the wild-type PYR/PYL receptor polypeptide prior to contacting the one or more mutated PYR/PYL receptor polypeptides with the chemical.

12. An expression cassette comprising a promoter operably linked to a polynucleotide encoding a mutated PYR/PYL receptor polypeptide, wherein the mutated PYR/PYL receptor polypeptide comprises a mutation at an amino acid residue corresponding to position K59 of PYR1 (SEQ ID NO:1), wherein the amino acid residue is mutated to alanine, cysteine, phenylalanine, glycine, histidine, leucine, methionine, arginine, serine, threonine, tyrosine, valine, asparagine, or tryptophan, wherein the mutated PYR/PYL receptor polypeptide is agonized by a chemical when the chemical is contacted to the mutated PYR/PYL receptor polypeptide and wherein the chemical does not significantly agonize a wild-type PYR/PYL receptor polypeptide when the chemical is contacted to the wild-type PYR/PYL receptor polypeptide;
or an expression vector comprising said expression cassette.

13. A method of producing a plant having increased stress tolerance, the method comprising growing a transgenic plant comprising at least one polynucleotide sequence encoding a mutated PYR/PYL receptor polypeptide, wherein the mutated PYR/PYL receptor polypeptide:
(i) has at least 70% sequence identity to any of SEQ ID NOs:124-139, 143, 144, 146, or 164-178 and comprises a mutation at an amino acid residue corresponding to position K59 of PYR1 (SEQ ID NO:1), wherein the amino acid residue is mutated to alanine, cysteine, phenylalanine, glycine, histidine, leucine, methionine, arginine, serine, threonine, tyrosine, valine, asparagine, or tryptophan, and/or
(ii) has at least 70% sequence identity to any of SEQ ID NO:147, 148, 164 and comprises a mutation at an amino acid residue corresponding to position F159 of PYR1 (SEQ ID NO 1), wherein the amino acid residue is mutated to leucine or serine;
wherein the mutated PYR/PYL receptor polypeptide is agonized by a chemical when the chemical is contacted to the mutated PYR/PYL receptor polypeptide, wherein the chemical does not significantly agonize a wild-type PYR/PYL receptor polypeptide when the chemical is contacted to the wild-type PYR/PYL receptor polypeptide, whereby the transgenic plant expresses the mutated PYR/PYL receptor polypeptide.

14. A polypeptide comprising a mutated PYR/PYL receptor polypeptide, wherein the mutated PYR/PYL receptor polypeptide:
(i) has at least 70% identity to any of SEQ ID NOs:124-139, 143, 144, 146, or 164-178 and comprises a mutation at an amino acid residue corresponding to position K59 of PYR1 (SEQ ID NO:1), wherein the amino acid residue is mutated to alanine, cysteine, phenylalanine, glycine, histidine, leucine, methionine, arginine, serine, threonine, tyrosine, valine, asparagine, or tryptophan, and/or
(ii) has at least 70% sequence identity to any of SEQ ID NO:147, 148, 164 and comprises a mutation at an amino acid residue corresponding to position F159 of PYR1 (SEQ ID NO 1), wherein the amino acid residue is mutated to leucine or serine;
wherein the mutated PYR/PYL receptor polypeptide is agonized by a chemical when the chemical is contacted to the mutated PYR/PYL receptor polypeptide, wherein the chemical does not significantly agonize a wild-type PYR/PYL receptor polypeptide when the chemical is contacted to the wild-type PYR/PYL receptor polypeptide;
or a polynucleotide encoding said polypeptide.

## Patentansprüche

1. Pflanze, umfassend eine heterologe Expressionskassette, wobei die Expressionskassette einen Promoter umfasst, der operabel mit einem Polynukleotid verbunden ist, das ein mutiertes PYR/PYL-Rezeptorpolypeptid kodiert, wobei das mutierte PYR/PYL-Rezeptorpolypeptid eine Mutation an einem Aminosäurerest umfasst, der der Position K59 von PYR1 (SEQ ID NO:1) entspricht, wobei der Aminosäurerest zu Alanin, Cystein, Phenylalanin, Glycin, Histidin, Leucin, Methionin, Arginin, Serin, Threonin, Tyrosin, Valin, Asparagin oder Tryptophan mutiert ist, wobei das mutierte PYR/PYL-Rezeptorpolypeptid durch eine Chemikalie agonisiert wird, wenn die Chemikalie mit dem mutierten PYR/PYL-Rezeptorpolypeptid in Kontakt gebracht wird und wobei die Chemikalie ein Wildtyp-PYR/PYL-Rezeptorpolypeptid nicht signifikant agonisiert, wenn die Chemikalie mit dem Wildtyp-PYR/PYL-Rezeptorpolypeptid in Kontakt gebracht wird.

2. Pflanze nach Anspruch 1, wobei:
(i) die Chemikalie ein Pestizid, Fungizid, Herbizid, Nematizid, einen Pflanzenaktivator, einen Synergisten, einen Herbizid-Safener, einen Pflanzenwachstumsregulator, ein Insektenschutzmittel oder ein Düngemittel umfasst; oder
(ii) die Chemikalie aus der Gruppe bestehend aus Fenhexamid, Bromoxynil, Chloroxynil, Ioxynil, Coumatetralyl, Dichlobenil, Benoxacor und BTH (Acibenzolar-s-methyl) ausgewählt ist.

3. Pflanze nach Anspruch 1, wobei der Aminosäurerest, der der Position K59 von SEQ ID NO: 1 entspricht, zu Arginin mutiert ist.

4. Pflanze nach Anspruch 1, wobei:
(i) die Chemikalie Fenhexamid, Bromoxynil, Chloroxynil, Ioxynil, Dichlobenil oder Benoxacor ist; oder
(ii) das mutierte PYR/PYL-Rezeptorpolypeptid ferner mindestens eine zusätzliche Mutation an einer Aminosäure umfasst, die den Positionen 21, 41, 50, 57, 60, 82, 92, 102, 116, 125, 141 und/oder 151 in PYR1 (SEQ ID NO: 1) entspricht, wobei die Mutation aus H21Y, P41L, R50G, T57A, H60R, I82N, S92T, E102G, R116K, T125A, E141Q, E141D, N151D oder Kombinationen davon ausgewählt ist und wobei das mutierte PYR/PYL-Rezeptorpolypeptid durch Bromoxynil, Chloroxynil oder Ioxynil agonisiert wird, wenn das Bromoxynil, Chloroxynil oder Ioxynil mit dem mutierten PYR/PYL-Rezeptorpolypeptid in Kontakt gebracht wird; oder
(iii) das mutierte PYR/PYL-Rezeptorpolypeptid ferner mindestens eine zusätzliche Mutation an einer Aminosäure umfasst, die den Positionen 10, 12, 25, 27, 29, 33, 42, 43, 44, 47, 49, 74, 75, 81, 97, 110, 120, 123, 124, 133, 138, 139, 144, 154, 158, 163, 172, 173, 174 und/oder 177 in PYR1 (SEQ ID NO: 1) entspricht, wobei die Mutation aus R10Q, E12G, E12K, L25R, P27L, S29N, L33F, P42S, E43G, L44F, S47P, V49I, R74C, V75I, V81M, D97N, I110S, Y120C, Y120H, V123I, T124M, N133D, V138M, V139I, V144A, E154G, M158I, V163I, A172T, T173A, V174I und/oder A177T oder Kombinationen davon ausgewählt ist und wobei das mutierte PYR/PYL-Rezeptorpolypeptid durch Fenhexamid agonisiert wird, wenn das Fenhexamid mit dem mutierten PYR/PYL-Rezeptorpolypeptid in Kontakt gebracht wird.

5. Pflanze nach Anspruch 1, wobei das mutierte PYR/PYL-Rezeptorpolypeptid Mutationen an Aminosäuren umfasst, die den Positionen 59, 120 und 158 in PYR1 (SEQ ID NO: 1) entsprechen, wobei die Mutationen K59R, Y120H und M158I sind und wobei das mutierte PYR/PYL-Rezeptorpolypeptid durch Fenhexamid agonisiert wird, wenn das Fenhexamid mit dem mutierten PYR/PYL-Rezeptorpolypeptid in Kontakt gebracht wird;
wobei das mutierte PYR/PYL-Rezeptorpolypeptid bevorzugt ferner Isoleucinreste an den Aminosäurepositionen umfasst, die den Positionen 62 und 110 in PYR1 (SEQ ID NO: 1) entsprechen.

6. Pflanze nach Anspruch 1, wobei:
(i) das mutierte PYR/PYL-Rezeptorpolypeptid ferner mindestens eine zusätzliche Mutation an einer Aminosäure umfasst, die den Positionen 27 und/oder 63 in PYR1 (SEQ ID NO: 1) entspricht, wobei die Mutation aus P27L, K63N oder Kombinationen davon ausgewählt ist und wobei das mutierte PYR/PYL-Rezeptorpolypeptid durch Dichlobenil agonisiert wird, wenn das Dichlobenil mit dem mutierten PYR/PYL-Rezeptorpolypeptid in Kontakt gebracht wird; oder
(ii) das mutierte PYR/PYL-Rezeptorpolypeptid ferner eine Mutation an einer Aminosäure umfasst, die der Position 119 in PYR1 (SEQ ID NO: 1) entspricht, wobei die Mutation N119Y ist und wobei das mutierte PYR/PYL-Rezeptorpolypeptid durch Benoxacor agonisiert wird, wenn das Benoxacor mit dem mutierten PYR/PYL-Rezeptorpolypeptid in Kontakt gebracht wird; oder
(iii) das mutierte PYR/PYL-Rezeptorpolypeptid ferner mindestens eine Mutation an einer Aminosäure umfasst, die den Positionen 24, 82, 159 und/oder 161 in PYR1 (SEQ ID NO: 1) entspricht, wobei die Mutation aus Q24R, I82T, F159L, D161G oder Kombinationen davon ausgewählt ist und wobei das mutierte PYR/PYL-Rezeptorpolypeptid durch BTH (Acibenzolar-s-methyl) agonisiert wird, wenn das BTH mit dem mutierten PYR/PYL-Rezeptorpolypeptid in Kontakt gebracht wird.

7. Pflanze nach Anspruch 1, wobei das mutierte PYR/PYL-Rezeptorpolypeptid mindestens 70 % Sequenzidentität hat mit:
(i) einer beliebigen der SEQ ID NOs: 131-139 und wobei das mutierte PYR/PYL-Rezeptorpolypeptid durch Bromoxynil, Chloroxynil oder Ioxynil agonisiert wird, wenn das Bromoxynil, Chloroxynil oder Ioxynil mit dem mutierten PYR/PYL-Rezeptorpolypeptid in Kontakt gebracht wird; oder
(ii) einer beliebigen der SEQ ID NOs: 124-130 oder 165-178 und wobei das mutierte PYR/PYL-Rezeptorpolypeptid durch Fenhexamid agonisiert wird, wenn das Fenhexamid mit dem mutierten PYR/PYL-Rezeptorpolypeptid in Kontakt gebracht wird; oder
(iii) einer beliebigen der SEQ ID NOs: 143-144 und wobei das mutierte PYR/PYL-Rezeptorpolypeptid durch Dichlobenil agonisiert wird, wenn das Dichlobenil mit dem mutierten PYR/PYL-Rezeptorpolypeptid in Kontakt gebracht wird; oder
(iv) SEQ ID NO: 146 und wobei das mutierte PYR/PYL-Rezeptorpolypeptid durch Benoxacor agonisiert wird, wenn das Benoxacor mit dem mutierten PYR/PYL-Rezeptorpolypeptid in Kontakt gebracht wird; oder
(v) SEQ ID NO: 164 und wobei das mutierte PYR/PYL-Rezeptorpolypeptid durch BTH (Acibenzolar-s-methyl) agonisiert wird, wenn das BTH mit dem mutierten PYR/PYL-Rezeptorpolypeptid in Kontakt gebracht wird.

8. Pflanze nach einem der Ansprüche 1 bis 7, wobei die Pflanze, wenn sie mit der Chemikalie in Kontakt gebracht wird, verbesserte abiotische Stresstoleranz hat, verglichen mit einer Pflanze, der die Expressionskassette fehlt.

9. Pflanzenzelle, -Samen, -Blüte, -Blatt oder -Frucht von der Pflanze nach einem der Ansprüche 1 bis 7, wobei die Pflanzenzelle, der Pflanzensamen, die Pflanzenblüte, das Pflanzenblatt oder die Pflanzenfrucht die heterologe Expressionskassette umfasst.

10. Verfahren für ein Verbessern von abiotischer Stresstoleranz in der Pflanze nach einem der Ansprüche 1 bis 7 durch In-Kontakt-Bringen der Pflanze mit einer Chemikalie, die aus der Gruppe bestehend aus Fenhexamid, Bromoxynil, Chloroxynil, Ioxynil, Coumatetralyl, Dichlobenil, Benoxacor und BTH (Acibenzolar-s-methyl) ausgewählt ist.

11. Verfahren für ein Herstellen eines mutierten PYR/PYL-Rezeptorpolypeptids, das durch eine Chemikalie agonisiert wird, wenn die Chemikalie mit dem mutierten PYR/PYL-Rezeptorpolypeptid in Kontakt gebracht wird, wobei die Chemikalie ein Wildtyp-PYR/PYL-Rezeptorpolypeptid nicht signifikant agonisiert, wenn die Chemikalie mit dem Wildtyp-PYR/PYL-Rezeptorpolypeptid in Kontakt gebracht wird, das Verfahren umfassend
(a) ein Mutagenisieren des Wildtyp-PYR/PYL-Rezeptorpolypeptids;
(b) ein In-Kontakt-Bringen von einem oder mehreren mutierten PYR/PYL-Rezeptorpolypeptiden mit der Chemikalie; und
(c) ein Bestimmen, ob die Chemikalie das eine oder die mehreren mutierten PYR/PYL-Rezeptorpolypeptide aktiviert, wobei Aktivierung das eine oder die mehreren mutierten PYR/PYL-Rezeptorpolypeptide als durch die Chemikalie agonisiert identifiziert;
wobei das Verfahren bevorzugt ferner vor Schritt (b) ein Screenen der Chemikalie umfasst, um zu bestimmen, ob die Chemikalie vor dem In-Kontakt-Bringen des einen oder der mehreren mutierten PYR/PYL-Rezeptorpolypeptide mit der Chemikalie an das Wildtyp-PYR/PYL-Rezeptorpolypeptid bindet.

12. Expressionskassette, umfassend einen Promoter, der operabel mit einem Polynukleotid verbunden ist, das ein mutiertes PYR/PYL-Rezeptorpolypeptid kodiert, wobei das mutierte PYR/PYL-Rezeptorpolypeptid eine Mutation an einem Aminosäurerest umfasst, der der Position K59 von PYR1 (SEQ ID NO: 1) entspricht, wobei der Aminosäurerest zu Alanin, Cystein, Phenylalanin, Glycin, Histidin, Leucin, Methionin, Arginin, Serin, Threonin, Tyrosin, Valin, Asparagin oder Tryptophan mutiert ist, wobei das mutierte PYR/PYL-Rezeptorpolypeptid durch eine Chemikalie agonisiert wird, wenn die Chemikalie mit dem mutierten PYR/PYL-Rezeptorpolypeptid in Kontakt gebracht wird und wobei die Chemikalie ein Wildtyp-PYR/PYL-Rezeptorpolypeptid nicht signifikant agonisiert, wenn die Chemikalie mit dem Wildtyp-PYR/PYL-Rezeptorpolypeptid in Kontakt gebracht wird;
oder ein Expressionsvektor umfassend die Expressionskassette.

13. Verfahren für ein Herstellen einer Pflanze mit erhöhter Stresstoleranz, das Verfahren umfassend ein Züchten einer transgenen Pflanze, die mindestens eine Polynukleotidsequenz umfasst, die ein mutiertes PYR/PYL-Rezeptorpolypeptid kodiert, wobei das mutierte PYR/PYL-Rezeptorpolypeptid:
(i) mindestens 70 % Sequenzidentität mit einer beliebigen der SEQ ID NOs: 124-139, 143, 144, 146 oder 164-178 hat und eine Mutation an einem Aminosäurerest umfasst, der der Position K59 von PYR1 (SEQ ID NO: 1) entspricht, wobei der Aminosäurerest zu Alanin, Cystein, Phenylalanin, Glycin, Histidin, Leucin, Methionin, Arginin, Serin, Threonin, Tyrosin, Valin, Asparagin oder Tryptophan mutiert ist, und/oder
(ii) mindestens 70 % Sequenzidentität mit einer beliebigen der SEQ ID NOs: 147, 148, 164 hat und eine Mutation an einem Aminosäurerest umfasst, der der Position F159 von PYR1 (SEQ ID NO: 1) entspricht,
wobei der Aminosäurerest zu Leucin oder Serin mutiert ist;
wobei das mutierte PYR/PYL-Rezeptorpolypeptid durch eine Chemikalie agonisiert wird, wenn die Chemikalie mit dem mutierten PYR/PYL-Rezeptorpolypeptid in Kontakt gebracht wird, wobei die Chemikalie ein Wildtyp-PYR/PYL-Rezeptorpolypeptid nicht signifikant agonisiert, wenn die Chemikalie mit dem Wildtyp-PYR/PYL-Rezeptorpolypeptid in Kontakt gebracht wird, wodurch die transgene Pflanze das mutierte PYR/PYL-Rezeptorpolypeptid exprimiert.

14. Polypeptid, umfassend ein mutiertes PYR/PYL-Rezeptorpolypeptid, wobei das mutierte PYR/PYL-Rezeptorpolypeptid:
(i) mindestens 70 % Identität mit einer beliebigen der SEQ ID NOs: 124-139, 143, 144, 146 oder 164-178 hat und eine Mutation an einem Aminosäurerest umfasst, der der Position K59 von PYR1 (SEQ ID NO:1) entspricht, wobei der Aminosäurerest zu Alanin, Cystein, Phenylalanin, Glycin, Histidin, Leucin, Methionin, Arginin, Serin, Threonin, Tyrosin, Valin, Asparagin oder Tryptophan mutiert ist, und/oder
(ii) mindestens 70 % Sequenzidentität mit einer beliebigen der SEQ ID NOs: 147, 148, 164 hat und eine Mutation mit einem Aminosäurerest umfasst, der der Position F159 von PYR1 (SEQ ID NO: 1) entspricht, wobei der Aminosäurerest zu Leucin oder Serin mutiert ist;
wobei das mutierte PYR/PYL-Rezeptorpolypeptid durch eine Chemikalie agonisiert wird, wenn die Chemikalie mit dem mutierten PYR/PYL-Rezeptorpolypeptid in Kontakt gebracht wird, wobei die Chemikalie ein Wildtyp-PYR/PYL-Rezeptorpolypeptid nicht signifikant agonisiert, wenn die Chemikalie mit dem Wildtyp-PYR/PYL-Rezeptorpolypeptid in Kontakt gebracht wird;
oder ein Polynukleotid, das das Polypeptid kodiert.

## Revendications

1. Plante, comprenant une cassette d'expression hétérologue, la cassette d'expression comprenant un promoteur fonctionnellement lié à un polynucléotide encodant un polypeptide récepteur PYR/PYL muté, dans laquelle le polypeptide récepteur PYR/PYL muté comprend une mutation à un résidu d'acide aminé correspondant à la position K59 de PYR1 (SEQ ID n° : 1), dans laquelle le résidu d'acide aminé est muté en alanine, cystéine, phénylalanine, glycine, histidine, leucine, méthionine, arginine, serine, thréonine, tyrosine, valine, asparagine, ou tryptophane, dans laquelle le polypeptide récepteur PYR/PYL muté est rendu agoniste par un produit chimique lorsque le produit chimique est mis en contact avec le polypeptide récepteur PYR/PYL muté et dans laquelle le produit chimique ne rend sensiblement pas agoniste un polypeptide récepteur PYR/PYL de type sauvage lorsque le produit chimique est mis en contact avec le polypeptide récepteur PYR/PYL de type sauvage.

2. Plante selon la revendication 1, dans laquelle :
(i) le produit chimique comprend un pesticide, un fongicide, un herbicide, un nématicide, un activateur de plante, un synergiste, un phytoprotecteur herbicide, un régulateur de croissance de plante, un insectifuge, ou un engrais ; ou
(ii) le produit chimique est sélectionné parmi le groupe constitué de : fenhexamide, bromoxynil, chloroxynil, ioxynil, coumatétralyl, dichlobénil, bénoxacor, et BTH (acibenzolar-s-méthyl).

3. Plante selon la revendication 1, dans laquelle le résidu d'acide aminé correspondant à la position K59 de SEQ ID n° : 1 est muté en arginine.

4. Plante selon la revendication 1, dans laquelle :
(i) le produit chimique est : fenhexamide, bromoxynil, chloroxynil, ioxynil, dichlobénil ou bénoxacor ; ou
(ii) le polypeptide récepteur PYR/PYL muté comprend en outre au moins une mutation supplémentaire, à un acide aminé correspondant aux positions 21, 41, 50, 57, 60, 82, 92, 102, 116, 125, 141, et/ou 151 dans PYR1 (SEQ ID n° : 1) dans laquelle la mutation est sélectionnée parmi H21Y, P41L, R50G, T57A, H60R, I82N, S92T, E102G, R116K, T125A, E141Q, E141D, N151D, ou des associations de celles-ci, et dans laquelle le polypeptide récepteur PYR/PYL muté est rendu agoniste par bromoxynil, chloroxynil, ou ioxynil lorsque le bromoxynil, le chloroxynil, ou l'ioxynil est mis en contact avec le polypeptide récepteur PYR/PYL muté ; ou
(iii) le polypeptide récepteur PYR/PYL muté comprend en outre au moins une mutation supplémentaire, à un acide aminé correspondant aux positions 10, 12, 25, 27, 29, 33, 42, 43, 44, 47, 49, 74, 75, 81, 97, 110, 120, 123, 124, 133, 138, 139, 144, 154, 158, 163, 172, 173, 174, et/ou 177 dans PYR1 (SEQ ID n° : 1), dans laquelle la mutation est sélectionnée parmi R10Q, E12G, E12K, L25R, P27L, S29N, L33F, P42S, E43G, L44F, S47P, V49I, R74C, V75I, V81M, D97N, I110S, Y120C, Y120H, V123I, T124M, N133D, V138M, V139I, V144A, E154G, M158I, V163I, A172T, T173A, V174I, et/ou A177T ou des associations de celles-ci, et dans laquelle le polypeptide récepteur PYR/PYL muté est rendu agoniste par fenhexamide lorsque le fenhexamide est mis en contact avec le polypeptide récepteur PYR/PYL muté.

5. Plante selon la revendication 1, dans laquelle le polypeptide récepteur PYR/PYL muté comprend des mutations à des acides aminés correspondant aux positions 59, 120, et 158 dans PYR1 (SEQ ID n° : 1) dans laquelle les mutations sont K59R, Y120H, et M158I, et dans laquelle le polypeptide récepteur PYR/PYL muté est rendu agoniste par fenhexamide lorsque le fenhexamide est mis en contact avec le polypeptide récepteur PYR/PYL muté ;
de préférence dans laquelle le polypeptide récepteur PYR/PYL muté comprend en outre des résidus d'isoleucine aux positions d'acides aminés correspondant aux positions 62 et 110 dans PYR1 (SEQ ID n° : 1).

6. Plante selon la revendication 1, dans laquelle :
(i) le polypeptide récepteur PYR/PYL muté comprend en outre au moins une mutation supplémentaire, à un acide aminé correspondant aux positions 27 et/ou 63 dans PYR1 (SEQ ID n° : 1), dans laquelle la mutation est sélectionnée parmi P27L, K63N, ou des associations de celles-ci, et dans laquelle le polypeptide récepteur PYR/PYL muté est rendu agoniste par dichlobénil lorsque le dichlobénil est mis en contact avec le polypeptide récepteur PYR/PYL muté ; ou
(ii) le polypeptide récepteur PYR/PYL muté comprend en outre une mutation à un acide aminé correspondant à la position 119 dans PYR1 (SEQ ID n° : 1) dans laquelle la mutation est N119Y, et dans laquelle le polypeptide récepteur PYR/PYL muté est rendu agoniste par bénoxacor lorsque le bénoxacor est mis en contact avec le polypeptide récepteur PYR/PYL muté ; ou
(iii) le polypeptide récepteur PYR/PYL muté comprend en outre au moins une mutation à un acide aminé correspondant aux positions 24, 82, 159, et/ou 161 dans PYR1 (SEQ ID n° : 1), dans laquelle la mutation est sélectionnée parmi Q24R, I82T, F159L, D161G, ou des associations de celles-ci, et dans laquelle le polypeptide récepteur PYR/PYL muté est rendu agoniste par BTH (acibenzolar-s-méthyl) lorsque le BTH est mis en contact avec le polypeptide récepteur PYR/PYL muté.

7. Plante selon la revendication 1, dans laquelle le polypeptide récepteur PYR/PYL muté possède au moins 70 % d'identité de séquence avec :
(i) l'un quelconque de SEQ ID n° : 131-139 et dans laquelle le polypeptide récepteur PYR/PYL muté est rendu agoniste par bromoxynil, chloroxynil, ou ioxynil lorsque le bromoxynil, le chloroxynil, ou l'ioxynil est mis en contact avec le polypeptide récepteur PYR/PYL muté ; ou
(ii) l'un quelconque de SEQ ID n° : 124-130 ou 165-178 et dans laquelle le polypeptide récepteur PYR/PYL muté est rendu agoniste par fenhexamide lorsque le fenhexamide est mis en contact avec le polypeptide récepteur PYR/PYL muté ; ou
(iii) l'un quelconque de SEQ ID n° : 143-144 et dans laquelle le polypeptide récepteur PYR/PYL muté est rendu agoniste par dichlobénil lorsque le dichlobénil est mis en contact avec le polypeptide récepteur PYR/PYL muté ; ou
(iv) SEQ ID n° : 146 et dans laquelle le polypeptide récepteur PYR/PYL muté est rendu agoniste par bénoxacor lorsque le bénoxacor est mis en contact avec le polypeptide récepteur PYR/PYL muté ; ou
(v) SEQ ID n° : 164, et dans laquelle le polypeptide récepteur PYR/PYL muté est rendu agoniste par BTH (acibenzolar-s-méthyl) lorsque le BTH est mis en contact avec le polypeptide récepteur PYR/PYL muté.

8. Plante selon l'une quelconque des revendications 1 à 7, dans laquelle la plante présente une tolérance améliorée aux contraintes abiotiques, lorsqu'elle est mise en contact avec le produit chimique, par rapport à une plante sans la cassette d'expression.

9. Cellule, graine, fleur, feuille, ou fruit de plante de la plante selon l'une quelconque des revendications 1 à 7, dans lequel la cellule, la graine, la fleur, la feuille, ou le fruit de plante comprend la cassette d'expression hétérologue.

10. Procédé d'amélioration de tolérance aux contraintes abiotiques dans la plante selon l'une quelconque des revendications 1 à 7 en mettant la plante en contact avec un produit chimique sélectionné parmi le groupe constitué de : fenhexamide, bromoxynil, chloroxynil, ioxynil, coumatétralyl, dichlobénil, bénoxacor, et BTH (acibenzolar-s-méthyl).

11. Procédé de fabrication d'un polypeptide récepteur PYR/PYL muté qui est rendu agoniste par un produit chimique lorsque le produit chimique est mis en contact avec le polypeptide récepteur PYR/PYL muté, dans lequel le produit chimique ne rend sensiblement pas agoniste un polypeptide récepteur PYR/PYL de type sauvage lorsque le produit chimique est mis en contact avec le polypeptide récepteur PYR/PYL de type sauvage, le procédé comprenant
(a) la mutagénèse du polypeptide récepteur PYR/PYL de type sauvage ;
(b) la mise en contact d'un ou de plusieurs polypeptides récepteurs PYR/PYL mutés avec le produit chimique ; et
(c) la détermination que le produit chimique active ou non le ou les polypeptides récepteurs PYR/PYL mutés, dans lequel l'activation identifie le ou les polypeptides récepteurs PYR/PYL mutés comme étant rendus agonistes par le produit chimique ;
de préférence dans lequel le procédé comprend en outre, avant l'étape (b), le contrôle du produit chimique pour déterminer si le produit chimique se lie au polypeptide récepteur PYR/PYL de type sauvage avant de mettre en contact le ou les polypeptides récepteurs PYR/PYL mutés avec le produit chimique.

12. Cassette d'expression, comprenant un promoteur fonctionnellement lié à un polynucléotide encodant un polypeptide récepteur PYR/PYL muté, dans lequel le polypeptide récepteur PYR/PYL muté comprend une mutation à un résidu d'acide aminé correspondant à la position K59 de PYR1 (SEQ ID n° : 1), dans lequel le résidu d'acide aminé est muté en alanine, cystéine, phénylalanine, glycine, histidine, leucine, méthionine, arginine, serine, thréonine, tyrosine, valine, asparagine, ou tryptophane, dans lequel le polypeptide récepteur PYR/PYL muté est rendu agoniste par un produit chimique lorsque le produit chimique est mis en contact avec le polypeptide récepteur PYR/PYL muté et dans lequel le produit chimique ne rend sensiblement pas agoniste un polypeptide récepteur PYR/PYL de type sauvage lorsque le produit chimique est mis en contact avec le polypeptide récepteur PYR/PYL de type sauvage ; ou
un vecteur d'expression comprenant ladite cassette d'expression.

13. Procédé de production d'une plante présentant une tolérance améliorée aux contraintes, le procédé comprenant la culture d'une plante transgénique comprenant au moins une séquence polynucléotidique encodant un polypeptide récepteur PYR/PYL muté, dans lequel le polypeptide récepteur PYR/PYL muté :
(i) possède au moins 70 % d'identité de séquence avec l'un quelconque de SEQ ID n° : 124-139, 143, 144, 146, ou 164-178 et comprend une mutation à un résidu d'acide aminé correspondant à la position K59 de PYR1 (SEQ ID n° : 1), dans lequel le résidu d'acide aminé est muté en alanine, cystéine, phénylalanine, glycine, histidine, leucine, méthionine, arginine, serine, thréonine, tyrosine, valine, asparagine, ou tryptophane, et/ou
(ii) possède au moins 70 % d'identité de séquence avec l'un quelconque de SEQ ID n° : 147, 148, 164 et comprend une mutation à un résidu d'acide aminé correspondant à la position F159 de PYR1 (SEQ ID n° 1),
dans lequel le résidu d'acide aminé est muté en leucine ou serine ;
dans lequel le polypeptide récepteur PYR/PYL muté est rendu agoniste par un produit chimique lorsque le produit chimique est mis en contact avec le polypeptide récepteur PYR/PYL muté, dans lequel le produit chimique ne rend sensiblement pas agoniste un polypeptide récepteur PYR/PYL de type sauvage lorsque le produit chimique est mis en contact avec le polypeptide récepteur PYR/PYL de type sauvage,
moyennant quoi la plante transgénique exprime le polypeptide récepteur PYR/PYL muté.

14. Polypeptide comprenant un polypeptide récepteur PYR/PYL muté, dans lequel le polypeptide récepteur PYR/PYL muté :
(i) possède au moins 70 % d'identité avec l'un quelconque de SEQ ID n° : 124-139, 143, 144, 146, ou 164-178 et comprend une mutation à un résidu d'acide aminé correspondant à la position K59 de PYR1 (SEQ ID n° : 1), dans lequel le résidu d'acide aminé est muté en alanine, cystéine, phénylalanine, glycine, histidine, leucine, méthionine, arginine, serine, thréonine, tyrosine, valine, asparagine, ou tryptophane, et/ou
(ii) possède au moins 70 % d'identité de séquence avec l'un quelconque de SEQ ID n° : 147, 148, 164 et comprend une mutation à un résidu d'acide aminé correspondant à la position F159 de PYR1 (SEQ ID n° : 1),
dans lequel le résidu d'acide aminé est muté en leucine ou serine ;
dans lequel le polypeptide récepteur PYR/PYL muté est rendu agoniste par un produit chimique lorsque le produit chimique est mis en contact avec le polypeptide récepteur PYR/PYL muté, dans lequel le produit chimique ne rend sensiblement pas agoniste un polypeptide récepteur PYR/PYL de type sauvage lorsque le produit chimique est mis en contact avec le polypeptide récepteur PYR/PYL de type sauvage ;
ou un polynucléotide encodant ledit polypeptide.
